# EUROPEAN PATENT APPLICATION

(11) **EP 4 825 847 A1**
(43) Date of publication of application: **21.10.2026**
(21) Application number: 24902986.9
(22) Date of filing: 13.12.2024
(51) Int. Cl.: C07D 265/36, C07D 265/28, C07D 217/00, C07D 409/02, C07D 409/14, C07D 413/02, C07D 215/00, A61K 31/538, A61K 31/472, A61P 23/00, A61P 25/00, A61P 25/04, A61P 25/08, A61P 25/24, A61P 9/10, A61P 29/00

(54) **NMDA RECEPTOR ANTAGONIST AND USE THEREOF**

(30) Priority: 14.12.2023 CN 202311731669; 02.01.2024 CN 202410005398; 04.02.2024 CN 202410159055; 16.05.2024 CN 202410614085; 12.09.2024 CN 202411283214
(71) Applicant: Synphatec (Shanghai) Biopharmaceutical Technology Co., Ltd., Shanghai 201210 (CN)
(72) Inventor: GENG, Yang, Shanghai 201210 (CN); CHEN, Zhengxia, Shanghai 201210 (CN); LI, Ziwen, Shanghai 201210 (CN); SU, Tonghui, Shanghai 201210 (CN)
(74) Representative: Simmons & Simmons LLP (Munich)
(86) International application number: PCT/CN2024/139325
(87) International publication number: WO 2025/124571

(57) **Abstract**

The present invention relates to a NMDA receptor antagonist and a use thereof. The NMDA receptor antagonist of the present invention is a compound of formulas II and III below, or a pharmaceutically acceptable salt, an enantiomer, a diastereomer, a tautomer, a solvate, an isotopic substituent, a polymorphic substance, a prodrug or a metabolite thereof. The present invention also provides a pharmaceutical composition comprising these compounds, and a use of these compounds in treating or preventing NMDA receptor-mediated diseases.

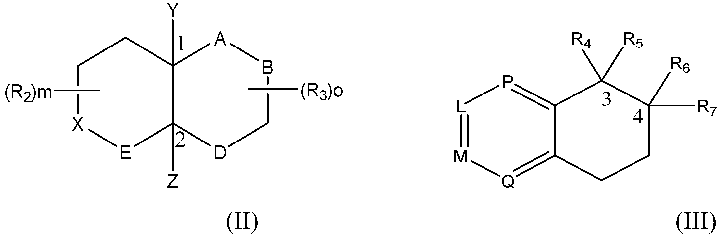

## Description

### FIELD OF THE INVENTION

The present invention relates to a NMDA receptor antagonist and use thereof.

### RELATED ART

Glutamate is a neurotransmitter that regulates excitatory synaptic transmission in mammalian brain. The glutamate system plays an important role in synaptic plasticity, learning and memory. N-methyl-D-aspartate (NMDA) receptor is the most important ionic glutamate receptor, and also a ligand-gated ion channel. The NMDA receptor (NMDAR) is a tetramer composed of various subunits, and most natural NMDAR found at present consists of two GluN1 and two GluN2 (or GluN3). The characteristics of NMDAR depend on the subtypes of GluN2 or GluN3. NMDAR with different GluN2 subtypes has great differences in ion permeability, sensitivity to Mg²⁺ block, sensitivity to antagonist, affinity to agonist, and channel dynamics.

As a transmembrane ion channel protein, the NMDA receptor has a complex molecular structure, has various subunits that are specific in brain distribution and pharmacological characteristics, shows dynamic changes in different developmental stages, and participates in many physiological activities, thus providing a molecular basis for complex neural activities to ensure the normal activities of neural networks. The integration, localization, recovery, distribution of NMDAR inside and outside synapses, and other processes depend on the regulation of neural activity. NMDAR is considered as a key factor for synaptic plasticity. The disruption of its functional homeostasis is highly related to many brain diseases, such as depression, schizophrenia, and epilepsy. The NMDA receptor as a target can be used for the treatment of these diseases.

In addition, since NMDA plays a key role in synaptic plasticity and excitotoxicity, its dysfunction is also highly related to many neurodegenerative diseases that lead to cognitive impairment, such as Alzheimer's disease, Parkinson's disease and Huntington's disease. Autoantibodies produced by the human immune system against NMDA receptors will interfere with the normal function of NMDA receptors, leading to schizophrenia symptoms and producing anti-NMDA receptor encephalitis. NMDA receptors are potential targets for the development of treatments for such diseases. Therefore, the NMDA receptor is expected to be used as a target of new rapid antidepressants, in the treatment of diseases such as schizophrenia, epilepsy, Alzheimer's disease, Parkinson's

disease, Huntington's disease, and anti-NMDA receptor encephalitis. and in anesthesia, sedation and analgesia, and pruritus (for human and animal use).

### SUMMARY OF THE INVENTION

An object of the present invention provides a NMDA receptor antagonist and use thereof.

The compound of the present invention is a NMDA receptor antagonist. Therefore, the compound of the present invention, or a pharmaceutically acceptable salt, enantiomer, diastereomer, tautomer, solvate, isotopologue, polymorph, prodrug, or metabolite thereof can be used to regulate the activity of NMDA receptors, to treat and/or prevent NMDA receptor-mediated diseases. The compound of the present invention has NMDAR-mediated current inhibiting activity, good in-vivo efficacy such as anti-depression, analgesic and antipruritic effects, excellent in-vivo safety (for example, the no observed adverse effect level (NOAEL) is higher), and no hallucinogenic side effects, and will not induce psychotomimetic-like behavior. Moreover, the compound of the present invention also has excellent pharmacokinetic performances (for example, good in-vivo metabolic stability, excellent oral drug absorption and exposure, and good oral drug absorption and bioavailability).

In a first aspect of the present invention, a compound of Formula II: 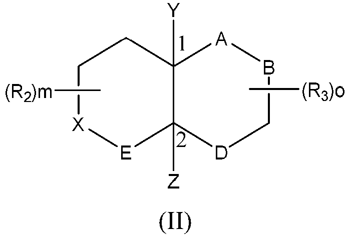
or a pharmaceutically acceptable salt, enantiomer, diastereomer, tautomer, solvate, isotopologue, polymorph, prodrug, or metabolite thereof is provided.
wherein,
   X is -O- or -CH₂-;
   E is -O- or -CH₂-;
   A is -NRₐ- or -CH₂-;
   B is -NRₐ- or -CH₂-;
   Rₐ is selected from H or C₁₋₆ alkyl;
   D is -O- or -CH₂-;
   Y is selected from a substituted or unsubstituted 6- to 14-membered aryl, a substituted or unsubstituted 5- to 14-membered heteroaryl, or a substituted or unsubstituted C₁₋₆ alkyl, and Y is optionally substituted with 1, 2, 3, 4 or 5 R₁, where each R₁ is independently selected from deuterium, cyano, C₁₋₃ alkyl, halogen, a 5- to 14-membered heteroaryl optionally substituted with 1, 2, 3, 4 or 5 halogens, or a 6- to 14-membered aryl optionally substituted with 1, 2, 3, 4 or 5 halogens, and the 5- to 14-membered heteroaryl includes 1, 2, 3 or 4 heteroatoms selected from N, O or S;
   Z is selected from H, hydroxyl, C₁₋₆ alkyl, and C₁₋₆ alkoxy;
   R₂ is selected from deuterium, C₁₋₃ alkyl, or halogen;
   R₃ is selected from deuterium, C₁₋₃ alkyl, or halogen;
   m is 0, 1, 2 or 3;
   o is 0, 1, 2 or 3;
   the chiral carbon atom at position 1 is in S or R configuration; and
   the chiral carbon atom at position 2 is in S or R configuration;
   provided that the compound of Formula II is not the following compounds and salt thereof: (4aR,8aS)-4α-(2-thienyl)octahydro-2H-benzo[b][1,4]oxazine, (4aR,8aR)-4α-(2-chloro-3-thienyl)octahydro-2H-benzo[b][1,4]oxazine, (4aS,8aR)-4α-(2-thienyl)octahydro-2H-benzo[b][1,4]oxazine, (4aR,8aS)-4α-(3-methyl-2-thienyl)octahydro-2H-benzo[b][1,4]oxazine, (4aR,8aR)-4α-(4-methyl-3-thienyl)octahydro-2H-benzo[b][1,4]oxazine, (4aR,8aR)-4α-(3-chlorophenyl)octahydro-2H-benzo[b][1,4]oxazine, (4aS,8aS)-4α-(3-chlorophenyl)octahydro-2H-benzo[b][1,4]oxazine, (4aR,8aR)-4α-(2-chlorophenyl)octahydro-2H-benzo[b][1,4]oxazine, (4aS,8aS)-4α-(2-chlorophenyl)octahydro-2H-benzo[b][1,4]oxazine, 4a-phenyloctahydro-2H-benzo[b][1,4]oxazine, 4a-(2-methoxyphenyl)octahydro-2H-benzo[b][1,4]oxazine, 4a-(3-fluorophenyl)octahydro-2H-benzo[b][1,4]oxazine, 4a-(4-fluorophenyl)octahydro-2H-benzo[b][1,4]oxazine, 4a-(2,3-difluorophenyl)octahydro-2H-benzo[b][1,4]oxazine, 4a-(2-fluorophenyl)octahydro-2H-benzo[b][1,4]oxazine, 6-methyl-4a-phenyloctahydro-2H-benzo[b][1,4]oxazine, 6-ethyl-4a-phenyloctahydro-2H-benzo[b][1,4]oxazine, 4a-(3-chlorophenyl)octahydro-2H-benzo[b][1,4]oxazine, 4a-(3-methylphenyl)octahydro-2H-benzo[b][1,4]oxazine, 4a-(2-chlorophenyl)octahydro-2H-benzo[b][1,4]oxazine, 4a-(4-chlorophenyl)octahydro-2H-benzo[b][1,4]oxazine, 4a-(4-methylphenyl)octahydro-2H-benzo[b][1,4]oxazine, 4a-(3-(trifluoromethyl)phenyl)octahydro-2H-benzo[b][1,4]oxazine, 8-methyl-4a-phenyloctahydro-2H-benzo[b][1,4]oxazine, 5-methyl-4a-phenyloctahydro-2H-benzo[b][1,4]oxazine, 7-methyl-4a-phenyloctahydro-2H-benzo[b][1,4]oxazine, 4-methyl-4a-phenyloctahydro-2H-benzo[b][1,4]oxazine, 3,3-dimethyl-5a-phenyldecahydrobenzo[b] [1,4]olanzapine, 4a-(3-methoxyphenyl)octahydro-2H-benzo[b][1,4]oxazine, 4a-(3-(trifluoromethoxy)phenyl)octahydro-2H-benzo[b][1,4]oxazine, 4*a*-(4-(trifluoromethyl)phenyl)octahydro-2H-benzo[*b*][1,4]oxazine, 4*a*-(2,6-dimethylphenyl)octahydro-2H-benzo[*b*][1,4]oxazine, 4*a*-(4-(t-butyl)phenyl)octahydro-2H-benzo[*b*][1,4]oxazine, 4*a*-(2,3-dichlorophenyl)octahydro-2H-benzo[*b*][1,4]oxazine, 4*a*-(2-isopropylphenyl)octahydro-2H-benzo[*b*] [1,4]oxazine, 4*a*-(2,5-dimethylphenyl)octahydro-2*H*-benzo[*b*][1,4]oxazine, 4*a*-(2-chloro-3-fluorophenyl)octahydro-2H-benzo[*b*][1,4]oxazine, 4*a*-(3,4-difluorophenyl)octahydro-2H-benzo[*b*][1,4]oxazine, 6,6-dimethyl-4*a-*phenyloctahydro-2H-benzo[*b*][1,4]oxazine, 4*a*-(2-chloro-5-fluorophenyl)octahydro-2H-benzo[*b*][1,4]oxazine, 4*α*-(3-ethoxyphenyl)octahydro-2H-benzo[*b*][1,4]oxazine, 4*α*-(2-chloro-4-methoxyphenyl)octahydro-2H-benzo[*b*][1,4]oxazine, and 4a-(2-chloro-6-fluorophenyl)octahydro-2H-benzo[b] [1,4]oxazine.

In a second aspect of the present invention, a crystal form of a salt of Compound 2 is provided, which is selected from Crystal Forms A, B, C, D, E, F, L, and M.

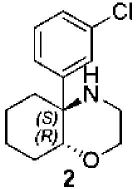

The X-ray powder diffraction pattern of Crystal Form A of the hydrochloride comprises characteristic peaks at the following 2θ angles: 14.44° ± 0.20°, 17.16° ± 0.20°, 20.59° ± 0.20°, and 23.38° ± 0.20°.

The X-ray powder diffraction pattern of Crystal Form B of the phosphate comprises characteristic peaks at the following 2θ angles: 5.48° ± 0.20°, 18.48° ± 0.20°, 22.49° ± 0.20°, and 22.92° ± 0.20°.

The X-ray powder diffraction pattern of Crystal Form C of the methanesulfonate comprises characteristic peaks at the following 2θ angles: 8.05° ± 0.20°, 14.37± 0.20°, 16.18° ± 0.20°, 18.49° ± 0.20°, and 22.65± 0.20°.

The X-ray powder diffraction pattern of Crystal Form D of the methanesulfonate comprises characteristic peaks at the following 2θ angles: 12.13° ± 0.20°, 14.80° ± 0.20°, 20.54° ± 0.20°, 23.37° ± 0.20°, and 26.09° ± 0.20°.

The X-ray powder diffraction pattern of Crystal Form E of the methanesulfonate comprises characteristic peaks at the following 2θ angles: 14.81° ± 0.20°, 16.30° ± 0.20°, 18.60° ± 0.20°, and 22.75° ± 0.20°.

The X-ray powder diffraction pattern of Crystal Form F of the formate comprises characteristic peaks at the following 2θ angles: 11.35° ± 0.20°, 13.53° ± 0.20°, and 23.76° ± 0.20°.

The X-ray powder diffraction pattern of Crystal Form L of the oxalate comprises characteristic peaks at the following 2θ angles: 9.44° ± 0.20°, 11.11° ± 0.20°, 15.61° ± 0.20°, 19.42° ± 0.20°, and 26.30° ± 0.20°.

The X-ray powder diffraction pattern of Crystal Form M of the pamoate comprises characteristic peaks at the following 2θ angles: 5.44° ± 0.20°, 6.49° ± 0.20°, 10.75° ± 0.20°, and 21.01° ± 0.20°.

In a third aspect of the present invention, a crystal form of a salt of Compound 3 is provided, which is selected from Crystal Forms G, H, I, J, K, N, or O.

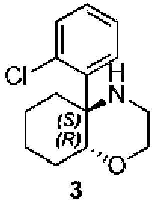

The X-ray powder diffraction pattern of Crystal Form G of the hydrochloride comprises characteristic peaks at the following 2θ angles: 6.18° ± 0.20°, 11.42° ± 0.20°, 12.45° ± 0.20°, 20.02° ± 0.20°, and 23.41° ± 0.20°.

The X-ray powder diffraction pattern of Crystal Form H of the hydrochloride comprises characteristic peaks at the following 2θ angles: 6.64° ± 0.20°, 13.12° ± 0.20°, 13.31° ± 0.20°, 21.83° ± 0.20°, and 23.06° ± 0.20°.

The X-ray powder diffraction pattern of Crystal Form I of the phosphate comprises characteristic peaks at the following 2θ angles: 5.94° ± 0.20°, 12.29° ± 0.20°, 13.34° ± 0.20°, and 17.99° ± 0.20°.

The X-ray powder diffraction pattern of Crystal Form J of the methanesulfonate comprises characteristic peaks at the following 2θ angles: 14.75° ± 0.20°, 15.81° ± 0.20°, and 21.89° ± 0.20°.

The X-ray powder diffraction pattern of Crystal Form K of the formate comprises characteristic peaks at the following 2θ angles: 11.92° ± 0.20°, 15.15° ± 0.20°, and 24.04° ± 0.20°.

The X-ray powder diffraction pattern of Crystal Form N of the oxalate comprises characteristic peaks at the following 2θ angles: 6.20° ± 0.20°, 7.49° ± 0.20°, 12.25° ± 0.20°, 12.47° ± 0.20°, 16.36° ± 0.20°, and 23.43° ± 0.20°.

The X-ray powder diffraction pattern of Crystal Form O of the pamoate comprises characteristic peaks at the following 2θ angles: 6.18° ± 0.20°, 9.41° ± 0.20°, 11.10° ± 0.20°, 15.59° ± 0.20°, and 26.25° ± 0.20°.

In a fourth aspect of the present invention, a pharmaceutical composition is provided. The pharmaceutical composition contains a pharmaceutically acceptable carrier and
(i) the compound or the pharmaceutically acceptable salt, enantiomer, diastereomer, tautomer, solvate, isotopologue, polymorph, prodrug, or metabolite thereof according to the first aspect of the present invention, and a pharmaceutically acceptable carrier; or
(ii) the crystal form of the salt of Compound 2 according to the second aspect of the present invention; or
(iii) the crystal form of the salt of Compound 3 according to the third aspect of the present invention.

In a fifth aspect of the present invention, use of the compound or the pharmaceutically acceptable salt, enantiomer, diastereomer, tautomer, solvate, isotopologue, polymorph, prodrug, or metabolite thereof according to the first aspect of the present invention, the crystal form of the salt of Compound **2** according to the second aspect of the present invention, the crystal form of the salt of Compound **3** according to the third aspect of the present invention, or the pharmaceutical composition according to the fourth aspect of the present invention in the manufacture of a medicament for treating or preventing NMDA receptor-mediated diseases, or in the manufacture of an anesthetic or analgesic.

In one or more embodiments, the NMDA receptor-mediated diseases are selected from: cerebral ischemia, traumatic brain injury, infarction, stroke, Alzheimer's disease, Parkinson's disease, Huntington's diseases, depression, anxiety, bipolar disorder, schizophrenia, autism, epilepsy, anti-NMDA receptor encephalitis, neuropathic pain, anorexia, sleep disorder, pruritus related diseases, or other nervous system events or neurodegeneration caused by the activation of NMDA receptors.

In one or more embodiments, the NMDA receptor-mediated disease is depression, schizophrenia or epilepsy.

In one or more embodiments, the sleep disorder related disease is insomnia, sleep apnea syndrome, atypical circadian rhythm sleep-wake disorder or Down syndrome.

In one or more embodiments, the NMDA receptor-mediated disease is pain or pruritus.

### BRIEF DESCRIPTION OF THE DRAWINGS

FIG. 1 shows the XRPD pattern of Crystal Form A of Compound **2** hydrochloride.
FIG. 2 shows the DSC profile and TGA curve of Crystal Form A of Compound **2** hydrochloride.
FIG. 3 shows the XRPD pattern of Crystal Form B of Compound **2** phosphate.
FIG. 4 shows the DSC profile and TGA curve of Crystal Form B of Compound **2** phosphate.
FIG. 5 shows the XRPD pattern of Crystal Form C of Compound **2** methanesulfonate.
FIG. 6 shows the DSC profile and TGA curve of Crystal Form C of Compound **2** methanesulfonate.
FIG. 7 shows the XRPD pattern of Crystal Form D of Compound **2** methanesulfonate.
FIG. 8 shows the DSC profile of Crystal Form D of Compound **2** methanesulfonate.
FIG. 9 shows the TGA curve of Crystal Form D of Compound **2** methanesulfonate.
FIG. 10 shows the XRPD pattern of Crystal Form E of Compound **2** methanesulfonate.
FIG. 11 shows the DSC profile of Crystal Form E of Compound **2** methanesulfonate.
FIG. 12 shows the TGA profile of Crystal Form E of Compound **2** methanesulfonate.
FIG. 13 shows the XRPD pattern of Crystal Form F of Compound **2** formate.
FIG. 14 shows the DSC profile and TGA curve of Crystal Form F of Compound **2** formate.
FIG. 15 shows the XRPD pattern of Crystal Form L of Compound **2** oxalate.
FIG. 16 shows the XRPD pattern of Crystal Form M of Compound **2** pamoate.
FIG. 17 shows the XRPD pattern of Crystal Form G of Compound **3** hydrochloride.
FIG. 18 shows the DSC profile and TGA curve of Crystal Form G of Compound **3** hydrochloride.
FIG. 19 shows the XRPD pattern of Crystal Form H of Compound **3** hydrochloride.
FIG. 20 shows the DSC profile and TGA curve of Crystal Form H of Compound **3** hydrochloride.
FIG. 21 shows the XRPD pattern of Crystal Form I of Compound **3** phosphate.
FIG. 22 shows the DSC profile and TGA curve of Crystal Form I of Compound **3** phosphate.
FIG. 23 shows the XRPD pattern of Crystal Form J of Compound **3** methanesulfonate.
FIG. 24 shows the DSC profile and TGA curve of Crystal Form J of Compound **3** methanesulfonate.
FIG. 25 shows the XRPD pattern of Crystal Form K of Compound **3** formate.
FIG. 26 shows the DSC profile and TGA curve of Crystal Form K of Compound **3** formate.
FIG. 27 shows the XRPD pattern of Crystal Form N of Compound **3** oxalate.
FIG. 28 shows the DSC profile and TGA curve of Crystal Form O of Compound **3** pamoate.
FIG. 29 shows the anti-depressant effect of Compound **1** and Crystal Form A of Compound **2** hydrochloride in a forced swimming test.
FIG. 30 shows the anti-depressant effect of Crystal Form C of Compound **2** methanesulfonate in a forced swimming test.
FIG. 31 shows the anti-depressant effect of Crystal Form G of Compound **3** hydrochloride and a comparative compound in a forced swimming test.
FIG. 32 shows the anti-depressant effect of Compound **34** hydrochloride in a forced swimming test.
FIG. 33 shows the result of an open field test of Crystal Form A of Compound **2** hydrochloride.
FIG. 34 shows the result of an open field test of Crystal Form C of Compound **2** methanesulfonate.
FIG. 35 shows the result of an open field test of Crystal Form G of Compound **3** hydrochloride.
FIG. 36 shows the result of an open field test of Compound **34** hydrochloride.
FIG. 37 shows the result of a pre-pulse inhibition test of Crystal Form C of Compound **2** methanesulfonate.

### DETAILED DESCRIPTION

To make the features and effects of the present invention comprehensible to those skilled in the art, a general description and definition of terms and expressions mentioned in the specification and claims are provided below. Unless otherwise indicated, all technical and scientific words used herein have common meanings understood by those skilled in the art. In case of conflict, the definitions in this specification shall prevail.

The theories or mechanisms described and disclosed herein, whether right or wrong, should not limit the scope of the present invention in any way. That is, the present invention can be implemented without being limited by any particular theory or mechanism.

Herein, "comprise", "include", "contain" and similar terms cover the meanings of "consisting essentially of" and "consisting of". For example, when "A contains B and C" is disclosed herein, "A consists essentially of B and C" and "A consists of B and C" are considered as being disclosed herein.

Herein, all characteristics defined in the form of numerical range or percentage range, such as numerical value, quantity, content and concentration, are merely for simplicity and convenience. Accordingly, the description of numerical range or percentage range shall be deemed to cover and specifically disclose all possible sub-ranges and individual values within the range (including integers and fractions).

Herein, unless otherwise specified, the percentage refers to the percentage by weight and the proportion refers to weight proportion.

Herein, when embodiments or examples are described, it is to be understood that the present invention is not limited to these embodiments or examples. Conversely, all alternatives, modifications and equivalents of the method and materials described in the present invention can be covered within the scope defined by the claims.

Herein, for the brevity of description, not all possible combinations of various technical features in various embodiments or examples are described. Therefore, the various technical features in various embodiments and examples can be combined at will as long as there are no contradictions in the combinations of these technical features, and all possible combinations should be considered to fall within the scope of the present application.

### I. Terms

As used herein, the "alkyl" refers to a linear or branched monovalent saturated hydrocarbon group having a specified number of carbon atoms. In some embodiments, the alkyl is those alkyl groups having 1 to 6 carbon atoms ("C1-C6 alkyl"), including alkyl groups having 6 carbon atoms (C6 alkyl), 5 carbon atoms (C5 alkyl), 4 carbon atoms (C4 alkyl), 3 carbon atoms (C3 alkyl), 2 carbon atoms (C2 alkyl), or 1 carbon atom (C1 alkyl), or alkyl groups having any number of carbon atoms that is within the above range. Examples of alkyl include, but are not limited to, methyl, ethyl, n-propyl, isopropyl, n-butyl, isobutyl, s-butyl, t-butyl, n-pentyl, n-hexyl and the like. Unless otherwise specified, the alkyl can be optionally substituted with a suitable substituent.

As used herein, the "alkoxy" is alkyl-O-, where the alkyl is as defined above.

As used herein, "aryl" or "Ar" refers to an aromatic carbocyclic group having one (e.g., phenyl) or multiple (e.g., fused) ring (e.g., naphthyl or anthracenyl), where each of the multiple rings may or may not be aromatic and at least one of the multiple rings is aromatic. For aryl with multiple rings, the aryl can be attached to a parent structure via the aromatic or non-aromatic ring. In some embodiments, the aryl with multiple rings is attached to the parent structure via the aromatic ring. In some embodiments, the aryl is a 6- to 14-membered aryl group (i.e., an aryl group containing 6 to 14 ring carbon atoms), and preferably a 6- to 10-membered aryl group. Examples of aryl include, but are not limited to, phenyl, naphthalenyl, phenanthryl, anthracenyl, indenyl, azulenyl, biphenyl, biphenylene and fluorenyl. Unless otherwise specified, the aryl can be optionally substituted with a suitable substituent.

As used herein, "heteroaryl" refers to a group containing 5 to 14 ring atoms and preferably 5 to 10 ring atoms, and having at least one aromatic ring. The ring atoms contained in heteroaryl include carbon atoms and 1 to 3 heteroatoms selected from O, N or S (including oxidized forms of heteroatoms). In the present invention, the preferred heteroaryl is a heteroaryl group containing N or S atom, and more preferably a 5-membered heteroaryl group containing S atom. Examples of heteroaryl include, but are not limited to, triazolyl, thienyl, furyl, pyranyl, pyrrolyl, imidazolyl, pyrazolyl, pyridinyl, pyrazinyl, pyrimidinyl, pyridazinyl, indolizinyl, isoindolyl, indolyl, benzimidazolyl and pyrazolopyrimidinyl. Unless otherwise specified, the heteroaryl can be optionally substituted with a suitable substituent.

As used herein, "oxo" refers to =O.

As used herein, "halogen" includes F, Cl, Br, and I.

As used herein, "hydroxyl" refers to -OH.

As used herein, the terms "moiety", "structural moiety", "chemical moiety", "group" and "chemical group" refer to a specific fragment or functional group in a molecule. The chemical moiety is generally considered as a chemical entity embedded or attached to a molecule.

Herein, English letters are used to indicate the designation of a specific crystal form. The English letters before the term "crystal forms" and the English letters after the term "crystal forms" indicate the same crystal form and can be used interchangeably. For example, "A Crystal Form" and "Crystal Form A" represent the same crystal form of designation A. It should also be understood by those skilled in the art that in the method described below, the functional group of an intermediate compound may need to be protected by an appropriate protecting group. Such functional groups include hydroxyl, amino, sulfhydryl, and carboxylic acids. Suitable hydroxyl protecting groups include trialkylsilyl or diarylalkylsilyl (such as tert-butyldimethylsilyl, t-butyldiphenylsilyl, or trimethylsilyl), tetrahydropyranyl, benzyl, and the like. Suitable protecting groups for amino, amidino and guanidino groups include tertbutoxycarbonyl, and benzyloxycarbonyl, etc. Suitable mercapto protecting groups include - C(O)-R" (where R" is alkyl, aryl or aralkyl), p-methoxybenzyl, and triphenylmethyl, etc. Suitable carboxyl protecting groups include alkyl esters, aryl esters or aralkyl esters.

The protecting groups can be introduced and removed according to standard techniques known to those skilled in the art and described herein. The use of protecting groups is described in detail in Greene, T. W. and P. G. M. Wuts, Protective Groups in Organic Synthesis, (1999), 4th Ed., Wiley. The protecting group may further be a polymer resin.

As used herein, the "subject" can be a human, a non-human primate, a mammal, rats, mice, cattle, horses, pigs, sheep, goats, dogs, and cats. The subject may be suspected to have or suffer from neurodegenerative diseases, such as NMDA receptor-mediated diseases, including cerebral ischemia, traumatic brain injury, infarction, stroke, Alzheimer's disease, Parkinson's disease, Huntington's diseases, depression, anxiety, bipolar disorder, schizophrenia, autism, epilepsy, anti-NMDA receptor encephalitis, neuropathic pain, psychosis, anorexia, sleep disorders (such as insomnia, sleep apnea syndrome, atypical circadian rhythm sleep-wake disorder or Down syndrome), or pruritus related diseases. The subject may further be suspected to have or suffer from other nervous system events or neurodegeneration caused by the activation of NMDA receptors.

The "mammal" includes: humans; domesticated animals, such as laboratory animals, family pets (for example, cats, cats, dogs, pigs, cattle, sheep, goats, horses, rabbits) and zoo animals (such as tigers, monkeys, and bears, etc.), and non-domesticated animals, such as wild animals, etc.

The "pharmaceutically acceptable carrier, diluent or excipient" includes, but is not limited to, any adjuvant, carrier, excipient, glidant, sweetener, diluent, preservative, dye/colorant, flavor enhancer, surfactant, wetting agent, dispersant, suspension, stabilizer, isotonic agent, solvent or emulsifier that has been approved by, for example, the Food and Drug Administration (FDA) of the United States for use in humans or domesticated animals.

In the present application, the term "pharmaceutically acceptable salt" includes a pharmaceutically acceptable acid addition salt and a pharmaceutically acceptable base addition salt.

The "pharmaceutically acceptable acid addition salt" refers to a salt formed with an inorganic acid or an organic acid that can retain the bioavailability of a free base without other side effects. The salts with inorganic acids include, but are not limited to, hydrochloride, hydrobromide, sulfate, nitrate, and phosphate, etc. The salts with organic acids include, but are not limited to, formate, acetate, 2,2-dichloroacetate, trifluoroacetate, propionate, caproate, octoate, decanoate, undecylenate, glycolate, gluconate, lactate, sebacate, adipate, glutarate, malonate, oxalate, maleate, succinate, fumarate, tartrate, citrate, palmitate, stearate, oleate, cinnamate, laurate, malate, glutamate, pyroglutamate, aspartate, benzoate, mesylate, benzenesulfonate, p-toluenesulfonate, alginate, ascorbate, salicylate, 4-aminosalicylate, naphthalene disulfonate, and pamoate, etc. These salts can be prepared by methods known in the art.

The "pharmaceutically acceptable base addition salt" refers to a salt formed with an inorganic base or an organic base that can retain the bioavailability of a free acid without other side effects. Salts derived from inorganic bases include, but are not limited to, potassium salt, lithium salt, ammonium salt, calcium salt, magnesium salt, iron salt, zinc salt, copper salt, manganese salt, aluminum salt, and the like. Preferred inorganic salts are ammonium salt, sodium salt, potassium salt, calcium salt and magnesium salt. Salts derived from organic bases include, but are not limited to, primary amines, secondary amines and tertiary amines, substituted amines, including natural substituted amines, cyclic amines and basic ion exchange resins, for example, ammonia, isopropylamine, trimethylamine, diethylamine, triethylamine, tripropylamine, ethanolamine, diethanolamine, triethanolamine, dimethylethanolamine, 2-dimethylaminoethanol, 2-diethylaminoethanol, dicyclohexylamine, lysine, arginine, histidine, caffeine, procaine, choline, betaine, ethylenediamine, glucosamine, methylglucosamine, theobromine, purine, piperazine, piperidine, N-ethylpiperidine, and polyamine resin, etc. Preferred organic bases include isopropylamine, diethylamine, ethanolamine, trimethylamine, dicyclohexylamine, choline and caffeine. These salts can be prepared by methods known in the art.

Examples of prodrugs of the compound of the present invention may include simple esters of compounds containing a carboxylic acid (for example, esters obtained by condensation with a C1-4 alcohol according to methods known in the art); esters of compounds containing a hydroxyl group (for example, esters obtained by condensation with a C1-4 carboxylic acid, a C3-6 diacid or an anhydride thereof such as succinic anhydride and fumaric anhydride according to methods known in the art); imines of compounds containing an amino group (for example, imines obtained by condensation with a C1-4 aldehyde or ketone according to methods known in the art); carbamates of compounds containing an amino group, such as those described by Leu et al. (J. Med. Chem., 42:3623-3628(1999)) and Greenwald et al. (J. Med. Chem., 42:3657-3667(1999)); and acetals or ketals of an alcoholcontaining compound (such as those obtained by condensation with chloromethyl methyl ether or chloromethyl ethyl ether according to methods known in the art).

As used herein, the term "solvate" refers to an aggregate comprising one or more molecules of the compound of the present invention and one or more solvent molecules. The solvent may be water, and the solvate may be a hydrate in this case. Alternatively, the solvent may be an organic solvent. Therefore, the compound of the present invention can exist as a hydrate, including monohydrate, dihydrate, hemihydrate, sesquihydrate, trihydrate, and tetrahydrate, and in a corresponding solvated form. The compound of the present invention can be a true solvate. In other cases, the compound of the present invention can only retain non-stoichiometric water or a mixture of water and some non-stoichiometric solvents.

The "pharmaceutical composition" refers to a preparation of the compound of the present invention and a medium known in the art for delivering a bioactive compound to a mammal (e.g., a human). The medium includes all pharmaceutically acceptable carriers, diluents or excipients.

The "effective amount" refers to a therapeutically effective amount or a prophylactically effective amount. The "therapeutically effective amount" refers to an amount that can effectively achieve the required therapeutic results (such as reducing pain and relieving pruritus) at a necessary dosage and for a necessary period of time. The therapeutically effective amount of the compound may vary depending on such factors as the disease state, age, sex and weight of the subject, and the ability of the compound to induce a desired response in the subject. The dosage regimen can be adjusted to provide an optimum therapeutic response. The "prophylactically effective amount" refers to an amount that can effectively achieve the required preventive results at a necessary dosage and for a necessary period of time. Generally, a prophylactic dose is given to a subject before or at an early stage of a disease, so that the prophylactically effective amount can be less than the therapeutically effective amount.

As used herein, the "treatment" covers the treatment of a disease or disorder of interest in a mammal (preferably human) suffering from the disease or disorder of interest and includes:
(i) preventing the occurrence of the disease or disorder in the mammal, especially when the mammal is susceptible to the disorder but has not been diagnosed with the disorder;
(ii) inhibiting the disease or condition, that is, that is, arresting its development;
(iii) alleviating the disease or disorder, that is, causing regression of the disease or disorder; or
(iv) alleviating symptoms caused by the disease or disorder, that is, that is, alleviating pain without solving the underlying disease or disorder.

As used herein, the terms "oral administration", "administration", "administering" and the like refer to a method able to deliver a compound or composition to a desired site for biological action. The administration methods well known in the art can be used in the present invention. These methods include, but are not limited to, oral route, duodenal route, parenteral injection (including intrapulmonary, intranasal, intrathecal, intravenous, subcutaneous, intraperitoneal, intramuscular, intra-arterial injection or infusion), local administration and rectal administration. The administration techniques that can be used for the compounds and methods described herein are known to those skilled in the art, for example, those discussed in Goodman and Gilman, The Pharmacological Basis of Therapeutics, current ed.; Pergamon; and Remington's, Pharmaceutical Sciences (current edition), Mack Publishing Co., Easton, Pa. In a preferred embodiment, the compound of the present invention, or the pharmaceutically acceptable salt, enantiomer, diastereomer, tautomer, solvate, isotopologue, polymorph, prodrug or metabolite, or pharmaceutical composition thereof is orally administered.

Herein, the "stereoisomer" refer to a compound that is composed of the same atoms and bonded by the same connection method, but has a different three-dimensional structure. The present invention will cover various stereoisomers and their mixtures, such as geometric isomers, enantiomers, diastereomers, and racemic mixtures.

When the compound of the present invention contains a double bond, unless otherwise indicated, the compound of the present invention is intended to include E- and Z-geometric isomers.

The "tautomer" refers to an isomer formed by the transfer of a proton from one atom of a molecule to another atom of the same molecule. All tautomeric forms of the compound of the present invention will also be included in the scope of the present invention.

The compound of the present invention or the pharmaceutically acceptable salt thereof may include one or more chiral carbon atoms, and thus enantiomeric, diastereomeric and other stereoisomeric forms may be formed. Each chiral carbon atom can be defined as (R)- or (S)- based on stereochemistry. The present invention is intended to include all possible isomers, as well as their racemates and optically pure forms. The racemate, diastereomer or enantiomer can be used as a raw material or intermediate in the preparation of the compound of the invention. The optically active isomer can be prepared with a chiral synthon or chiral reagent, or resolved by conventional techniques, such as crystallization and chiral chromatography.

Those skilled in the art will understand that a solid wedge-shaped bond ( 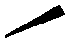 ) or a dashed wedge-shaped bond ( 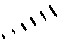 ) is used to indicate the absolute configuration of the chiral center, and a thick solid bond ( 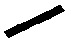 ) or a thick dashed bond ( 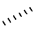 ) is used to indicate the relative configuration of the chiral center.

Conventional techniques for preparing/separating individual isomers include chiral synthesis with a suitable optically pure precursor, or resolution of racemates (or racemates of a salt or derivative) by, for example, chiral high performance liquid chromatography, see, for example, GeRald Gübitz and Martin G. Schmid(Eds.), ChiRal SepaRations, Methods and Protocols, Methods in Molecular Biology, Vol. 243, 2004; A.M. Stalcup, ChiRal SepaRations, Annu. Rev. Anal. Chem. 3: 341-63, 2010; Fumiss et al.(eds.), VOGEL'S ENCYCLOPEDIA OF PRaCTICAL ORGANIC CHEMISTRY.sup.TH ED., Longman Scientific and Technical Ltd., Essex, 1991, 809-816; Heller, Acc. Chem. Res. 1990, 23, 128.

The present invention also includes all suitable isotopically substituted variants of the compound of the present invention or pharmaceutically acceptable salts thereof. The isotopically substituted variants of the compound of the present invention or pharmaceutically acceptable salts thereof are defined as those in which at least one atom is replaced by an atom with the same atomic number, but an atomic weight that is different from the atomic weight often found in nature. Isotopes that can be incorporated into the compound of the present invention and pharmaceutically acceptable salt thereof include, but are not limited to, isotopes of H, C, N, and O, for example, ²H, ³H, ¹¹C, ¹³C, ¹⁴C, ¹⁵N, ¹⁷O, ¹⁸O, ³⁵S, ¹⁸F, ³⁶Cl, and ¹²⁵I. The isotopically substituted variants of the compound of the present invention or pharmaceutically acceptable salt thereof can be prepared by conventional techniques with appropriate isotopically substituted variants of appropriate reagents.

### II. Compounds

The present invention provides a compound of Formula II: 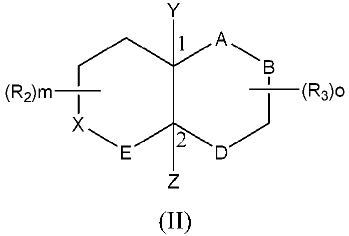
or a pharmaceutically acceptable salt, enantiomer, diastereomer, tautomer, solvate, isotopologue, polymorph, prodrug, or metabolite thereof
wherein,
   X is -O- or -CH₂-;
   E is -O- or -CH₂-;
   A is -NRₐ- or -CH₂-;
   B is -NRₐ- or -CH₂-;
   Rₐ is selected from H or C₁₋₆ alkyl;
   D is -O- or -CH₂-;
   Y is selected from a substituted or unsubstituted 6- to 14-membered aryl, a substituted or unsubstituted 5- to 14-membered heteroaryl, or a substituted or unsubstituted C₁₋₆ alkyl, and Y is optionally substituted with 1, 2, 3, 4 or 5 R₁, where each R₁ is independently selected from deuterium, cyano, C₁₋₃ alkyl, halogen, a 5- to 14-membered heteroaryl optionally substituted with 1, 2, 3, 4 or 5 halogens, or a 6- to 14-membered aryl optionally substituted with 1, 2, 3, 4 or 5 halogens, and the 5- to 14-membered heteroaryl includes 1, 2, 3 or 4 heteroatoms selected from N, O or S;
   Z is selected from H, hydroxyl, C₁₋₆ alkyl, or C₁₋₆ alkoxy;
   R₂ is selected from deuterium, C₁₋₃ alkyl, or halogen;
   R₃ is selected from deuterium, C₁₋₃ alkyl, or halogen;
   m is 0, 1, 2 or 3;
   o is 0, 1, 2 or 3;
   the chiral carbon atom at position 1 is in S or R configuration; and
   the chiral carbon atom at position 2 is in S or R configuration;
   provided that the compound of Formula II is not the following compounds and salts thereof: (4aR,8aS)-4α-(2-thienyl)octahydro-2H-benzo[b][1,4]oxazine, (4aR,8aR)-4α-(2-chloro-3-thienyl)octahydro-2H-benzo[b][1,4]oxazine, (4aS,8aR)-4α-(2-thienyl)octahydro-2H-benzo[b][1,4]oxazine, (4aR,8aS)-4α-(3-methyl-2-thienyl)octahydro-2H-benzo[b][1,4]oxazine, (4aR,8aR)-4α-(4-methyl-3-thienyl)octahydro-2H-benzo[b][1,4]oxazine, (4aR,8aR)-4α-(3-chlorophenyl)octahydro-2H-benzo[b][1,4]oxazine, (4aS,8aS)-4α-(3-chlorophenyl)octahydro-2H-benzo[b][1,4]oxazine, (4aR,8aR)-4α-(2-chlorophenyl)octahydro-2H-benzo[b][1,4]oxazine, (4aS,8aS)-4α-(2-chlorophenyl)octahydro-2H-benzo[b][1,4]oxazine, 4a-phenyloctahydro-2H-benzo[b][1,4]oxazine, 4a-(2-methoxyphenyl)octahydro-2H-benzo[b][1,4]oxazine, 4a-(3-fluorophenyl)octahydro-2H-benzo[b][1,4]oxazine, 4a-(4-fluorophenyl)octahydro-2H-benzo[b][1,4]oxazine, 4a-(2,3-difluorophenyl)octahydro-2H-benzo[b][1,4]oxazine, 4a-(2-fluorophenyl)octahydro-2H-benzo[b][1,4]oxazine, 6-methyl-4a-phenyloctahydro-2H-benzo[b][1,4]oxazine, 6-ethyl-4a-phenyloctahydro-2H-benzo[b][1,4]oxazine, 4a-(3-chlorophenyl)octahydro-2H-benzo[b][1,4]oxazine, 4a-(3-methylphenyl)octahydro-2H-benzo[b][1,4]oxazine, 4a-(2-chlorophenyl)octahydro-2H-benzo[b][1,4]oxazine, 4a-(4-chlorophenyl)octahydro-2H-benzo[b][1,4]oxazine, 4a-(4-methylphenyl)octahydro-2H-benzo[b][1,4]oxazine, 4a-(3-(trifluoromethyl)phenyl)octahydro-2H-benzo[b][1,4]oxazine, 8-methyl-4a-phenyloctahydro-2H-benzo[b][1,4]oxazine, 5-methyl-4a-phenyloctahydro-2H-benzo[b][1,4]oxazine, 7-methyl-4a-phenyloctahydro-2H-benzo[b][1,4]oxazine, 4-methyl-4a-phenyloctahydro-2H-benzo[b][1,4]oxazine, 3,3-dimethyl-5a-phenyldecahydrobenzo[b] [1,4]olanzapine, 4a-(3-methoxyphenyl)octahydro-2H-benzo[b][1,4]oxazine, 4a-(3-(trifluoromethoxy)phenyl)octahydro-2H-benzo[b][1,4]oxazine, 4*a*-(4-(trifluoromethyl)phenyl)octahydro-2*H*-benzo[*b*][1,4]oxazine, 4*a*-(2,6-dimethylphenyl)octahydro-2*H*-benzo[*b*][1,4]oxazine, 4*a*-(4-(t-butyl)phenyl)octahydro-2*H-*benzo[*b*][1,4]oxazine, 4*a*-(2,3-dichlorophenyl)octahydro-2*H*-benzo[*b*][1,4]oxazine, 4*a*-(2-isopropylphenyl)octahydro-2*H*-benzo[*b*] [1,4]oxazine, 4*a*-(2,5-dimethylphenyl)octahydro-2*H*-benzo[*b*][1,4]oxazine, 4*a*-(2-chloro-3-fluorophenyl)octahydro-2*H*-benzo[*b*][1,4]oxazine, 4*a*-(3,4-difluorophenyl)octahydro-2*H*-benzo[*b*][1,4]oxazine, 6,6-dimethyl-4*a-*phenyloctahydro-2*H*-benzo[*b*][1,4]oxazine, 4*a*-(2-chloro-5-fluorophenyl)octahydro-2*H-*benzo[*b*][1,4]oxazine, 4*α*-(3-ethoxyphenyl)octahydro-2*H*-benzo[*b*][1,4]oxazine, 4*α*-(2-chloro-4-methoxyphenyl)octahydro-2*H*-benzo[*b*][1,4]oxazine, and 4a-(2-chloro-6-fluorophenyl)octahydro-2H-benzo[b] [1,4]oxazine.

In some embodiments of the compound of Formula II, one of X and E is -O-, and the other is -CH₂-; or X and E are both -CH₂-.

In some embodiments of the compound of Formula II, one of A and B is - NRₐ-, and the other is -CH₂-.

In some embodiments of the compound of Formula II, Z is selected from H or hydroxyl. In some embodiments, Z is H. In some embodiments, Z is hydroxyl.

In some embodiments of the compound of Formula II, Y is selected from a substituted or unsubstituted 6- to 10-membered aryl, a substituted or unsubstituted 5- to 7-membered heteroaryl, or a substituted or unsubstituted C₁₋₃ alkyl; preferably, Y is selected from a substituted or unsubstituted phenyl, a substituted or unsubstituted 5- to 6-membered heteroaryl, or a substituted or unsubstituted methyl; and preferably, Y is selected from a substituted or unsubstituted 6- to 10-membered aryl, a substituted or unsubstituted 5- to 7-membered heteroaryl, or a substituted or unsubstituted C₁₋₃ alkyl, where the 6- to 10-membered aryl and 5- to 7-membered heteroaryl are each independently substituted with 1, 2, 3 or 4 groups selected from deuterium, cyano, C₁₋₃ alkyl or halogens, and the C₁₋₃ alkyl is optionally substituted with 1 or 2 groups selected from the group consisting of: a 5- to 7-membered heteroaryl optionally substituted with 1, 2, 3, 3 or 4 halogens, and a 6- to 10-membered aryl optionally substituted with 1, 2, 3 or 4 halogens. In some embodiments, Y is phenyl or thienyl optionally substituted with 1, 2, 3, 4 or 5 R₁.

In some embodiments of the compound of Formula II, each R₁ is independently selected from deuterium, cyano, C₁₋₃ alkyl, halogen, a 5- to 7-membered heteroaryl optionally substituted with 1, 2, 3, or 4 halogens, or a 6- to 10-membered aryl optionally substituted with 1, 2, 3 or 4 halogens; preferably, each R₁ is independently selected from deuterium, cyano, C₁₋₃ alkyl, halogen, or a 6- to 10-membered aryl optionally substituted with 1, 2, 3 or 4 halogens; and preferably, the halogen is selected from F or Cl.

In some embodiments of the compound of Formula II, D is -CH₂-; or D is -O-, provided that X and E are both -CH₂-.

In some embodiments, the present invention provides a compound of Formula A: 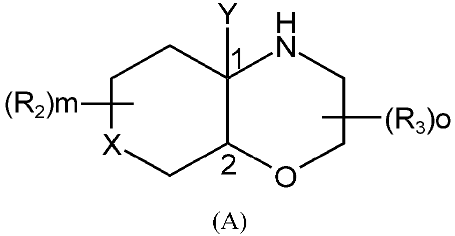 or a pharmaceutically acceptable salt, enantiomer, diastereomer, tautomer, solvate, isotopologue, polymorph, prodrug, or metabolite thereof
wherein,
Y is a 6- to 14-membered aryl or a 5- to 7-membered heteroaryl optionally substituted with 1, 2, 3, 4 or 5 R₁ substitutents, where each R₁ is independently selected from deuterium, cyano, C₁₋₃ alkyl or halogen;
R₂ is selected from deuterium, C₁₋₃ alkyl, or halogen;
R₃ is selected from deuterium, C₁₋₃ alkyl, or halogen;
m is 0, 1, 2 or 3;
o is 0, 1, 2 or 3;
X is -O- or -CH₂-;
the chiral carbon atom at position 1 is in S or R configuration; and
the chiral carbon atom at position 2 is in S or R configuration;
provided that the compound of Formula A is not the following compounds and salts thereof: (4aR,8aS)-4α-(2-thienyl)octahydro-2H-benzo[b][1,4]oxazine, (4aR,8aR)-4α-(2-chloro-3-thienyl)octahydro-2H-benzo[b][1,4]oxazine, (4aS,8aR)-4α-(2-thienyl)octahydro-2H-benzo[b][1,4]oxazine, (4aR,8aS)-4α-(3-methyl-2-thienyl)octahydro-2H-benzo[b][1,4]oxazine, (4aR,8aR)-4α-(4-methyl-3-thienyl)octahydro-2H-benzo[b][1,4]oxazine, (4aR,8aR)-4α-(3-chlorophenyl)octahydro-2H-benzo[b][1,4]oxazine, (4aS,8aS)-4α-(3-chlorophenyl)octahydro-2H-benzo[b][1,4]oxazine, (4aR,8aR)-4α-(2-chlorophenyl)octahydro-2H-benzo[b][1,4]oxazine, (4aS,8aS)-4α-(2-chlorophenyl)octahydro-2H-benzo[b][1,4]oxazine, 4a-phenyloctahydro-2H-benzo[b][1,4]oxazine, 4a-(2-methoxyphenyl)octahydro-2H-benzo[b][1,4]oxazine, 4a-(3-fluorophenyl)octahydro-2H-benzo[b][1,4]oxazine, 4a-(4-fluorophenyl)octahydro-2H-benzo[b][1,4]oxazine, 4a-(2,3-difluorophenyl)octahydro-2H-benzo[b][1,4]oxazine, 4a-(2-fluorophenyl)octahydro-2H-benzo[b][1,4]oxazine, 6-methyl-4a-phenyloctahydro-2H-benzo[b][1,4]oxazine, 6-ethyl-4a-phenyloctahydro-2H-benzo[b][1,4]oxazine, 4a-(3-chlorophenyl)octahydro-2H-benzo[b][1,4]oxazine, 4a-(3-methylphenyl)octahydro-2H-benzo[b][1,4]oxazine, 4a-(2-chlorophenyl)octahydro-2H-benzo[b][1,4]oxazine, 4a-(4-chlorophenyl)octahydro-2H-benzo[b][1,4]oxazine, 4a-(4-methylphenyl)octahydro-2H-benzo[b][1,4]oxazine, 4a-(3-(trifluoromethyl)phenyl)octahydro-2H-benzo[b][1,4]oxazine, 8-methyl-4a-phenyloctahydro-2H-benzo[b][1,4]oxazine, 5-methyl-4a-phenyloctahydro-2H-benzo[b][1,4]oxazine, 7-methyl-4a-phenyloctahydro-2H-benzo[b][1,4]oxazine, 4-methyl-4a-phenyloctahydro-2H-benzo[b][1,4]oxazine, 3,3-dimethyl-5a-phenyldecahydrobenzo[b] [1,4]olanzapine, 4a-(3-methoxyphenyl)octahydro-2H-benzo[b][1,4]oxazine, 4a-(3-(trifluoromethoxy)phenyl)octahydro-2H-benzo[b][1,4]oxazine, 4*a*-(4-(trifluoromethyl)phenyl)octahydro-2H-benzo[*b*][1,4]oxazine, 4*a*-(2,6-dimethylphenyl)octahydro-2H-benzo[*b*][1,4]oxazine, 4*a*-(4-(t-butyl)phenyl)octahydro-2H-benzo[*b*][1,4]oxazine, 4*a*-(2,3-dichlorophenyl)octahydro-2H-benzo[*b*][1,4]oxazine, 4*a*-(2-isopropylphenyl)octahydro-2H-benzo[*b*] [1,4]oxazine, 4*a*-(2,5-dimethylphenyl)octahydro-2*H*-benzo[*b*][1,4]oxazine, 4*a*-(2-chloro-3-fluorophenyl)octahydro-2H-benzo[*b*][1,4]oxazine, 4*a*-(3,4-difluorophenyl)octahydro-2H-benzo[*b*][1,4]oxazine, 6,6-dimethyl-4*a-*phenyloctahydro-2*H*-benzo[*b*][1,4]oxazine, 4*a*-(2-chloro-5-fluorophenyl)octahydro-2H-benzo[*b*][1,4]oxazine, 4*α*-(3-ethoxyphenyl)octahydro-2H-benzo[*b*][1,4]oxazine, 4*α*-(2-chloro-4-methoxyphenyl)octahydro-2H-benzo[*b*][1,4]oxazine, and 4a-(2-chloro-6-fluorophenyl)octahydro-2H-benzo[b] [1,4]oxazine.

In some embodiments of the compound of Formula A, when Y is phenyl substituted with 1 chloro, X is -CH₂-, and when m and o are both 0, the chiral carbon atom at position 1 and the chiral carbon atom at position 2 are not both in S configuration or R configuration (for example, the chiral carbon atom at position 1 is in S configuration and the chiral carbon atom at position 2 is in R configuration; or the chiral carbon atom at position 1 is in R configuration and the chiral carbon atom at position 2 is in S configuration); and when Y is an unsubstituted 2-thienyl, X is -CH₂-, and when m and o are both 0, the chiral carbon atom at position 1 and the chiral carbon atom at position 2 are both in S configuration or R configuration (for example, the chiral carbon atoms at position 1 and position 2 are both in S configuration; the chiral carbon atoms at position 1 and position 2 are both in R configuration).

In some embodiments of the compound of Formula A, Y is phenyl or thienyl optionally substituted with 1, 2, 3, 4 or 5 substituents selected from deuterium, cyano, C₁₋₃ alkyl, or halogens. The thienyl may be 2-thienyl or 3-thienyl.

In some embodiments of the compound of Formula A, R₂ is selected from deuterium or halogen.

In some embodiments of the compound of Formula A, R₃ is selected from deuterium or halogen.

In some embodiments, the present invention provides a compound of Formula I: 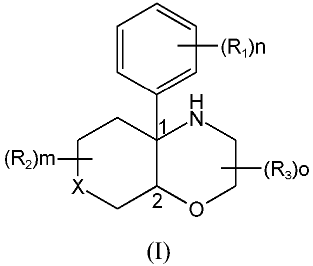
or a pharmaceutically acceptable salt, enantiomer, diastereomer, tautomer, solvate, isotopologue, polymorph, prodrug, or metabolite thereof,
wherein:
   R₁ is selected from deuterium, C₁₋₃ alkyl, or halogen;
   R₂ is selected from deuterium, C₁₋₃ alkyl, or halogen;
   R₃ is selected from deuterium, C₁₋₃ alkyl, or halogen;
   n is 0, 1 or 2;
   m is 0, 1, 2 or 3;
   o is 0, 1, 2 or 3;
   X is -O- or -CH₂-;
   the chiral carbon atom at position 1 is in S or R configuration; and
   the chiral carbon atom at position 2 is in S or R configuration;
   provided that the compound of Formula I is not the following compounds and salts thereof: (4aR,8aR)-4α-(3-chlorophenyl)octahydro-2H-benzo[b][1,4]oxazine, (4aS,8aS)-4α-(3-chlorophenyl)octahydro-2H-benzo[b][1,4]oxazine, (4aR,8aR)-4α-(2-chlorophenyl)octahydro-2H-benzo[b][1,4]oxazine, and (4aS,8aS)-4α-(2-chlorophenyl)octahydro-2H-benzo[b][1,4]oxazine.

In some embodiments of the compound of Formula I, when n is 1, R₁ is chloro, X is -CH₂-, and when m and o are both 0, the chiral carbon atom at position 1 and the chiral carbon atom at position 2 are not both in S configuration or R configuration (for example, the chiral carbon atom at position 1 is in S configuration and the chiral carbon atom at position 2 is in R configuration; or the chiral carbon atom at position 1 is in R configuration and the chiral carbon atom at position 2 is in S configuration).

In some embodiments of the compounds of Formulas A and I, R₁ is halogen, and further preferably Cl.

In some embodiments of the compounds of Formulas A and I, n is 1; and preferably, R₁ is at the ortho or para position.

In some embodiments of the compounds of Formulas A and I, m is 0.

In some embodiments of the compounds of Formulas A and I, R₂ is deuterium, and preferably m is 1 or 2.

In some embodiments of the compounds of Formulas A and I, R₂ is methyl, and preferably m is 1 or 2.

In some embodiments of the compounds of Formulas A and I, R₂ is F; preferably, m is 1 in this case; and preferably, X is -CH₂- in this case.

In some embodiments of the compounds of Formulas A and I, o is 0.

In some embodiments of the compounds of Formulas A and I, R₃ is deuterium, and preferably o is 1 or 2.

In some embodiments of the compounds of Formulas A and I, m is 0, o is 1 or 2, and R₃ is deuterium.

In some embodiments of the compounds of Formulas A and I, X is O, at least one of m and o is not 0, and correspondingly at least one of R₂ and R₃ is deuterium.

In some embodiments of the compound of Formula A, Y is an unsubstituted thienyl (e.g. 3-thienyl), m and o are 0, and X is -CH₂-.

It is to be understood that when X is -CH₂- and m is 1 or 2, X may be substituted with 1 or 2 R₂.

In some embodiments, the compound of Formula I has a structure of Formula I-1, I-2 or I-3: 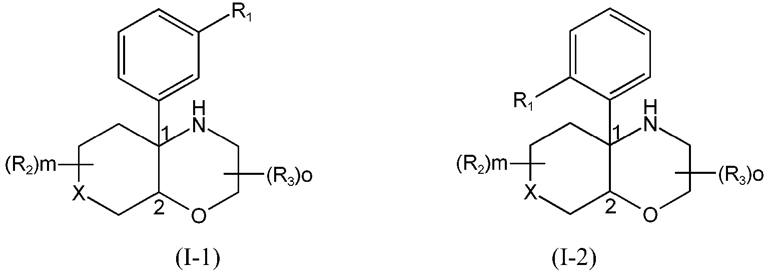 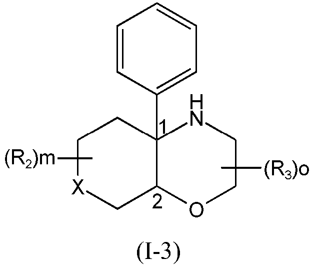 in which, R₁, R₂, R₃, m, o and X are as defined in any of the foregoing embodiments.

In some embodiments of the compounds of Formulas I-1 and I-2, R₁ is Cl.

In some embodiments of the compounds of Formulas I-1, I-2, and I-3, X is - CH₂-, where the chiral carbon atom at position 1 is in S configuration, and the chiral carbon atom at position 2 is in R configuration; or the chiral carbon atom at position 1 is in R configuration, and the chiral carbon atom at position 2 is in S configuration.

In some embodiments of the compounds of Formulas I-1 and I-2, X is -CH₂-; preferably, R₁ is Cl; further preferably, the chiral carbon atom at position 1 is in S configuration, and the chiral carbon atom at position 2 is in R configuration; or the chiral carbon atom at position 1 is in R configuration, and the chiral carbon atom at position 2 is in S configuration.

In some embodiments of the compounds of Formulas I-1, I-2, and I-3, X is O, m and o are both 0. Preferably, the chiral carbon atom at position 1 and the chiral carbon atom at position 2 are both in the same configuration in this case. For example, the chiral carbon atom at position 1 and the chiral carbon atom at position 2 are both in S configuration, or the chiral carbon atom at position 1 and the chiral carbon atom at position 2 are both in R configuration.

In some embodiments of the compounds of Formulas I-1, I-2, and I-3, X is O, m is 1 or 2, and R₂ is deuterium. In some embodiments of the compounds of Formulas I-1, I-2, and I-3, X is O, o is 1 or 2, and R₃ is deuterium.

In some embodiments of the compounds of Formulas I-1 and I-2, X is O or - CH₂-, R₁ is Cl, o is 1 or 2, and R₃ is deuterium.

In some embodiments of the compound of Formula I-3, X is -CH₂-; R₂ is F and m is 1, or R₂ is deuterium and m is 2; o is 0.

In some embodiments, the compound of Formula I has a structure of Formulas I-4 and I-5: 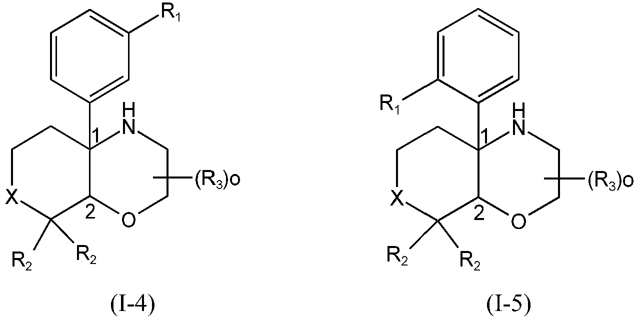 in which, R₁, R₂, R₃, m, o and X are as defined in any of the foregoing embodiments.

In some embodiments of the compounds of Formulas I-4 and I-5, R₁ is Cl.

In some embodiments of the compounds of Formulas I-4 and I-5, R₂ is deuterium.

In some embodiments of the compounds of Formulas I-4 and I-5, o is 0.

In some embodiments, the compound of Formula A is selected from the following compounds:

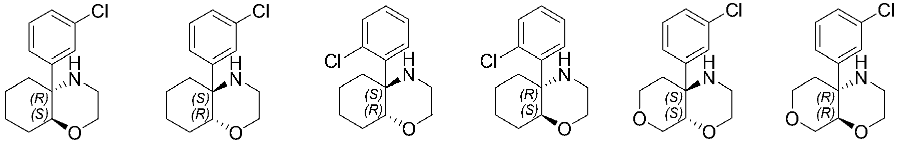

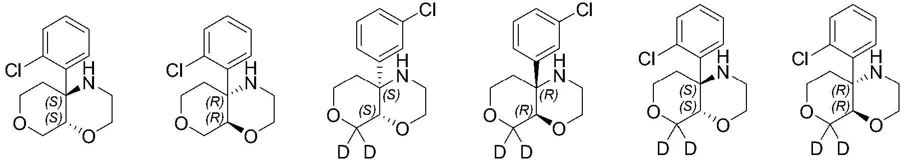

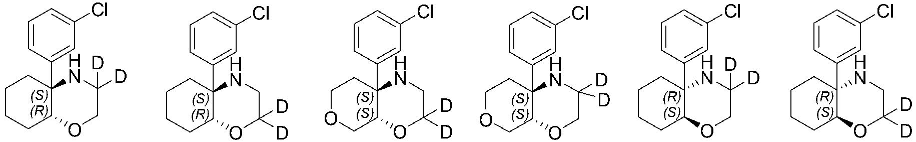

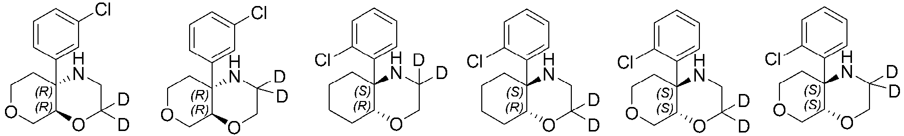

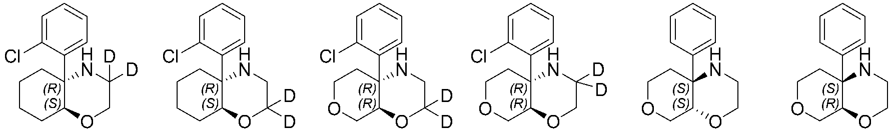

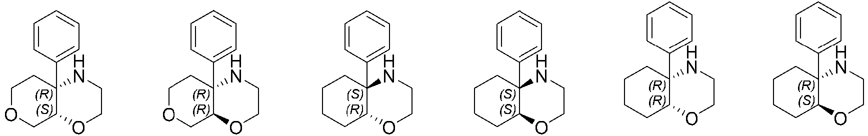

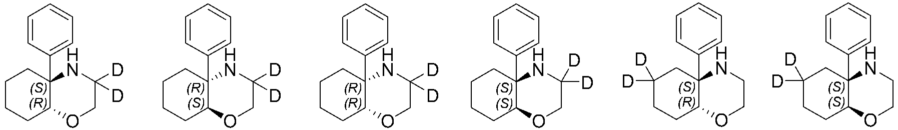

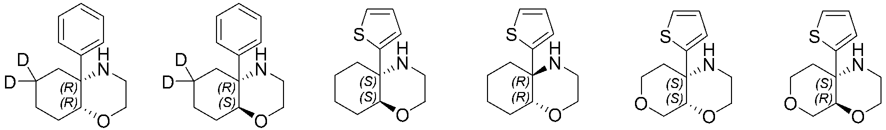

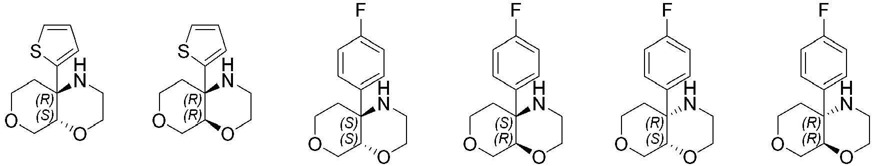

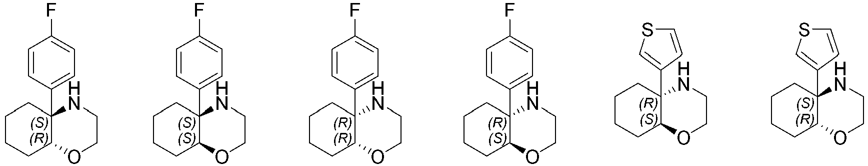

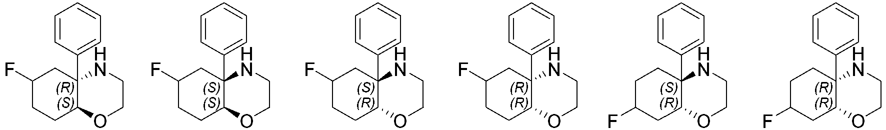

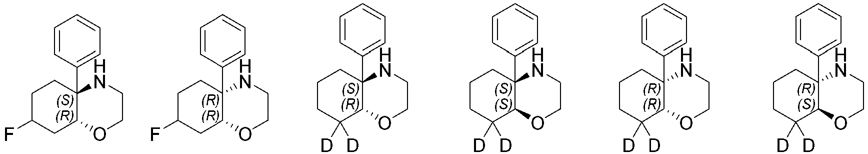

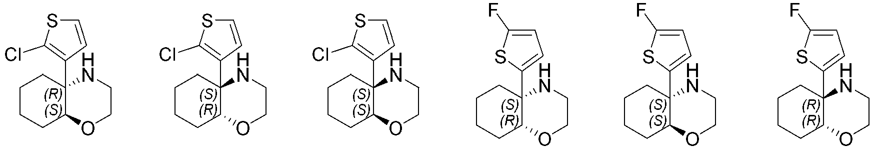

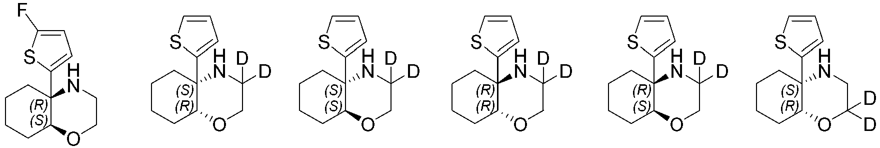

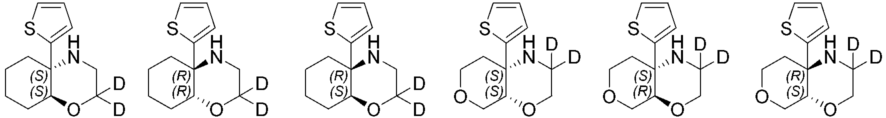

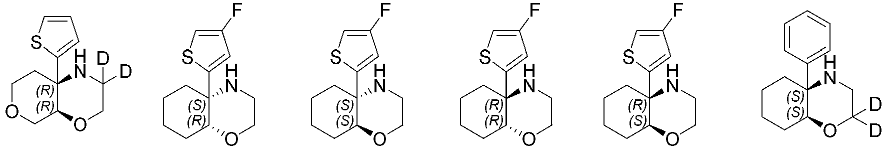

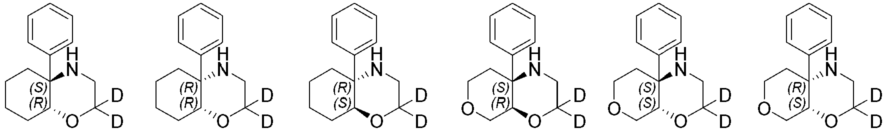

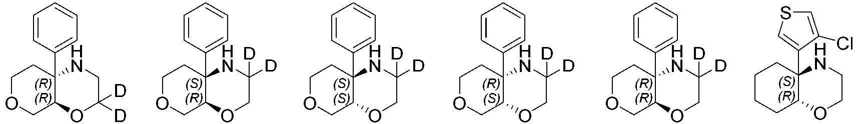

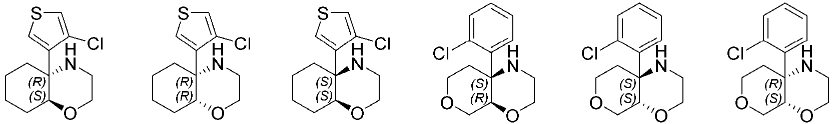

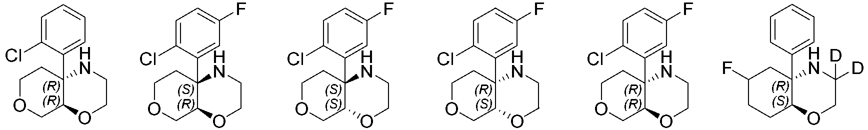

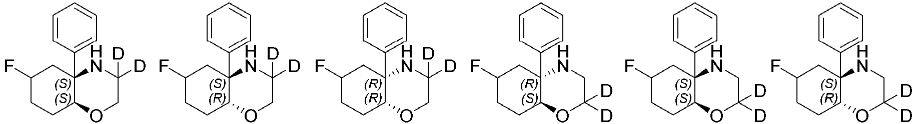

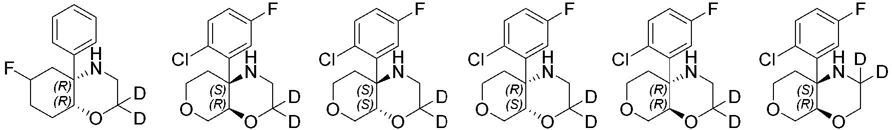

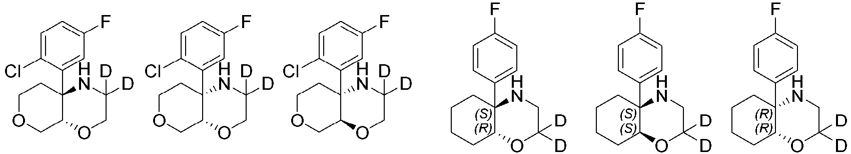

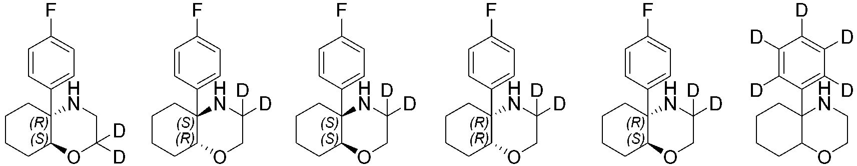

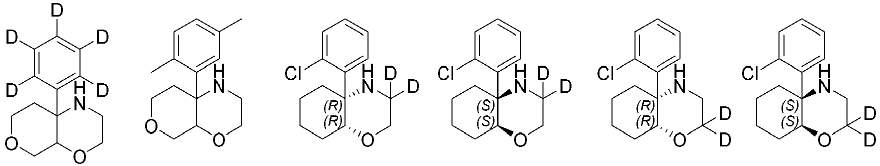

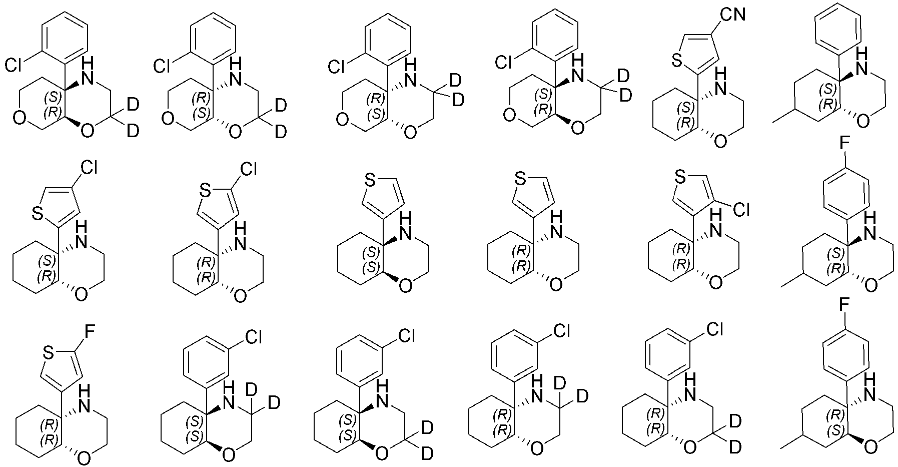

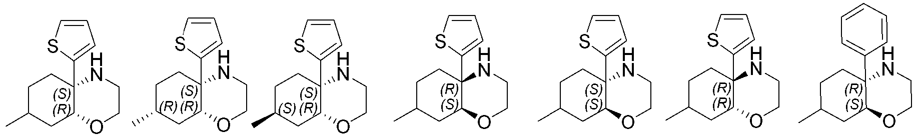

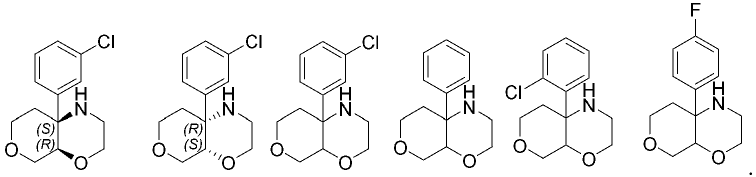

In some embodiments, the present invention further provides a pharmaceutically acceptable salt of the compounds of Formulas A and I, including, but not limited to, one or more of a formate, a hydrochloride, a sulfate, a phosphate, an oxalate, a pamoate, a citrate, a succinate, a D-tartrate, a fumarate, a maleate, a p-toluenesulfonate, a mesylate, and a benzenesulfonate.

In some embodiments, the pharmaceutically acceptable salt of the compound of Formula A is selected from:
Compound **2** hydrochloride, Compound **2** phosphate, Compound **2** mesylate, Compound **2** formate, Compound **2** oxalate, Compound **2** pamoate, Compound **3** hydrochloride, Compound **3** phosphate, Compound **3** mesylate, Compound **3** formate, Compound **3** oxalate, Compound **3** pamoate, Compound **13** hydrochloride, Compound **17** hydrochloride, Compound **33** hydrochloride, Compound **36** hydrochloride, Compound **34** hydrochloride, Compound **35** hydrochloride, Compound **39** hydrochloride, Compound **40** hydrochloride, Compound **45** hydrochloride, Compound **46** hydrochloride, Compound **74** hydrochloride, Compound **80** hydrochloride, Compound **96** hydrochloride, Compound **98** hydrochloride, Compound **144** hydrochloride, Compound **145** hydrochloride, Compound **158** hydrochloride, Compound **55** hydrochloride, Compound **56** hydrochloride, Compound **57** hydrochloride, Compound **58** hydrochloride, Compound **84** hydrochloride, Compound **92** hydrochloride, Compound **98** hydrochloride, Compound **139** hydrochloride, Compound **141** hydrochloride, Compound **155** hydrochloride, Compound **156** hydrochloride, Compound **157** hydrochloride, Compound **158** hydrochloride, Compound **159** hydrochloride, Compound **160** hydrochloride, Compound **161** hydrochloride, Compound **177** hydrochloride, Compound **178** hydrochloride, Compound **179** hydrochloride, Compound **180** hydrochloride, Compound **218** mesylate, Compound **219** mesylate, Compound **242** mesylate, Compound **243** mesylate, Compound **167**a mesylate, Compound **167**b mesylate, Compound **29** mesylate, Compound **32** mesylate, Compound **51** mesylate, Compound **54** mesylate, Compound **62** mesylate, Compound **68** mesylate, Compound **230** mesylate, Compound **231** mesylate, Compound **196** mesylate, Compound **199** mesylate, Compound **226** mesylate, Compound **14** mesylate, Compound **163** mesylate, or Compound **164** mesylate.

In some embodiments, the present invention provides a compound of Formula A': 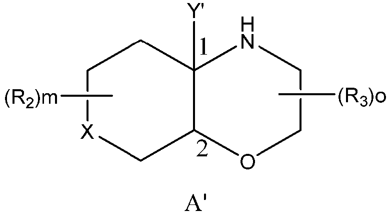
or a pharmaceutically acceptable salt, enantiomer, diastereomer, tautomer, solvate, isotopologue, polymorph, prodrug, or metabolite thereof,
wherein,
   Y' is a C₁₋₆ alkyl optionally substituted with a 6- to 14-membered aryl, in which the 6- to 14-membered aryl is optionally substituted with 1, 2, 3, 4 or 5 halogens; and
   R₂ is selected from deuterium, C₁₋₃ alkyl, or halogen;
   R₃ is selected from deuterium, C₁₋₃ alkyl, or halogen;
   m is 0, 1, 2 or 3;
   o is 0, 1, 2 or 3;
   X is -O- or -CH₂-;
   the chiral carbon atom at position 1 is in S or R configuration; and
   the chiral carbon atom at position 2 is in S or R configuration;
   provided that the compound of Formula A' is not the following compounds and salts thereof: (4aR,8aS)-4α-(2-thienyl)octahydro-2H-benzo[b][1,4]oxazine, (4aR,8aR)-4α-(2-chloro-3-thienyl)octahydro-2H-benzo[b][1,4]oxazine, (4aS,8aR)-4α-(2-thienyl)octahydro-2H-benzo[b][1,4]oxazine, (4aR,8aS)-4α-(3-methyl-2-thienyl)octahydro-2H-benzo[b][1,4]oxazine, (4aR,8aR)-4α-(4-methyl-3-thienyl)octahydro-2H-benzo[b][1,4]oxazine, (4aR,8aR)-4α-(3-chlorophenyl)octahydro-2H-benzo[b][1,4]oxazine, (4aS,8aS)-4α-(3-chlorophenyl)octahydro-2H-benzo[b][1,4]oxazine, (4aR,8aR)-4α-(2-chlorophenyl)octahydro-2H-benzo[b][1,4]oxazine, (4aS,8aS)-4α-(2-chlorophenyl)octahydro-2H-benzo[b][1,4]oxazine, 4a-phenyloctahydro-2H-benzo[b][1,4]oxazine, 4a-(2-methoxyphenyl)octahydro-2H-benzo[b][1,4]oxazine, 4a-(3-fluorophenyl)octahydro-2H-benzo[b][1,4]oxazine, 4a-(4-fluorophenyl)octahydro-2H-benzo[b][1,4]oxazine, 4a-(2,3-difluorophenyl)octahydro-2H-benzo[b][1,4]oxazine, 4a-(2-fluorophenyl)octahydro-2H-benzo[b][1,4]oxazine, 6-methyl-4a-phenyloctahydro-2H-benzo[b][1,4]oxazine, 6-ethyl-4a-phenyloctahydro-2H-benzo[b][1,4]oxazine, 4a-(3-chlorophenyl)octahydro-2H-benzo[b][1,4]oxazine, 4a-(3-methylphenyl)octahydro-2H-benzo[b][1,4]oxazine, 4a-(2-chlorophenyl)octahydro-2H-benzo[b][1,4]oxazine, 4a-(4-chlorophenyl)octahydro-2H-benzo[b][1,4]oxazine, 4a-(4-methylphenyl)octahydro-2H-benzo[b][1,4]oxazine, 4a-(3-(trifluoromethyl)phenyl)octahydro-2H-benzo[b][1,4]oxazine, 8-methyl-4a-phenyloctahydro-2H-benzo[b][1,4]oxazine, 5-methyl-4a-phenyloctahydro-2H-benzo[b][1,4]oxazine, 7-methyl-4a-phenyloctahydro-2H-benzo[b][1,4]oxazine, 4-methyl-4a -phenyloctahydro-2H-benzo[b] [1,4]oxazine, 3,3-dimethyl-5a-phenyldecahydrobenzo[b][1,4]olanzapine, 4a-(3-methoxyphenyl)octahydro-2H-benzo[b][1,4]oxazine, 4a-(3-(trifluoromethoxy)phenyl)octahydro-2H-benzo[b][1,4]oxazine, 4*a*-(4-(trifluoromethyl)phenyl)octahydro-2*H*-benzo[*b*][1,4]oxazine, 4*a*-(2,6-dimethylphenyl)octahydro-2*H*-benzo[*b*] [1,4]oxazine, 4*a*-(4-(t-butyl)phenyl)octahydro-2H-benzo[*b*] [1,4]oxazine, 4*a*-(2,3-dichlorophenyl)octahydro-2H-benzo[*b*][1,4]oxazine, 4*a*-(2-isopropylphenyl)octahydro-2H-benzo[*b*] [1,4]oxazine, 4*a*-(2,5-dimethylphenyl)octahydro-2*H*-benzo[*b*] [1,4]oxazine, 4*a*-(2-chloro-3-fluorophenyl)octahydro-2H-benzo[*b*][1,4]oxazine, 4*a*-(3,4-difluorophenyl)octahydro-2H-benzo[*b*][1,4]oxazine, 6,6-dimethyl-4*a-*phenyloctahydro-2*H*-benzo[*b*][1,4]oxazine, 4*a*-(2-chloro-5-fluorophenyl)octahydro-2H-benzo[*b*] [1,4]oxazine, 4*α*-(3-ethoxyphenyl)octahydro-2H-benzo[b][1,4]oxazine, 4*α*-(2-chloro-4-methoxyphenyl)octahydro-2H-benzo[*b*][1,4]oxazine, and 4a-(2-chloro-6-fluorophenyl)octahydro-2H-benzo[b] [1,4]oxazine.

In some embodiments of the compound of Formula A', X is -CH₂-.

In some embodiments of the compound of Formula A', m is 0.

In some embodiments of the compound of Formula A', o is 0.

In some embodiments of the compound of Formula A', Y' is a C₁₋₃ alkyl optionally substituted with a 6- to 10-membered aryl, in which the 6- to 10-membered aryl is optionally substituted with 1, 2, 3, 4 or 5 halogens; preferably, Y' is methyl optionally substituted with a 6- to 10-membered aryl, in which the 6- to 10-membered aryl is optionally substituted with 1, 2, 3, 4 or 5 halogens selected from Cl or F; and preferably, Y' is methyl substituted with phenyl, in which the phenyl is optionally substituted with 1, 2 or 3 Cl.

In some embodiments, the compound of Formula A' is selected from the following compounds:

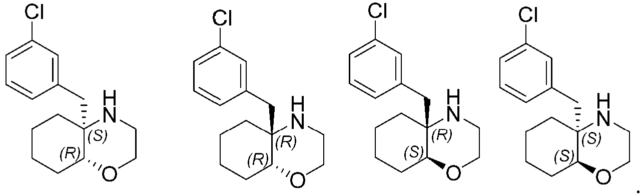

In some embodiments, the present invention provides a compound of Formula II-1: 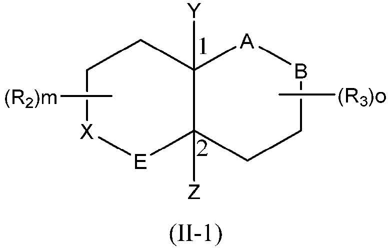
or a pharmaceutically acceptable salt, enantiomer, diastereomer, tautomer, solvate, isotopologue, polymorph, prodrug, or metabolite thereof,
wherein,
   X is -O- or -CH₂-;
   E is -O- or -CH₂-;
   A is -NRₐ- or -CH₂-;
   B is -NRₐ- or -CH₂-;
   Rₐ is selected from H or C₁₋₆ alkyl;
   Y is selected from a substituted or unsubstituted 6- to 14-membered aryl and a substituted or unsubstituted 5- to 14-membered heteroaryl, and Y is optionally substituted with 1, 2, 3, 4 or 5 R₁, where each R₁ is independently selected from deuterium, cyano, C₁₋₃ alkyl, or halogen, and the 5- to 14-membered heteroaryl includes 1, 2, 3 or 4 heteroatoms selected from N, O or S;
   Z is selected from H, hydroxyl, C₁₋₆ alkyl, or C₁₋₆ alkoxy;
   R₂ is selected from deuterium, C₁₋₃ alkyl, or halogen;
   R₃ is selected from deuterium, C₁₋₃ alkyl, or halogen;
   m is 0, 1, 2 or 3;
   o is 0, 1, 2 or 3;
   the chiral carbon atom at position 1 is in S or R configuration; and
   the chiral carbon atom at position 2 is in S or R configuration.

In some embodiments of the compound of Formula II-1, X and E are both - CH₂-; or one of X and E is -O-, and the other is -CH₂-.

In some embodiments of the compound of Formula II-1, one of A and B is - NRₐ-, and the other is -CH₂-.

In some embodiments of the compound of Formula II-1, Z is selected from H or hydroxyl.

In some embodiments of the compound of Formula II-1, Y is selected from a substituted or unsubstituted 6- to 10-membered aryl or a substituted or unsubstituted 5- to 7-membered heteroaryl; preferably, Y is optionally substituted with 1, 2, 3, 4 or 5 halogens; and preferably, the halo is selected from Cl or F.

In some embodiments of the compound of Formula II-1, m is 0.

In some embodiments of the compound of Formula II-1, o is 0.

In some embodiments, the present invention provides a compound of Formula II-2: 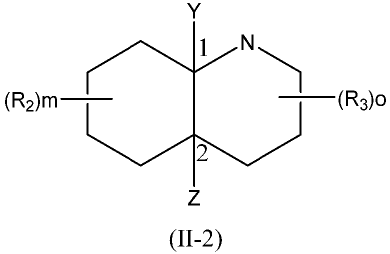
or a pharmaceutically acceptable salt, enantiomer, diastereomer, tautomer, solvate, isotopologue, polymorph, prodrug, or metabolite thereof
wherein,
   Y is selected from a substituted or unsubstituted 6- to 14-membered aryl or a substituted or unsubstituted 5- to 14-membered heteroaryl, and Y is optionally substituted with 1, 2, 3, 4 or 5 R₁, where each R₁ is independently selected from deuterium, cyano, C₁₋₃ alkyl, or halogen, and the 5- to 14-membered heteroaryl includes 1, 2, 3 or 4 heteroatoms selected from N, O or S;
   Z is selected from hydroxyl, C₁₋₆ alkyl, or C₁₋₆ alkoxy;
   R₂ is selected from deuterium, C₁₋₃ alkyl, or halogen;
   R₃ is selected from deuterium, C₁₋₃ alkyl, or halogen;
   m is 0, 1, 2 or 3;
   o is 0, 1, 2 or 3;
   the chiral carbon atom at position 1 is in S or R configuration; and
   the chiral carbon atom at position 2 is in S or R configuration.

In some embodiments of the compound of Formula II-2, Z is hydroxyl.

In some embodiments of the compound of Formula II-2, Y is selected from a substituted or unsubstituted 6- to 10-membered aryl or a substituted or unsubstituted 5- to 7-membered heteroaryl; preferably, Y is optionally substituted with 1, 2, 3, 4 or 5 halogens; and preferably, the halo is selected from Cl or F.

In some embodiments of the compound of Formula II-2, m is 0.

In some embodiments of the compound of Formula II-2, o is 0.

In some embodiments, the compound of Formula II-2 is selected from the following compounds:

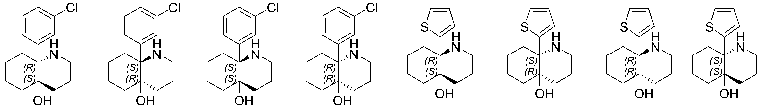

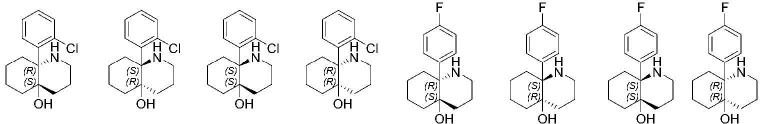

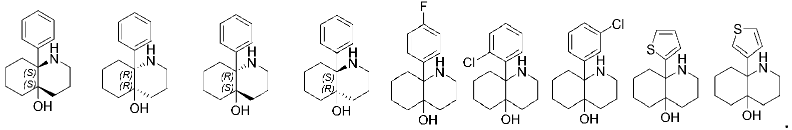

In some embodiments, the present invention provides a compound of Formula II-3: 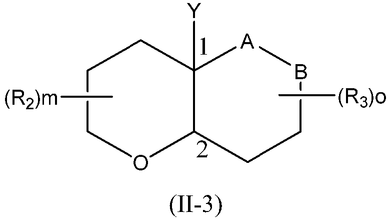
or a pharmaceutically acceptable salt, enantiomer, diastereomer, tautomer, solvate, isotopologue, polymorph, prodrug, or metabolite thereof,
wherein,
   A is -NRₐ- or -CH₂-;
   B is -NRₐ- or -CH₂-;
   Rₐ is selected from H or C₁₋₆ alkyl;
   Y is selected from a substituted or unsubstituted 6- to 14-membered aryl or a substituted or unsubstituted 5- to 14-membered heteroaryl, and Y is optionally substituted with 1, 2, 3, 4 or 5 R₁, where each R₁ is independently selected from deuterium, cyano, C₁₋₃ alkyl, or halogen, and the 5- to 14-membered heteroaryl includes 1, 2, 3 or 4 heteroatoms selected from N, O or S;
   R₂ is selected from deuterium, C₁₋₃ alkyl, or halogen;
   R₃ is selected from deuterium, C₁₋₃ alkyl, or halogen;
   m is 0, 1, 2 or 3;
   o is 0, 1, 2 or 3;
   the chiral carbon atom at position 1 is in S or R configuration; and
   the chiral carbon atom at position 2 is in S or R configuration.

In some embodiments of the compound of Formula II-3, one of A and B is - NRₐ-, and the other is -CH₂-.

In some embodiments of the compound of Formula II-3, Y is selected from a substituted or unsubstituted 6- to 10-membered aryl or a substituted or unsubstituted 5- to 7-membered heteroaryl; preferably, Y is optionally substituted with 1, 2, 3, 4 or 5 halogens; and preferably, the halo is selected from Cl or F.

In some embodiments of the compound of Formula II-3, m is 0.

In some embodiments of the compound of Formula II-3, o is 0.

In some embodiments, the compound of Formula II-3 is selected from the following compounds:

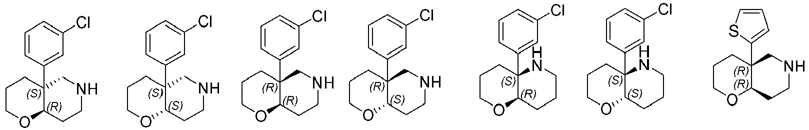

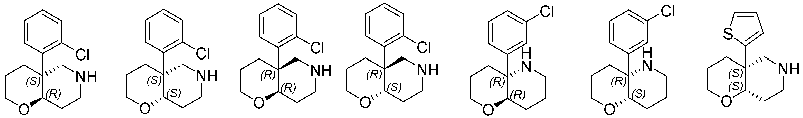

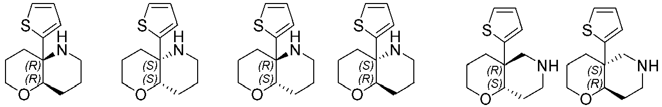

In some embodiments, the present invention provides a compound of Formula II-4: 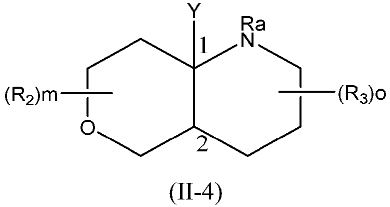
or a pharmaceutically acceptable salt, enantiomer, diastereomer, tautomer, solvate, isotopologue, polymorph, prodrug, or metabolite thereof,
wherein,
   Rₐ is selected from H or C₁₋₆ alkyl;
   Y is selected from a substituted or unsubstituted 6- to 14-membered aryl or a substituted or unsubstituted 5- to 14-membered heteroaryl, and Y is optionally substituted with 1, 2, 3, 4 or 5 R₁, where each R₁ is independently selected from deuterium, cyano, C₁₋₃ alkyl, or halogen, and the 5- to 14-membered heteroaryl includes 1, 2, 3 or 4 heteroatoms selected from N, O or S;
   R₂ is selected from deuterium, C₁₋₃ alkyl, or halogen;
   R₃ is selected from deuterium, C₁₋₃ alkyl, or halogen;
   m is 0, 1, 2 or 3;
   o is 0, 1, 2 or 3;
   the chiral carbon atom at position 1 is in S or R configuration; and
   the chiral carbon atom at position 2 is in S or R configuration.

In some embodiments of the compound of Formula II-4, Y is selected from a substituted or unsubstituted 6- to 10-membered aryl or a substituted or unsubstituted 5- to 7-membered heteroaryl; preferably, Y is optionally substituted with 1, 2, 3, 4 or 5 halogens; and preferably, the halo is selected from Cl or F.

In some embodiments of the compound of Formula II-4, m is 0.

In some embodiments of the compound of Formula II-4, o is 0.

In some embodiments, the compound of Formula II-4 is selected from the following compounds:

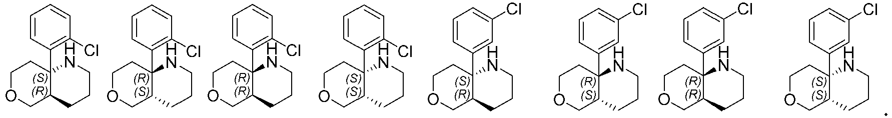

In some embodiments, the present invention further provides a compound of Formula III: 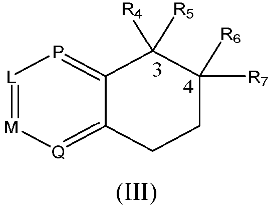
or a pharmaceutically acceptable salt, enantiomer, diastereomer, tautomer, solvate, isotopologue, polymorph, prodrug, or metabolite thereof,
wherein,
   L, M, P, and Q are each independently selected from N or CH;
   R₄, R₅, R₆, and R₇ are each independently selected from C₁₋₆ alkyl, C₁₋₆ alkoxy, -NR_{b}R_{c}, a substituted or unsubstituted 6- to 14-membered aryl, or a substituted or unsubstituted 5- to 14-membered heteroaryl, where the 6- to 14-membered aryl and the 5- to 14-membered heteroaryl are optionally substituted with a group selected from halogen, deuterium, C₁₋₆ alkyl, or C₁₋₆ alkoxy, and the 5- to 14-membered heteroaryl includes 1, 2, 3 or 4 heteroatoms selected from N, O or S; R₄ is further selected from oxo, provided that R₅ is absent; and R₆ is further selected from oxo, provided that R₇ is absent; and
   R_{b} and R_{c} are each independently selected from H or C₁₋₆ alkyl; and
   the carbon atom at position 3 or 4 is a chiral carbon atom that is in S configuration or R configuration.

In some embodiments of the compound of Formula III, R₄ is oxo, R₅ is absent, and R₆ and R₇ are each independently selected from C₁₋₆ alkyl, C₁₋₆ alkoxy, -NR_{b}R_{c}, a substituted or unsubstituted 6- to 14-membered aryl, or a substituted or unsubstituted 5- to 14-membered heteroaryl; or
R₆ is oxo, R₇ is absent, and R₄ and R₅ are each independently selected from C₁₋₆ alkyl, C₁₋₆ alkoxy, -NR_{b}R_{c}, a substituted or unsubstituted 6- to 14-membered aryl, or a substituted or unsubstituted 5- to 14-membered heteroaryl.

In some embodiments of the compound of Formula III, P is CH.

In some embodiments of the compound of Formula III, Q is CH.

In some embodiments of the compound of Formula III, one of L and M is CH, and the other is N.

In some embodiments of the compound of Formula III, M is N; L is CH; P is CH; Q is CH; R₆ is oxo, R₇ is absent, and R₄ and R₅ are each independently selected from C₁₋₆ alkyl, C₁₋₆ alkoxy, -NR_{b}R_{c}, a substituted or unsubstituted 6- to 14-membered aryl, or a substituted or unsubstituted 5- to 14-membered heteroaryl.

In some embodiments of the compound of Formula III, M is N; L is CH; P is CH; Q is CH; R₄ is oxo, R₅ is absent, and R₆ and R₇ are each independently selected from C₁₋₆ alkyl, C₁₋₆ alkoxy, -NR_{b}R_{c}, a substituted or unsubstituted 6- to 14-membered aryl, or a substituted or unsubstituted 5- to 14-membered heteroaryl.

In some embodiments of the compound of Formula III, M is CH; L is N; P is CH; Q is CH; and R₄ is oxo, R₅ is absent, and R₆ and R₇ are each independently selected from C₁₋₆ alkyl, C₁₋₆ alkoxy, -NR_{b}R_{c}, a substituted or unsubstituted 6- to 14-membered aryl, or a substituted or unsubstituted 5- to 14-membered heteroaryl.

In some embodiments of the compound of Formula III, M is CH; L is N; P is CH; Q is CH; and R₆ is oxo, R₇ is absent, and R₄ and R₅ are each independently selected from C₁₋₆ alkyl, C₁₋₆ alkoxy, -NR_{b}R_{c}, a substituted or unsubstituted 6- to 14-membered aryl, or a substituted or unsubstituted 5- to 14-membered heteroaryl.

In some embodiments of the compound of Formula III, R₄, R₅, R₆, and R₇ are each independently selected from C₁₋₆ alkoxy, -NR_{b}R_{c}, a substituted or unsubstituted 6- to 14-membered aryl, or a substituted or unsubstituted 5- to 14-membered heteroaryl; R₄ is further selected from oxo, provided that R₅ is absent; and R₆ is further selected from oxo, provided that R₇ is absent. In some embodiments, R₄, R₅, R₆, and R₇ are each independently selected from -NR_{b}R_{c} or a substituted or unsubstituted 6- to 14-membered aryl; R₄ is further selected from oxo, provided that R₅ is absent; and R₆ is further selected from oxo, provided that R₇ is absent. In some embodiments, R₄, R₅, R₆, and R₇ are each independently selected from - NR_{b}R_{c}, or a 6- to 10-membered aryl optionally substituted with 1, 2, 3 or 4 substituents selected from halogen, C₁₋₆ alkyl, or deuterium; R₄ is further selected from oxo, provided that R₅ is absent; and R₆ is further selected from oxo, provided that R₇ is absent.

In some embodiments of the compound of Formula III, R₄, and R₅ are each independently selected from -NR_{b}R_{c}, or a 6- to 10-membered aryl optionally substituted with 1, 2, 3 or 4 substituents selected from halogen, C₁₋₆ alkyl, or deuterium, R₆ is oxo, and R₇ is absent; or
R₆ and R₇ are each independently selected from -NR_{b}R_{c}, or a 6- to 10-membered aryl optionally substituted with 1, 2, 3 or 4 substituents selected from halogen, C₁₋₆ alkyl, or deuterium, R₄ is oxo, and R₅ is absent.

In some embodiments of the compound of Formula III, R_{b} and R_{c} are each independently selected from H or C₁₋₃ alkyl; and preferably, one of R_{b} and R_{c} is H, and the other is C₁₋₃ alkyl.

In some embodiments, the compound of Formula III is selected from the following compounds:

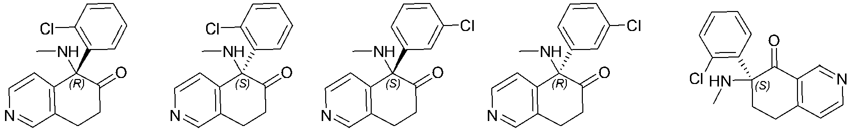

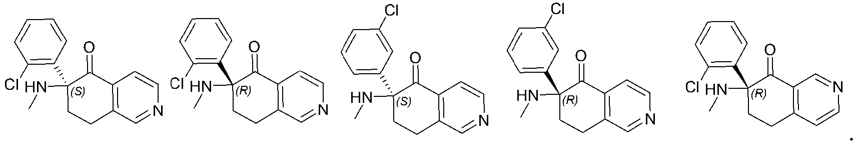

### III. Preparation of compounds

The compound of the present invention can be prepared by a reaction process similar to that described in Examples 1-25 of the present application.

### IV. Data for compounds in salt forms

The present invention provides Crystal Form A of Compound **2** hydrochloride, which has an X-ray powder diffraction pattern with characteristic peaks at at least 3, at least 4, at least 5, at least 6, at least 7, at least 8, at least 9, at least 10, at least 11, at least 12 or all the 13 of the following 2θ angles: 6.79° ± 0.20°, 11.44° ± 0.20°, 13.15° ± 0.20°, 14.44° ± 0.20°, 15.00° ± 0.20°, 16.36° ± 0.20°, 17.16° ± 0.20°, 20.59° ± 0.20°, 23.38° ± 0.20°, 24.11° ± 0.20°, 24.30° ± 0.20°, 25.74° ± 0.20°, and 26.72° ± 0.20°.

In some embodiments, Crystal Form A comprises characteristic peaks at at least the following 2θ angles: 14.44° ± 0.20°, 17.16° ± 0.20°, 20.59° ± 0.20°, and 23.38° ± 0.20°, and optionally comprises characteristic peak(s) at at least 1, at least 2, at least 3, at least 4, at least 5, at least 6, at least 7, at least 8 or all the 9 of the following 2θ angles: 6.79° ± 0.20°, 11.44° ± 0.20°, 13.15° ± 0.20°, 15.00° ± 0.20°, 16.36° ± 0.20°, 24.11° ± 0.20°, 24.30° ± 0.20°, 25.74° ± 0.20°, and 26.72° ± 0.20°.

In some embodiments, Crystal Form A comprises characteristic peaks at at least the following 2θ angles: 14.44° ± 0.20°, 17.16° ± 0.20°, 20.59° ± 0.20°, and 23.38° ± 0.20°, comprises characteristic peak(s) at at least 1, at least 2, at least 3, at least 4, at least 5 or all the 6 of the following 2θ angles: 11.44° ± 0.20°, 15.00° ± 0.20°, 16.36° ± 0.20°, 24.11° ± 0.20°, 24.30° ± 0.20°, and 26.72° ± 0.20°, and optionally comprises characteristic peak(s) at at least 1, at least 2 or all the 3 of the following 2θ angles: 6.79° ± 0.20°, 13.15° ± 0.20°, and 25.74° ± 0.20°.

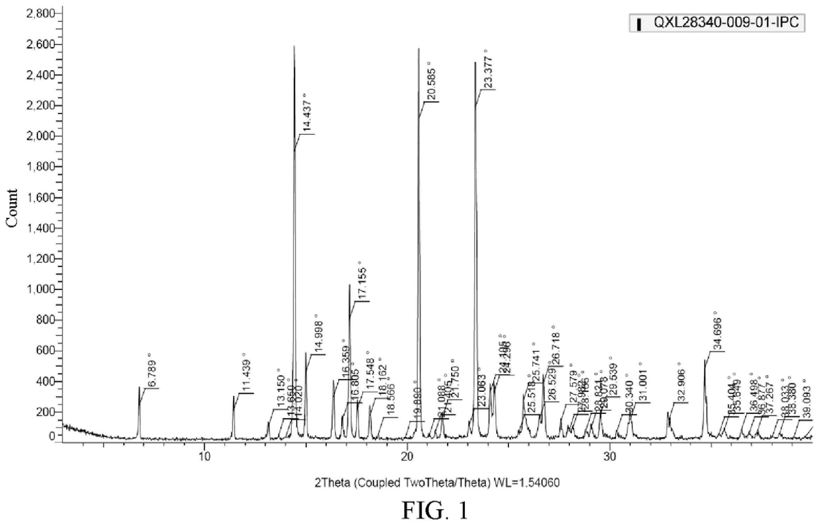

The XRPD pattern of Crystal Form A of Compound **2** hydrochloride is shown in FIG. 1.

The XRPD pattern analysis data of Crystal Form A of Compound **2** hydrochloride is shown in Table 1.

**Table 1: XRPD pattern analysis data of Crystal Form A of Compound 2 hydrochloride**

| No. | 2θ (°) | Intensity | Relative intensity (%) | No. | 2θ (°) | Intensity | Relative intensity (%) |
|---|---|---|---|---|---|---|---|
| 1 | 6.789 | 244.28 | 10.2 | 9 | 20.585 | 2111.91 | 96.7 |
| 2 | 11.439 | 210.58 | 8.8 | 10 | 23.377 | 2190.92 | 100.0 |
| 3 | 13.150 | 108.45 | 4.0 | 11 | 24.105 | 331.32 | 13.7 |
| 4 | 14.437 | 1901.9 | 87.0 | 12 | 24.296 | 329.56 | 13.7 |
| 5 | 14.998 | 463.05 | 20.1 | 13 | 25.741 | 278.11 | 11.5 |
| 6 | 16.359 | 299.67 | 12.6 | 14 | 26.718 | 388.70 | 16.7 |
| 7 | 17.155 | 809.24 | 36.2 | 15 | 29.539 | 187.07 | 7.3 |
| 8 | 17.548 | 222.59 | 9.0 | 16 | 34.696 | 482.40 | 21.0 |

No absorption peak is detected in the differential scanning calorimetry (DSC) profile of Crystal Form A of Compound **2** hydrochloride.

The weight loss starting temperature in the thermogravimetric analysis curve of Crystal Form A of Compound **2** hydrochloride is 205.5°C.

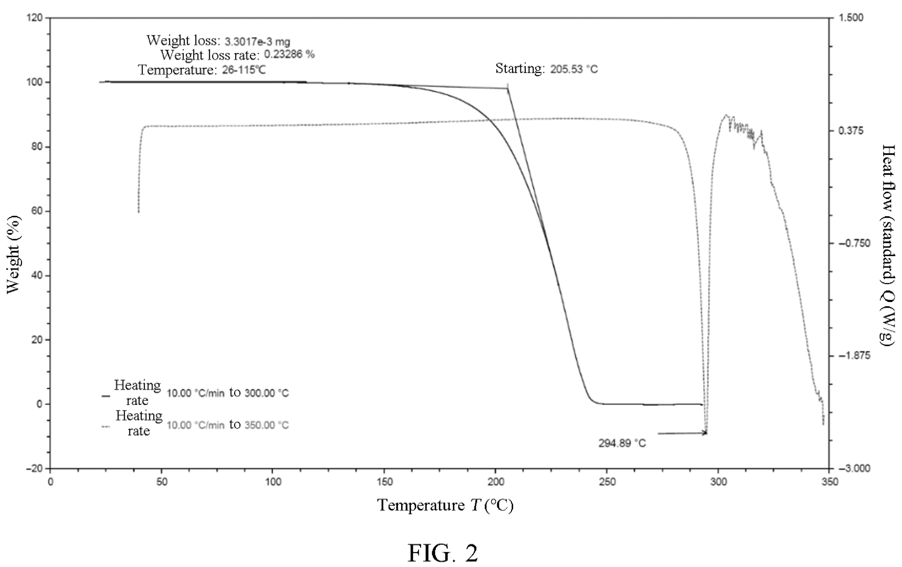

The DSC profile and TGA curve of Crystal Form A of Compound **2** hydrochloride is shown in FIG. 2.

The present invention provides Crystal Form B of Compound **2** phosphate, which has an X-ray powder diffraction pattern with characteristic peaks at at least 3, at least 4, at least 5, at least 6, at least 7, at least 8, at least 9, at least 10, at least 11 or all the 12 of the following 2θ angles: 5.48° ± 0.20°, 10.96° ± 0.20°, 12.32° ± 0.20°, 13.49° ± 0.20°, 14.83° ± 0.20°, 15.44° ± 0.20°, 16.50° ± 0.20°, 18.48° ± 0.20°, 20.63° ± 0.20°, 22.49° ± 0.20°, 22.92° ± 0.20°, and 27.64° ± 0.20°.

In some embodiments, Crystal Form B comprises characteristic peaks at at least the following 2θ angles: 5.48° ± 0.20°, 18.48° ± 0.20°, 22.49° ± 0.20°, and 22.92° ± 0.20°, and optionally comprises characteristic peak(s) at at least 1, at least 2, at least 3, at least 4, at least 5, at least 6, at least 7 or all the 8 of the following 2θ angles: 10.96° ± 0.20°, 12.32° ± 0.20°, 13.49° ± 0.20°, 14.83° ± 0.20°, 15.44° ± 0.20°, 16.50° ± 0.20°, 20.63° ± 0.20°, and 27.64° ± 0.20°.

In some embodiments, Crystal Form B comprises characteristic peaks at at least the following 2θ angles: 5.48° ± 0.20°, 18.48° ± 0.20°, 22.49° ± 0.20°, and 22.92° ± 0.20°, comprises characteristic peak(s) at at least 1, at least 2, at least 3, at least 4 or all the 5 of the following 2θ angles: 13.49° ± 0.20°, 15.44° ± 0.20°, 16.50° ± 0.20°, 20.63° ± 0.20°, and 27.64° ± 0.20°, and optionally comprises characteristic peak(s) at at least 1, at least 2 or all the 3 of the following 2θ angles: 10.96° ± 0.20°, 12.32° ± 0.20°, and 14.83° ± 0.20°.

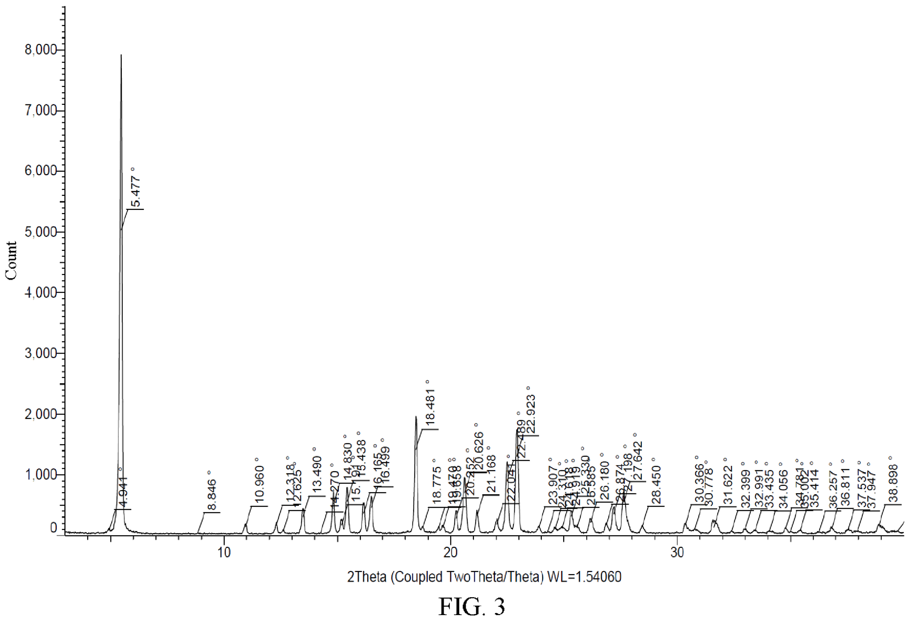

The XRPD pattern of Crystal Form B of Compound **2** phosphate is shown in FIG. 3.

The XRPD pattern analysis data of Crystal Form B of Compound **2** phosphate is shown in Table 2.

**Table 2: XRPD pattern analysis data of Crystal Form B of Compound 2 phosphate**

| No. | 2θ (°) | Intensity | Relative intensity (%) | No. | 2θ (°) | Intensity | Relative intensity (%) |
|---|---|---|---|---|---|---|---|
| 1 | 5.477 | 5033.32 | 100.0 | 9 | 20.627 | 698.80 | 13.0 |
| 2 | 10.960 | 140.76 | 2.3 | 10 | 21.168 | 326.80 | 5.5 |
| 3 | 12.318 | 162.93 | 2.6 | 11 | 22.489 | 900.29 | 16.9 |
| 4 | 13.490 | 309.58 | 5.5 | 12 | 22.923 | 1313.07 | 25.2 |
| 5 | 14.830 | 520.78 | 9.7 | 13 | 25.330 | 311.26 | 5.2 |
| 6 | 15.438 | 565.86 | 10.5 | 14 | 27.198 | 348.77 | 6.0 |
| 7 | 16.499 | 461.12 | 8.4 | 15 | 27.642 | 546.22 | 10.0 |
| 8 | 18.481 | 1415.12 | 27.4 | | | | |

The differential scanning calorimetry (DSC) profile of Crystal Form B of Compound **2** phosphate shows an endothermic peak starting at 119.4 ± 3.0°C and another endothermic peak starting at 181.1 ± 3.0°C.

The thermogravimetric analysis curve of Crystal Form B of Compound **2** phosphate shows a weight loss of up to 2.87% at 150.0 ± 3.0°C.

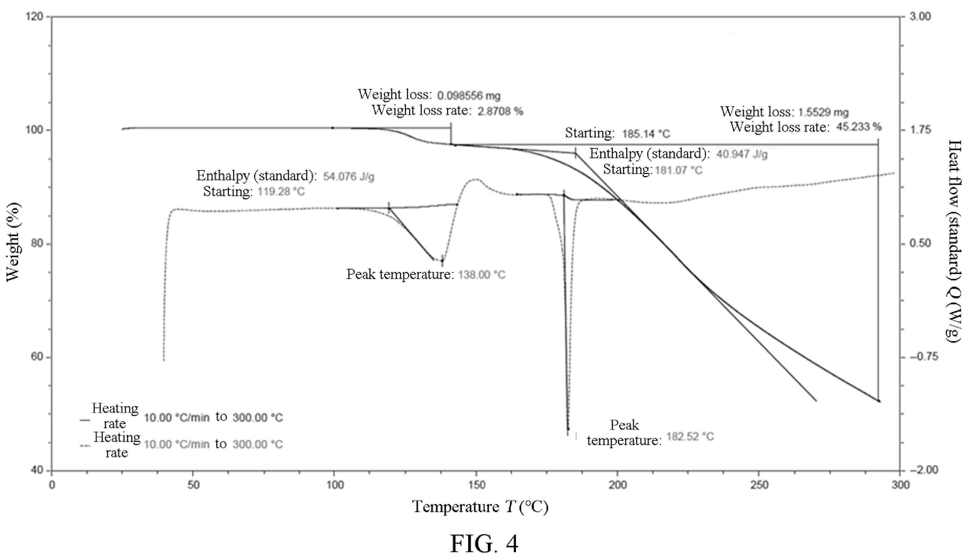

The DSC profile and TGA curve of Crystal Form B of Compound **2** phosphate are shown in FIG. 4.

The present invention provides Crystal Form C of Compound **2** mesylate, which has an X-ray powder diffraction pattern with characteristic peaks at at least 3, at least 4, at least 5, at least 6, at least 7, at least 8, at least 9 or all the 10 of the following 2θ angles: 8.05° ± 0.20°, 9.71° ± 0.20°, 12.00° ± 0.20°, 13.91° ± 0.20°, 14.37° ± 0.20°, 15.03° ± 0.20°, 16.18° ± 0.20°, 18.49° ± 0.20°, 22.65° ± 0.20°, and 24.42° ± 0.20°.

In some embodiments, Crystal Form C comprises characteristic peaks at at least the following 2θ angles: 8.05° ± 0.20°, 14.37± 0.20°, 16.18° ± 0.20°, 18.49° ± 0.20°, and 22.65° ± 0.20°, and optionally comprises characteristic peak(s) at at least 1, at least 2, at least 3, at least 4 or all the 5 of the following 2θ angles: 9.71° ± 0.20°, 12.00° ± 0.20°, 13.91° ± 0.20°, 15.03° ± 0.20°, and 24.42° ± 0.20°.

In some embodiments, Crystal Form C comprises characteristic peaks at at least the following 2θ angles: 8.05° ± 0.20°, 14.37± 0.20°, 16.18° ± 0.20°, 18.49° ± 0.20°, and 22.65° ± 0.20°, comprises characteristic peak(s) at at least 1, at least 2 or all the 3 of the following 2θ angles: 13.91° ± 0.20°, 15.03° ± 0.20°, and 24.42° ± 0.20°, and optionally comprises characteristic peak(s) at any 1 or all the 2 of the following 2θ angles, 9.71° ± 0.20°, and 12.00° ± 0.20°.

In some embodiments, Crystal Form C comprises characteristic peaks at at least the following 2θ angles: 8.05° ± 0.20°, 14.37± 0.20°, 16.18° ± 0.20°, and 18.49° ± 0.20°, comprises characteristic peak(s) at at least 1, at least 2, at least 3 or all the 4 of the following 2θ angles: 13.91° ± 0.20°, 15.03° ± 0.20°, 22.65° ± 0.20°, and 24.42° ± 0.20°, and

optionally comprises characteristic peak(s) at any 1 or all the 2 of the following 2θ angles, 9.71° ± 0.20°, and 12.00° ± 0.20°.

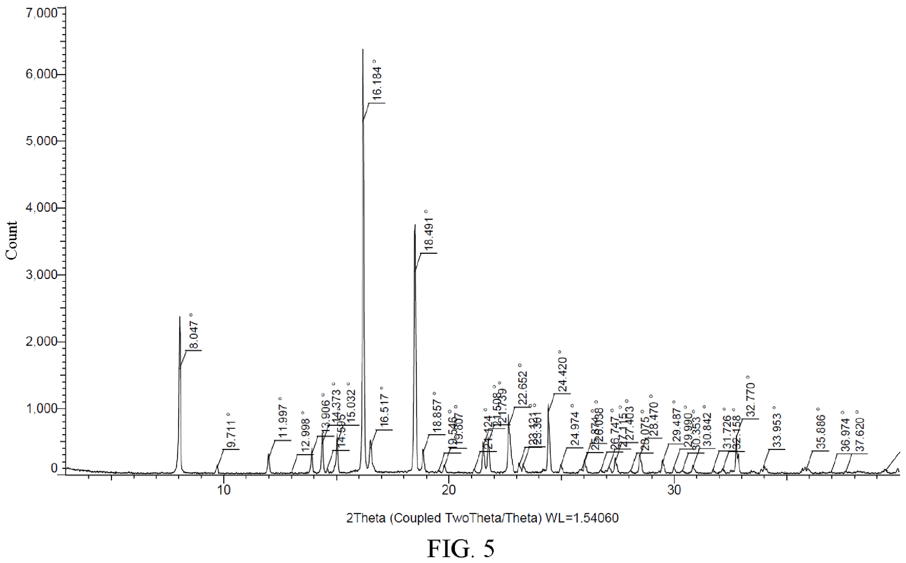

The XRPD pattern of Crystal Form C of Compound **2** mesylate is shown in FIG. 5.

The XRPD pattern analysis data of Crystal Form C of Compound **2** mesylate is shown in Table 3.

**Table 3: XRPD pattern analysis data of Crystal Form C of Compound 2 mesylate**

| No. | 2θ (°) | Intensity | Relative intensity (%) | No. | 2θ (°) | Intensity | Relative intensity (%) |
|---|---|---|---|---|---|---|---|
| 1 | 8.047 | 1593.31 | 29.7 | 9 | 18.491 | 3047.90 | 57.2 |
| 2 | 9.711 | 108.54 | 1.5 | 10 | 18.857 | 324.95 | 5.4 |
| 3 | 11.997 | 247.19 | 4.2 | 11 | 21.508 | 422.86 | 7.4 |
| 4 | 13.906 | 307.80 | 5.3 | 12 | 21.739 | 479.72 | 8.5 |
| 5 | 14.373 | 470.03 | 8.3 | 13 | 22.652 | 745.60 | 13.5 |
| 6 | 15.032 | 470.06 | 8.2 | 14 | 24.420 | 946.06 | 17.2 |
| 7 | 16.184 | 5299.34 | 100.0 | 15 | 28.470 | 280.69 | 4.7 |
| 8 | 16.517 | 400.36 | 6.7 | 16 | 32.770 | 569.56 | 10.3 |

The differential scanning calorimetry profile of Crystal Form C of Compound **2** mesylate shows an endothermic peak starting at 186.9 ± 3.0°C.

The weight loss starting temperature in the thermogravimetric analysis curve of Crystal Form C of Compound **2** mesylate is about 259.9°C.

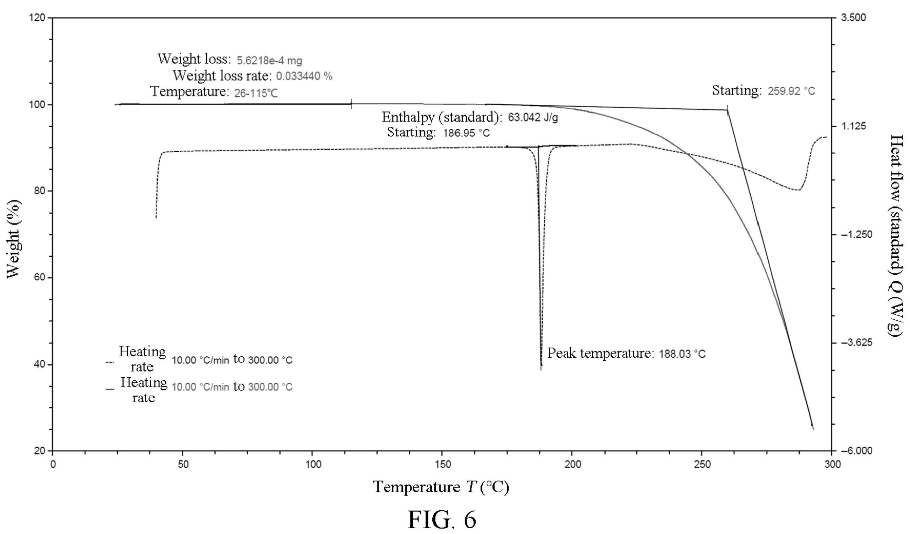

The DSC profile and TGA curve of Crystal Form C of Compound **2** mesylate are shown in FIG. 6.

The present invention provides Crystal Form D of Compound **2** mesylate, which has an X-ray powder diffraction pattern with characteristic peaks at at least 3, at least 4, at least 5, at least 6, at least 7, at least 8, at least 9, at least 10 or all the 11 of the following 2θ angles: 6.28° ± 0.20°, 11.33° ± 0.20°, 12.13° ± 0.20°, 12.53° ± 0.20°, 14.80° ± 0.20°, 17.14° ± 0.20°, 18.08° ± 0.20°, 20.54° ± 0.20°, 21.46° ± 0.20°, 22.70° ± 0.20°, 23.37° ± 0.20°, 23.93° ± 0.20°, 24.66° ± 0.20°, and 26.09° ± 0.20°.

In some embodiments, Crystal Form D comprises characteristic peaks at at least the following 2θ angles: 12.13° ± 0.20°, 14.80° ± 0.20°, 20.54° ± 0.20°, 23.37° ± 0.20°, and 26.09° ± 0.20°, and optionally comprises characteristic peak(s) at at least 1, at least 2, at least 3, at least 4, at least 5, at least 6, at least 7, at least 8 or all the 9 of the following 2θ angles: 6.28° ± 0.20°, 11.33° ± 0.20°, 12.53° ± 0.20°, 17.14° ± 0.20°, 18.08° ± 0.20°, 21.46° ± 0.20°, 22.70° ± 0.20°, 23.93° ± 0.20°, and 24.66° ± 0.20°.

In some embodiments, Crystal Form D comprises characteristic peaks at at least the following 2θ angles: 12.13° ± 0.20°, 14.80° ± 0.20°, 20.54° ± 0.20°, 23.37° ± 0.20°, and 26.09° ± 0.20°, comprises characteristic peak(s) at at least 1, at least 2, at least 3 or all the 4 of the following 2θ angles: 17.14° ± 0.20°, 18.08° ± 0.20°, 22.70° ± 0.20°, 24.66° ± 0.20°, and optionally comprises characteristic peak(s) at at least 1, at least 2, at least 3, at least 4 or all the 5 of the following 2θ angles: 6.28° ± 0.20°, 11.33° ± 0.20°, 12.53° ± 0.20°, 21.46° ± 0.20°, and 23.93° ± 0.20°.

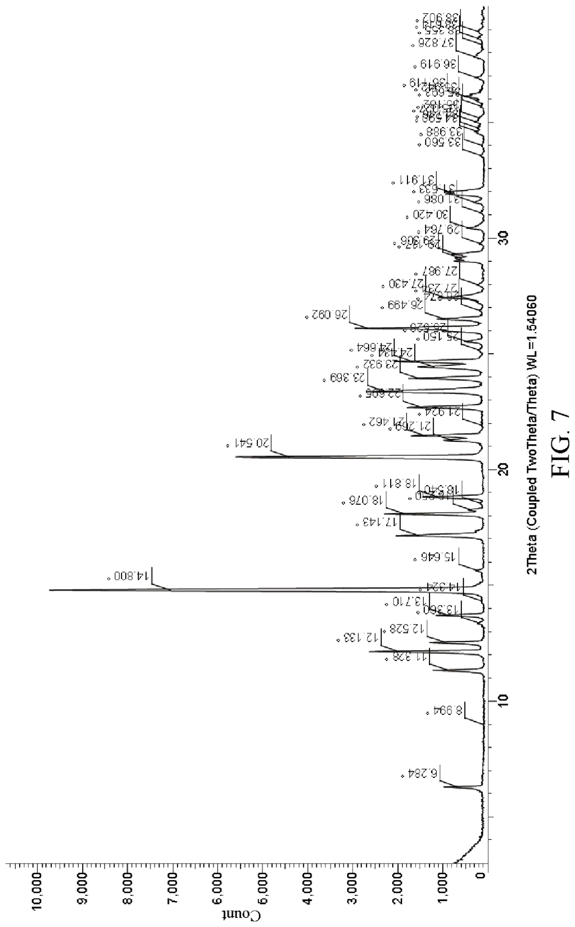

The XRPD pattern of Crystal Form D of Compound **2** mesylate is shown in FIG. 7.

The XRPD pattern analysis data of Crystal Form D of Compound **2** mesylate is shown in Table 4.

**Table 4: XRPD pattern analysis data of Crystal Form D of Compound 2 mesylate**

| No. | 2θ (°) | Intensity | Relative intensity (%) | No. | 2θ (°) | Intensity | Relative intensity (%) |
|---|---|---|---|---|---|---|---|
| 1 | 6.284 | 658.40 | 7.8 | 14 | 23.369 | 2251.74 | 30.3 |
| 2 | 11.328 | 871.34 | 11.3 | 15 | 23.932 | 1525.94 | 19.9 |
| 3 | 12.133 | 1958.21 | 26.9 | 16 | 24.434 | 1197.59 | 15.2 |
| 4 | 12.528 | 927.38 | 12.0 | 17 | 24.664 | 1661.13 | 22.0 |
| 5 | 13.710 | 875.92 | 11.3 | 18 | 25.528 | 469.21 | 4.8 |
| 6 | 14.800 | 7049.92 | 100.0 | 19 | 26.092 | 2657.40 | 36.3 |
| 7 | 17.143 | 1527.61 | 20.5 | 20 | 26.499 | 965.82 | 12.0 |
| 8 | 18.076 | 1831.36 | 24.8 | 21 | 27.430 | 950.75 | 12.0 |
| 9 | 18.811 | 1100.11 | 14.3 | 22 | 29.137 | 592.92 | 7.1 |
| 10 | 20.541 | 4401.86 | 61.7 | 23 | 29.306 | 690.21 | 8.5 |
| 11 | 21.269 | 791.31 | 9.7 | 24 | 30.420 | 415.13 | 4.6 |
| 12 | 21.462 | 1383.06 | 18.2 | 25 | 31.911 | 736.95 | 9.2 |
| 13 | 22.695 | 1464.56 | 19.1 | 26 | 36.119 | 487.47 | 5.6 |

The differential scanning calorimetry profile of Crystal Form D of Compound **2** mesylate shows an endothermic peak starting at 136.4 ± 3.0°C and another endothermic peak starting at 185.6 ± 3.0°C.

The thermogravimetric analysis curve of Crystal Form D of Compound **2** mesylate shows a weight loss of up to 7.76% at 155.0 ± 3.0°C.

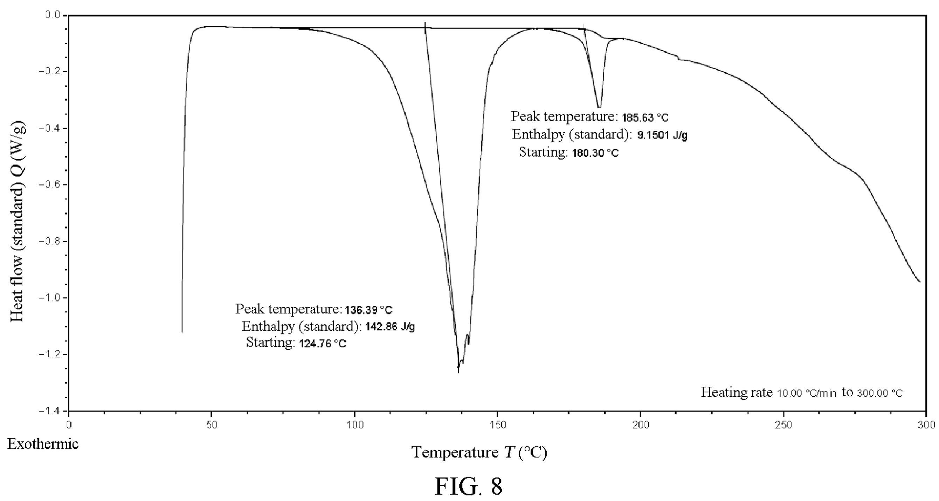

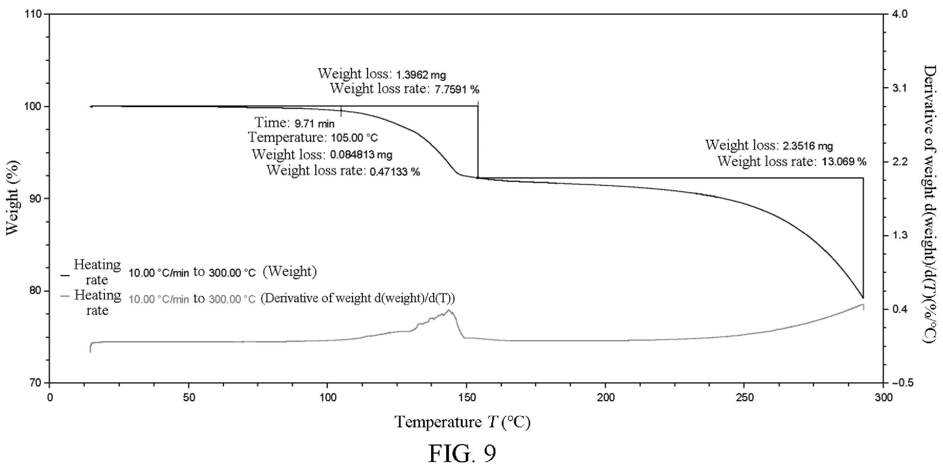

The DSC profile of Crystal Form D of Compound **2** mesylate is shown in FIG. 8, and the TGA curve is shown in FIG. 9.

The present invention provides Crystal Form E of Compound **2** mesylate, which has an X-ray powder diffraction pattern with characteristic peaks at at least 3, at least 4, at least 5, at least 6, at least 7, at least 8, at least 9, at least 10, at least 11, at least 12, at least 13 or all the 14 of the following 2θ angles: 6.24° ± 0.20°, 8.16° ± 0.20°, 12.13° ± 0.20°, 12.47° ± 0.20°, 14.48° ± 0.20°, 14.81° ± 0.20°, 16.30° ± 0.20°, 17.15° ± 0.20°, 18.60° ± 0.20°, 20.53° ± 0.20°, 21.45° ± 0.20°, 21.62° ± 0.20°, 22.75° ± 0.20°, and 26.08° ± 0.20°.

In some embodiments, Crystal Form E comprises characteristic peaks at at least the following 2θ angles: 14.81° ± 0.20°, 16.30° ± 0.20°, 18.60° ± 0.20°, and 22.75° ± 0.20°, and optionally comprises characteristic peak(s) at at least 1, at least 2, at least 3, at least 4, at least 5, at least 6, at least 7, at least 8, at least 9 or all the 10 of the following 2θ angles: 6.24° ± 0.20°, 8.16° ± 0.20°, 12.13° ± 0.20°, 12.47° ± 0.20°, 14.48° ± 0.20°, 17.15° ± 0.20°, 20.53° ± 0.20°, 21.45° ± 0.20°, 21.62° ± 0.20°, and 26.08° ± 0.20°.

In some embodiments, Crystal Form E comprises characteristic peaks at at least the following 2θ angles: 14.81° ± 0.20°, 16.30° ± 0.20°, 18.60° ± 0.20°, and 22.75° ± 0.20°, comprises characteristic peak(s) at at least 1, at least 2, at least 3, at least 4, at least 5 or all the 6 of the following 2θ angles: 12.13° ± 0.20°, 14.48° ± 0.20°, 17.15° ± 0.20°, 20.53° ± 0.20°, 21.45° ± 0.20°, and 21.62° ± 0.20°, and optionally comprises characteristic peak(s) at at least 1, at least 2, at least 3 or all the 4 of the following 2θ angles: 6.24° ± 0.20°, 8.16° ± 0.20°, 12.47° ± 0.20°, and 26.08° ± 0.20°.

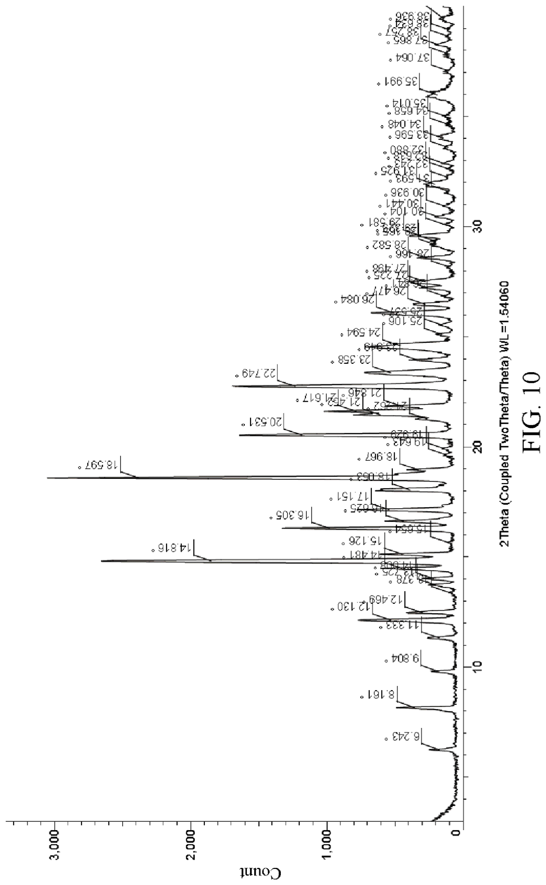

The XRPD pattern of Crystal Form E of Compound **2** mesylate is shown in FIG. 10.

The XRPD pattern analysis data of Crystal Form E of Compound **2** mesylate is shown in Table 5.

**Table 5: XRPD pattern analysis data of Crystal Form E of Compound 2 mesylate**

| No. | 2θ (°) | Intensity | Relative intensity (%) | No. | 2θ (°) | Intensity | Relative intensity (%) |
|---|---|---|---|---|---|---|---|
| 1 | 6.243 | 174.33 | 5.5 | 17 | 18.967 | 329.05 | 10.6 |
| 2 | 8.161 | 353.81 | 13.0 | 18 | 20.531 | 1182.93 | 47.5 |
| 3 | 9.804 | 178.13 | 5.4 | 19 | 21.452 | 604.85 | 22.2 |
| 4 | 11.333 | 173.48 | 5.3 | 20 | 21.617 | 784.44 | 30.1 |
| 5 | 12.130 | 529.10 | 20.6 | 21 | 21.846 | 450.07 | 15.6 |
| 6 | 12.469 | 296.14 | 10.4 | 22 | 22.749 | 1231.03 | 49.6 |
| 7 | 13.725 | 201.47 | 5.8 | 23 | 23.358 | 528.50 | 18.7 |
| 8 | 14.008 | 215.18 | 6.2 | 24 | 23.949 | 332.21 | 10.1 |
| 9 | 14.481 | 444.73 | 16.0 | 25 | 24.594 | 459.34 | 15.8 |
| 10 | 14.816 | 1848.02 | 77.0 | 26 | 26.084 | 504.66 | 18.1 |
| 11 | 15.126 | 444.90 | 15.9 | 27 | 27.498 | 272.79 | 8.3 |
| 12 | 16.305 | 979.357 | 39.2 | 28 | 28.582 | 269.31 | 8.4 |
| 13 | 16.625 | 433.86 | 15.4 | 29 | 29.581 | 307.94 | 9.9 |
| 14 | 17.151 | 541.46 | 20.1 | 30 | 31.925 | 207.50 | 5.9 |
| 15 | 18.053 | 389.98 | 13.4 | 31 | 35.991 | 188.55 | 4.9 |
| 16 | 18.597 | 2381.76 | 100.0 | | | | |

The differential scanning calorimetry profile of Crystal Form E of Compound **2** mesylate shows an endothermic peak starting at 86.3 ± 3.0°C and another endothermic peak starting at 187.7 ± 3.0°C.

The thermogravimetric analysis curve of Crystal Form E of Compound **2** mesylate shows a weight loss of up to 2.53% at 105.0 ± 3.0°C.

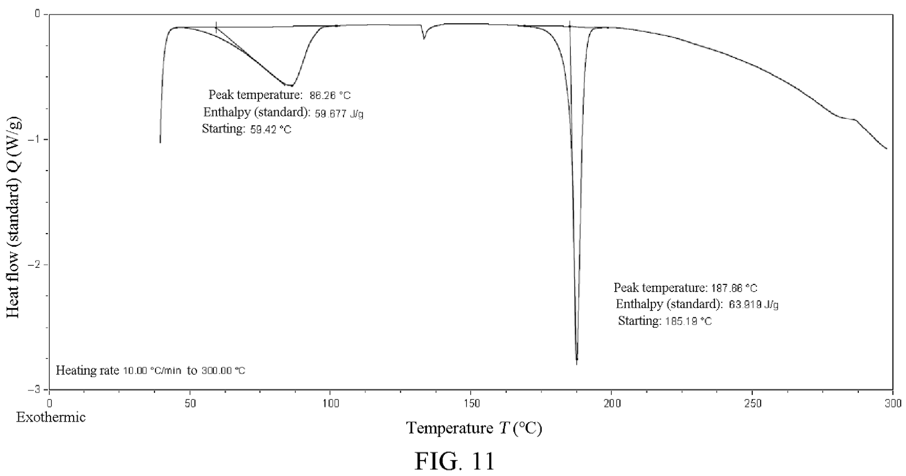

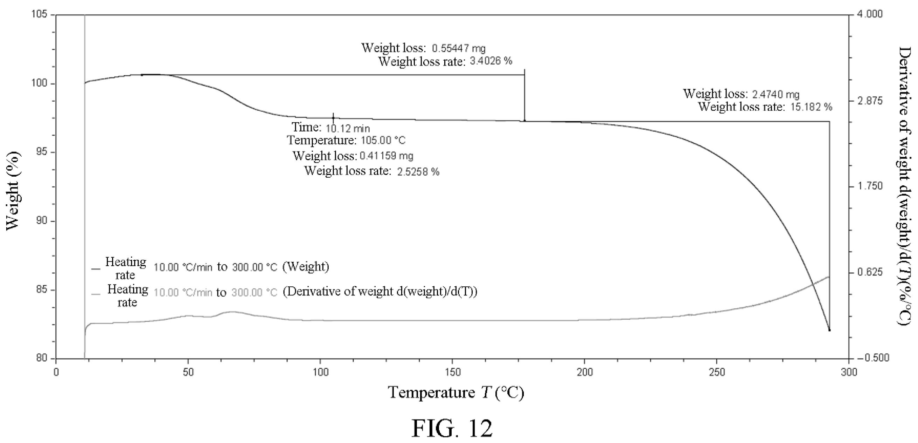

The DSC profile of Crystal Form E of Compound **2** mesylate is shown in FIG. 11, and the TGA curve is shown in FIG. 12.

The present invention provides Crystal Form F of Compound **2** formate, which has an X-ray powder diffraction pattern with characteristic peaks at at least 3, at least 4, at least 5, at least 6, at least 7, at least 8, at least 9 or all the 10 of the following 2θ angles: 5.85° ± 0.20°, 11.35° ± 0.20°, 11.79° ± 0.20°, 12.99° ± 0.20°, 13.53° ± 0.20°, 15.89° ± 0.20°, 17.34° ± 0.20°, 22.37° ± 0.20°, 22.87° ± 0.20°, and 23.76° ± 0.20°.

In some embodiments, Crystal Form F comprises characteristic peaks at at least the following 2θ angles: 11.35° ± 0.20°, 13.53° ± 0.20°, and 23.76° ± 0.20°, and optionally comprises characteristic peak(s) at at least 1, at least 2, at least 3, at least 4, at least 5, at least 6 or all the 7 of the following 2θ angles: 5.85° ± 0.20°, 11.79° ± 0.20°, 12.99° ± 0.20°, 15.89° ± 0.20°, 17.34° ± 0.20°, 22.37° ± 0.20°, and 22.87° ± 0.20°.

In some embodiments, Crystal Form F comprises characteristic peaks at at least the following 2θ angles: 11.35° ± 0.20°, 13.53° ± 0.20°, and 23.76° ± 0.20°, comprises characteristic peak(s) at at least 1, at least 2, at least 3, at least 4 or all the 5 of the following 2θ angles: 11.79° ± 0.20°, 12.99° ± 0.20°, 15.89° ± 0.20°, 17.34° ± 0.20°, and 22.87° ± 0.20°,

and optionally comprises characteristic peak(s) at any one or all the two of the following 2θ angles, 5.85° ± 0.20°, and 22.37° ± 0.20°.

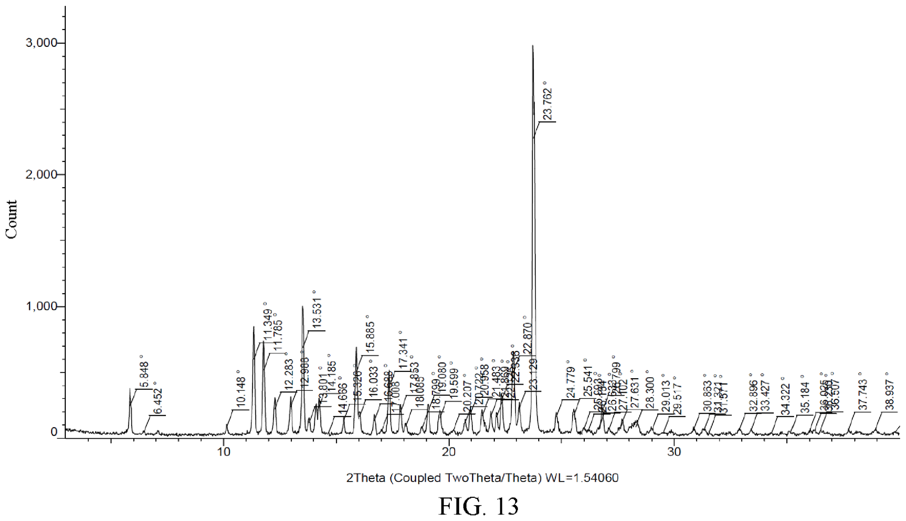

The XRPD pattern of Crystal Form F of Compound **2** mesylate is shown in FIG. 13.

The XRPD pattern analysis data of Crystal Form F of Compound **2** formate is shown in Table 6:

**Table 6: XRPD pattern analysis data of Crystal Form F of Compound 2 formate**

| No. | 2θ (°) | Intensity | Relative intensity (%) | No. | 2θ (°) | Intensity | Relative intensity (%) |
|---|---|---|---|---|---|---|---|
| 1 | 5.848 | 246.72 | 9.6 | 12 | 19.080 | 199.56 | 7.5 |
| 2 | 10.148 | 86.06 | 2.7 | 13 | 19.599 | 151.22 | 5.4 |
| 3 | 11.349 | 599.77 | 25.5 | 14 | 20.958 | 182.84 | 6.8 |
| 4 | 11.785 | 518.36 | 21.7 | 15 | 21.483 | 169.18 | 6.0 |
| 5 | 12.283 | 221.61 | 8.3 | 16 | 22.336 | 283.33 | 10.7 |
| 6 | 12.988 | 236.52 | 9.0 | 17 | 22.870 | 497.91 | 20.2 |
| 7 | 13.531 | 688.64 | 29.3 | 18 | 23.129 | 225.39 | 7.9 |
| 8 | 14.185 | 208.90 | 7.9 | 19 | 23.762 | 2276.64 | 100.0 |
| 9 | 15.885 | 503.26 | 21.2 | 20 | 25.541 | 173.97 | 6.0 |
| 10 | 17.341 | 383.87 | 15.7 | 21 | 26.799 | 197.84 | 7.3 |
| 11 | 17.853 | 198.33 | 7.3 | | | | |

The differential scanning calorimetry profile of Crystal Form F of Compound **2** formate shows an endothermic peak starting at 116.8 ± 3.0°C.

The weight loss starting temperature in the thermogravimetric analysis curve of Crystal Form F of Compound **2** formate is about 107.0°C.

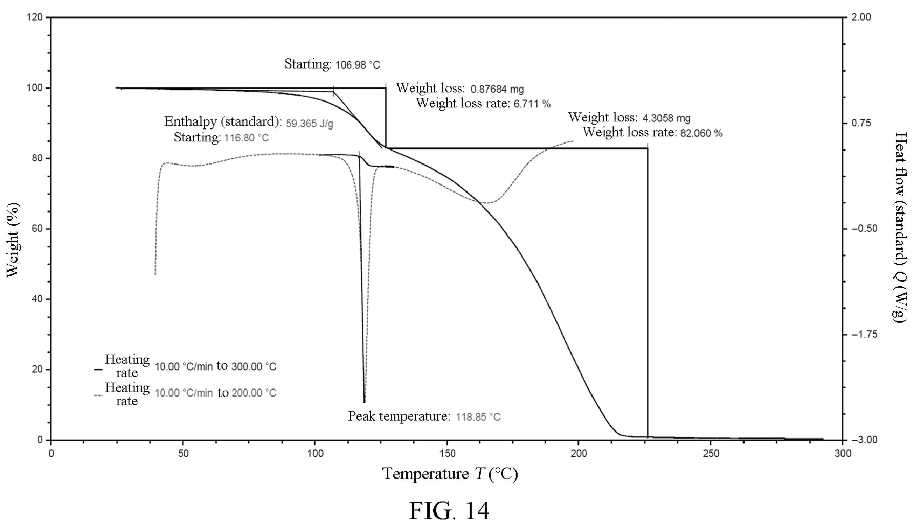

The DSC profile and TGA curve of Crystal Form F of Compound **2** formate is shown in FIG. 14.

The present invention provides Crystal Form L of Compound **2** oxalate, which has an X-ray powder diffraction pattern with characteristic peaks at at least 3, at least 4, at least 5, at least 6, at least 7, at least 8 or all the 9 of the following 2θ angles: 9.43° ± 0.20°, 11.11° ± 0.20°, 15.61° ± 0.20°, 18.98° ± 0.20°, 19.42° ± 0.20°, 23.17° ± 0.20°, 26.30° ± 0.20°, 29.92° ± 0.20°, and 31.69° ± 0.20°.

In some embodiments, Crystal Form L comprises characteristic peaks at at least the following 2θ angles: 9.43° ± 0.20°, 15.61° ± 0.20°, and 26.30° ± 0.20°, and optionally comprises characteristic peak(s) at at least 1, at least 2, at least 3, at least 4, at least 5 or all the 6 of the following 2θ angles: 11.11° ± 0.20°, 18.98° ± 0.20°, 19.42° ± 0.20°, 23.17° ± 0.20°, 29.92° ± 0.20°, and 31.69° ± 0.20°.

In some embodiments, Crystal Form L comprises characteristic peaks at at least the following 2θ angles: 9.43° ± 0.20°, 15.61° ± 0.20°, and 26.30° ± 0.20°, comprises characteristic peak(s) at at least 1, at least 2, at least 3 or all the 4 of the following 2θ angles: 18.98° ± 0.20°, 19.42° ± 0.20°, 23.17° ± 0.20°, 29.92° ± 0.20°, and optionally comprises characteristic peak(s) at any one or all the two of the following 2θ angles, 11.11° ± 0.20°, and 31.69° ± 0.20°.

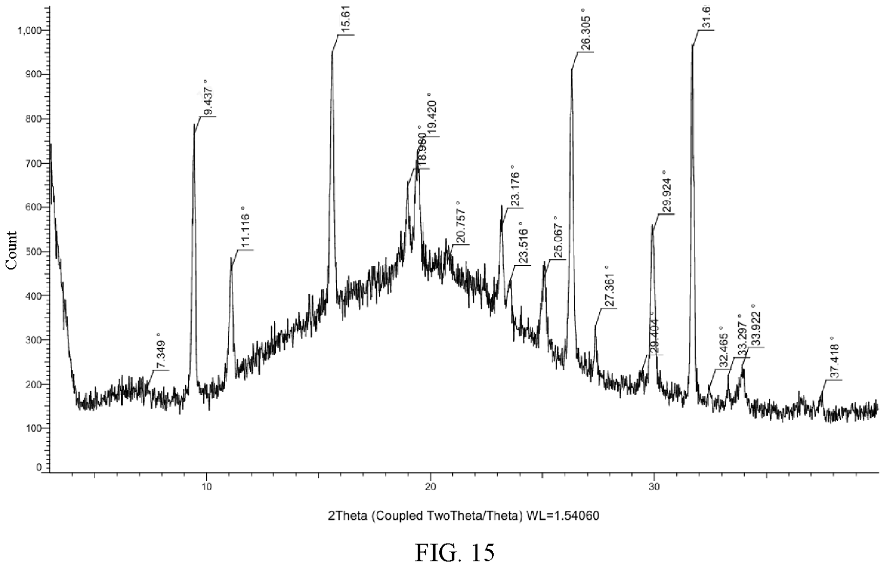

The XRPD pattern of Crystal Form L of Compound **2** oxalate is shown in FIG. 15.

The XRPD pattern analysis data of Crystal Form L of Compound **2** oxalate is shown in Table 7:

**Table 7: XRPD pattern analysis data of Crystal Form L of Compound 2 oxalate**

| No. | 2θ (°) | Intensity | No. | 2θ (°) | Intensity |
|---|---|---|---|---|---|
| 1 | 7.439 | 190.58 | 11 | 26.305 | 909.74 |
| 2 | 9.437 | 763.31 | 12 | 27.361 | 329.98 |
| 3 | 11.116 | 462.50 | 13 | 29.404 | 224.83 |
| 4 | 15.612 | 948.91 | 14 | 29.924 | 543.04 |
| 5 | 18.980 | 646.09 | 15 | 31.696 | 959.19 |
| 6 | 19.420 | 718.63 | 16 | 32.465 | 192.48 |
| 7 | 20.757 | 473.67 | 17 | 33.297 | 219.08 |
| 8 | 23.176 | 556.60 | 18 | 33.922 | 241.78 |
| 9 | 23.516 | 427.95 | 19 | 37.418 | 168.42 |
| 10 | 25.067 | 441.01 | | | |

The present invention provides Crystal Form M of Compound **2** pamoate, which has an X-ray powder diffraction pattern with characteristic peaks at at least 3, at least 4, at least 5 or all the 6 of the following 2θ angles: 5.35° ± 0.20°, 6.49° ± 0.20°, 10.00° ± 0.20°, 10.75° ± 0.20°, 12.51° ± 0.20°, and 21.01° ± 0.20°.

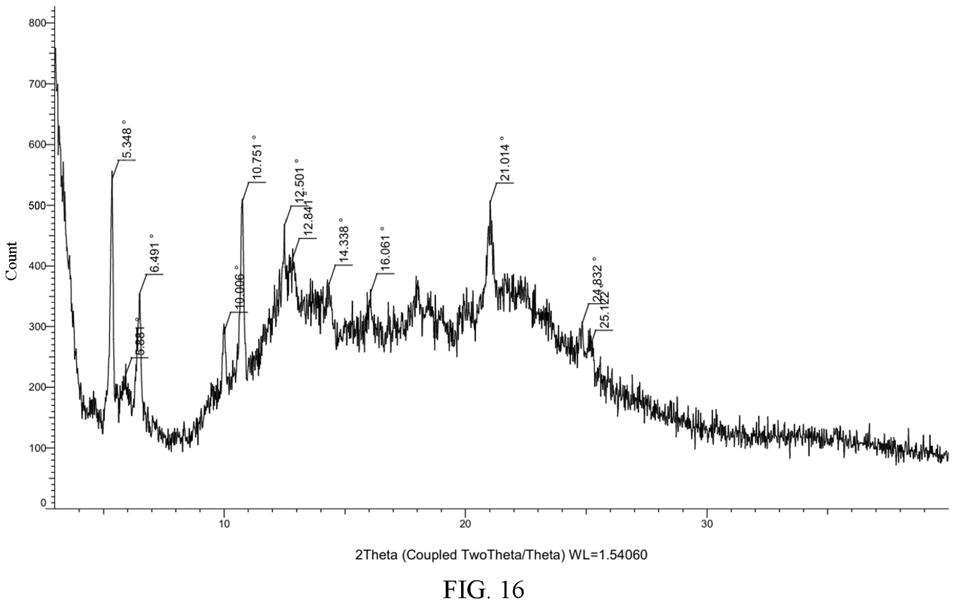

The XRPD pattern of Crystal Form M of Compound **2** pamoate is shown in FIG. 16.

The XRPD pattern analysis data of Crystal Form M of Compound **2** pamoate is shown in Table 8:

**Table 8: XRPD pattern analysis data of Crystal Form M of Compound 2 pamoate**

| No. | 2θ (°) | Intensity | No. | 2θ (°) | Intensity |
|---|---|---|---|---|---|
| 1 | 5.348 | 541.81 | 7 | 12.841 | 413.23 |
| 2 | 5.881 | 216.38 | 8 | 14.338 | 369.26 |
| 3 | 6.491 | 354.00 | 9 | 16.061 | 355.26 |
| 4 | 10.006 | 291.08 | 10 | 21.014 | 504.39 |
| 5 | 10.751 | 505.51 | 11 | 24.832 | 305.11 |
| 6 | 12.501 | 466.43 | 12 | 25.122 | 261.54 |

The present invention provides Crystal Form G of Compound **3** hydrochloride, which has an X-ray powder diffraction pattern with characteristic peaks at at least 3, at least 4, at least 5, at least 6, at least 7, at least 8, at least 9, at least 10, at least 11, at least 12, at least 13, at least 14, at least 15 or all the 16 of the following 2θ angles: 6.18° ± 0.20°, 8.52° ± 0.20°, 10.42° ± 0.20°, 10.96° ± 0.20°, 11.42° ± 0.20°, 12.45° ± 0.20°, 13.57° ± 0.20°, 14.15° ± 0.20°, 17.14° ± 0.20°, 19.42° ± 0.20°, 20.02° ± 0.20°, 20.46° ± 0.20°, 23.41° ± 0.20°, 24.50° ± 0.20°, 24.88° ± 0.20°, and 26.74° ± 0.20°.

In some embodiments, Crystal Form G comprises characteristic peaks at at least the following 2θ angles: 6.18° ± 0.20°, 11.42° ± 0.20°, 12.45° ± 0.20°, 20.02° ± 0.20°, and 23.41° ± 0.20°, and optionally comprises characteristic peak(s) at at least 1, at least 2, at least 3, at least 4, at least 5, at least 6, at least 7, at least 8, at least 9, at least 10 or all the 11 of the following 2θ angles: 8.52° ± 0.20°, 10.42° ± 0.20°, 10.96° ± 0.20°, 13.57° ± 0.20°, 14.15° ± 0.20°, 17.14° ± 0.20°, 19.42° ± 0.20°, 20.46° ± 0.20°, 24.50° ± 0.20°, 24.88° ± 0.20°, and 26.74° ± 0.20°.

In some embodiments, Crystal Form G comprises characteristic peaks at at least the following 2θ angles: 6.18° ± 0.20°, 11.42° ± 0.20°, 12.45° ± 0.20°, 20.02° ± 0.20°, and 23.41° ± 0.20°, comprises characteristic peak(s) at at least 1, at least 2, at least 3, at least 4, at least 5, at least 6 or all the 7 of the following 2θ angles: 10.42° ± 0.20°, 13.57° ± 0.20°, 14.15° ± 0.20°, 17.14° ± 0.20°, 19.42° ± 0.20°, 24.50° ± 0.20°, and 24.88° ± 0.20°, and optionally comprises characteristic peak(s) at at least 1, at least 2, at least 3 or all the 4 of the following 2θ angles: 8.52° ± 0.20°, 10.96° ± 0.20°, 20.46° ± 0.20°, and 26.74° ± 0.20°.

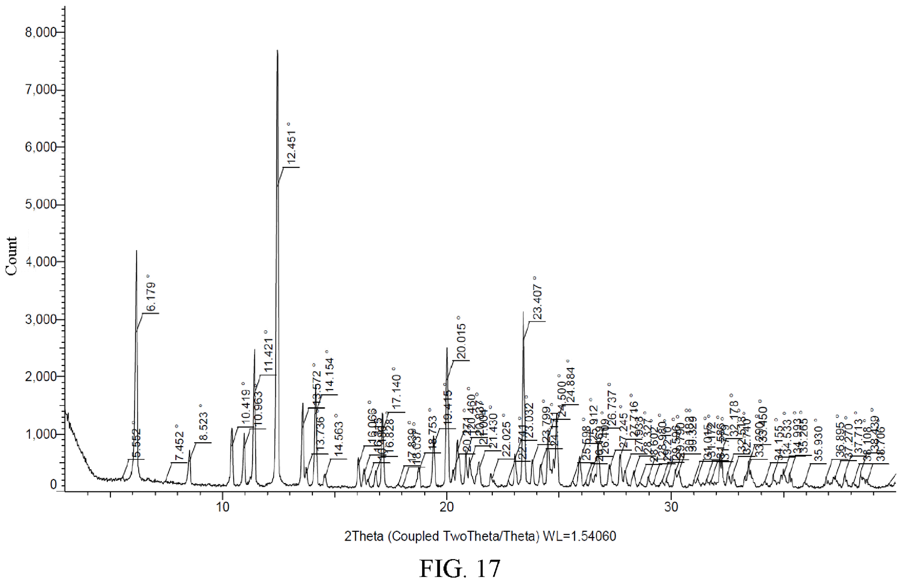

The XRPD pattern of Crystal Form G of Compound **3** hydrochloride is shown in FIG. 17.

The XRPD pattern analysis data of Crystal Form G of Compound **3** hydrochloride is shown in Table 9.

**Table 9: XRPD pattern analysis data of Crystal Form G of Compound 3 hydrochloride**

| No. | 2θ (°) | Intensity | Relative intensity (%) | No. | 2θ (°) | Intensity | Relative intensity (%) |
|---|---|---|---|---|---|---|---|
| 1 | 6.179 | 2781.06 | 49.7 | 14 | 20.015 | 1946.14 | 35.5 |
| 2 | 8.523 | 524.00 | 7.7 | 15 | 20.460 | 708.53 | 11.7 |
| 3 | 10.419 | 794.71 | 13.2 | 16 | 20.837 | 608.59 | 9.7 |
| 4 | 10.963 | 761.80 | 12.6 | 17 | 21.004 | 460.46 | 6.9 |
| 5 | 11.421 | 1702.09 | 30.7 | 18 | 23.032 | 565.88 | 9.0 |
| 6 | 12.451 | 5319.28 | 100.0 | 19 | 23.407 | 2631.29 | 48.5 |
| 7 | 13.572 | 1121.83 | 19.5 | 20 | 23.799 | 488.31 | 7.3 |
| 8 | 13.736 | 324.37 | 4.3 | 21 | 24.500 | 937.59 | 16.0 |
| 9 | 14.154 | 1352.36 | 24.1 | 22 | 24.884 | 1186.65 | 20.9 |
| 10 | 16.066 | 466.64 | 7.5 | 23 | 25.912 | 516.32 | 8.0 |
| 11 | 17.140 | 1055.16 | 18.8 | 24 | 26.737 | 780.72 | 13.1 |
| 12 | 18.753 | 342.24 | 5.1 | 25 | 27.716 | 587.63 | 9.3 |
| 13 | 19.415 | 776.46 | 13.3 | 26 | 32.178 | 592.31 | 9.5 |

The differential scanning calorimetry profile of Crystal Form G of Compound **3** hydrochloride shows an endothermic peak starting at 121.3 ± 3.0°C.

The thermogravimetric analysis curve of Crystal Form G of Compound **3** hydrochloride shows a weight loss of up to 4.49% at 135.0 ± 3.0°C.

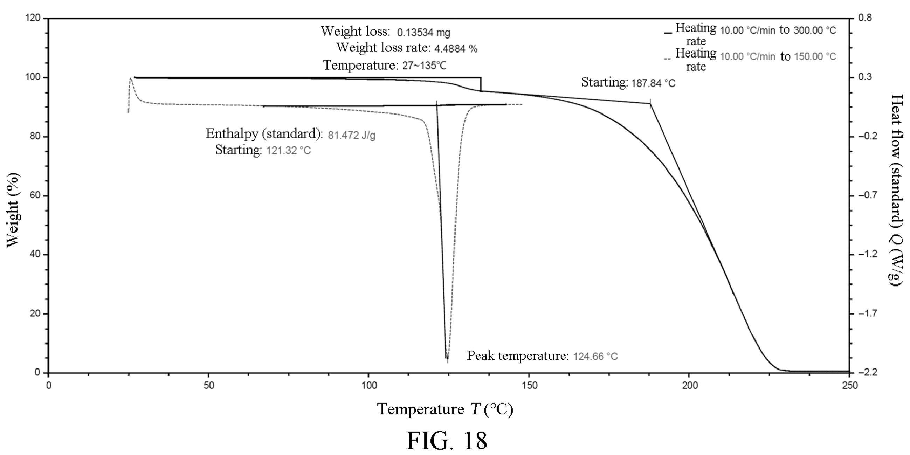

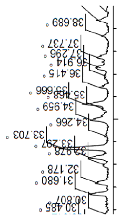

The DSC profile and TGA curve of Crystal Form G of Compound **3** hydrochloride is shown in FIG. 18.

The present invention provides Crystal Form G of Compound **3** hydrochloride, which has an X-ray powder diffraction pattern with characteristic peaks at at least 3, at least 4, at least 5, at least 6, at least 7, at least 8, at least 9, at least 10, at least 11, at least 12, at least 13, at least 14, at least 15 or all the 16 of the following 2θ angles: 6.64° ± 0.20°, 12.64° ± 0.20°, 13.12° ± 0.20°, 13.31° ± 0.20°, 14.16° ± 0.20°, 14.43° ± 0.20°, 16.99° ± 0.20°, 18.37° ± 0.20°, 21.48° ± 0.20°, 21.83° ± 0.20°, 23.06° ± 0.20°, 23.71° ± 0.20°, 26.17° ± 0.20°, 26.61° ± 0.20°, 29.21° ± 0.20°, and 33.70° ± 0.20°.

In some embodiments, Crystal Form H comprises characteristic peaks at at least the following 2θ angles: 6.64° ± 0.20°, 13.12° ± 0.20°, 13.31° ± 0.20°, 21.83° ± 0.20°, and 23.06° ± 0.20°, and optionally comprises characteristic peak(s) at at least 1, at least 2, at least 3, at least 4, at least 5, at least 6, at least 7, at least 8, at least 9, at least 10 or all the 11 of the following 2θ angles: 12.64° ± 0.20°, 14.16° ± 0.20°, 14.43° ± 0.20°, 16.99° ± 0.20°, 18.37° ± 0.20°, 21.48° ± 0.20°, 23.71° ± 0.20°, 26.17° ± 0.20°, 26.61° ± 0.20°, 29.21° ± 0.20°, and 33.70° ± 0.20°.

In some embodiments, Crystal Form H comprises characteristic peaks at at least the following 2θ angles: 6.64° ± 0.20°, 13.12° ± 0.20°, 13.31° ± 0.20°, 21.83° ± 0.20°, and 23.06° ± 0.20°, comprises characteristic peak(s) at at least 1, at least 2, at least 3, at least 4, at least 5, at least 6 or all the 7 of the following 2θ angles: 14.16° ± 0.20°, 18.37° ± 0.20°, 21.48° ± 0.20°, 23.71° ± 0.20°, 26.17° ± 0.20°, 29.21° ± 0.20°, and 33.70° ± 0.20°, and optionally comprises characteristic peak(s) at at least 1, at least 2, at least 3 or all the 4 of the following 2θ angles: 12.64° ± 0.20°, 14.43° ± 0.20°, 16.99° ± 0.20°, and 26.61° ± 0.20°.

The XRPD pattern of Crystal Form H of Compound **3** hydrochloride is shown in FIG. 19.

The XRPD pattern analysis data of Crystal Form H of Compound **3** hydrochloride is shown in Table 10.

**Table 10: XRPD pattern analysis data of Crystal Form H of Compound 3 hydrochloride**

| No. | 2θ (°) | Intensity | Relative intensity (%) | No. | 2θ (°) | Intensity | Relative intensity (%) |
|---|---|---|---|---|---|---|---|
| 1 | 6.637 | 2769.10 | 50.7 | 14 | 23.058 | 3195.29 | 60.3 |
| 2 | 12.642 | 672.53 | 11.1 | 15 | 23.522 | 644.04 | 10.2 |
| 3 | 13.121 | 4021.12 | 76.7 | 16 | 23.708 | 1145.87 | 20.1 |
| 4 | 13.312 | 5207.92 | 100.0 | 17 | 24.006 | 471.82 | 7.0 |
| 5 | 14.160 | 1539.47 | 27.9 | 18 | 26.170 | 1467.98 | 26.6 |
| 6 | 14.427 | 1017.74 | 17.7 | 19 | 26.608 | 776.65 | 13.0 |
| 7 | 15.385 | 506.62 | 8.0 | 20 | 28.608 | 465.72 | 6.7 |
| 8 | 16.992 | 904.53 | 16.0 | 21 | 29.207 | 1166.78 | 20.4 |
| 9 | 17.501 | 437.17 | 6.8 | 22 | 29.500 | 639.79 | 10.0 |
| 10 | 18.369 | 1392.92 | 25.7 | 23 | 31.680 | 409.83 | 5.9 |
| 11 | 21.479 | 1154.32 | 20.5 | 24 | 33.297 | 422.01 | 6.2 |
| 12 | 21.832 | 1833.70 | 33.7 | 25 | 33.703 | 961.39 | 16.8 |
| 13 | 22.727 | 453.25 | 6.4 | 26 | 35.666 | 525.62 | 8.2 |

The differential scanning calorimetry profile of Crystal Form H of Compound **3** hydrochloride shows an endothermic peak starting at 73.6 ± 3.0°C.

The weight loss starting temperature in the thermogravimetric analysis curve of Crystal Form H of Compound **3** hydrochloride is 142.8°C.

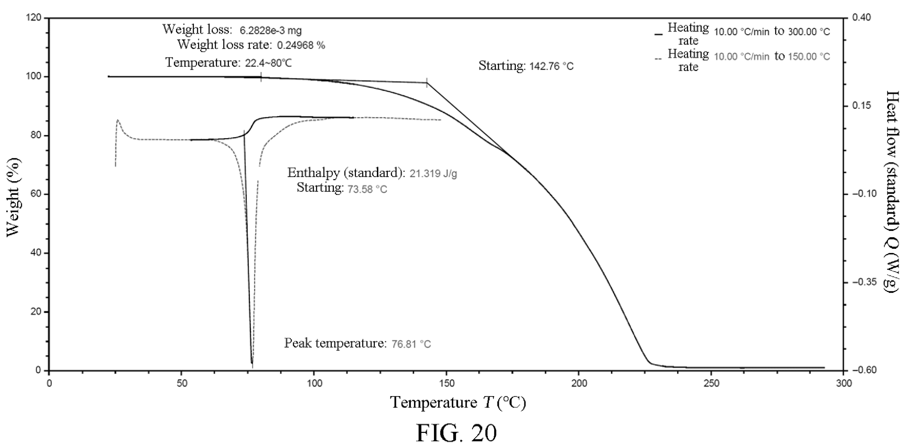

The DSC profile and TGA curve of Crystal Form H of Compound **3** hydrochloride is shown in FIG. 20.

The present invention provides Crystal Form I of Compound **3** phosphate, which has an X-ray powder diffraction pattern with characteristic peaks at at least 3, at least 4, at least 5, at least 6, at least 7, at least 8, at least 9 or all the 10 of the following 2θ angles: 5.94° ± 0.20°, 12.29° ± 0.20°, 13.34° ± 0.20°, 14.94° ± 0.20°, 17.99° ± 0.20°, 19.20° ± 0.20°, 21.54° ± 0.20°, 23.80° ± 0.20°, 24.12° ± 0.20°, and 24.54° ± 0.20°.

In some embodiments, Crystal Form I comprises characteristic peaks at at least the following 2θ angles: 5.94° ± 0.20°, 12.29° ± 0.20°, 13.34° ± 0.20°, and 17.99° ± 0.20°, and optionally comprises characteristic peak(s) at at least 1, at least 2, at least 3, at least 4, at least 5 or all the 6 of the following 2θ angles: 14.94° ± 0.20°, 19.20° ± 0.20°, 21.54° ± 0.20°, 23.80° ± 0.20°, 24.12° ± 0.20°, and 24.54° ± 0.20°.

In some embodiments, Crystal Form I comprises characteristic peaks at at least the following 2θ angles: 5.94° ± 0.20°, 12.29° ± 0.20°, 13.34° ± 0.20°, and 17.99° ± 0.20°, comprises characteristic peak(s) at at least 1, at least 2, at least 3, at least 4, at least 5 or all the 6 of the following 2θ angles: 14.94° ± 0.20°, 19.20° ± 0.20°, 21.54° ± 0.20°, 23.80° ± 0.20°, 24.12° ± 0.20°, and 24.54° ± 0.20°, and optionally comprises a characteristic peak at an 2θ angle of 7.21° ± 0.20°.

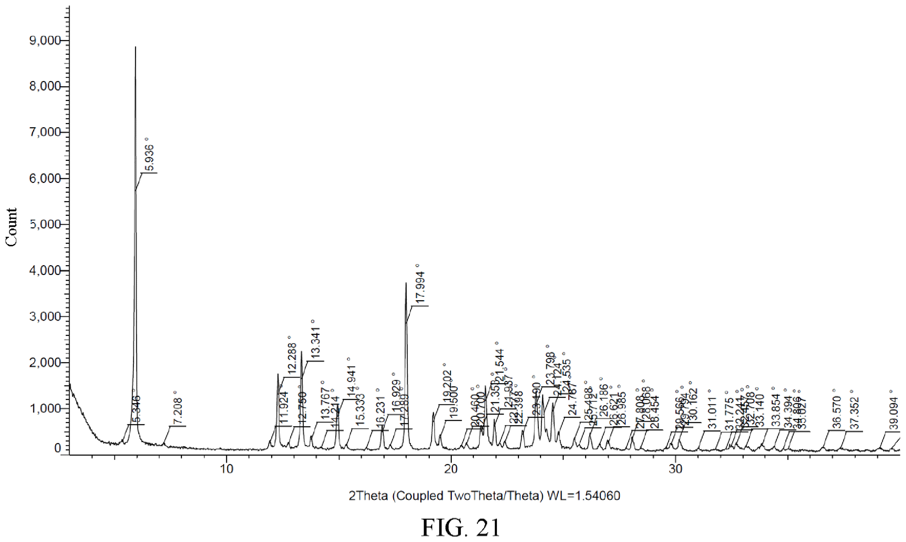

The XRPD pattern of Crystal Form I of Compound **3** phosphate is shown in FIG. 21.

The XRPD pattern analysis data of Crystal Form I of Compound **3** phosphate is shown in Table 11.

**Table 11: XRPD pattern analysis data of Crystal Form I of Compound 3 phosphate**

| No. | 2θ (°) | Intensity | Relative intensity (%) | No. | 2θ (°) | Intensity | Relative intensity (%) |
|---|---|---|---|---|---|---|---|
| 1 | 5.936 | 5732.69 | 100.0 | 10 | 21.544 | 1171.62 | 18.7 |
| 2 | 7.208 | 241.22 | 1.2 | 11 | 21.937 | 613.76 | 8.6 |
| 3 | 12.288 | 1304.77 | 21.5 | 12 | 23.190 | 440.46 | 5.4 |
| 4 | 13.341 | 1649.50 | 27.6 | 13 | 23.798 | 1104.11 | 17.3 |
| 5 | 14.941 | 826.38 | 12.9 | 14 | 24.124 | 867.36 | 13.0 |
| 6 | 16.929 | 499.69 | 7.0 | 15 | 24.535 | 947.57 | 14.5 |
| 7 | 17.994 | 2849.82 | 49.5 | 16 | 24.787 | 438.71 | 5.3 |
| 8 | 19.202 | 709.85 | 10.7 | 17 | 26.186 | 404.59 | 5.2 |
| 9 | 21.353 | 503.59 | 6.7 | | | | |

The differential scanning calorimetry (DSC) profile of Crystal Form I of Compound **3** phosphate shows an endothermic peak starting at 73.3 ± 3.0°C and another endothermic peak starting at 90.4 ± 3.0°C.

The thermogravimetric analysis curve of Crystal Form I of Compound **3** phosphate shows a weight loss of up to 3.18% at 115.0 ± 3.0°C.

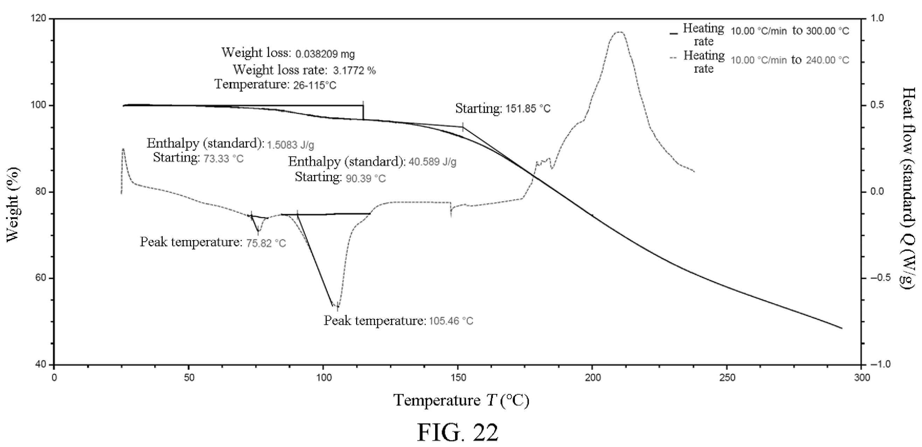

The DSC profile and TGA curve of Crystal Form I of Compound **3** phosphate are shown in FIG. 22.

The present invention provides Crystal Form J of Compound **3** mesylate, which has an X-ray powder diffraction pattern with characteristic peaks at at least 3, at least 4, at least 5, at least 6, at least 7, at least 8, at least 9, at least 10, at least 11, at least 12, at least 13 or all the 14 of the following 2θ angles: 7.87° ± 0.20°, 11.94° ± 0.20°, 12.32° ± 0.20°, 14.75° ± 0.20°, 15.27° ± 0.20°, 15.81° ± 0.20°, 16.75° ± 0.20°, 18.21° ± 0.20°, 18.96° ± 0.20°, 21.89° ± 0.20°, 23.51° ± 0.20°, 24.44° ± 0.20°, 27.96° ± 0.20°, and 30.06° ± 0.20°.

In some embodiments, Crystal Form J comprises characteristic peaks at at least the following 2θ angles: 14.75° ± 0.20°, 15.81° ± 0.20°, and 21.89° ± 0.20°, and optionally comprises characteristic peak(s) at at least 1, at least 2, at least 3, at least 4, at least 5, at least 6, at least 7, at least 8, at least 9, at least 10 or all the 11 of the following 2θ angles: 7.87° ± 0.20°, 11.94° ± 0.20°, 12.32° ± 0.20°, 15.27° ± 0.20°, 16.75° ± 0.20°, 18.21° ± 0.20°, 18.96° ± 0.20°, 23.51° ± 0.20°, 24.44° ± 0.20°, 27.96° ± 0.20°, and 30.06° ± 0.20°.

In some embodiments, Crystal Form J comprises characteristic peaks at at least the following 2θ angles: 14.75° ± 0.20°, 15.81° ± 0.20°, and 21.89° ± 0.20°, comprises characteristic peak(s) at at least 1, at least 2, at least 3, at least 4 or all the 5 of the following 2θ angles: 15.27° ± 0.20°, 16.75° ± 0.20°, 18.96° ± 0.20°, 23.51° ± 0.20°, 24.44° ± 0.20°, and optionally comprises characteristic peak(s) at at least 1, at least 2, at least 3, at least 4, at least 5 or all the 6 of the following 2θ angles: 7.87° ± 0.20°, 11.94° ± 0.20°, 12.32° ± 0.20°, 18.21° ± 0.20°, 27.96° ± 0.20°, and 30.06° ± 0.20°.

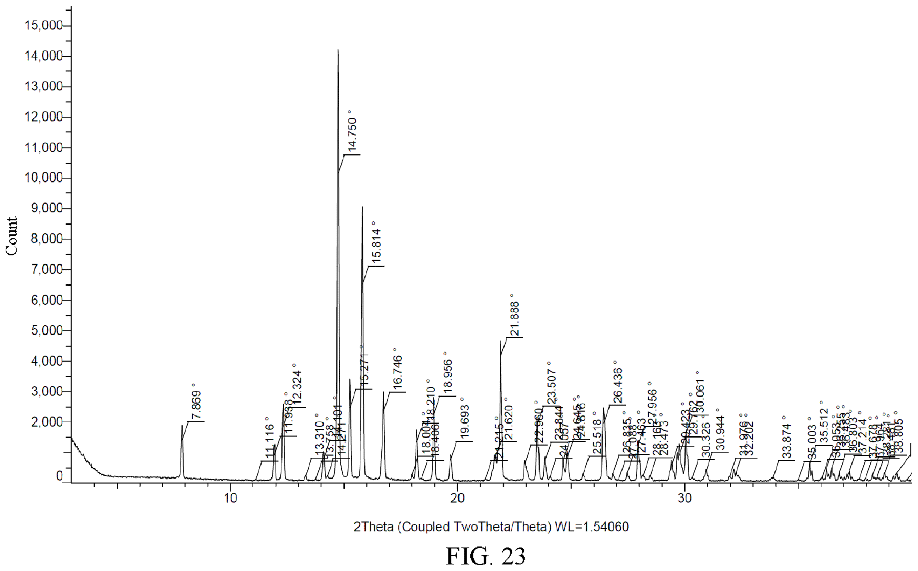

The XRPD pattern of Crystal Form J of Compound **3** mesylate is shown in FIG. 23.

The XRPD pattern analysis data of Crystal Form J of Compound **3** mesylate is shown in Table 12.

**Table 12: XRPD pattern analysis data of Crystal Form J of Compound 3 mesylate**

| No. | 2θ (°) | Intensity | Relative intensity (%) | No. | 2θ (°) | Intensity | Relative intensity (%) |
|---|---|---|---|---|---|---|---|
| 1 | 7.869 | 1419.71 | 12.7 | 13 | 21.888 | 4152.32 | 40.5 |
| 2 | 11.938 | 920.94 | 8.3 | 14 | 22.960 | 646.19 | 5.5 |
| 3 | 12.324 | 1869.34 | 17.7 | 15 | 23.507 | 1921.10 | 18.2 |
| 4 | 14.101 | 785.56 | 6.9 | 16 | 23.844 | 736.41 | 6.4 |
| 5 | 14.750 | 10153.6 | 100.0 | 17 | 24.645 | 744.18 | 6.5 |
| 6 | 15.271 | 2473.21 | 23.4 | 18 | 24.816 | 823.01 | 7.3 |
| 7 | 15.814 | 6512.18 | 63.6 | 19 | 24.436 | 1956.24 | 18.6 |
| 8 | 16.746 | 2370.89 | 22.5 | 20 | 27.956 | 1261.97 | 11.7 |
| 9 | 18.210 | 1343.45 | 12.5 | 21 | 29.762 | 897.24 | 8.1 |
| 10 | 18.956 | 2247.86 | 21.5 | 22 | 30.061 | 1618.34 | 15.3 |
| 11 | 19.693 | 746.52 | 6.7 | 23 | 35.512 | 640.74 | 5.6 |
| 12 | 21.620 | 739.16 | 6.5 | | | | |

The differential scanning calorimetry profile of Crystal Form J of Compound **3** mesylate shows an endothermic peak starting at 247.2 ± 3.0°C and
The weight loss starting temperature in the thermogravimetric analysis curve of Crystal Form J of Compound **3** mesylate is about 247.4°C.

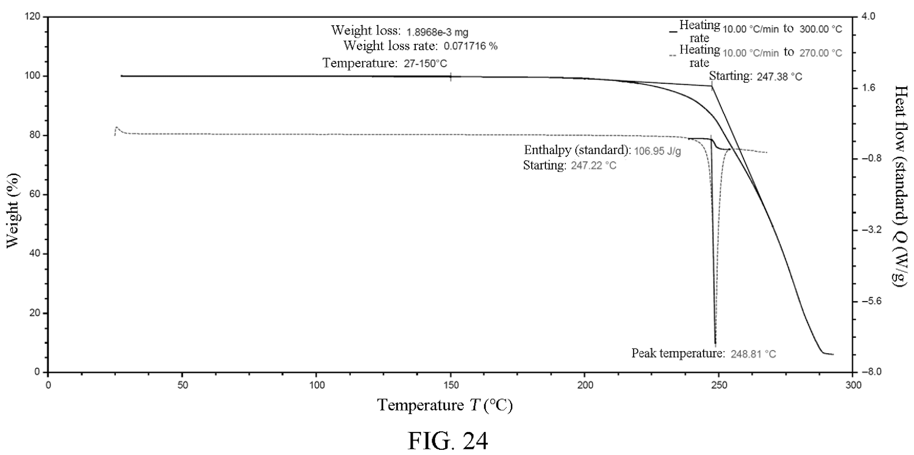

The DSC profile and TGA curve of Crystal Form J of Compound **3** mesylate are shown in FIG. 24.

The present invention provides Crystal Form K of Compound **3** formate, which has an X-ray powder diffraction pattern with characteristic peaks at at least 3, at least 4, at least 5, at least 6, at least 7, at least 8 or all the 9 of the following 2θ angles: 10.76° ± 0.20°, 11.92° ± 0.20°, 13.91° ± 0.20°, 14.38° ± 0.20°, 15.15° ± 0.20°, 18.41° ± 0.20°, 24.04° ± 0.20°, 24.90° ± 0.20°, and 30.61° ± 0.20°.

In some embodiments, Crystal Form K comprises characteristic peaks at at least the following 2θ angles: 11.92° ± 0.20°, 15.15° ± 0.20°, and 24.04° ± 0.20°, and optionally comprises characteristic peak(s) at at least 1, at least 2, at least 3, at least 4, at least 5 or all the 6 of the following 2θ angles: 10.76° ± 0.20°, 13.91° ± 0.20°, 14.38° ± 0.20°, 18.41° ± 0.20°, 24.90° ± 0.20°, and 30.61° ± 0.20°.

In some embodiments, Crystal Form K comprises characteristic peaks at at least the following 2θ angles: 11.92° ± 0.20°, 15.15° ± 0.20°, and 24.04° ± 0.20°, comprises characteristic peak(s) at at least 1, at least 2, at least 3 or all the 4 of the following 2θ angles: 13.91° ± 0.20°, 14.38° ± 0.20°, 18.41° ± 0.20°, and 30.61° ± 0.20°, and optionally comprises characteristic peak(s) at any 1 or all the 2 of the following 2θ angles, 10.76° ± 0.20°, and 24.90° ± 0.20°.

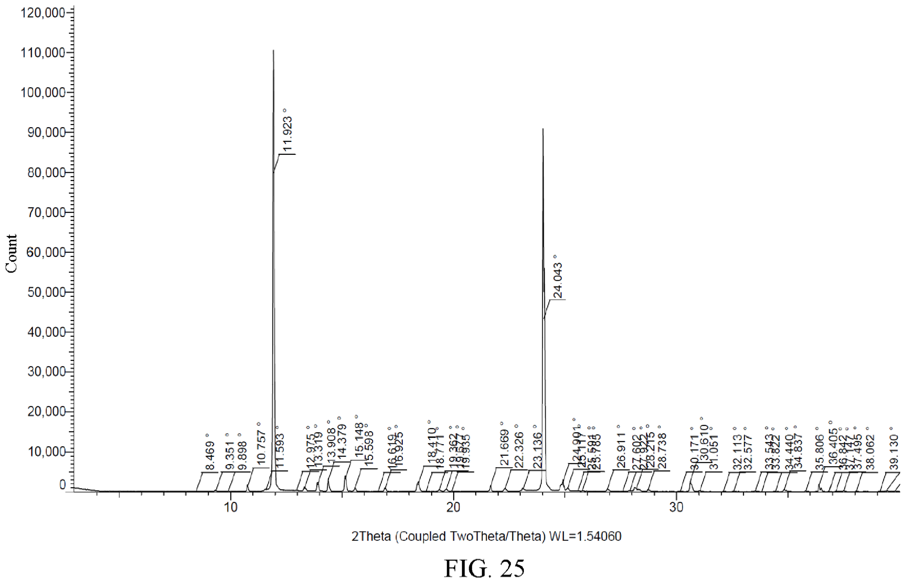

The XRPD pattern of Crystal Form K of Compound **3** formate is shown in FIG. 25.

The XRPD pattern analysis data of Crystal Form K of Compound **3** formate is shown in Table 13:

**Table 13: XRPD pattern analysis data of Crystal Form K of Compound 3 formate**

| No. | 2θ (°) | Intensity | Relative intensity (%) | No. | 2θ (°) | Intensity | Relative intensity (%) |
|---|---|---|---|---|---|---|---|
| 1 | 10.757 | 1270.74 | 1.4 | 6 | 18.410 | 1750.0 | 2.0 |
| 2 | 11.923 | 79911.1 | 100.0 | 7 | 24.043 | 43341.4 | 54.0 |
| 3 | 13.908 | 1737.99 | 1.9 | 8 | 24.901 | 2399.34 | 2.7 |
| 4 | 14.379 | 2756.11 | 3.2 | 9 | 30.610 | 2641.37 | 3.2 |
| 5 | 15.148 | 3208.22 | 3.8 | 10 | 36.405 | 1627.42 | 1.9 |

The differential scanning calorimetry profile of Crystal Form K of Compound **3** formate shows an endothermic peak starting at 195.4 ± 3.0°C.

The weight loss starting temperature in the thermogravimetric analysis curve of Crystal Form K of Compound **3** formate is about 200.5°C.

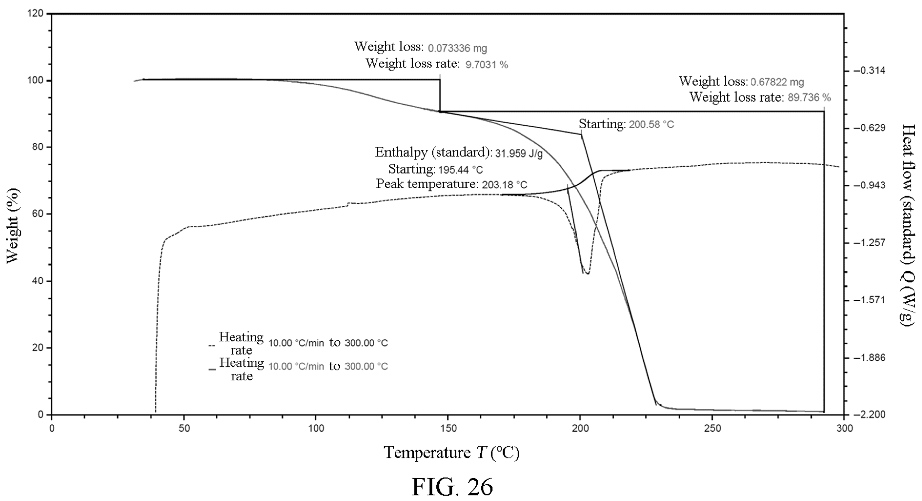

The DSC profile and TGA curve of Crystal Form K of Compound **3** formate is shown in FIG. 26.

The present invention provides Crystal Form N of Compound **3** oxalate, which has an X-ray powder diffraction pattern with characteristic peaks at at least 3, at least 4, at least 5, at least 6, at least 7, at least 8, at least 9, at least 10, at least 11, at least 12 or all the 13 of the following 2θ angles: 6.20° ± 0.20°, 7.49° ± 0.20°, 10.45° ± 0.20°, 11.45° ± 0.20°, 12.25° ± 0.20°, 12.47° ± 0.20°, 14.00° ± 0.20°, 15.06° ± 0.20°, 16.36° ± 0.20°, 23.43° ± 0.20°, 23.78° ± 0.20°, 24.07° ± 0.20°, and 24.95° ± 0.20°.

In some embodiments, Crystal Form N comprises characteristic peaks at at least the following 2θ angles: 6.20° ± 0.20°, 12.47° ± 0.20°, and 16.36° ± 0.20°, and optionally comprises characteristic peak(s) at at least 1, at least 2, at least 3, at least 4, at least 5, at least 6, at least 7, at least 8 or all the 9 of the following 2θ angles: 7.49° ± 0.20°, 11.45° ± 0.20°, 12.25° ± 0.20°, 14.00° ± 0.20°, 15.06° ± 0.20°, 23.43° ± 0.20°, 23.78° ± 0.20°, 24.07° ± 0.20°, and 24.95° ± 0.20°.

In some embodiments, Crystal Form N comprises characteristic peaks at at least the following 2θ angles: 6.20° ± 0.20°, 12.47° ± 0.20°, and 16.36° ± 0.20°, comprises characteristic peak(s) at at least 1, at least 2, at least 3, at least 4, at least 5, at least 6 or all the 7 of the following 2θ angles: 7.49° ± 0.20°, 11.45° ± 0.20°, 12.25° ± 0.20°, 14.00° ± 0.20°, 15.06° ± 0.20°, 23.43° ± 0.20°, 23.78° ± 0.20°, and optionally comprises characteristic peak(s) at any 1 or all the 2 of the following 2θ angles, 24.07° ± 0.20°, and 24.95° ± 0.20°.

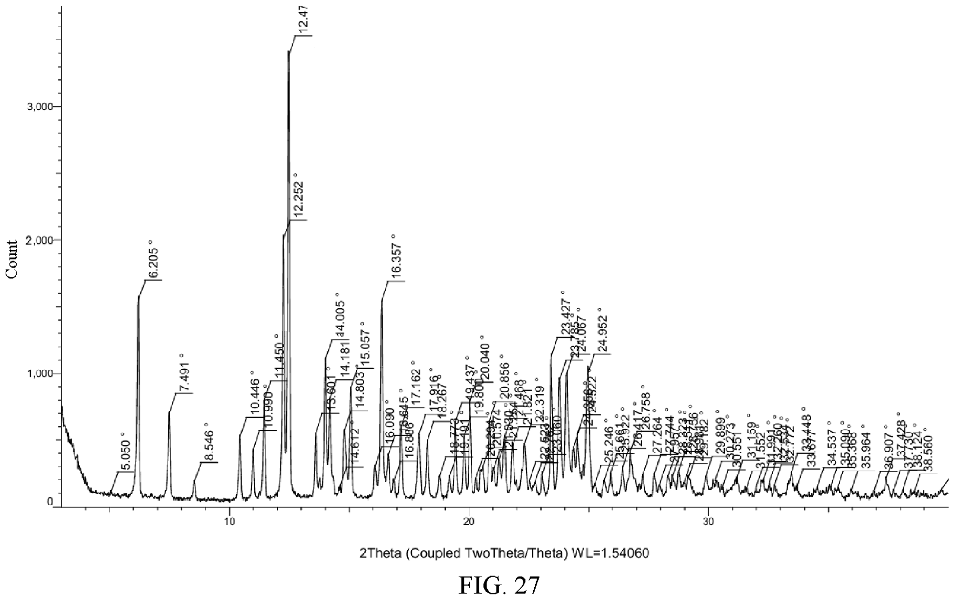

The XRPD pattern of Crystal Form N of Compound **3** oxalate is shown in FIG. 27.

The XRPD pattern analysis data of Crystal Form N of Compound **3** oxalate is shown in Table 14:

**Table 14: XRPD pattern analysis data of Crystal Form N of Compound 3 oxalate**

| No. | 2θ (°) | Intensity | No. | 2θ (°) | Intensity |
|---|---|---|---|---|---|
| 1 | 6.205 | 1151.78 | 17 | 17.916 | 545.66 |
| 2 | 7.491 | 701.99 | 18 | 18.267 | 493.27 |
| 3 | 8.546 | 185.27 | 19 | 19.437 | 634.05 |
| 4 | 10.446 | 522.65 | 20 | 19.800 | 526.82 |
| 5 | 10.990 | 424.35 | 21 | 20.040 | 788.94 |
| 6 | 11.450 | 797.52 | 22 | 20.856 | 644.50 |
| 7 | 12.252 | 2003.68 | 23 | 21.468 | 515.77 |
| 8 | 12.470 | 3382.23 | 24 | 21.821 | 454.09 |
| 9 | 13.601 | 553.92 | 25 | 22.319 | 471.96 |
| 10 | 14.005 | 1106.99 | 26 | 23.427 | 1125.75 |
| 11 | 14.181 | 806.53 | 27 | 23.785 | 954.34 |
| 12 | 14.803 | 572.94 | 28 | 24.067 | 997.99 |
| 13 | 15.057 | 892.48 | 29 | 24.356 | 445.56 |
| 14 | 16.357 | 1542.77 | 30 | 24.522 | 541.07 |
| 15 | 16.645 | 386.99 | 31 | 24.952 | 996.94 |
| 16 | 17.162 | 597.49 | 32 | 26.758 | 418.79 |

The present invention provides Crystal Form O of Compound **3** pamoate, which has an X-ray powder diffraction pattern with characteristic peaks at at least 3, at least 4, at least 5, at least 6, at least 7, at least 8, at least 9, at least 10, at least 11, at least 12, at least 13 or all the 14 of the following 2θ angles: 6.18° ± 0.20°, 9.41° ± 0.20°, 10.40° ± 0.20°, 11.15° ± 0.20°, 12.46° ± 0.20°, 15.59° ± 0.20°, 18.96° ± 0.20°, 19.34° ± 0.20°, 23.12° ± 0.20°, 23.43° ± 0.20°, 25.00° ± 0.20°, 26.25° ± 0.20°, 29.90° ± 0.20°, and 31.65° ± 0.20°.

In some embodiments, Crystal Form O comprises characteristic peaks at at least the following 2θ angles: 9.41° ± 0.20°, 11.10° ± 0.20°, 15.59° ± 0.20°, and 26.25° ± 0.20°, and optionally comprises characteristic peak(s) at at least 1, at least 2, at least 3, at least 4, at least 5, at least 6, at least 7, at least 8, at least 9 or all the 10 of the following 2θ angles: 6.18° ± 0.20°, 10.40° ± 0.20°, 12.46° ± 0.20°, 18.96° ± 0.20°, 19.34° ± 0.20°, 23.12° ± 0.20°, 23.43° ± 0.20°, 25.00° ± 0.20°, 29.90° ± 0.20°, and 31.46° ± 0.20°.

In some embodiments, Crystal Form O comprises characteristic peaks at at least the following 2θ angles: 9.41° ± 0.20°, 11.10° ± 0.20°, 15.59° ± 0.20°, and 26.25° ± 0.20°, and optionally comprises characteristic peak(s) at at least 1, at least 2, at least 3, at least 4, at least 5, at least 6, at least 7 or all the 8 of the following 2θ angles: 10.40° ± 0.20°, 12.46° ± 0.20°, 18.96° ± 0.20°, 19.34° ± 0.20°, 23.12° ± 0.20°, 23.43° ± 0.20°, 29.90° ± 0.20°, and 31.46° ± 0.20°, and optionally comprises characteristic peak(s) at any 1 or all the 2 of the following 2θ angles, 6.18° ± 0.20°, and 25.00° ± 0.20°.

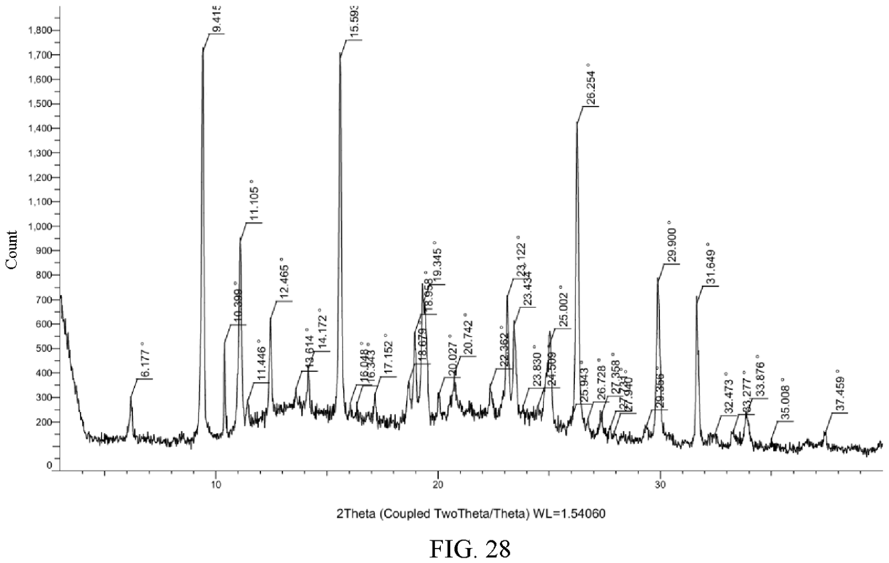

The XRPD pattern of Crystal Form O of Compound **3** pamoate is shown in FIG. 28.

The XRPD pattern analysis data of Crystal Form O of Compound **3** hydrochloride is shown in Table 15.

**Table 15: XRPD pattern analysis data of Crystal Form O of Compound 3 pamoate**

| No. | 2θ (°) | Intensity | No. | 2θ (°) | Intensity |
|---|---|---|---|---|---|
| 1 | 6.177 | 300.70 | 9 | 19.345 | 687.32 |
| 2 | 9.415 | 1710.90 | 10 | 23.122 | 710.25 |
| 3 | 10.399 | 522.64 | 11 | 23.434 | 594.19 |
| 4 | 11.105 | 939.08 | 12 | 25.002 | 507.82 |
| 5 | 12.465 | 619.39 | 13 | 26.254 | 1415.25 |
| 6 | 14.173 | 414.00 | 14 | 29.900 | 770.56 |
| 7 | 15.593 | 1686.18 | 15 | 31.649 | 681.19 |
| 8 | 18.958 | 565.17 | | | |

The X-ray powder diffractometry (XRPD) in the present invention is shown in Table A.

**Table A**

| | |
|---|---|
| Model | Bruker D8 Advance |
| No. | XRPD-02 |
| Technical indexes | The wavelength of copper target is 1.54Å Kα radiation (40 kV, 40 mA) |
| Acquisition software | DIFFRAC.MEA.CENTER |
| Calibration substance | Corundum (Al₂O₃) |
| Analysis software | DIFFRAC.EVA V5.2 |
| Detection angle | 3-40° 2θ |
| Step length | 0.02° 2θ |
| speed | 0.1 s/step |

The differential scanning calorimetry (DSC) and thermal gravimetric analysis (TGA) in the present invention are shown in Table B:

**Table B**

| | | | |
|---|---|---|---|
| Instrument | Model | TGA Discovery 55 | DSC 250 |
| | No. | TGA-01 | DSC-01 |
| | Sample pan | Platinum pan | Aluminum pan |
| Parameter | Sample amount detected | 1 mg-10 mg | 0.5 mg-5 mg |
| | Protection gas | Nitrogen | Nitrogen |
| | Gas flow rate | 60 mL/min | 200 mL/min |
| | Common detection method | Heating the sample to 300°C or to a weight loss of 20% at a heating rate of 10.0°C/min | Heating the sample to 300°C or to a weight loss of 20% at a heating rate of 10.0°C/min |

### V. Uses, treatments, and pharmaceutical compositions

The compound of the present invention is a NMDA receptor antagonist. Therefore, the compound of the present invention, or the pharmaceutically acceptable salt, enantiomer, diastereomer, tautomer, solvate, isotopologue, polymorph, prodrug, or metabolite thereof can be used to regulate the activity of NMDA receptors,

The present invention further provides a pharmaceutical composition, which contains a pharmaceutically acceptable carrier and a therapeutically amount of the compound of the present invention or the pharmaceutically acceptable salt, enantiomer, diastereomer, tautomer, solvate, isotopologue, polymorph, prodrug, or metabolite thereof, and a pharmaceutically acceptable carrier or excipient. The pharmaceutical composition of the present invention can be used to regulate the activity of NMDA receptors, to treat and/or prevent NMDA receptor-mediated diseases. The pharmaceutically acceptable salt of the compound of the present invention is as described in any embodiment herein. Herein, the activity of NMDA receptors can be regulated to treat and/or prevent NMDA receptor-mediated diseases. Therefore, in some embodiments, the present invention provides use of the compound of the present invention or the pharmaceutically acceptable salt, enantiomer, diastereomer, tautomer, solvate, isotopologue, polymorph, prodrug, or metabolite thereof, or the pharmaceutical composition of the present invention in the manufacture of a medicament for treating or preventing NMDA receptor-mediated diseases. The pharmaceutically acceptable salt of the compound of the present invention is as described in any embodiment herein. In some other embodiments, the present invention provides the compound of the present invention or the pharmaceutically acceptable salt, enantiomer, diastereomer, tautomer, solvate, isotopologue, polymorph, prodrug, or metabolite thereof for use in the treatment or prevention of NMDA receptor-mediated diseases.

In some embodiments, the present invention provides a method for treating or preventing a NMDA receptor-mediated disease in a subject. The method includes administering, to the subject, a therapeutically effective amount or prophylactically effective amount of the compound of the present invention or the pharmaceutically acceptable salt, enantiomer, diastereomer, tautomer, solvate, isotopologue, polymorph, prodrug, or metabolite thereof, or the pharmaceutical composition containing a therapeutically effective amount or prophylactically effective amount of the compound of the present invention or the pharmaceutically acceptable salt, enantiomer, diastereomer, tautomer, solvate, isotopologue, polymorph, prodrug or metabolite, through oral route, duodenal route, parenteral injection (including intrapulmonary, intranasal, intrathecal, intravenous, subcutaneous, intraperitoneal, intramuscular, intra-arterial injection or infusion), local administration and rectal administration. The administration techniques that can be used for the compounds and methods described herein are known to those skilled in the art, for example, those discussed in Goodman and Gilman, The Pharmacological Basis of Therapeutics, current ed.; Pergamon; and Remington's, Pharmaceutical Sciences (current edition), Mack Publishing Co., Easton, Pa. In a preferred embodiment, the compound of the present invention, or the pharmaceutically acceptable salt, enantiomer, diastereomer, tautomer, solvate, isotopologue, polymorph, prodrug or metabolite, or pharmaceutical composition thereof is orally administered.

A suitable pharmaceutical composition can be prepared by methods known in the art according to the administration mode and dosage determined by skilled practitioners. For parenteral administration, the compound can be dissolved in sterile water, physiological saline, or a pharmaceutically acceptable vehicle (such as those for vitamin K) for administering a water-insoluble compound. For enteral administration, the compound can be administered in the form of tablets or capsules or dissolved in a liquid form. The tablets or capsules may be coated with an enteric coating or in the form of sustained-release preparations. A variety of suitable preparations are known, including polymer or protein microparticles encapsulating the compound to be released, ointments, pastes, gels, hydrogels or solutions, which allows the compound to be topically or locally administered. Sustained-release patches or implants can be used to provide release over an extended period of time. Preparations for parenteral administration may, for example, contain the excipient polyalkylene glycol (such as polyethylene glycol), plant-derived oils, or hydrogenated naphthalene. Biocompatible and biodegradable lactide polymers, lactide/glycolide copolymers or polyoxyethylene-polyoxypropylene copolymers can be used to control the release of the compound. Other potentially useful parenteral delivery systems for modulating the compound include ethylene-vinyl acetate copolymer particles, osmotic pumps, implantable infusion systems and liposomes. Preparations for inhalation may contain an excipient such as lactose, may be aqueous solutions containing, for example, polyoxyethylene-9-lauryl ether, a glycocholate and a deoxycholate, or oily solutions for administration in the form of nasal drops, or may be in the form of gels.

It should be noted that the dose may vary with the precise imaging scheme. For any particular subject, the specific dosage regimen can be adjusted over time according to the individual needs and the professional judgment of the person administering or supervising the administration of the composition. The dosage range described herein is merely exemplary and does not limit the dosage range that medical practitioners can choose. The amount of the active compound in the composition may vary according to factors such as the disease state, age, sex and weight of the subject. The dosage regimen can be adjusted to provide an optimum imaging results. For example, a single bolus can be administered, several divided doses can be administered over time, or the dose can be reduced or increased proportionally as indicated by the imaging results. It may be advantageous to prepare the parenteral composition in a unit dosage form that is easy to administer and achieves dose uniformity.

Herein, the diseases related to NMDA receptor activity include, but are not limited to, cerebral ischemia, traumatic brain injury, infarction, stroke, Alzheimer's disease, Parkinson's disease, Huntington's diseases, depression, anxiety, bipolar disorder, schizophrenia, autism, epilepsy, anti-NMDA receptor encephalitis, neuropathic pain, and other nervous system events or neurodegeneration caused by the activation of NMDA receptors. In some embodiments, the neuropathic pain includes peripheral diabetic neuropathy, post-herpetic neuralgia, complex local pain syndrome, peripheral neuropathy, chemotherapy-induced neuropathic pain, cancer-related neuropathic pain, neuropathic low back pain, HIV neuropathic pain, trigeminal neuralgia, and central post-stroke pain. Especially, the diseases related to NMDA receptor activity or NMDA receptor-mediated diseases described herein include depression, schizophrenia or epilepsy. The present invention also finds that the regulation of NMDAR activity has the effect of increasing the appetite, and the NMDAR target is also expected to be used in the treatment of anorexia caused by various reasons. It is also found in the present invention that the regulation of NMDAR activity is also expected to be used in the treatment of diseases related to sleep disorder, such as insomnia, sleep apnea syndrome, atypical circadian rhythm sleep-wake disorder or Down syndrome; as well as in the treatment of pruritus and other diseases.

In some embodiments, the compound provided in the present invention or the pharmaceutically acceptable salt, enantiomer, diastereomer, tautomer, solvate, isotopologue, polymorph, prodrug or metabolite thereof, or the composition thereof can also be used for anesthesia and analgesia of subjects. Therefore, in some embodiments, the present invention provides an anesthetic or analgesic, which contains the compound of the present invention or the pharmaceutically acceptable salt, enantiomer, diastereomer, tautomer, solvate, isotopologue, polymorph, prodrug or metabolite thereof as an active ingredient with anesthetic or analgesic effects, and a safe carrier or excipient suitable for administration to human or animal bodies. In some other embodiments, the present invention further provides use of the compound of the present invention, or the pharmaceutically acceptable salt, enantiomer, diastereomer, tautomer, solvate, isotopologue, polymorph, prodrug or metabolite thereof in the preparation of anesthetics or analgesics. In some other embodiments, the present invention further provides an anesthetic or analgesic method. The method includes administering, to an individual or subject in need thereof, a therapeutically effective amount of the compound of the present invention or the pharmaceutically acceptable salt, enantiomer, diastereomer, tautomer, solvate, isotopologue, polymorph, prodrug or metabolite thereof, or the pharmaceutical composition thereof.

The following abbreviations are used in the present invention: Pd₂(dba)₃: tris(dibenzylideneacetone) dipalladium, SFC: supercritical fluid chromatography, prep-HPLC: preparative high-performance liquid chromatography, Pd(dppf)Cl₂: (1,1'-bis(diphenylphosphino)ferrocene) palladium dichloride, Xantphos: 4,5-bis(diphenylphosphino)-9,9-dimethylxanthene, (DHQ)₂ PHAL: hydroquinine 1,4-phthalazinediyl diether, (DHQD)₂PHAL: hydroquinidine 1,4-phthalazinediyl diether, PCC: pyridinium chlorochromate, NH₄Cl: ammonium chloride, THF: tetrahydrofuran, K₂CO₃: potassium carbonate, TfOH: trifluoromethanesulfonic acid, EA: ethyl acetate, PE: petroleum ether, DCM: dichloromethane Na₂SO₄: sodium sulfate.

The present invention is further described below in conjunction with specific examples. It is to be understood that these examples are merely illustrative of the present invention and are not intended to limit the scope of the present invention. For experimental methods where no specific conditions are given in the following examples, usually carried out according to conventional conditions or conditions recommended by the manufacturer are followed. Unless otherwise specified, the percentages and parts are percentages and parts by weight.

### Example 1: Preparation of Compound 1

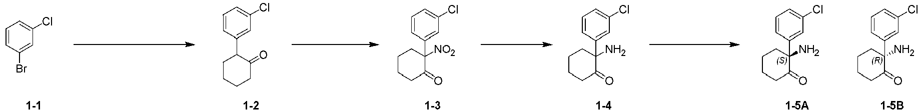

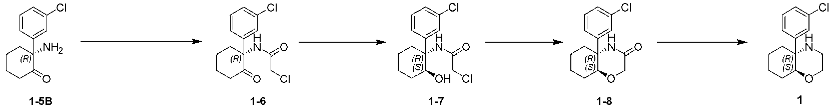

### Step I: Synthesis of Compound 1-2

At 25°C, cyclohexanone (40.0 g, 418 mmol), 3-bromo-1-chlorobenzene (40.0 g, 209 mmol, **1-1**), and cesium carbonate (149.8 g,459.6 mmol) were dissolved in dioxane (400 mL). Pd₂(dba)₃ (1.9 g, 2.1 mmol) and Xantphos (2.4 g, 4.2 mmol) were added to the reaction solution. The system was purged with nitrogen. The reaction solution was heated to 100°C and reacted for 20 hrs. After cooling and filtration, the filtrate was concentrated, and dried. The residue was separated and purified by column chromatography on silica gel, to obtain Compound **1-2.** LCMS: 209.0 (M+H)⁺.

### Step II: Synthesis of Compound 1-3

At 25°C, Compound **1-2** (20.0 g, 95.4 mmol), copper acetate (9.6 g, 48 mmol), and ceric ammonium nitrate (131.4 g, 239.6 mmol) were added to 1,2-dichloroethane (400 mL). The system was purged with nitrogen. The reaction solution was reacted at 80°C for 16 hrs. After filtration, the filtrate was concentrated. The residue was separated and purified by column chromatography on silica gel, to obtain Compound **1-3**. LCMS: 207.0 (M-NO₂)⁺.

### Step III: Synthesis of Compound 1-4

At 0 °C, Compound **1-3** (9.0 g, 35 mmol) was added to acetic acid (100 mL), and then a zinc powder (11.6 g, 177 mmol) was added batchwise to the mixed solution. The reaction solution was heated to 80°C, and stirred for 16 hrs. The reaction solution was concentrated, added with H₂O (100 mL), and adjusted to pH 12 with a sodium hydroxide aqueous solution (2 M). The reaction solution was extracted with EA (100 mL x 2). The organic phases were combined, washed with saturated brine (50 mL), dried over anhydrous Na₂SO₄, and filtered. The filtrate was concentrated under reduced pressure. The residue was separated and purified by column chromatography on silica gel, to obtain Compound **1-4**. LCMS: 224.2 (M+H)⁺.

### Step IV: Synthesis of Compound 1-5A and Compound 1-5B

Compound **1-4** (4 g, 17.88 mmol) was separated by SFC, to obtain Compound **1-5B** (1.70 g, retention time Rt= 2.190 min). LCMS: *m*/*z*= 224.2 (M+H)⁺ and Compound **1-5A** (1.75 g, retention time Rt= 4.340 min). LCMS: *m*/*z*= 224.2 (M+H)⁺. (SFC conditions: column: ChiralPak IE, 250 x 40mm I.D., 10µm; mobile phase: A CO₂ and B 50% ethanol (0.1% aqueous ammonia) + 50% acetonitrile (0.1%NH₃·H3O); gradient: B 40%, flow rate: 120 mL /min).

### Step V: Synthesis of Compound 1-6

At 0 °C, Compound **1-5B** (1.0 g, 4.5 mmol) and triethyl amine (0.90 g, 8.9 mmol) were dissolved in DCM (20 mL), and chloroacetyl chloride (0.50 g, 4.4 mmol) was slowly added dropwise to the above solution. The reaction was continued at 0°C for 2 hrs. The reaction was quenched with water (10 mL), and extracted with EA (50 mL x 2). The organic phases were combined, washed with saturated brine (10 mL x 1), dried over anhydrous Na₂SO₄, and filtered. The filtrate was concentrated under reduced pressure. The residue was separated and purified by column chromatography on silica gel, to obtain Compound **1-6**. LCMS: 300.2 (M+H)⁺.

### Step VI: Synthesis of Compound 1-7

At 0°C, Compound **1-6** (1.2 g, 4.0 mmol) was dissolved in methanol (25 mL), sodium borohydride (0.3 g, 8.0 mmol) was slowly added batchwise, and reacted at 25°C for 3 hrs. The reaction solution was concentrated. The residue was separated and purified by column chromatography on silica gel, to obtain Compound **1-7.** LCMS: 304.2 (M+H)⁺.

### Step VII: Synthesis of Compound 1-8

At 0°C, Compound **1-7** (400 mg, 1.32 mmol) was dissolved in THF (15 mL), and sodium hydride (106 mg, 2.65 mmol, 60%) was slowly added batchwise. The reaction solution was reacted at room temperature for 2 hrs, quenched with saturated NH₄Cl (10 mL), and extracted with EA (50 mL x 2). The organic phases were combined, washed with saturated brine, dried over anhydrous Na₂SO₄, and filtered and concentrated. The residue was separated and purified by column chromatography on silica gel, to obtain Compound **1-8.** LCMS: 266.2 (M+H)⁺.

### Step VIII: Synthesis of Compound 1

At 0°C, **1-8** (300 mg, 1.13 mmol) was dissolved in THF (10 mL), and a borane dimethyl sulfide complex (565 uL, 5.65 mmol) was slowly added dropwise. The reaction solution was heated to 70°C and reacted for 4 hrs. The reaction was quenched with methanol (5 mL) at 0°C, and stirred at room temperature for 1 hr. The reaction solution was concentrated, and purified by prep-HPLC, to obtain Compound **1.**

MS: 252.0 (M+H)⁺. ¹H NMR (400 MHz, DMSO) δ 7.77 (s, 1H), 7.69 (d, *J =* 7.2 Hz, 1H), 7.38-7.34 (m, 1H), 7.28-7.25 (m, 1H), 3.82 (dd, *J* = 10.8 Hz, 10.8 Hz 1H), 3.64-3.58 (m, 1H), 3.54-3.50 (m, 1H), 2.52-2.50 (m, 3H), 2.38-2.37 (m, 1H), 1.70-1.69 (m, 3H), 1.40-1.37 (m, 3H), 0.85-0.75 (m, 1H).

### Example 2: Preparation of Compound 2

### Preparation method I (chemical resolution of intermediate):

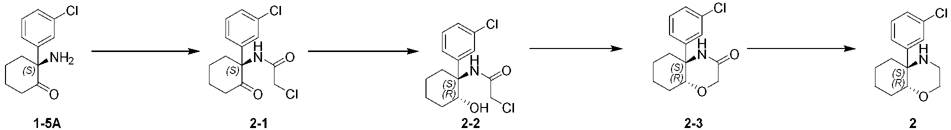

### Step I: Synthesis of Compound 2-1

At 0°C, Compound **1-5A** (1.0 g, 4.47 mmol) and triethyl amine (1.2 mL, 8.6 mmol) were dissolved in DCM (20 mL). Chloroacetyl chloride (0.5 g, 4.5 mmol) was slowly added dropwise to the above solution. The reaction solution was reacted at 0°C for 3 hrs. The reaction solution was diluted with iced water (10 mL) and extracted three times with DCM (30 mL). The organic phases were combined, washed with saturated brine, dried over anhydrous Na₂SO₄, filtered, and concentrated. The residue was separated and purified by column chromatography on silica gel, to obtain Compound **2-1**. LCMS: 322.2 (M+Na)⁺.

### Step II: Synthesis of Compound 2-2

At 0°C, Compound **2-1** (1.1 g, 3.7 mmol) was dissolved in methanol (20 mL), and sodium borohydride (0.37 g, 9.78 mmol) were added. The reaction solution was reacted at 25°C for 16 hrs. The reaction solution was quenched with saturated NH₄Cl (20 mL), and extracted three times with EA (30 mL). The organic phases were combined, washed with saturated brine, dried over anhydrous Na₂SO₄, and concentrated. The residue was separated and purified by column chromatography on silica gel, to obtain Compound **2-2**.

### Step III: Synthesis of Compound 2-3

At 0°C, Compound **2-2** (300 mg, 0.993 mmol) was dissolved in THF (5 mL), and sodium hydride (80 mg, 2.0 mmol, 60%) was added batchwise. and reacted at room temperature for 3 hrs. The reaction solution was quenched with a saturated NH₄Cl solution, and extracted two times with EA (30 mL). The organic phases were combined, washed with saturated brine, dried over anhydrous Na₂SO₄, and concentrated. The residue was separated and purified by column chromatography on silica gel, to obtain Compound **2-3**. LCMS: *m*/*z*= 266.1 (M+H)⁺.

### Step IV: Synthesis of Compound 2

At 0°C, Compound **2-3** (240 mg, 0.903 mmol) was dissolved in THF (20 mL), and a borane dimethyl sulfide complex (1.0 mL ,10 M, 10 mmol) was slowly added dropwise. The reaction solution was heated to 60°C and reacted for 16 hrs. The reaction solution was quenched with methanol (20 mL), stirred at room temperature for 1 hr, and concentrated. The residue was purified by prep-HPLC, to obtain Compound **2**. LCMS: 252.2 (M+H)⁺.

¹H NMR (400 MHz, DMSO) δ 7.82 - 7.76 (m, 1H), 7.73 - 7.66 (m, 1H), 7.38 (t, *J* = 8.0 Hz, 1H), 7.32 - 7.25 (m, 1H), 3.90 - 3.77 (m, 1H), 3.67 - 3.58 (m, 1H), 3.56 - 3.49 (m, 1H), 2.47 - 2.35 (m , 2H), 2.24 - 2.14 (m, 1H), 1.77 - 1.59 (m, 3H), 1.49 - 1.33 (m, 3H), 0.92 - 0.74 (m, 1H).

### Method II (chiral synthesis route):

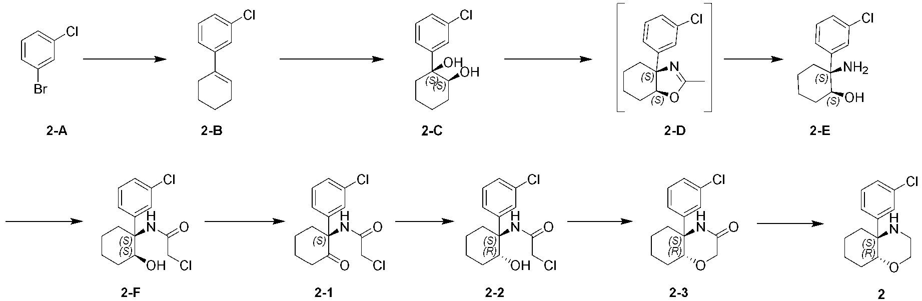

### Step I: Synthesis of Compound 2-B

At room temperature, **2-A** (50.0 g, 261 mmol), cyclohexene-1-boronic acid pinacol ester (32.9 g, 261 mmol), and sodium carbonate (83.04 g, 783.5 mmol) were dissolved in a mixed solvent of dioxane (500 mL) and water (50 mL). Pd(dppf)Cl₂ (3.8 g, 5.2 mmol) was added. The system was purged with nitrogen, heated to 90°C, and refluxed for 16 hrs. The reaction solution was concentrated. The residue was separated and purified by column chromatography on silica gel, to obtain Compound **2-B.**

¹H NMR (400 MHz, DMSO) δ 7.42 - 7.40 (m, 1H), 7.38 - 7.31 (m, 2H), 7.29 - 7.25 (m, 1H), 6.24 - 6.20 (m, 1H), 2.36 - 2.31 (m, 2H), 2.21 - 2.15 (m, 2H), 1.75 - 1.68 (m, 2H), 1.62 - 1.56 (m, 2H).

### Step II: Synthesis of Compound 2-C

At room temperature, K₂CO₃ (86.0 g, 622.8 mmol), potassium ferricyanide (205.0 g, 622.8 mmol), methylsulfonylamide (23.7 g, 249.1 mmol), potassium osmate dihydrate (3.8 g, 10.4 mmol), and (DHQ)₂ PHAL (32.3 g, 41.5 mmol) were dissolved in water (400 mL). **2-B** (40.0 g, 208 mmol) was dissolved in t-butanol (250 mL) and added to the above reaction solution. The reaction was carried out at room temperature for 16 hrs. The reaction solution was added with EA (500 mL), and washed three times with a 15% sodium hydroxide aqueous solution (200 mL x 3). The organic phase was dried over anhydrous Na₂SO₄, and filtered. The filtrate was concentrated. The residue was separated and purified by column chromatography on silica gel, to obtain Compound **2-C.** LCMS: 209.4 (M-18)⁺.

### Step III: Synthesis of Compound 2-E

At -40°C, TfOH (32.2 mL, 364 mmol) was added dropwise to **2-C** (37.5 g, 165 mmol) in acetonitrile (550 mL). Then, the dry ice bath was removed, the reaction solution was naturally warmed to room temperature and further reacted for 1 hr. Water (370 mL) was added to the reaction solution and stirred for 10 min. Acetonitrile was removed by concentration under vacuum. The remaining aqueous solution was heated to 100°C and refluxed for 16 hrs. The reaction solution was adjusted to pH 10 with a sodium hydroxide aqueous solution, and extracted with EA (800 mL). The organic phase was washed with water (300 mL x 3) and saturated brine (300 mL), dried over anhydrous Na₂SO₄, and filtered. The filtrate was concentrated. PE:EA (300 mL, v/v=5/1) was added, and stirred for 10 min. After filtration, the filer cake was collected, and dried under reduced pressure to obtain Compound **2-E.** LCMS: 226.4 (M+H)⁺.

### Step IV: Synthesis of Compound 2-F

**2-E** (54.0 g, 239 mmol) and triethyl amine (83.0 mL, 597 mmol) were added to methyl tetrahydrofuran (1 L) at 0°C. Chloroacetyl chloride (22.8 mL, 286.5 mmol) was slowly added dropwise, and reacted at 0°C for 1 hr. The reaction solution was diluted with water (500 mL) and extracted three times with EA (300 mL). The organic phases were combined, washed with saturated brine, dried over anhydrous Na₂SO₄, filtered, and concentrated. The residue was separated and purified by column chromatography on silica gel, to obtain Compound **2-E.** LCMS: 302.4 (M+H)⁺.

### Step V: Synthesis of Compound 2-1

At room temperature, **2-E** (40.0 g, 132 mmol) was dissolved in DCM (700 mL), and then PCC (71.3 g, 331 mmol) was added, heated to 30°C, and reacted for 16 hrs. The reaction solution was directly concentrated. The residue was separated and purified by column chromatography on silica gel, to obtain Compound **2-1.** LCMS: 300.0 (M+H)⁺.

### Step VI: Synthesis of Compound 2-2

At 0°C, Compound **2-1** (1.1 g, 3.7 mmol) was dissolved in methanol (20 mL), and sodium borohydride (0.37 g, 9.78 mmol) were added. The reaction solution was reacted at 25°C for 16 hrs. The reaction solution was quenched with saturated NH₄Cl (20 mL), and extracted three times with EA (30 mL). The organic phases were combined, washed with saturated brine, dried over anhydrous Na₂SO₄, and concentrated. The residue was separated and purified by column chromatography on silica gel, to obtain Compound **2-2.**

### Step VII: Synthesis of Compound 2-3

At 0°C, Compound **2-2** (300 mg, 0.993 mmol) was dissolved in THF (5 mL), and sodium hydride (80 mg, 2.0 mmol, 60%) was added batchwise. The reaction solution was reacted at room temperature for 3 hrs. The reaction solution was quenched with a saturated NH₄Cl solution, and extracted two times with EA (30 mL). The organic phases were combined, washed with saturated brine, dried over anhydrous Na₂SO₄, and concentrated. The residue was separated and purified by column chromatography on silica gel, to obtain Compound **2-3.** LCMS: *m*/*z=* 266.1 (M+H)⁺.

### Step VIII: Synthesis of Compound 2

At 0°C, Compound **2-3** (240 mg, 0.903 mmol) was dissolved in THF (20 mL), and a borane dimethyl sulfide complex (1.0 mL ,10 M in DMS, 10 mmol) was slowly added dropwise. The reaction solution was heated to 60°C and reacted for 16 hrs. The reaction solution was quenched with methanol (20 mL), stirred at room temperature for 1 hr, and concentrated to obtain a crude product. The crude product was purified by prep-HPLC, to obtain Compound **2.** LCMS: 252.2 (M+H)⁺.

¹H NMR (400 MHz, DMSO) δ 7.82 - 7.76 (m, 1H), 7.73 - 7.66 (m, 1H), 7.38 (t, *J =* 8.0 Hz, 1H), 7.32 - 7.25 (m, 1H), 3.90 - 3.77 (m, 1H), 3.67 - 3.58 (m, 1H), 3.56 - 3.49 (m, 1H), 2.47 - 2.35 (m , 2H), 2.24 - 2.14 (m, 1H), 1.77 - 1.59 (m, 3H), 1.49 - 1.33 (m, 3H), 0.92 - 0.74 (m, 1H).

### Example 3: Preparation of Compound 3 hydrochloride

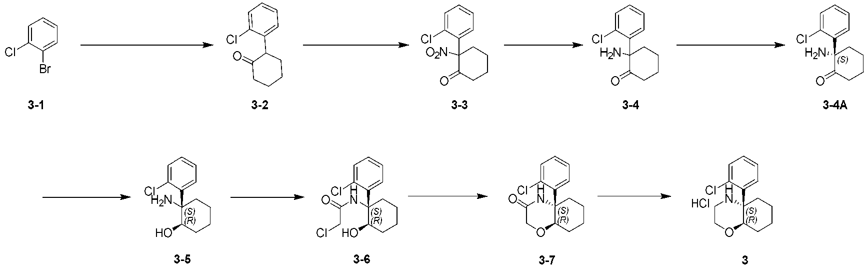

### Step I: Synthesis of Compound 3-2

At 25°C, cyclohexanone (25.6 g, 261 mmol), 1-bromo-2-chlorobenzene (25.0 g, 131 mmol, **3-1**), and cesium carbonate (93.6 g, 287 mmol) were dissolved in dioxane (250 mL), and Pd₂(dba)₃(1.2 g, 1.3 mmol) and Xantphos (1.5 g, 2.6 mmol) were added. The system was purged with nitrogen, heated to 100°C, and reacted for 20 hrs. The reaction solution was cooled to room temperature, and filtered. The filtrate was concentrated to obtain a crude product. The crude product was separated and purified by column chromatography on silica gel, to obtain Compound **3-2.** LCMS: 209.0 (M+H)⁺.

### Step II: Synthesis of Compound 3-3

At 25°C, Compound **3-2** (24.0 g, 115 mmol), copper acetate (11.5 g, 57.5 mmol), and ceric ammonium nitrate (157.6 g, 287.5 mmol) were added to 1,2-dichloroethane (350 mL). The system was purged with nitrogen, and reacted at 80°C for 16 hrs. The reaction solution was filtered and concentrated to obtain a crude product. The crude product was separated and purified by column chromatography on silica gel, to obtain Compound **3-3.** LCMS: 276.1 (M+Na)⁺.

### Step III: Synthesis of Compound 3-4

At 0 °C, Compound **3-3** (16.0 g, 63.0 mmol) was added to acetic acid (200 mL), and a zinc powder (20.6 g, 315.3 mmol) were added batchwise. The reaction solution was heated to 80°C, and stirred for 16 hrs. The reaction solution was concentrated to obtain a crude product. H₂O (200 mL) and a sodium hydroxide aqueous solution (2 M) were added to adjust the pH to 12. The reaction solution was extracted with EA (200 mL x 2). The organic phases were combined, washed with saturated brine (50 mL), dried over anhydrous Na₂SO₄, and filtered. The filtrate was concentrated under reduced pressure. The residue was separated and purified by column chromatography on silica gel, to obtain Compound **3-4**. LCMS: 224.2 (M+H)⁺.

### Step IV: Synthesis of Compound 3-4A

At 25°C, Compound **3-4** (6.0 g, 26.8 mmol) was added to anhydrous ethanol (25 mL). The system was stirred at 65°C until the solution became clear. L-pyroglutamic acid (1.9 g, 14.7 mmol) was dissolved in a mixed solution of ethanol (12 mL) and H₂O (3 mL). The mixed solution was slowly added dropwise to the above reaction solution, heated to 75°C, and reacted for 1 hr. The reaction solution was allowed to stand overnight, and a white solid was precipitated out. After filtration, the filter cake was washed two times with ethanol (10 ml). The filter cake was dissolved in H₂O (30 mL), adjusted to pH 10 with a 2 M sodium hydroxide aqueous solution, and extracted two times with EA (100 mL). The organic phases were combined, dried over anhydrous Na₂SO₄, and filtered. The filtrate was concentrated under reduced pressure to obtain Compound **3-4A** as a crude product. Compound **3-4A** was dissolved in anhydrous ethanol. The above process was repeated. to obtain Compound **3-4A**. LCMS: *m*/*z*= 224.2 (M+H)⁺.

### Step V: Synthesis of Compound 3-5

At 0°C, **3-4A** (26.0 g, 116 mmol) was dissolved in methanol (500 mL), and then sodium borohydride (13.2 g, 349 mmol) was slowly added batchwise and reacted at 25°C for 2 hrs. The reaction solution was concentrated to obtain a crude product. The residue was separated and purified by column chromatography on silica gel, to obtain Compound **3-5**. LCMS: 226.2 (M+H)⁺.

### Step VI: Synthesis of Compound 3-6

At 0°C, Compound **3-5** (23.0 g, 102 mmol) and triethyl amine (22.0 g, 217.1 mmol) were dissolved in DCM (500 mL), and chloroacetyl chloride (14.7 g, 130.3 mmol) was slowly added dropwise to the above solution, and reacted at 0°C for 2 hrs. The reaction solution was quenched with water (100 mL), and extracted two times with EA (200 mL). The organic phases were combined, washed with saturated brine (100 mL), dried over anhydrous Na₂SO₄, and filtered. The filtrate was concentrated under reduced pressure to obtain a crude product. The crude product was separated and purified by column chromatography on silica gel, to obtain Compound **3-6.** LCMS: 302.2 (M+H)⁺.

### Step VII: Synthesis of Compound 3-7

At 0°C, Compound **3-6** (1.5 g, 5.0 mmol) was dissolved in THF (30 mL), and sodium hydride (0.5 g, 12.4 mmol, 60%) was slowly added batchwise. The reaction was continued at room temperature for 2 hrs. The reaction solution was quenched with saturated NH₄Cl (20 mL), and extracted two times with EA (50 mL). The organic phases were combined, washed with saturated brine. dried over anhydrous Na₂SO₄, filtered, and concentrated to obtain a crude product. The crude product was separated and purified by column chromatography on silica gel, to obtain Compound **3-7.** LCMS: 266.2 (M+H)⁺.

### Step VIII: Synthesis of Compound 3

At 0°C, Compound **3-7** (1.4 g, 5.27 mmol) was dissolved in THF (50 mL), and a borane dimethyl sulfide complex (2.1 mL, 21.07 mmol) was slowly added dropwise. The reaction solution was heated to 70°C and reacted for 4 hrs. The reaction solution was cooled to 0°C, slowly quenched with methanol (10 mL), stirred at room temperature for 1 hr, and concentrated under reduced pressure to obtain a crude product. The residue was purified by prep-HPLC, to obtain Compound **3** hydrochloride.

LCMS: 252.0 (M+H)⁺. ¹H NMR (400 MHz, DMSO) 8.28 - 8.26 (m, 1H), 7.63-7.59 (m, 1H), 7.51-7.48 (m, 2H), 4.25 - 4.21 (m, 1H), 4.19 - 4.13 (m, 1H), 4.05-4.01 (m, 1H), 3.42 - 3.36 (m, 1H), 3.08 (d, *J =* 12.8 Hz, 1H), 2.72-2.69 (m, 1H), 2.11 - 1.96 (m, 3H), 1.71 - 1.55 (m, 2H), 1.47 - 1.38 (m, 1H), 0.80 - 0.69 (m, 1H).

### Example 4: Preparation of Compounds 13 and 17 hydrochloride

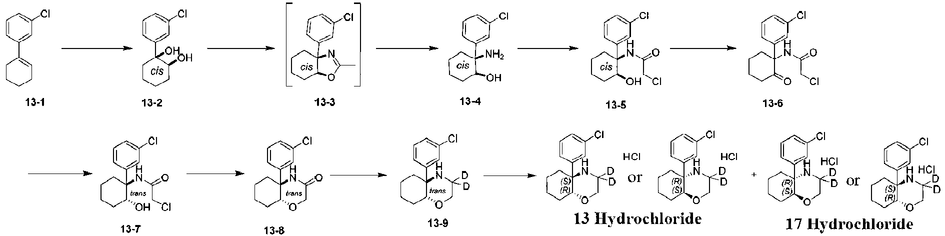

### Step I: Synthesis of Compound 13-2

At room temperature, 3-chloro-1-(cyclohex-1-enyl)benzene (15.0 g, 77.8 mmol, **13-1**), NMO (18.5 g, 158 mmol), and potassium osmate dihydrate (1.4 g, 3.8 mmol) were added to a mixed solvent of t-butanol (100 mL) and H₂O (150 mL). The system was purged three times with nitrogen, and stirred at room temperature for 12 hrs. The reaction solution was added with EA (500 mL), and washed three times with a 15% sodium hydroxide aqueous solution (100 mL x 3). The organic phase was dried over anhydrous Na₂SO₄, and filtered. The filtrate was concentrated under reduced pressure to obtain a crude product. The crude product was separated and purified by column chromatography on silica gel, to obtain Compound **13-2**. LCMS: 209.0 (M-18)⁺.

### Step II: Synthesis of Compound 13-4

At -40°C, TfOH (13.0 mL, 147 mmol) was added to a solution of **13-2** (14.0 g, 61.8 mmol) in acetonitrile (250 mL). Then, the dry ice bath was removed. The reaction solution naturally warmed to room temperature and stirred for 1 hr. Water (150 mL) was added to the reaction solution and stirred for 30 min. Acetonitrile was removed by concentration under vacuum. The remaining aqueous solution was heated to 100°C and refluxed for 16 hrs. The reaction solution was adjusted to pH 10 with a 1 N sodium hydroxide aqueous solution, and extracted with EA (300 mL). The organic phase was washed with water (100 mL x 3) and saturated brine (100 mL), dried over anhydrous Na₂SO₄, and filtered. The filtrate was concentrated under reduced pressure, and PE:EA (200 mL, v/v=5/1) was added, and stirred for 10 min. After filtration, the filer cake was collected, and dried under reduced pressure to obtain Compound **13-4**. LCMS: 226.0 (M+H)⁺.

### Step III: Synthesis of Compound 13-5

**13-4** (8.50 g, 37.7 mmol) and triethyl amine (13.0 mL, 93.5 mmol) was dissolved in methyl tetrahydrofuran (100 mL) at 0°C. Chloroacetyl chloride (3.60 mL, 45.2 mmol) were slowly added. Then, the reaction solution was further stirred at 0°C for 2 hrs. The reaction solution was diluted with water (200 mL) and extracted three times with EA (100 mL x 3). The organic phases were combined, washed with saturated brine (100 mL), dried over anhydrous Na₂SO₄, and filtered. The filtrate was concentrated to obtain a crude product. The crude product was separated and purified by column chromatography on silica gel, to obtain Compound **13-5**. LCMS: 302.0 (M+H)⁺.

### Step IV: Synthesis of Compound 13-6

At room temperature, **13-5** (8.5 g, 28 mmol) was dissolved in DCM (100 mL), and PCC (15 g, 69.6 mmol) was added. The reaction solution was heated to 30°C, stirred for 12 hrs, and directly concentrated. The residue was separated and purified by column chromatography on silica gel, to obtain Compound **13-6**. LCMS: 300.0 (M+H)⁺.

### Step V: Synthesis of Compound 13-7

**13-6** (6.0 g, 20 mmol) was dissolved in methanol (80 mL) at 0°C, and then sodium borohydride (1.5 g, 39.7 mmol) was slowly added batchwise. The reaction was continued at 0°C for 2 hrs. The reaction solution was directly concentrated. The residue was separated and purified by column chromatography on silica gel, to obtain Compound **13-7**. LCMS: 302.0 (M+H)⁺.

### Step VI: Synthesis of Compound 13-8

**13-7** (4.7 g, 15.5 mmol) was dissolved in isopropanol (60 mL) at 0°C, and then potassium tert-butoxide (5.20 g, 46.3 mmol) was added batchwise. After reaction at 0°C for 30 min, the ice bath was removed, and the reaction solution was naturally warmed to room temperature and reacted for 2 hrs. The reaction solution was directly concentrated, added with water (50 mL), stirred for 10 min, and filtered. The filer cake was collected, and dried under vacuum to obtain Compound **13-7**. LCMS: 266.0 (M+H)⁺.

### Step VII: Synthesis of Compound 13-9

At 0°C, **13-8** (100.0 mg, 0.376 mmol) was dissolved in THF (3 mL), and boron trifluorideetherate (252.0 uL, 0.941 mmol) was slowly added dropwise and reacted at 0°C for 20 min. Then, NaBD₄ (220.0 mg, 1.128 mmol) was added batchwise to the reaction solution, heated to 65°C, and reacted for 2 hrs. The reaction solution was cooled to room temperature, and quenched with methanol (5 mL). The reaction solution was concentrated. The residue was separated and purified by column chromatography on silica gel, to obtain Compound **13-9**. LCMS: 254.4 (M+H)⁺.

### Step VIII: Synthesis of Compounds 13 hydrochloride and 17 hydrochloride

Compound **13-9** (40 mg, 0.138 mmol) was separated by SFC. A first fraction (Rt=1.027 min) was collected, and rotary dried to obtain a compound as a colorless oil. Pure water (10 mL) and 1 M dilute hydrochloric acid (200 uL) were added to the compound. A first P1 (Rt=1.027 min) was collected, and freeze dried, to obtain Compound **13** hydrochloride. LCMS: 254.1 (M+H)⁺.

¹H NMR (400 MHz, DMSO) δ 10.33 (s, 1H), 9.37 (s, 1H), 7.96 (s, 1H), 7.92 - 7.91 (m, 1H), 7.58 - 7.50 (m, 2H), 4.12 - 3.97 (m, 3H), 2.59 - 2.55 (m, 1H), 1.98 (td, *J* = 13.2, 3.2 Hz, 1H), 1.92 - 1.85 (m, 1H), 1.81 - 1.71 (m, 1H), 1.70 - 1.62 (m, 1H), 1.57 - 1.53 (m, 1H), 1.47 - 1.35 (m, 1H), 0.87 - 0.76 (m, 1H).

A second fraction (Rt=1.235 min) was collected, and rotary dried to obtain a compound as a colorless oil. Pure water (10 mL) and 1 M dilute hydrochloric acid (200 uL) were added to the compound, and freeze dried, to obtain Compound **17** hydrochloride. LCMS: 254.1 (M+H)⁺.

¹H NMR (400 MHz, DMSO) δ 10.32 (s, 1H), 9.36 (s, 1H), 7.96 (s, 1H), 7.92 - 7.90 (m, 1H), 7.57 - 7.51 (m, 2H), 4.11 - 3.97 (m, 3H), 2.59 - 2.55 (m, 1H), 1.98 (td, *J =* 13.6, 3.2 Hz, 1H), 1.92 - 1.85 (m, 1H), 1.82 - 1.71 (m, 1H), 1.68 - 1.64 (m, 1H), 1.59 - 1.51 (m, 1H), 1.47 - 1.34 (m, 1H), 0.87 - 0.76 (m, 1H).

(SFC conditions: column: ChiralPAK-IG_100x3.0mm_3µm; mobile phase: A for CO₂ and B for IPA (0.1%DEA); gradient: 10% in 3 min, flow rate: 2.0 mL /min).

### Example 5: Preparation of Compounds 30 and 31

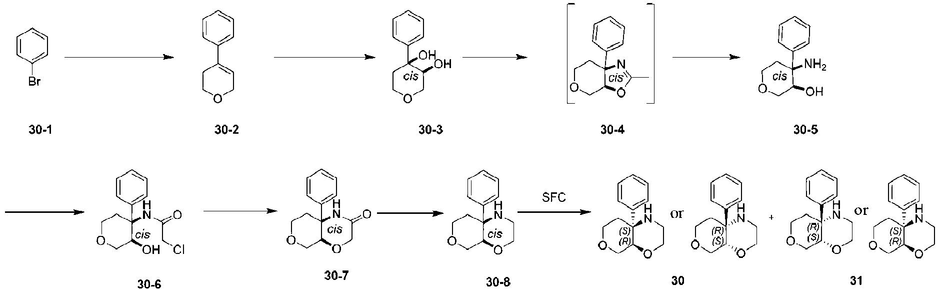

### Step I: Synthesis of Compound 30-2

At 25°C, 1,4-dioxane (50 mL) and H₂O (10 mL) were added to bromobenzene (5.0 g, 32 mmol, 30-1), 2-(3,6-dihydro-2*H*-pyran-4-yl)-4,4,5,5-tetramethyl-1,3,2-dioxaborane (6.7 g, 32 mmol), and Na₂CO₃ (6.75 g, 63.7 mmol), and then purged three times with nitrogen. Pd(dppf)Cl₂.CH₂Cl₂ (1.30 g, 1.59 mmol) were added. The system was purged 3 times with N₂, heated to 100°C and reacted for 16 hrs. The reaction solution was cooled to 25 °C, quenched with H₂O (100 mL), and extracted three times with EA (100 mL*3). The organic phases were combined, washed with saturated brine (100 mL), dried over anhydrous Na₂SO₄, filtered, and concentrated to obtain a crude product. The crude product was separated and purified by column chromatography on silica gel, to obtain Compound **30-2.** LCMS: 161.2 (M+H)⁺.

### Step II: Synthesis of Compound 30-3

At 25°C, t-butanol (50 mL) and H₂O (25 mL) were added to **30-2** (4.00 g, 19.5 mmol) and potassium osmate dihydrate (300 mg, 0.976 mmol). Then, N-methylmorpholine oxide (4.60 g, 39.3 mmol) was slowly added to the reaction solution and purged 3 times with N₂. The reaction was continued at 25°C for 16 hrs. The reaction solution was diluted with EA (30 mL), and filtered. The filtrate was extracted with EA (50 mL x 3), and the organic layer was washed with saturated brine (30 mL), dried over anhydrous Na₂SO₄, filtered, and concentrated to obtain a crude product. The crude product was separated and purified by column chromatography on silica gel, to obtain Compound **30-3**.

### Step III: Synthesis of Compound 30-5

**30-3** (500 mg, 2.49 mmol) was dissolved in acetonitrile (10 mL), and purged 3 times with N₂. The reaction solution was cooled to -40°C, and TfOH(0.55 mL, 6.2 mmol) was slowly added dropwise. After that, the cooling bath was removed, and the reaction solution was stirred at room temperature for 2 hrs. H₂O (20 mL) was added, and stirred for 10 min. The reaction solution was concentrated to remove acetonitrile, and The remaining mother liquor was heated to 100°C and refluxed for 12 hrs. The reaction solution was cooled to room temperature, adjusted to pH 10 with 1 N NaOH solution, and extracted two times with EA (50 mL x 2). The organic layer was washed with saturated brine (30 mL), dried over anhydrous Na₂SO₄, filtered, and concentrated to obtain a crude product. The crude product was slurried in 50 mL of a mixed solvent (PE/EA=5/1), filtered, and concentrated under reduced pressure to obtain **30-5**. LCMS: 194.2 (M+H)⁺.

### Step IV: Synthesis of Compound 30-6

Under a nitrogen atmosphere, **30-5** (250 mg, 0.996 mmol) was dissolved in DCM (10 mL), and cooled to 0°C. Triethyl amine (0.35 mL, 2.518 mmol) was slowly added dropwise, and stirred for 30 min. Chloroacetyl chloride (0.10 mL, 1.3 mmol) was added dropwise, and then heated to room temperature for 2.5 hrs. The reaction solution was concentrated under reduced pressure to obtain a crude product. The crude product was separated and purified by column chromatography on silica gel, to obtain Compound **30-6**. LCMS: 270.0 (M+H)⁺.

### Step V: Synthesis of Compound 30-7

At 25°C, **30-6** (250 mg, 0.927 mmol) was dissolved in isopropanol (10 mL), and then potassium tert-butoxide (310 mg, 2.76 mmol) was added, purged 3 times with N₂, and stirred for 5 hrs. The reaction solution was directly concentrated to obtain a crude product. Water (20 mL) was added, stirred for 10 min, and filtered. The filer cake was collected, and dried under vacuum to obtain **30-7**. LCMS: 234.2 (M+H)⁺.

### Step VI: Synthesis of Compound 30-8

At 0°C, **30-7** (150 mg, 0.643 mmol) was dissolved in THF (10 mL), and a borane dimethyl sulfide complex (0.35 mL, 3.500 mmol) was slowly added dropwise, heated to 70°C, and reacted for 16 hrs. After cooling to 0°C, the reaction was quenched with methanol (1 mL). The reaction solution was stirred at room temperature for 1 hr. Sodium borohydride (50 mg, 1.322 mmol) was slowly added batchwise. The reaction solution was heated to 50°C and further reacted for 16 hrs. The reaction solution was concentrated, to obtain a crude product. The crude product was separated and purified by column chromatography on silica gel, to obtain Compound **30-8.**

### Step VII: Synthesis of Compounds 30 and 31

**30-8** (100 mg, 0.456 mmol) was separated by SFC. A second fraction (*Rt*=4.4 min) was collected, and rotary dried to obtain a compound as a colorless oil. Compound **30** was obtained after freezing drying with pure water. LCMS: 220.2 (M+H)⁺.

¹H NMR (400 MHz, CDCl₃) δ 7.55 - 7.48 (m, 2H), 7.43 - 7.34 (m, 2H), 7.32 - 7.27 (m, 1H), 4.12 - 4.07 (m, 1H), 4.05 - 3.91 (m, 3H), 3.72 - 3.63 (m, 1H), 3.62 - 3.51 (m, 2H), 3.27 - 3.15 (m, 1H), 2.78 - 2.59 (m, 2H), 1.92 - 1.83 (m, 1H).

A first fraction (Rt=3 min) was collected, and rotary dried to obtain a compound as a colorless oil. Compound 31 was obtained after freezing drying with pure water. LCMS: 220.2 (M+H)⁺.

¹H NMR (400 MHz, CDCl₃) δ 7.55 - 7.49 (m, 2H), 7.42 - 7.36 (m, 2H), 7.32 - 7.27 (m, 1H), 4.10 - 4.06 (m, 1H), 4.05 - 3.91 (m, 3H), 3.71 - 3.63 (m, 1H), 3.61 - 3.52 (m, 2H), 3.24 - 3.14 (m, 1H), 2.78 - 2.59 (m, 2H), 1.94 - 1.82 (m, 1H).

(SFC conditions: column: ChiralPak IH, 40 mm I.D. x 250 mm, 10 µm; mobile phase: A for Supercritical CO₂ and B for 0.1% NH₃.H₂O MeOH; gradient: 30% B; flow rate: 120 mL /min).

### Example 6: Preparation of Compounds 33 and 36 hydrochloride

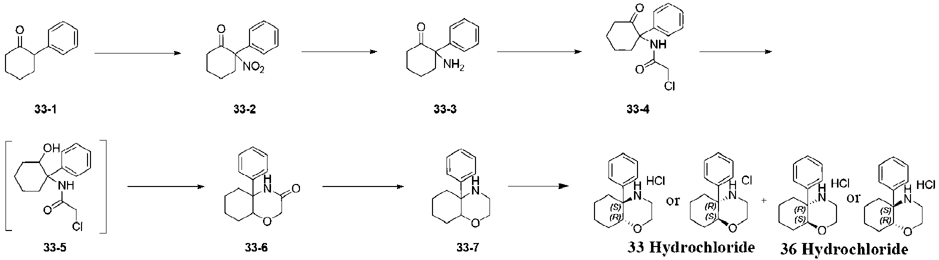

### Step I: Synthesis of Compound 33-2

2-phenylcyclohexanone (5 g, 28.70 mmol, **33-1**) was dissolved in 1,2-dichloroethane (100 mL), and ceric ammonium nitrate (47.2 g, 86.10 mmol) and copper acetate (5.21 g, 28.70 mmol) were added. The system was purged with nitrogen, heated to 85°C and refluxed for 12 hrs. The reaction solution was cooled to room temperature, and filtered through diatomite. The filter cake was washed with DCM, and The filtrate was concentrated under reduced pressure to obtain a crude product. The crude product was separated and purified by column chromatography on silica gel, to obtain Compound **33-2.** LCMS: 173.0 (M-NO₂)⁺.

### Step II: Synthesis of Compound 33-3

**33-2** (2.89 g, 13.18 mmol) was dissolved in a mixed solution of methanol and acetic acid (30 mL: 20 mL). The system was purged with nitrogen, slowly added with a zinc powder (4.28 g, 65.91 mmol), heated to 70°C and refluxed for 12 hrs. The reaction solution was cooled to room temperature, adjusted to a basic pH with 2 M NaOH aqueous solution and extracted two times with EA (100 mL). The organic phase was dried over anhydrous Na₂SO₄, and concentrated under reduced pressure to obtain a crude product. The crude product was separated and purified by column chromatography on silica gel, to obtain Compound 33-3. LCMS: 190.0 (M+H)⁺.

### Step III: Synthesis of Compound 33-4

**33-3** (1.89 g, 10.01 mmol) was dissolved in DCM (40 mL), and the system was purged with nitrogen and added with triethyl amine (1.22 g, 12.01 mmol). The reaction solution was cooled to 0°C, and chloroacetyl chloride (1.24 g, 11.01 mmol) was slowly added dropwise. Then, the reaction solution was heated to room temperature, stirred for 1 hr, water was added, and the reaction solution was extracted with EA (50 mL). The organic phase was dried over anhydrous Na₂SO₄, and concentrated under reduced pressure to obtain a crude product. The crude product was separated and purified by column chromatography on silica gel, to obtain Compound **33-4.** LCMS: 266.0 (M+H)⁺.

### Step IV: Synthesis of Compound 33-6

**33-4** (2.04 g, 7.68 mmol) was dissolved in methanol (30 mL), and cooled to 0°C. Sodium borohydride (291 mg, 7.68 mmol) were added batchwise. The reaction solution was heated to room temperature, stirred for 1 hr, and rotary dried under reduced pressure to obtain Compound **33-5. 33-5** was dissolved in THF (30 mL), cooled to 0°C, and then added with NaH (369 mg, 9.22 mmol, 60% in mineral oil). The reaction solution was heated to room temperature, stirred for 12 hr, quenched with a saturated NH₄Cl solution, and extracted three times with DCM (40 mL). The organic phase was dried over anhydrous Na₂SO₄, and concentrated under reduced pressure to obtain a crude product. The crude product was separated and purified by column chromatography on silica gel, to obtain Compound **33-6.** LCMS: 232.0 (M+H)⁺.

### Step V: Synthesis of Compound 33-7

**33-6** (1.0 g, 4.32 mmol) were dissolved in THF (16 mL). The system was purged three times with nitrogen, and then a borane dimethyl sulfide complex (10.8 mL, 21.62 mmol) was added dropwise. Then, the system was heated to 70°C and refluxed for 12 hrs. The reaction solution was cooled to room temperature, and quenched with methanol added dropwise. 1 N HCl (8 mL) was added and stirred for 1 hr. The reaction solution was adjusted to a basic pH with a saturated sodium bicarbonate solution and extracted two times with EA (50 mL). The organic phase was dried over anhydrous Na₂SO₄, and concentrated under reduced pressure to obtain a crude product. The crude product was separated and purified by column chromatography on silica gel, to obtain Compound **33-7**. LCMS: 218.0 (M+H)⁺.

### Step VI: Synthesis of Compounds 33 and 36

**33-7** (100 mg, 0.46 mmol) was separated by SFC. A first fraction (Rt=3.785 min) was collected. Pure water (10 mL) and 1 M dilute hydrochloric acid (200 uL) were added to the compound, and freeze dried, to obtain Compound **33** as a hydrochloride. LCMS: 218.2 (M+H)⁺.

¹H NMR (400 MHz, MeOD) δ 7.87 (d, *J* = 7.6 Hz, 2H), 7.48 - 7.32 (m, 3H), 4.16 - 4.07 (m, 1H), 3.98 - 3.89 (m, 1H), 3.87 - 3.81 (m, 1H), 2.98 - 2.86 (m, 2H), 2.69 - 2.55 (m, 1H), 1.95 - 1.85 (m, 2H), 1.80 - 1.63 (m, 2H), 1.59 - 1.43 (m, 2H), 1.03 - 0.90 (m, 1H).

A second fraction (Rt=4.525 min) was collected. Pure water (10 mL) and 1 M dilute hydrochloric acid (200 uL) were added to the compound, and freeze dried, to obtain Compound **36** which was a hydrochloride. LCMS: 218.2 (M+H)⁺.

¹H NMR (400 MHz, MeOD) δ 7.94 - 7.79 (m, 2H), 7.48 - 7.33 (m, 3H), 4.15 - 4.05 (m, 1H), 3.97 - 3.88 (m, 1H), 3.86 - 3.78 (m, 1H), 2.97 - 2.86 (m, 2H), 2.66 - 2.56 (m, 1H), 1.95 - 1.85 (m, 2H), 1.80 - 1.62 (m, 2H), 1.59 - 1.38 (m, 2H), 1.04 - 0.87 (m, 1H).

(SFC conditions: Column: ChiralPak IG, 250 x 40mm I.D., 10µm; mobile phase: A for Supercritical CO₂ and B for 0.1 NH₃.H₂O EtOH; Gradient: 10% B; Flow rate: 80 mL /min).

### Example 7: Preparation of Compound 34 hydrochloride

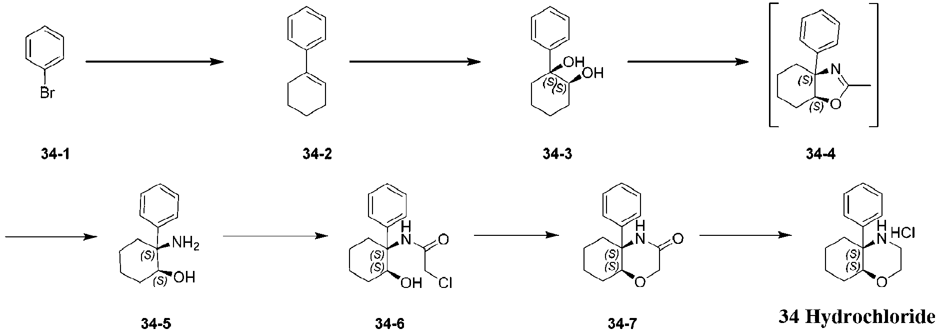

### Step I: Synthesis of Compound 34-2

At room temperature, bromobenzene (10.0 g, 63.7 mmol, **34-1**), cyclohexene-1-boronic acid pinacol ester (13.2 g, 63.7 mmol), and sodium carbonate (13.5 g, 127.0 mmol) were added to a mixed solvent of dioxane (100 mL) and water (20 mL). Pd(dppf)Cl₂ (2.33 g, 3.18 mmol) were added to the reaction solution. The system was purged three times with nitrogen. The reaction solution was heated to 90°C and reacted for 16 hrs. The reaction solution was concentrated to obtain a crude product. The crude product was separated and purified by column chromatography on silica gel, to obtain Compound **34-2.**

¹H NMR (400 MHz, DMSO) δ 7.40 - 7.37 (m, 2H), 7.33 - 7.27 (m, 2H), 7.24 - 7.19 (m, 1H), 6.17 - 6.11 (m, 1H), 2.38 - 2.33 (m, 2H), 2.21 - 2.14 (m, 2H), 1.77 - 1.69 (m, 2H), 1.64 - 1.56 (m, 2H).

### Step II: Synthesis of Compound 34-3

At room temperature, **34-2** (3.0 g, 18 mmol) was dissolved in water (25 mL) and t-butanol (25 mL). Then K₂CO₃ (7.86 g, 56.9 mmol), potassium ferricyanide (18.73 g, 56.88 mmol), Methylsulfonylamide (1.98 g, 20.9 mmol), potassium osmate dihydrate (0.35 g, 0.95 mmol), and (DHQ)₂PHAL (3.69 g, 4.74 mmol) were added batchwise, and reacted at room temperature for 16 hrs. A 15% sodium hydroxide aqueous solution (50 mL) was added to the reaction solution, and extracted three times with EA (50 mL x 3). The combined organic layer was washed with brine (50 mL), dried over anhydrous Na₂SO₄, filtered, and concentrated to obtain a crude product. The crude product was separated and purified by column chromatography on silica gel, to obtain Compound **34-3.**

¹H NMR (400 MHz, DMSO) δ 7.51 - 7.44 (m, 2H), 7.32 - 7.24 (m, 2H), 7.19 - 7.12 (m, 1H), 4.32 (s, 1H), 4.10 (d, *J =* 6.8 Hz, 1H), 3.78 - 3.68 (m, 1H), 1.71 - 1.54 (m, 6H), 1.42 - 1.29 (m, 2H).

### Step III: Synthesis of Compound 34-5

At -40°C, to a solution of **34-3** (3.00 g, 15.6 mmol) in acetonitrile (40 mL), TfOH (3.42 mL, 39.0 mmol) was added dropwise. Then, the dry ice bath was removed. The reaction solution was naturally warmed to room temperature and reacted for 1 hr. Water (40 mL) was added to the reaction solution and stirred for 10 min. Acetonitrile was removed by concentration under vacuum. The remaining aqueous phase was heated to 100°C and refluxed for 16 hrs. The reaction solution was added with a 15% NaOH solution (50 mL) and extracted three times with EA (50 mL x 3). The combined organic layer was washed with brine (50 mL), dried over anhydrous Na₂SO₄, filtered, and concentrated to obtain a crude product. The crude product was added with PE:EA=10:1(20 mL), slurried, and filtered, to obtain Compound **34-5**. LCMS: 192.4 (M+H)⁺.

### Step IV: Synthesis of Compound 34-6

**34-5** (2.5 g, 13 mmol) was dissolved in THF (25 mL). Triethyl amine (3.6 mL, 26 mmol) was added at 0°C, and then chloroacetyl chloride (1.14 mL, 14.4 mmol) was slowly added to the mixed solution. The reaction was continued at 0°C for 1 hr. The reaction solution was diluted with water (25 mL) and extracted three times with EA (25 mL x 3). The combined organic layer was washed with brine (25 mL), dried over anhydrous Na₂SO₄, filtered, and concentrated to obtain a crude product. The crude product was separated and purified by column chromatography on silica gel, to obtain Compound **34-6**.

¹H NMR (400 MHz, DMSO) δ 7.60 (s, 1H), 7.36 - 7.25 (m, 4H), 7.22 - 7.15 (m, 1H), 4.32 (s, 2H), 3.71 - 3.61 (m, 1H), 2.81 - 2.70 (m, 1H), 1.79 - 1.56 (m, 4H), 1.47-1.24 (m, 3H).

### Step V: Synthesis of Compound 34-7

At 0°C, **34-6**(2.00 g, 7.47 mmol) was dissolved in isopropanol (20 mL). Potassium tert-butoxide (1.68 g, 14.9 mmol) was added batchwise to the reaction solution, and reacted in an ice bath for 1 hr. The reaction solution was directly rotary dried to obtain a crude product. H₂O(20 ml) was added, and a solid was precipitated out, filtered, and washed with H₂O (20 mL) and PE(20 mL). The filer cake was collected, and dried under vacuum to obtain Compound **34-7**.

¹H NMR (400 MHz, DMSO) δ 8.47 (s, 1H), 7.49 - 7.41 (m, 2H), 7.40 - 7.31 (m, 2H), 7.28 - 7.21 (m, 1H), 4.17 (dd, *J* = 14.4, 4.3 Hz, 1H), 3.90 (dd, *J* = 68.8, 17.1 Hz, 2H), 1.93 - 1.70 (m, 4H), 1.66 - 1.55 (m, 1H), 1.54 - 1.40 (m, 3H).

### Step VI: Synthesis of Compound 34

At 0°C, **34-7** (200 mg, 0.865 mmol) was dissolved in THF (5 mL). A borane dimethyl sulfide complex (865 uL, 8.65 mmol) was slowly added dropwise to the above reaction solution. Then, the reaction solution was heated to 70°C and refluxed for 16 hrs. The reaction solution was cooled to 0°C, and quenched with methanol (5 mL). After quenching, the reaction solution was stirred for 1 hr. After cooling to 0°C, sodium borohydride (163 mg, 4.32 mmol) were slowly added batchwise to the reaction solution. The reaction solution was heated to 50°C, and further reacted for 16 hrs. The reaction solution was concentrated to obtain a crude product. The crude product was separated and purified by column chromatography on silica gel. Pure water (10 mL) and 1N dilute hydrochloric acid (100 uL) were added to the colorless oil compound, and freeze dried, to obtain Compound **34** hydrochloride. LCMS: 218.2 (M+H)⁺.

¹H NMR (400 MHz, DMSO) δ 9.96 (s, 1H), 9.33 (s, 1H), 7.83 - 7.67 (m, 2H), 7.57 - 7.39 (m, 3H), 4.50 (s, 1H), 4.09 (dd, *J =* 15.6, 3.3 Hz, 1H), 3.87 (td, *J=* 26.4, 2.4 Hz, 1H), 3.50 - 3.37 (m, 1H), 2.99 - 2.83 (m, 1H), 2.50 - 2.26 (m, 2H), 1.78 (s, 1H), 1.71 - 1.61 (m, 1H), 1.57 - 1.40 (m, 2H), 1.36 - 1.25 (m, 1H), 1.19 - 1.01 (m, 1H).

### Example 8: Preparation of Compound 35 hydrochloride

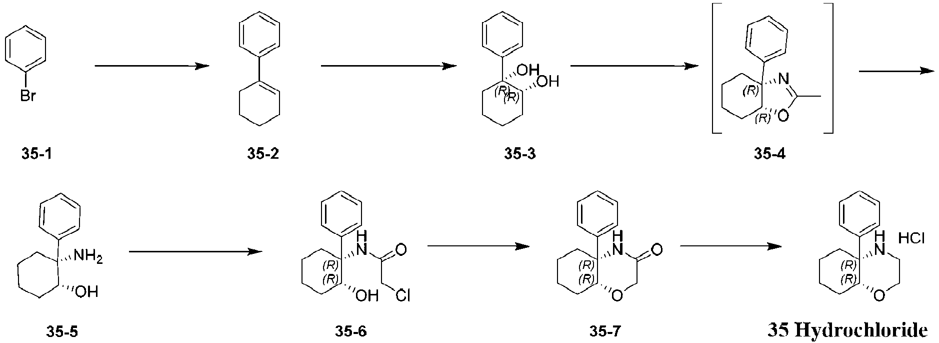

### Step I: Synthesis of Compound 35-2

At room temperature, bromobenzene (10.0 g, 63.7 mmol, **35-1**), cyclohexene-1-boronic acid pinacol ester (13.25 g, 63.69 mmol), and sodium carbonate (13.50 g, 127.4 mmol) were added to a mixed solvent of dioxane (100 mL) and water (20 mL). Pd(dppf)Cl₂ (2.33 g, 3.18 mmol) was added. The system was purged three times with nitrogen, heated to 90°C and refluxed for 16 hrs. The reaction solution was filtered, and the filtrate was concentrated to obtain a crude product. The crude product was separated and purified by column chromatography on silica gel to obtain Compound **35-2.**

¹H NMR (400 MHz, DMSO) δ 7.40 - 7.37 (m, 2H), 7.33 - 7.27 (m, 2H), 7.24 - 7.19 (m, 1H), 6.17 - 6.11 (m, 1H), 2.38 - 2.33 (m, 2H), 2.21 - 2.14 (m, 2H), 1.77 - 1.69 (m, 2H), 1.64 - 1.56 (m, 2H).

### Step II: Synthesis of Compound 35-3

At room temperature, K₂CO₃ (10.5 g, 76.0 mmol), potassium ferricyanide (25.0 g, 75.9 mmol), methylsulfonylamide (2.60 g, 27.3 mmol), potassium osmate dihydrate (470 mg, 1.26 mmol), and (DHQD)₂PHAL (4.0 g, 5.1 mmol) were added to water (40 mL). **35-2** (4.0 g, 25 mmol) was dissolved in t-butanol (25 mL), and added to the above reaction solution. The reaction was continued at room temperature for 16 hrs. The reaction solution was added with EA (200 mL), and washed three times with a 15% sodium hydroxide aqueous solution (50 mL). The organic phase was dried over anhydrous Na₂SO₄, and filtered. The filtrate was concentrated under reduced pressure to obtain a crude product. The crude product was separated and purified by column chromatography on silica gel to obtain Compound **35-3**.

¹H NMR (400 MHz, DMSO) δ 7.49 - 7.46 (m, 2H), 7.30 - 7.25 (m, 2H), 7.19 - 7.14(m, 1H), 4.33 (s, 1H), 4.11 (d, *J* = 6.8 Hz, 1H), 3.77 - 3.69 (m, 1H), 1.70 - 1.56 (m, 6H), 1.42 - 1.29 (m, 2H).

### Step III: Synthesis of Compound 35-5

At -40°C, to a solution of **35-3** (3.50 g, 18.2 mmol) in acetonitrile (70 mL), TfOH (3.50 mL, 39.6 mmol) was added dropwise. Then, the dry ice bath was removed. The reaction solution was naturally warmed to room temperature and reacted for 1 hr. Water (35 mL) was added to the reaction solution and stirred for 10 min. Acetonitrile was removed by concentration under vacuum. The remaining aqueous phase was heated to 100°C and further reacted for 16 hrs. The reaction solution was adjusted to pH 10 with a 1 N sodium hydroxide aqueous solution, and the mixed solution was extracted two times with EA (50 mL x 2). The organic phases were combined, washed with saturated brine, dried over anhydrous Na₂SO₄, and filtered. The filtrate was concentrated under reduced pressure to obtain a crude product. The crude product was added with PE:EA=3:1 (30 mL), stirred for 10 min, and filtered. The filer cake was collected, and dried under vacuum to obtain Compound **35-5**. LCMS: 192.4 (M+H)⁺.

### Step IV: Synthesis of Compound 35-6

At 0°C, **35-5** (1.3 g, 6.8 mmol) and triethyl amine (2.40 mL, 17.3 mmol) were added to methyl tetrahydrofuran (25 mL). Chloroacetyl chloride (0.60 mL, 7.5 mmol) was slowly added dropwise to the above solution, and reacted at 0°C for 1 hr. The reaction solution was diluted with water (50 mL) and extracted two times with EA (25 mL x 2). The organic phases were combined, washed with saturated brine, dried over anhydrous Na₂SO₄, filtered, and concentrated to obtain a crude product. The crude product was separated and purified by column chromatography on silica gel. to obtain Compound **35-6**. LCMS: 268.4 (M+H)⁺.

### Step V: Synthesis of Compound 35-7

At 0°C, **35-6** (900 mg, 3.36 mmol) was dissolved in isopropanol (20 mL). Potassium tert-butoxide (754 mg, 6.72 mmol) was added batchwise to the reaction solution, and reacted for 10 in an ice bath. Then the ice bath was removed, and the reaction solution was naturally warmed to room temperature and reacted for 2 hrs. The reaction solution was concentrated to obtain a crude product. The crude product was added with water (20 mL), stirred for 10 min, and filtered. The filer cake was collected, and dried under vacuum to obtain Compound **35-7**. LCMS: 232.4 (M+H)⁺.

### Step VI: Synthesis of Compound 35

At 0°C, **35-7** (150 mg, 0.649 mmol) was dissolved in THF (10 mL). A borane dimethyl sulfide complex (325 uL, 3.25 mmol) was slowly added dropwise to the above reaction solution. After that, the reaction solution was heated to 70°C and reacted for 16 hrs. The reaction solution was cooled to 0°C, and quenched with methanol (5 mL). After quenching, the reaction solution was further stirred for 1 hr. After cooling to 0°C, sodium borohydride (50 mg, 1.3 mmol) were slowly added batchwise to the reaction solution. The reaction solution was heated to 50°C, and further reacted for 16 hrs. The reaction solution was concentrated to obtain a crude product. The crude product was separated and purified by column chromatography on silica gel. Pure water (10 mL) and 1 N dilute hydrochloric acid (400 uL) were added to the colorless oil compound, and freeze dried, to obtain Compound **35**, which was a hydrochloride. LCMS: 218.4 (M+H)⁺.

¹H NMR (400 MHz, DMSO) δ 9.71 (s, 1H), 9.20 (s, 1H), 7.76 - 7.70 (m, 2H), 7.54 - 7.49 (m, 2H), 7.48 - 7.43 (m, 1H), 4.52 - 4.48 (m, 1H), 4.12 - 4.07 (m, 1H), 3.90 - 3.79 (m, 1H), 3.47 - 3.37 (m, 1H), 2.96 - 2.89 (m, 1H), 2.49 - 2.46 (m, 1H), 2.35 - 2.26 (m, 1H), 1.85 - 1.72 (m, 1H), 1.70 - 1.62 (m, 1H), 1.56 - 1.43 (m, 2H), 1.35 - 1.28 (m, 1H), 1.19 - 1.07 (m, 1H).

### Example 9: Preparation of Compound 39 hydrochloride

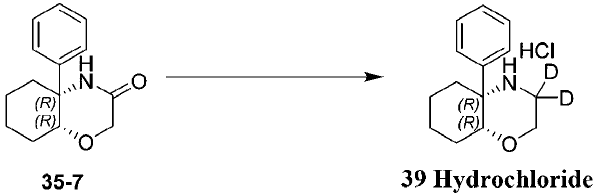

### Step I: Synthesis of Compound 39

At 0°C, **35-7** (80 mg, 0.35 mmol) was dissolved in THF (5 mL). Boron trifluoride etherate (232 uL, 0.865 mmol) was slowly added dropwise, and reacted for 1 hr. Sodium borodeuteride (73.0 mg, 1.74 mmol) was slowly added batchwise. The reaction solution was heated to 65°C and reacted for 2 hrs. The reaction solution was cooled to 0°C, quenched with methanol (5 mL), and concentrated to obtain a crude product. The crude product was purified by prep-HPLC. Pure water (10 mL) and 1 N dilute hydrochloric acid (400 uL) were added, and freeze dried, to obtain Compound **39** which was a hydrochloride. LCMS: 220.4 (M+H)⁺.

(Prep-HPLC conditions: Column: Xtimate C18, 21.2*250 mm, 5 um; Mobile phase: A (0.05% NH₃.H₂O), B (ACN); Gradient: 30%-60% B; Flow rate: 20 mL /min).

¹H NMR (400 MHz, DMSO) δ 9.85 (s, 1H), 9.23 (s, 1H), 7.89 - 7.65 (m, 2H), 7.63 - 7.38 (s, 3H), 4.50 (s, 1H), 4.10 - 3.83(m, 2H), 2.47 - 2.25 (s, 2H), 1.78 - 1.05 (m, 6H).

### Example 10: Preparation of Compound 40 hydrochloride

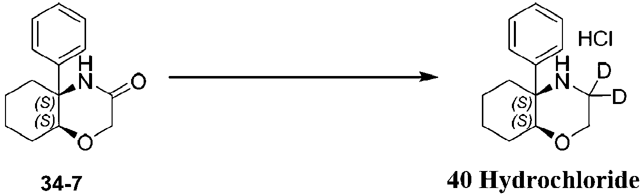

### Step I: Synthesis of Compound 40 hydrochloride

At 0°C, **34-7** (200 mg, 0.865 mmol) was dissolved in THF (5 mL). Boron trifluoride etherate (534 uL, 4.33 mmol) was slowly added dropwise. Sodium borodeuteride (181 mg, 4.32 mmol) was slowly added batchwise. The system was heated to 65°C, and reacted for 2 hrs. The reaction solution was cooled to 0°C, quenched with methanol (5 mL), and concentrated to remove the solvent. The residue was added with water (10 ml) and extracted with EA (10 mL x 3). The combined organic layer was washed with brine (10 mL), dried over anhydrous Na₂SO₄, filtered, and concentrated to obtain a crude product. The crude product was separated and purified by column chromatography on silica gel to obtain Compound 40 as a free base. Pure water (10 mL) and 1N dilute hydrochloric acid (100 uL) were added to the colorless oil compound, and freeze dried, to obtain Compound **40** hydrochloride. LCMS: 220.2 (M+H)⁺.

¹H NMR (400 MHz, DMSO) δ 9.38 (s, 1H), 8.97 (s, 1H), 7.74 - 7.67 (m, 2H), 7.58 - 7.44 (m, 3H), 4.57 - 4.46 (m, 1H), 4.10 (d, *J* = 12.8 Hz, 1H), 3.86 - 3.78 (m, 1H), 2.57 - 2.51 (m, 1H), 2.28 (d, *J* = 13.2 Hz, 1H), 1.83 - 1.61 (m, 2H), 1.58 - 1.43 (m, 2H), 1.35 - 1.26 (m, 1H), 1.18 - 1.05 (m, 1H).

### Example 11: Preparation of Compound 45 hydrochloride and Compound 46 hydrochloride

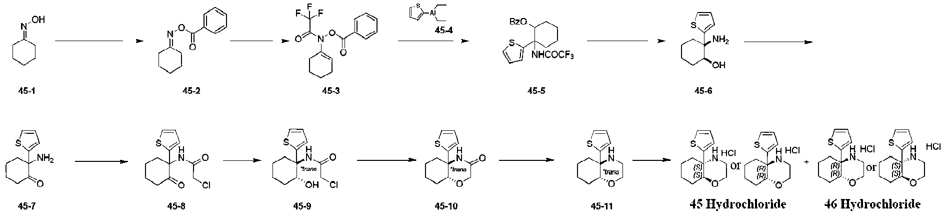

### Step I: Synthesis of Compound 45-2

At 0°C, cyclohexanone oxime **(45-1)** (10.0 g, 88.4 mmol) was dissolved in a mixed solution of DCM and n-hexane (1:15, 320 mL). The system was purged with nitrogen, and pyridine (7.1 mL, 88 mmol) was slowly added dropwise. The mixed solution was stirred at 0°C for 30 min, and then benzoyl chloride (10.3 mL, 88.4 mmol) was slowly added dropwise, heated to room temperature, and stirred for 2 hrs. Water (100 mL x 3) was added for separatory extraction. The organic layer was concentrated, to obtain Compound **45-2.**

### Step II: Synthesis of Compound 45-3

At 0°C, **45-2** (19 g, crude) was dissolved in DCM (120 mL). The system was purged with nitrogen. Then, triethyl amine (65.0 mL, 468 mmol) was slowly added dropwise, and slowly heated to room temperature and stirred for 12 hrs. After filtration, the filtrate was concentrated under reduced pressure to obtain a crude product. The crude product was separated and purified by column chromatography on silica gel, to obtain Compound **45-3.**

¹H NMR (400 MHz, CDCl₃) δ 8.08 (d, *J=* 7.6 Hz, 2H), 7.75 - 7.60 (m, 1H), 7.58 - 7.44 (m, 2H), 6.43 - 6.12 (m, 1H), 2.48 - 2.13 (m, 4H), 1.81 - 1.71 (m, 2H), 1.69 - 1.56 (m, 2H).

### Step III: Synthesis of Compound 45-5

**45-3** (6.5 g, 20.7 mmol) was dissolved in THF (60 ml), then slowly added dropwise to a solution of **45-4** at 0°C, heated to reflux, and stirred for 3 hrs. The reaction solution was concentrated under reduced pressure to obtain a crude product. The crude product was separated and purified by column chromatography on silica gel to obtain Compound **45-5.**

¹H NMR (400 MHz, CDCl₃) δ 7.99 - 7.89 (m, 2H), 7.63 - 7.56 (m, 1H), 7.53 - 7.41 (m, 2H), 7.18 - 7.13 (m, 1H), 7.04 - 6.95 (m, 2H), 6.90 - 6.86 (m, 1H), 5.33 - 5.24 (m, 1H), 3.35 - 3.25 (m, 1H), 2.23 - 2.04 (m, 2H), 1.90 (m, 1H), 1.94 - 1.85 (m, 2H), 1.59 - 1.41 (m, 2H).

### Step IV: Synthesis of Compound 45-6

**45-5** (4.0 g, 14.5 mmol) was dissolved in methanol (40 mL). At 0°C, a 10% sodium hydroxide aqueous solution (26 mL, 72.4 mmol) was slowly added dropwise, and stirred at room temperature for 12 hrs. The reaction solution was concentrated under reduced pressure to obtain a crude product. The crude product was separated and purified by column chromatography on silica gel to obtain Compound **45-6**.

¹H NMR (400 MHz, CDCl₃) δ 7.23 - 7.18 (m, 1H), 7.05 - 6.95 (m, 2H), 3.87 (dd, *J* = 9.6, 3.8 Hz, 1H), 2.19 (s, 3H), 1.98 - 1.90 (m, 1H), 1.89 - 1.73 (m, 3H), 1.72 - 1.49 (m, 3H), 1.44 - 1.32 (m, 1H).

### Step V: Synthesis of Compound 45-7

At room temperature, **45-6** (600 mg, 3.0 mmol) was dissolved in acetone (15 mL). Jones reagent (3 mL, 6.1 mmol) was slowly added dropwise, and reacted for 16 hrs. Water (10 mL) was added, and the reaction solution was adjusted to pH 10 with 15% sodium hydroxide and extracted with EA (20 mL x 2). The organic phases were combined, washed with saturated brine, dried over anhydrous Na₂SO₄, and filtered. The filtrate was concentrated under reduced pressure to obtain a crude product. The crude product was separated and purified by column chromatography on silica gel to obtain Compound **45-7**. LCMS: 196.3 (M+H)⁺.

### Step VI: Synthesis of Compound 45-8

At 0°C, **45-7**(100 mg, 0.51 mmol) and triethyl amine (104 mg, 1.02 mmol) were dissolved in DCM (5 mL), and chloroacetyl chloride (64 mg, 0.56 mmol) was slowly added dropwise, and reacted for 2 hrs. Water (5 mL) was added and the reaction solution was extracted with DCM (10 mL x 2). The organic phases were combined, washed with saturated brine, dried over anhydrous Na₂SO₄, and filtered. The filtrate was concentrated under reduced pressure to obtain a crude product. The crude product was separated and purified by column chromatography on silica gel to obtain Compound **45-8**. LCMS: 294.0 (M+H)⁺.

### Step VII: Synthesis of Compound 45-9

At 0°C, **45-8** (130 mg, 0.48 mmol) was dissolved in methanol (4 mL), and sodium borohydride (54 mg, 1.44 mmol) was slowly added batchwise. The system was heated to 25°C, and reacted for 1 hr. The reaction solution was concentrated to obtain a crude product. The crude product was separated and purified by column chromatography on silica gel to obtain Compound **45-9**.

¹H NMR (400 MHz, CDCl₃) δ 7.21-7.20 (m, 2H), 6.93 (dd, *J*=4.0, 4.8 Hz, 1H), 6.79 (s, 1H), 4.12 (dd, *J*=3.6, 8.0 Hz, 1H), 3.91 (s, 2H), 3.47 (s, 1H), 2.48-2.42 (m, 1H), 2.15-2.08 (m, 1H), 1.84-1.77 (m, 1H), 1.72-1.63 (m, 1H), 1.57-1.46 (m, 2H), 1.41-1.32 (m, 1H).

### Step VIII: Synthesis of Compound 45-10

At 0°C, **45-9** (130 mg, 0.48 mmol) was dissolved in THF (5 mL), and sodium hydride (57 mg, 1.43 mmol, 60%) was slowly added batchwise, heated to room temperature, and reacted for 2 hrs. A saturated NH₄Cl aqueous solution (5 mL) was added, and the reaction solution was extracted with EA (10 mL x 2). The organic phases were combined, washed with saturated brine, dried over anhydrous Na₂SO₄, filtered, and concentrated to obtain a crude product. The crude product was separated and purified by column chromatography on silica gel to obtain Compound **45-10.** LCMS: 238.2 (M+H)⁺.

### Step IX: Synthesis of Compound 45-11

At 0°C, **45-10** (100 mg, 0.42 mmol) was dissolved in THF (5 mL), and a borane dimethyl sulfide complex (210 uL, 2.10 mmol) was slowly added dropwise. The system was heated to 70°C, and reacted for 16 hrs. The reaction solution was cooled to 0°C, quenched with methanol (5 mL), and stirred at room temperature for 1 hr. The reaction solution was concentrated to obtain a crude product. The crude product was separated and purified by column chromatography on silica gel to obtain Compound **45-11.** LCMS: 224.2 (M+H)⁺.

### Step X: Synthesis of Compounds 45 and 46

**45-11** (60 mg, 0.27 mmol) was separated by SFC. A first fraction (Rt=3.69 min) was collected. After concentration under reduced pressure, pure water (10 mL) and 1 M dilute hydrochloric acid (50 uL) were added, and freeze dried, to obtain Compound **45** which was a hydrochloride. LCMS: 224.2 (M+H)⁺.

¹H NMR (400 MHz, DMSO) δ 10.56 (s, 1H), 9.39 (d, *J*=10.0 Hz, 1H), 7.74 (d, *J*=4.8 Hz, 1H), 7.52 (d, *J*=3.2 Hz, 1H), 7.17 (dd, *J*=3.6, 5.2 Hz, 1H), 4.10-3.95 (m, 3H), 3.09-3.01 (m, 2H), 2.54-2.50 (m, 1H), 2.18-2.11 (m, 1H), 1.79-1.75 (m, 1H), 1.65-1.57 (m, 3H), 1.41-1.31 (m, 1H), 1.17-1.07 (m, 1H).

A second fraction (Rt=5.74 min) was collected. After concentration under reduced pressure, pure water (10 mL) and 1 M dilute hydrochloric acid (50 uL) were added, and freeze dried, to obtain Compound **46** which was a hydrochloride. LCMS: 224.2 (M+H)⁺.

¹H NMR (400 MHz, DMSO) δ 10.62 (s, 1H), 9.41 (d, *J*=9.6 Hz, 1H), 7.73 (d, *J*=4.8 Hz, 1H), 7.52 (d, *J*=3.2 Hz, 1H), 7.17 (dd, *J =* 3.6, 5.2 Hz, 1H), 4.09-3.95 (m, 3H), 3.09-3.01 (m, 2H), 2.53-2.50 (m, 1H), 2.19-2.12 (m, 1H), 1.78-1.76 (m, 1H), 1.63-1.57 (m, 3H), 1.41-1.31 (m, 1H), 1.12-1.07 (m, 1H).

(SFC conditions: Column: ChiralPak IG, 250 x 40mm I.D., 10µm; Mobile phase: A for CO₂ and B for 50% MeOH (0.1% NH₃·H₂O) + 50% ACN (0.1% NH₃·H₂O); Gradient: 50% B; Flow rate: 120 mL /min).

### Example 12: Preparation of Compounds 52 and 53

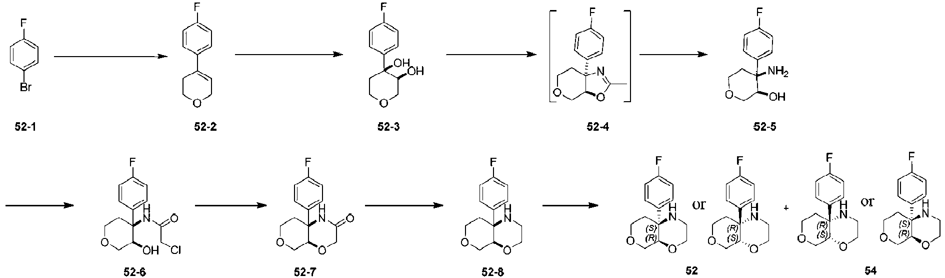

### Step I: Synthesis of Compound 52-2

At room temperature, **52-1** (5.00 g, 28.6 mmol), 3,6-dihydro-2H-pyran-4-boronic acid pinacol ester (6.00 g, 28.6 mmol), and sodium carbonate (9.08 g, 85.7 mmol) were added to a mixed solvent of dioxane (50 mL) and water (5 mL). Pd(dppf)Cl₂.CH₂Cl₂ (0.47 g, 0.57 mmol) was added, and the system was purged three times with nitrogen. The reaction solution was heated to 90°C, and reacted for 16 hrs. The reaction solution was concentrated to obtain a crude product. The crude product was separated and purified by flash column chromatography on silica gel to obtain Compound **52-2.**

¹H NMR (400 MHz, MeOD) δ 7.46 - 7.39 (m, 2H), 7.08 - 7.01 (m, 2H), 6.13 - 6.11 (m, 1H), 4.28 (q, *J =* 2.8 Hz, 2H), 3.91 (t, *J =* 5.6 Hz, 2H), 2.52 - 2.46 (m, 2H).

### Step II: Synthesis of Compound 52-3

At room temperature, **52-2** (3.00 g, 16.8 mmol) was dissolved in a mixed solution of water (30 mL) and t-butanol (45 mL), and potassium osmate dihydrate (310 mg, 0.842 mmol) and *N*-methylmorpholine oxide (3.94 g, 33.7 mmol) were slowly added dropwise, and reacted for 16 hrs. Water (50 mL) was added, and the reaction was extracted with EA (100 mL x 2). The organic phases were combined, washed with saturated brine, dried over anhydrous Na₂SO₄, and filtered. The filtrate was concentrated under reduced pressure to obtain a crude product. The crude product was separated and purified by column chromatography on silica gel to obtain Compound **52-3.**

¹H NMR (400 MHz, DMSO) δ 7.57 - 7.51 (m, 2H), 7.15 - 7.08 (m, 2H), 4.82 (d, *J =* 1.6 Hz, 1H), 4.61 (d, *J =* 6.8 Hz, 1H), 3.80 - 3.74 (m, 1H), 3.69 - 3.58 (m, 3H), 3.42 (t, *J* = 10.4 Hz, 1H), 1.98 - 1.89 (m, 1H), 1.58 - 1.53 (m, 1H).

### Step III: Synthesis of Compound 52-5

At -40°C, to a solution of **52-3** (2.0 g, 9.4 mmol) in acetonitrile(30 mL), TfOH (2.08 mL, 23.6 mmol) was slowly added dropwise. Then, the dry ice bath was removed, and the reaction solution was naturally warmed to room temperature and reacted for 1 hr. Water (20 mL) was added, and stirred for 10 min. Acetonitrile was removed by concentration under vacuum. The remaining aqueous phase was heated to 100°C and further reacted for 16 hrs. The reaction solution was adjusted to pH 10 with a sodium hydroxide aqueous solution, and extracted with EA (50 mL x 2). The organic phases were combined, washed with saturated brine (30 mL), dried over anhydrous Na₂SO₄, and filtered. The filtrate was concentrated under reduced pressure to obtain a product. The crude product was added with PE/EA (20 mL, v/v= 5/1), stirred for 10 min, and filtered. The filer cake was collected, and dried under vacuum to obtain Compound **52-5**. LCMS: 212.4 (M+H)⁺.

### Step IV: Synthesis of Compound 52-6

At 0°C, **52-5** (800 mg, 3.79 mmol) and triethyl amine (1.3 mL, 9.5 mmol) were added to methyl tetrahydrofuran (20 mL). Chloroacetyl chloride (362 uL, 4.55 mmol) was added dropwise, and reacted for 1 hr. The reaction solution was concentrated under reduced pressure to obtain a crude product. The crude product was separated and purified by flash column chromatography on silica gel to obtain Compound **52-6**.

¹H NMR (400 MHz, CDCl₃) δ 7.41 (s, 1H), 7.39 - 7.34 (m, 2H), 7.11 - 7.05 (m, 2H), 4.10 (d, *J* = 0.8 Hz, 2H), 3.96 - 3.91 (m, 1H), 3.89 - 3.81 (m, 2H), 3.60 (td, *J* = 11.6, 2.4 Hz, 1H), 3.51 (dd, *J* = 11.6, 9.6 Hz, 1H), 2.83 (dt, *J* = 14.4, 2.4 Hz, 1H), 2.62 (s, 1H), 2.33 - 2.24 (m, 1H).

### Step V: Synthesis of Compound 52-7

At room temperature, to a solution of **52-6** (250 mg, 0.869 mmol) in isopropanol (10 mL), potassium tert-butoxide (292 mg, 2.61 mmol) was added, and reacted for 2 hrs. The reaction solution was directly concentrated to obtain a crude product. The crude product was separated and purified by column chromatography on silica gel to obtain Compound **52-7**. LCMS: 252.0 (M+H)⁺.

### Step VI: Synthesis of Compound 52-8

At 0°C, to a solution of **52-7** (150 mg, 0.597 mmol) in THF (10 mL), a borane dimethyl sulfide complex (300 uL, 3.00 mmol) was slowly added dropwise. After that, the reaction solution was heated to 70°C, and reacted for 16 hrs. After cooling to 0°C, the reaction solution was quenched with methanol (1 mL), and stirred for 1 hr. At 0°C, sodium borohydride (45 mg, 1.2 mmol) was slowly added batchwise. The reaction solution was heated to 50°C, and further reacted for 16 hrs. The reaction solution was concentrated to obtain a crude product. The crude product was separated and purified by column chromatography on silica gel to obtain Compound **52-8.** LCMS: 238.4 (M+H)⁺.

### Step VII: Synthesis of Compounds 52 and 53

**52-8** (100 mg, 0.421 mmol) was separated by SFC. A second fraction (Rt=9.2 min) was collected. The reaction solution was concentrated under reduced pressure, and pure water (10 mL) was added, freeze dried, to obtain Compound **52.** LCMS: 238.4 (M+H)⁺.

¹H NMR (400 MHz, DMSO) δ 7.63 - 7.56 (m, 2H), 7.20 - 7.11 (m, 2H), 4.10 - 4.07 (m, 1H), 4.05 - 3.96 (m, 1H), 3.78 - 3.71 (m, 2H), 3.50 - 3.45 (m, 1H), 3.45 - 3.36 (m, 2H), 2.79 - 2.70 (m, 1H), 2.64 (s, 1H), 2.56 - 2.50 (m, 1H), 2.17 - 1.96 (m, 1H), 1.66 - 1.59 (m, 1H).

A first fraction (Rt=6.8 min) was collected. After concentration under reduced pressure, pure water (10 mL) was added, and freeze dried, to obtain Compound **53.** LCMS: 238.4 (M+H)⁺.

¹H NMR (400 MHz, DMSO) δ 7.63 - 7.56 (m, 2H), 7.20 - 7.12 (m, 2H), 4.10 - 4.07 (m, 1H), 4.06 - 3.96 (m, 1H), 3.78 - 3.72 (m, 2H), 3.50 - 3.46 (m, 1H), 3.45 - 3.36 (m, 2H), 2.79 - 2.69 (m, 1H), 2.65 (s, 1H), 2.55 - 2.51 (m,, 1H), 2.13 - 2.03 (m, 1H), 1.68 - 1.58 (m, 1H).

(SFC conditions: Column: ChiralPak IG, 250 x 40mm I.D., 10µm; Mobile phase: A for CO₂ and B for 30% MeOH (0.1% NH₃·H₂O); Gradient: 30% B; Flow rate: 120 mL /min).

### Example 13: Preparation of Compound 74 hydrochloride

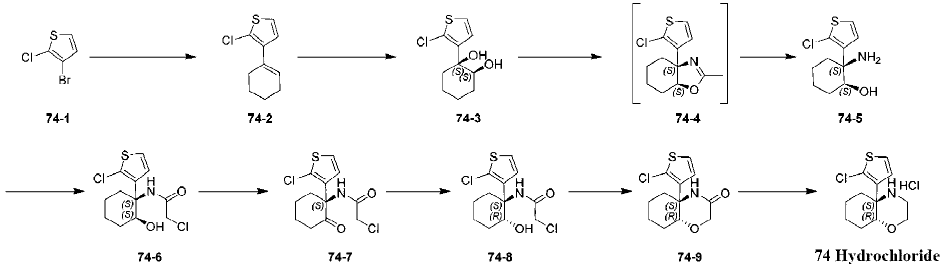

### Step I: Synthesis of Compound 74-2

At room temperature, **74-1** (5.00 g, 25.3 mmol), cyclohexene-1-boronic acid pinacol ester (5.50 g, 26.4 mmol), and sodium carbonate (5.37 g, 50.6 mmol) were added to a mixed solvent of dioxane (50 mL) and water (10 mL). Pd(dppf)Cl₂·CH₂Cl₂(0.41 g, 0.51 mmol) was added. The system was purged three times with nitrogen, heated to 90°C, and stirred for 16 hrs. The reaction solution was cooled to room temperature, and concentrated to obtain a crude product. The crude product was separated and purified by column chromatography on silica gel to obtain Compound **74-2**.

¹H NMR (400 MHz, DMSO) δ 7.39 (d, *J* = 5.6 Hz, 1H), 7.00 (d, *J* = 5.6 Hz, 1H), 6.01 - 5.98 (m, 1H), 2.32 - 2.26 (m, 2H), 2.19 - 2.12 (m, 2H), 1.71 - 1.65 (m, 2H), 1.63 - 1.56 (m, 2H).

### Step II: Synthesis of Compound 74-3

At room temperature, to H₂O (50 mL), potassium osmate dihydrate (0.42 g, 1.1 mmol), methylsulfonylamide (2.37 g, 24.9 mmol), potassium ferricyanide (22.4 g, 67.9 mmol), (DHQ)₂PHAL (3.5 g, 4.5 mmol), and K₂CO₃ (9.39 g, 67.9 mmol) were added. **74-2** (4.50 g, 22.6 mmol) was dissolved in t-butanol (50 mL), added to the above reaction solution, and reacted for 16 hrs. EA (200 mL) was added, and washed with 15% sodium hydroxide (50 mL x 3). The organic phase was dried over anhydrous Na₂SO₄, and concentrated to obtain a crude product. The crude product was separated and purified by column chromatography on silica gel to obtain Compound **74-3**.

¹H NMR (400 MHz, DMSO) δ 7.27 (d, *J* = 6.0 Hz, 1H), 7.10 (d, *J* = 6.0 Hz, 1H), 4.65 (d, *J* = 1.6 Hz, 1H), 4.40 (d, *J* = 6.8 Hz, 1H), 4.03 - 3.96 (m, 1H), 1.93 - 1.85 (m, 1H), 1.72 - 1.51 (m, 5H), 1.40 - 1.24 (m, 2H).

### Step III: Synthesis of Compound 74-5

At - 40°C, to a solution of **74-3** (2.90 g, 12.5 mmol) in acetonitrile(30 mL), TfOH (2.74 mL, 31.2 mmol) was slowly added dropwise. Then, the dry ice bath was removed, and the reaction solution was naturally warmed to room temperature and reacted for 1 hr. Water (30 mL) was added to the reaction solution and stirred for 10 min. Acetonitrile was removed by concentration under vacuum. The reaction solution was heated to 100°C, and refluxed for 16 hrs. The reaction solution was cooled to room temperature, adjusted to pH 10 with a 15% sodium hydroxide aqueous solution, and extracted with EA (100 mL). The organic phase was washed with water (50 mL x 3) and saturated brine (50 mL), dried over anhydrous Na₂SO₄, and filtered. The filtrate was concentrated under reduced pressure to obtain a crude product. The crude product was added with PE:EA (30 mL, v/v=5/1), stirred for 10 min, and filtered. The filer cake was collected, and dried under vacuum to obtain Compound **74-5**. LCMS: 232.4 (M+H)⁺.

### Step IV: Synthesis of Compound 74-6

At 0°C, **74-5** (2.40 g, 10.4 mmol) and triethyl amine (2.90 mL, 20.9 mmol) were added to THF (30 mL). Chloroacetyl chloride (1.00 mL, 12.6 mmol) was slowly added dropwise, and reacted for 1 hr. Water (30 mL) was added, and the reaction solution was extracted with EA (30 mL x 3). The organic phases were combined, washed with saturated brine (30 mL), dried over anhydrous Na₂SO₄, filtered, and concentrated to obtain a crude product. The crude product was separated and purified by column chromatography on silica gel to obtain Compound **74-6**.

¹H NMR (400 MHz, DMSO) δ 7.72 (s, 1H), 7.30 (d, *J* = 6.0 Hz, 1H), 6.93 (d, *J* = 6.0 Hz, 1H), 5.23 (d, *J* = 5.2 Hz, 1H), 4.33 - 4.24 (m, 2H), 3.91 - 3.81 (m, 1H), 2.80 - 2.71 (m, 1H), 2.04 - 1.95 (m, 1H), 1.73 - 1.56 (m, 3H), 1.45 - 1.23 (m, 3H).

### Step V: Synthesis of Compound 74-7

At room temperature, **74-6** (1.0 g, 3.2 mmol) was dissolved in DCM (20 mL), and then PCC (1.8 g, 8.4 mmol) was added, heated to 40°C, and reacted for 16 hrs. The reaction solution was concentrated to obtain a crude product. The crude product was separated and purified by column chromatography on silica gel to obtain Compound **74-7**. LCMS: 306.0 (M+H)⁺.

### Step VI: Synthesis of Compound 74-8

At 0°C, **74-7** (700 mg, 2.29 mmol) was dissolved in methanol (20 mL), and then sodium borohydride (173 mg, 4.57 mmol) was slowly added batchwise, and reacted for 1 hr. The reaction solution was concentrated to obtain a crude product. The crude product was separated and purified by column chromatography on silica gel, to obtain Compound **74-8**. LCMS: 308.2 (M+H)⁺.

### Step VII: Synthesis of Compound 74-9

At 0°C, to a solution of **74-8** (600 mg, 1.95 mmol) in isopropanol (10 mL), potassium tert-butoxide (546 mg, 4.87 mmol) was added batchwise. After reaction for 10 min, the reaction solution was naturally warmed to room temperature and reacted for 2 hrs. The reaction solution was concentrated to obtain a crude product. Water (10 mL) was added, stirred for 10 min, and filtered. The filer cake was collected, and dried under vacuum to obtain Compound **74-9**. LCMS: 272.2 (M+H)⁺.

### Step VIII: Synthesis of Compound 74 hydrochloride

At 0°C, to a solution of **74-9** (150 mg, 0.552 mmol) in THF (10 mL), a borane dimethyl sulfide complex (276 uL, 2.76 mmol) was slowly added dropwise. The reaction solution was heated to 70°C and refluxed for 16 hrs. The reaction solution was cooled to 0°C, and quenched with methanol (2 mL). After quenching, the reaction solution was stirred for 1 hr. At 0°C, sodium borohydride (21 mg, 0.55 mmol) was slowly added batchwise. The reaction solution was heated to 50°C, and further reacted for 16 hrs. The reaction solution was concentrated to obtain a crude product. Compound **74** hydrochloride was obtained by purification by prep-HPLC. LCMS: 258.4 (M+H)⁺.

¹H NMR (400 MHz, DMSO) δ 11.06 - 11.01 (m, 1H), 8.95 - 8.80 (m, 1H), 7.71 (d, J = 6.0 Hz, 1H), 7.60 (d, J = 6.0 Hz, 1H), 4.14 - 4.02 (m, 2H), 4.00 - 3.95 (m, 1H), 3.29 - 3.19 (m, 1H), 3.02 - 2.95 (m, 1H), 2.82 - 2.71 (m, 1H), 2.03 - 1.95 (m, 1H), 1.86 - 1.80 (m, 1H), 1.67 - 1.58 (m, 3H), 1.46 - 1.35 (m, 1H), 1.03 - 0.91 (m, 1H).

### Example 14: Preparation of Compound 80 hydrochloride

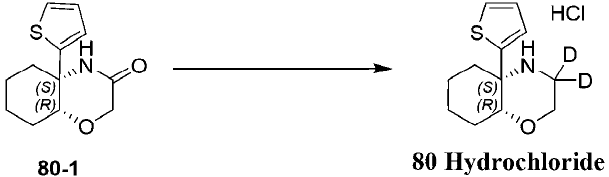

### Step I: Synthesis of Compound 80 hydrochloride

At 0°C, (4aS, 8aR)-4a-(thien-2-yl)hexahydro-2*H*-benzo[*b*][1,4]oxazin-3(4*H*)-one (100 mg, 0.421 mmol, **80-1**) (the synthesis was shown in WO2022237849, Compound **35**) was dissolved in THF (5 mL). Boron trifluoride etherate (0.2 uL, 1.62 mmol) was slowly added dropwise, and reacted for 1 hr. Sodium borodeuteride (53 mg, 1.26 mmol) was slowly added. The reaction solution was heated to 65°C and further reacted for 5 hrs. The reaction solution was cooled to 0°C, and quenched with methanol (3 mL). The reaction solution was directly concentrated to obtain a crude product. Compound **80** hydrochloride was obtained by purification by prep-HPLC. LCMS: 226.2 (M+H)⁺.

¹H NMR (400 MHz, DMSO) δ 9.68 (s, 1H), 9.15 (s, 1H), 7.75 (d, J = 5.2 Hz, 1H), 7.55 - 7.67 (m, 1H), 7.27 - 7.07 (m, 1H), 4.30 (s, 1H), 4.12 - 4.04 (m, 1H), 3.85 - 3.76 (m, 1H), 2.60 - 2.52 (m, 1H), 2.18 - 2.10 (m, 1H), 1.83 - 1.68 (m, 2H), 1.65 - 1.43 (m, 2H), 1.38 - 1.26 (m, 2H).

### Example 15: Preparation of Compound 96 hydrochloride

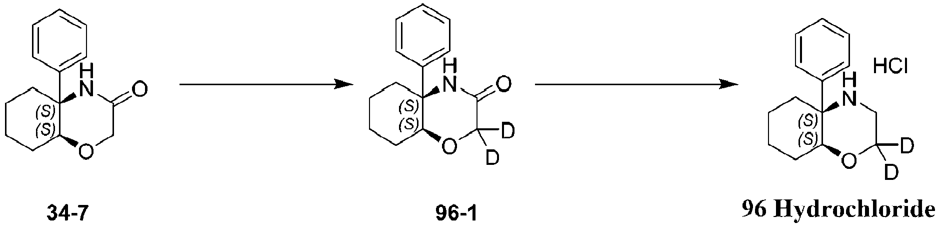

### Step I: Synthesis of Compound 96-1

**34-7** (150 mg, 0.649 mmol) was dissolved in deuterated methanol (2 mL), and then Cs₂CO₃ (1.05g, 3.24 mmol) was added, and reacted at 80°C for 2 hrs. The reaction solution was concentrated. The residue was dissolved in DCM (10 mL), and filtered. The filter cake was washed with DCM (10 mL), and the filtrate was dried over anhydrous Na₂SO₄ and concentrated to obtain Compound **96-1.** LCMS: 234.2 (M+H)⁺.

### Step II: Synthesis of Compound 96 hydrochloride

At 0°C, **96-1** (150 mg, 0.643 mmol) was dissolved in THF (5 mL), and a borane dimethyl sulfide complex (0.321 mL, 3.21 mmol) was slowly added dropwise. The system was heated to 70°C, and reacted for 16 hrs. The reaction solution was cooled to 0°C, and quenched with MeOH (5 mL). Then, NaBH₄ (122 mg, 3.22 mmol) was added. The system was heated to 50°C, and reacted for 16 hrs. The reaction solution was concentrated to remove the solvent. Water (5 ml) was added, and then the reaction solution was extracted with EA (5 mL x 3). The organic layers were combined, washed with saturated brine (5 mL), dried over anhydrous Na₂SO₄, and concentrated to obtain a crude product. The crude product was separated and purified by column chromatography on silica gel. After concentration, 1 N HCl (0.05 mL) was added and freeze dried to obtain Compound **96** hydrochloride. LCMS: 220.2 (M+H)⁺.

¹H NMR (400 MHz, DMSO) δ 7.67 - 7.58 (m, 2H), 7.45 - 7.36 (m, 2H), 7.31 (t, J = 14.4 Hz, 1H), 4.28 (s, 1H), 3.15 - 2.94 (m, 1H), 2.76 - 2.64 (m, 1H), 2.23 (s, 1H), 2.04 - 1.76 (m, 2H), 1.66 - 1.51 (m, 2H), 1.48 - 1.40 (m, 1H), 1.32 - 1.18 (m, 2H).

### Example 16: Preparation of Compound 98 hydrochloride

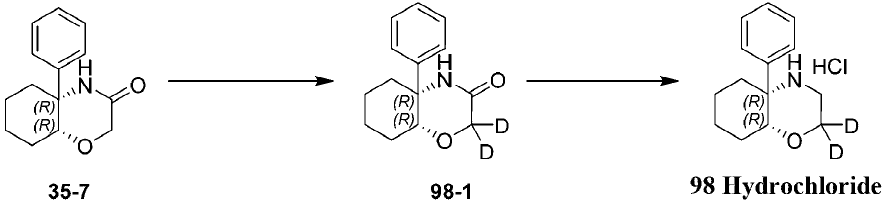

### Step I: Synthesis of Compound 98-1

At room temperature, **35-7** (200 mg, 0.865 mmol) was dissolved in deuterated methanol (2 mL). Cesium carbonate (1.1 g, 3.5 mmol) was added. The system was heated to 80°C, and reacted for 1 hr. The reaction solution was directly rotary dried to obtain a crude product. DCM (10 mL) was added. After filtration, the filtrate was concentrated under reduced pressure to obtain Compound **98-1.** LCMS: 234.4 (M+H)⁺.

### Step II: Synthesis of Compound 98

At 0°C, **98-1** (180 mg, 0.772 mmol) was dissolved in THF (10 mL). A borane dimethyl sulfide complex (380 uL, 3.80 mmol) was slowly added dropwise. The system was heated to 70°C, and reacted for 16 hrs. The reaction solution was cooled to 0°C, and quenched with methanol (5 mL). The reaction solution was further stirred for 1 hr. At 0°C, sodium borohydride (59 mg, 1.6 mmol) was added batchwise to the reaction solution. The reaction solution was heated to 50°C, and further stirred for 16 hrs. The reaction solution was concentrated to obtain a crude product. Compound **98** hydrochloride was obtained by purification by prep-HPLC. LCMS: 220.4 (M+H)⁺.

¹H NMR (400 MHz, DMSO) δ 10.2 - 9.99 (m, 1H), 9.40 - 9.20 (m, 1H), 7.77 - 7.72 (m, 2H), 7.53 - 7.48 (m, 2H), 7.47 - 7.42 (m, 1H), 4.52 - 4.48 (m, 1H), 3.47 - 3.35 (m, 1H), 2.94 - 2.87 (m, 1H), 2.50 - 2.43 (m, 1H), 2.36 - 2.32 (m, 1H), 1.84 - 1.61 (m, 2H), 1.58 - 1.43 (m, 2H), 1.35 - 1.28 (m, 1H), 1.19 - 1.06 (m, 1H).

### Example 17: Preparation of Compound 144 hydrochloride

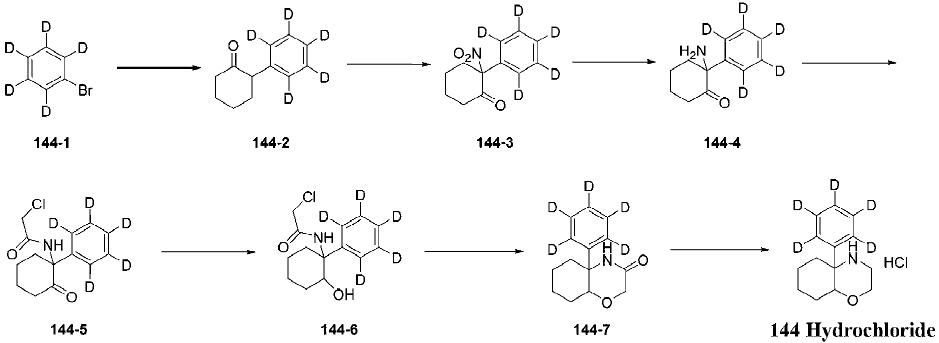

### Step I: Synthesis of Compound 144-2

Under a nitrogen atmosphere, Pd₂(dba)₃ (283 mg, 0.31 mmol), Xantphos (359 mg, 0.62 mmol), and cesium carbonate (22.1 g, 67.87 mmol) were dissolved in dioxane (35 ml), and then deuterated bromobenzene (5 g, 30.85 mmol, **144-1**) and cyclohexanone (6.05 g, 61.70 mmol) were added. The reaction solution was heated to 85°C and refluxed for 20 hrs. The reaction solution was cooled to room temperature, filtered through diatomite, extracted with EA (20 mL x 2), dried over anhydrous Na₂SO₄, and concentrated to obtain a crude product. The crude product was separated and purified by column chromatography on silica gel, to obtain Compound **144-2.** LCMS: 180.0 (M+H)⁺.

### Step II: Synthesis of Compound 144-3

Under a nitrogen atmosphere, ceric ammonium nitrate (36.5 g, 66.60 mmol), copper acetate (4.03 g, 22.20 mmol), and **144-2** (3.98 g, 22.20 mmol) were dissolved in 1,2-dichloroethane (60 ml). The reaction solution was heated to 80°C and refluxed for 12 hrs. The reaction solution was cooled to room temperature, diluted with DCM, and filtered through diatomite. The filter cake was washed with DCM. After concentration, a crude product was obtained. The crude product was separated and purified by column chromatography on silica gel to obtain Compound **144-3**. LCMS: 178.0 (M-NO₂)⁺.

### Step III: Synthesis of Compound 144-4

Under a nitrogen atmosphere, **144-3** (1.64 g, 7.31 mmol) was dissolved in methanol (20 mL), glacial acetic acid (20 mL) was added, and then a zinc powder (2.37 g) was slowly added. The reaction solution was heated to 80°C and refluxed for 12 hrs. The reaction solution was cooled to room temperature, adjusted to pH 10 with NaOH (2 M), and extracted with EA (20 mL x 2). The organic phases were combined, dried over anhydrous Na₂SO₄, and concentrated to obtain a crude product. The crude product was separated and purified by column chromatography on silica gel to obtain Compound **144-4**. LCMS: 195.0 (M+H)⁺.

### Step IV: Synthesis of Compound 144-5

Under a nitrogen atmosphere, **144-4** (1.36 g, 7.0 mmol) in DCM (30 mL) was added with triethyl amine (850 mg, 8.4 mmol). The reaction solution was cooled to 0°C, and chloroacetyl chloride (790 mg, 7.0 mmol) was added dropwise. The reaction solution was heated to room temperature, continuously stirred for 1 hr, and then extracted with EA. The organic phases were combined, dried over anhydrous Na₂SO₄, and concentrated to obtain a crude product. The crude product was separated and purified by column chromatography on silica gel to obtain Compound **144-5**. LCMS: 271.0 (M+H)⁺.

### Step V: Synthesis of Compound 144-7

Under a nitrogen atmosphere, **144-5** (1.22 g, 4.51 mmol) was dissolved in methanol (20 mL). The reaction solution was cooled to 0°C, and sodium borohydride (171 mg, 4.51 mmol) was added. The reaction solution was heated to room temperature, continuously stirred for 1 hr, and then concentrated under reduced pressure to remove the solvent, to obtain **144-6**, which was dissolved in THF (20 mL). The reaction solution was cooled to 0°C, and sodium hydride (216 mg, 5.41 mmol) was added. The reaction solution was heated to room temperature, continuously stirred for 12 hrs, quenched with 1 N HCl, and extracted with DCM. The organic phases were combined, dried over anhydrous Na₂SO₄, and concentrated to obtain a crude product. The crude product was separated and purified by column chromatography on silica gel to obtain Compound **144-7**. LCMS: 237.0 (M+H)⁺.

### Step VI: Synthesis of Compound 144 hydrochloride

Under a nitrogen atmosphere, **144-7** (876 mg, 3.70 mmol) was dissolved in THF (10 mL), and BH₃/Me₂S (9.2 mL, 18.53 mmol) was added dropwise. The reaction solution was heated to 70°C and refluxed for 12 hrs. After cooling to room temperature, MeOH was added dropwise, and then 1 N HCl was added and stirred for 1 hr. The reaction solution was adjusted to pH 8-9 with a saturated NaHCO₃ solution, and extracted with EA. The organic phases were combined, dried over anhydrous Na₂SO₄, and concentrated to obtain a crude product. The residue was purified by prep-HPLC, most of the water was spun off, 1 M HCl was added and freeze-dried to obtain Compound **144** hydrochloride. LCMS: 223.2 (M+H)⁺.

¹H NMR (400 MHz, CDCl₃) δ 10.4 (s, 1H), 9.7 (s, 1H), 4.4 (t, J = 11.4 Hz, 1H), 4.2 (dd, J = 12.0, 4.6 Hz, 1H), 4.0 (dd, J = 12.5, 3.7 Hz, 1H), 2.9 (m, 2H), 2.8 (d, J = 13.0 Hz, 1H), 2.3 (td, J = 13.3, 3.1 Hz, 1H), 2.1 - 1.9 (m, 2H), 1.8 - 1.5 (m, 4H).

### Example 18: Preparation of Compound 145 hydrochloride

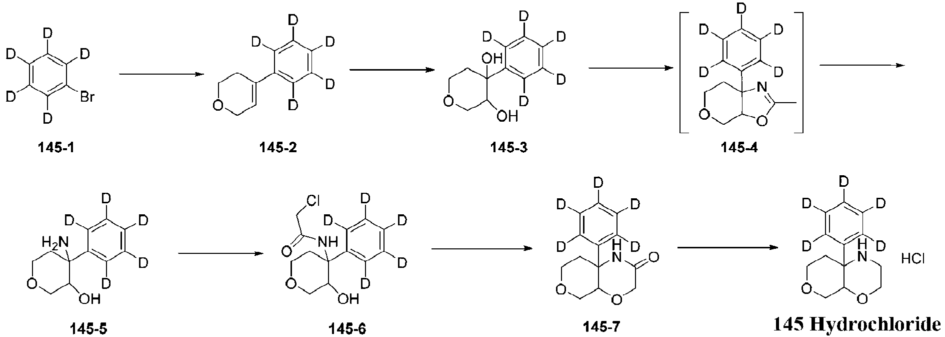

### Step I: Synthesis of Compound 145-2

At room temperature, **145-1**(3.50 g, 21.6 mmol), 3,6-dihydro-2H-pyran-4-boronic acid pinacol ester (5.00 g, 23.8 mmol), and K₂CO₃ (8.97 g, 64.92 mmol) were added to a mixed solvent of dioxane (40 mL) and water (10 mL). Pd(dppf)Cl₂ (0.88 g, 1.08 mmol) was added. The system was purged three times with nitrogen, heated to 80°C, and reacted for 12 hrs. The reaction solution was concentrated to obtain a crude product. The crude product was separated and purified by column chromatography on silica gel to obtain Compound **145-2.**

### Step II: Synthesis of Compound 145-3

At room temperature, **145-2** (3.42 g, 20.72 mmol) was added to a mixed solvent of water (27 mL) and t-butanol (18 mL). Potassium osmate dihydrate (378 mg, 1.03 mmol), K₂CO₃(8.59 g, 62.16 mmol), and potassium ferricyanide (20.46 g, 62.16 mmol) were added, and reacted for 16 hrs. Water (50 mL) was added, and the reaction solution was extracted with EA (100 mL x 2). The organic phases were combined, washed with saturated brine, dried over anhydrous Na₂SO₄, and filtered. The filtrate was concentrated under reduced pressure to obtain a crude product. The crude product was separated and purified by column chromatography on silica gel to obtain Compound **145-3**. LCMS: 182.0 (M-18)⁺.

### Step III: Synthesis of Compound 145-5

At -40°C, to a solution of **145-3** (3.36 g, 16.86 mmol) in acetonitrile(50 mL), TfOH (2.98 mL, 33.72 mmol) was added dropwise. After that, the dry ice bath was removed, and the reaction solution was naturally warmed to room temperature and reacted for 1 hr. Water (50 mL) was added to the reaction solution, and stirred for 10 min. Acetonitrile was removed by concentration under vacuum. The remaining aqueous phase was heated to 100°C and further reacted for 16 hrs. The reaction solution was cooled to room temperature, adjusted to pH 10 with a sodium hydroxide aqueous solution, and extracted with EA (50 mL x 2). The organic phases were combined, washed with saturated brine (30 mL), dried over anhydrous Na₂SO₄, and filtered. The filtrate was concentrated under reduced pressure to obtain a crude product. The crude product was added with PE/EA (20 mL, v/v= 5/1), stirred for 10 min, and filtered. The filer cake was collected, and dried under vacuum to obtain Compound **145-5**. LCMS: 199.0 (M+H)⁺.

### Step IV: Synthesis of Compound 145-6

Under a nitrogen atmosphere, **145-5** (1.80 g, 9.12 mmol) was dissolved in DCM (36 mL), and triethyl amine (1.11 g, 10.94 mmol) was added. The reaction solution was cooled to 0°C, and chloroacetyl chloride (1.13 g, 10.03 mmol) was added dropwise. The reaction solution was warmed to room temperature, stirred for 1 hr, and extracted with EA. The organic phase was dried over anhydrous Na₂SO₄, and concentrated to obtain a crude product. The crude product was separated and purified by column chromatography on silica gel to obtain Compound **145-6**. LCMS: 273.0 (M-H)⁻.

### Step V: Synthesis of Compound 145-7

At 0°C, **145-6** (795 mg, 2.90 mmol) was dissolved in THF (20 mL), and sodium hydride (139 mg, 3.48 mmol, 60%) was slowly added batchwise. The reaction was continued at room temperature for 2 hrs, quenched with a saturated NH₄Cl aqueous solution (5 mL), and extracted with EA (10 mL x 2). The organic phases were combined, washed with saturated brine, dried over anhydrous Na₂SO₄, filtered, and concentrated to obtain a crude product. The crude product was separated and purified by column chromatography on silica gel to obtain Compound **145-7**. LCMS: 239.0 (M+H)⁺.

### Step VI: Synthesis of Compound 145 hydrochloride

Under a nitrogen atmosphere, **145-7** (576 mg, 2.42 mmol) was dissolved in THF (12 mL), and BH₃/Me₂S (6.1 mL, 12.10 mmol) was added dropwise. The reaction solution was heated to 70°C and refluxed for 12 hrs. After cooling to room temperature, the reaction solution was quenched with methanol added dropwise, and then 1 N HCl was added and stirred for 1 hr. The reaction solution was adjusted to pH 8-9 with a saturated NaHCO₃ solution, and extracted with EA. The organic phases were combined, dried over anhydrous Na₂SO₄, and concentrated to obtain a crude product. The residue was purified by prep-HPLC, most of the water was removed by concentration, 1 M HCl was added and freeze-dried to obtain Compound **145** hydrochloride. LCMS: 225.0 (M+H)⁺.

¹H NMR (400 MHz, DMSO) δ 10.27 (s, 1H), 9.43 (s, 1H), 4.44 (d, J = 1.9 Hz, 1H), 4.11 (dd, J = 12.5, 3.8 Hz, 1H), 3.99 - 3.67 (m, 3H), 3.41 (d, J = 12.8 Hz, 1H), 3.15 (td, J = 11.9, 1.8 Hz, 1H), 2.97 (d, J = 13.0 Hz, 1H), 2.80 (td, J = 12.9, 12.4, 4.4 Hz, 1H), 2.35 (d, J = 12.7 Hz, 1H).

### Example 19: Preparation of Compound 158 hydrochloride

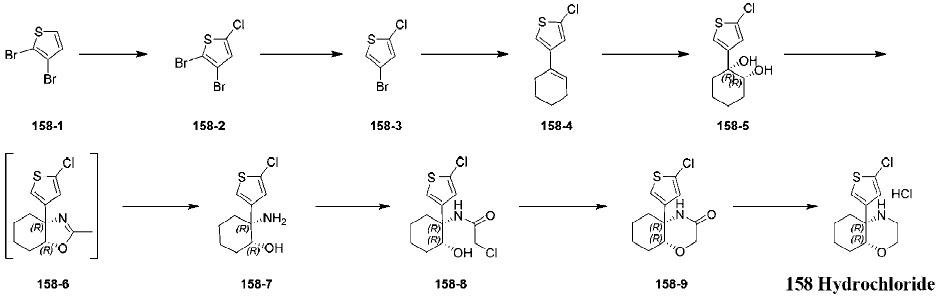

### Step I: Synthesis of Compound 158-2

**158-1** (1.0 g, 4.1 mmol) was dissolved in acetic acid (10 mL), and N-chlorosuccinimide (0.61 g, 4.5 mmol) was added. The reaction solution was heated to 120°C and refluxed for 2 hrs. The reaction solution was cooled to room temperature, water (10 mL) was added, and the reaction solution was extracted with EA (10 mL x 3). The organic layers were combined, washed with saturated brine (10 mL), dried over anhydrous Na₂SO₄, filtered, and concentrated to obtain a crude product. The crude product was separated and purified by column chromatography on silica gel to obtain Compound **158-2.**

¹H NMR (400 MHz, CDCl₃) δ 6.78 (s, 1H).

### Step II: Synthesis of Compound 158-3

**158-2** (1.0 g, 3.6 mmol) was dissolved in acetic acid (10 mL), and a zinc powder (2.37 g, 36.2 mmol) was slowly added. The reaction solution was heated to 100°C, stirred for 16 hrs, and then cooled to room temperature. Water (10 mL) was added, and the reaction solution was extracted with DCM (10 mL x 3). The organic phases were combined, washed with saturated Na₂CO₃ (10 mL), dried over anhydrous Na₂SO₄, filtered and concentrated to obtain Compound **158-3**.

### Step III: Synthesis of Compound 158-4

**158-3** (600 mg, 1.82 mmol, 60% purity), and 2-(cyclohex-1-en-1-yl)-4,4,5,5-tetramethyl-1,3,2-dioxaborane (417 mg, 2.00 mmol) was dissolved in a mixed solution of 1,4-dioxane (10 mL) and H₂O (2 mL). Sodium carbonate (386 mg, 3.64 mmol) was added. The system was purged with nitrogen, and Pd(dppf)Cl₂ (66.7 mg, 0.091 mmol) was added. The reaction solution was heated to 90°C and refluxed for 16 hrs. The reaction solution was filtered, and concentrated to obtain a crude product. The crude product was separated and purified by column chromatography on silica gel to obtain Compound **158-4**.

¹H NMR (400 MHz, DMSO) δ 7.35 (d, J = 1.6 Hz, 1H), 7.20 (d, J = 1.6 Hz, 1H), 6.22 - 6.19 (m, 1H), 1.73 - 1.62 (m, 4H), 1.61 - 1.54 (m, 4H).

### Step IV: Synthesis of Compound 158-5

At room temperature, **158-4** (500 mg, 1.25 mmol, 50% purity) was dissolved in a mixed solution of water (5 mL) and t-butanol (5 mL). Potassium osmate dihydrate (23 mg, 0.063 mmol), methylsulfonylamide (143 mg, 1.51 mmol), potassium ferricyanide (1.24 g, 3.77 mmol), (DHQD)₂PHAL(245 mg, 0.315 mmol), and K₂CO₃(522 mg, 3.77 mmol) were added, and reacted for 16 hrs. A 15% sodium hydroxide aqueous solution (10 mL) was added, and the reaction solution was extracted with EA (10 mL x 3). The organic phases were combined, washed with saturated brine (10 mL), dried over anhydrous Na₂SO₄, filtered, and concentrated to obtain a crude product. The crude product was separated and purified by column chromatography on silica gel to obtain Compound **158-5**.

¹H NMR (400 MHz, DMSO) δ 7.14 (d, J = 1.6 Hz, 1H), 7.11 (d, J = 2.0 Hz, 1H), 4.54 (s, 1H), 4.28 (d, J = 6.4 Hz, 1H), 3.58 - 3.49 (m, 1H), 1.65 - 1.54 (m, 6H), 1.37 - 1.26 (m, 2H).

### Step V: Synthesis of Compound 158-7

At -40°C, to a solution of **158-5** (300 mg, 1.16 mmol) in acetonitrile (5 mL), TfOH (0.28 mL, 3.2 mmol) was added dropwise. The dry ice bath was removed, and the reaction solution was naturally warmed to room temperature and further reacted for 1 hrs. Water (5 mL) was added, and stirred for 10 min. Acetonitrile was removed by concentration under vacuum. The remaining aqueous phase was heated to 100°C and refluxed for 16 hrs. The reaction solution was adjusted to pH 10 with 1N sodium hydroxide aqueous solution, and extracted with EA (10 mL x 3). The organic phases were combined, washed with saturated brine (10 mL), dried over anhydrous Na₂SO₄, filtered, and concentrated to obtain a crude product. The crude product was added with PE:EA=10:1 (5 mL), stirred for 10 min, and filtered. The filer cake was collected, and dried under vacuum to obtain Compound **158-7**. LCMS: 256.0 (M+H)⁺.

### Step VI: Synthesis of Compound 158-8

At 0°C, **158-7** (200 mg, 0.863 mmol) and triethyl amine (0.24 mL, 1.7 mmol) were added to methyl tetrahydrofuran (5 mL). Chloroacetyl chloride (0.082 mL, 1.0 mmol) was slowly added. After reaction for 1 hr, water (5 mL) was added, and the reaction solution was extracted with EA (5 mL x 3). The organic phases were combined, washed with saturated brine (5 mL), dried over anhydrous Na₂SO₄, filtered, and concentrated to obtain a crude product. The crude product was separated and purified by column chromatography on silica gel to obtain Compound **158-8**.

¹H NMR (400 MHz, DMSO) δ 7.58 (s, 1H), 7.10 (d, J = 1.6 Hz, 1H), 7.05 (d, J = 1.6 Hz, 1H), 5.17 (d, J = 5.2 Hz, 1H), 4.28 - 4.24 (m, 2H), 3.59 - 3.48 (m, 1H), 2.84 - 2.73 (m, 1H), 1.70 - 1.53 (m, 4H), 1.43 - 1.32 (m, 2H), 1.26 - 1.19 (m, 1H).

### Step VII: Synthesis of Compound 158-9

At 0 °C, **158-8** (150 mg, 0.487 mmol) was dissolved in isopropanol (5 mL), and potassium tert-butoxide (109 mg, 0.973 mmol) was added. After reaction for 1 hr, a crude product was obtained by concentration to remove most of the solvent. Water (5 mL) was added, and stirred for 10 min. After filtration, the filer cake was collected, and dried under vacuum to obtain Compound **158-9**.

¹H NMR (400 MHz, DMSO) δ 8.40 (s, 1H), 7.25 (s, 2H), 4.09 - 4.04 (m, 1H), 4.01 - 3.95 (m, 2H), 1.93 - 1.73 (m, 3H), 1.69 - 1.52 (m, 2H), 1.46 - 1.37 (m, 3H).

### Step VIII: Synthesis of Compound 158 hydrochloride

At 0°C, **158-9** (100 mg, 0.368 mmol) was dissolved in THF (2 mL). A borane dimethyl sulfide complex (368 uL, 3.68 mmol) was slowly added dropwise. The reaction solution was heated to 70°C and refluxed for 16 hrs. The reaction solution was cooled to 0°C, quenched with methanol (2 mL), further stirred for 1 hr, and then cooled to 0°C. Sodium borohydride (69 mg, 1.8 mmol) was slowly added batchwise. The reaction solution was heated to 50°C, and further reacted for 16 hrs. The reaction solution was concentrated to obtain a crude product. The crude product was separated and purified by column chromatography on silica gel to obtain Compound **158.** Pure water (10 mL) and 1 N dilute hydrochloric acid (100 uL) were added, and freeze dried, to obtain Compound **158** hydrochloride. LCMS: 258.0 (M+H)⁺.

¹H NMR (400 MHz, DMSO) δ 7.68 (s, 1H), 7.41 (d, J = 1.6 Hz, 1H), 4.19 (s, 1H), 4.03 - 3.94 (m, 1H), 3.75 - 3.66 (m, 1H), 3.43 - 3.35 (m, 1H), 2.83 (d, J = 12.8 Hz, 1H), 2.40 - 2.26 (m, 1H), 2.05 - 1.95 (m, 1H), 1.87 - 1.70 (m, 1H), 1.69 - 1.60 (m, 1H), 1.54 - 1.46 (m, 2H), 1.33 - 1.16 (m, 2H).

**Example 20:** The following compounds can be prepared by referring to the preparation method of the above analogues, as shown in Table 16.

**Table 16: Characterization data for compound preparation**

| No. | Structure | Preparation method | LCMS (M+H)⁺ | HNMR |
|---|---|---|---|---|
| Compound **14** mesylate | 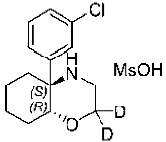 | Following the synthesis process for Compound **96** | 254.4 | ¹H NMR: (400 MHz, DMSO) δ 7.89 - 7.74 (m, 2H), 7.50 - 7.38 (m, 2H), 4.07 - 3.61 (m, 2H), 2.78 - 2.54 (m, 2H), 2.45 - 2.25 (m, 3H), 1.85 - 1.59 (m, 4H), 1.51 - 1.37 (m, 2H), 0.87 - 0.74 (m, 1H). |
| Compound **55** hydrochloride | 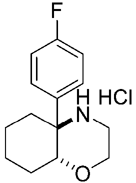 | Following the synthesis process for Compound **46.** SFC retention time RT: 9.108 | 236.2 | ¹H NMR: (400 MHz, DMSO) δ 10.36 (s, 1H), 9.38 (s, 1H), 8.15 - 7.82 (m, 2H), 7.33 (t, J = 8.8 Hz, 2H), 4.21 - 3.88 (m, 3H), 3.06 - 2.90 (m, 1H), 2.80 - 2.63 (m, 1H), 2.62 - 2.54 (m, 1H), 2.09 - 1.95 (m, 1H), 1.92 - 1.83 (m, 1H), 1.81 - 1.70 (m, 1H), 1.69 - 1.61 (m, 1H), 1.59 - 1.51 (m, 1H), 1.48 - 1.35 (m, 1H), 0.89 - 0.77 (m, 1H). |
| Compound **56** hydrochloride | 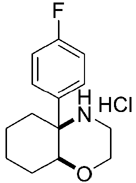 | Following the synthesis process for Compound **34** | 236.2 | ¹H NMR: (400 MHz, DMSO) δ 9.92 (s, 1H), 9.35 (s, 1H), 7.93 - 7.69 (m, 2H), 7.42 - 7.24 (m, 2H), 4.49 (s, 1H), 4.15 - 4.04 (m, 1H), 3.93 - 3.82 (m, 1H), 3.53 - 3.37 (m, 1H), 3.00 - 2.86 (m, 1H), 2.49 - 2.45 (m, 1H), 2.39 - 2.23 (m, 1H), 1.86 - 1.71 (m, 1H), 1.69 - 1.60 (m, 1H), 1.57 - 1.39 (m, 2H), 1.35 - 1.27 (m, 1H), 1.20 - 1.03 (m, 1H). |
| Compound **57** hydrochloride | 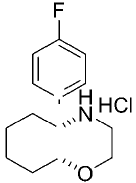 | Following the synthesis process for Compound **35** | 236.4 | ¹H NMR: (400 MHz, DMSO) δ 9.90 - 9.75 (m, 1H), 9.35 - 9.20 (m, 1H), 7.82 - 7.76 (m, 2H), 7.38 - 7.31 (m, 2H), 4.51 - 4.47 (m, 1H), 4.11 - 4.06 (m, 1H), 3.89 - 3.81 (m, 1H), 3.49 - 3.38 (m, 1H), 2.98 - 2.90 (m, 1H), 2.49 - 2.45 (m, 1H), 2.34 - 2.25 (m, 1H), 1.81 - 1.61 (m, 2H), 1.54 - 1.40 (m, 2H), 1.35 - 1.27 (m, 1H), 1.17 - 1.04 (m, 1H). |
| Compound **58** hydrochloride | 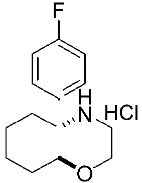 | Following the synthesis process for Compound **45.** SFC retention time RT: 6.280 | 236.2 | ¹H NMR:(400 MHz, DMSO) δ 10.30 (s, 1H), 9.35 (s, 1H), 8.05 - 7.89 (m, 2H), 7.34 (t, *J =* 8.8 Hz, 2H), 4.20 - 3.86 (m, 3H), 3.07 - 2.92 (m, 1H), 2.83 - 2.69 (m, 1H), 2.62 - 2.53 (m, 1H), 2.11 - 1.94 (m, 1H), 1.92 - 1.83 (m, 1H), 1.81 - 1.71 (m, 1H), 1.69 - 1.60 (m, 1H), 1.59 - 1.49 (m, 1H), 1.48 - 1.32 (m, 1H), 0.89 - 0.75 (m, 1H). |
| Compound **59** hydrochloride | 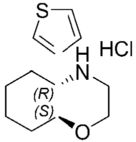 | Following the synthesis process for Compound **74** | 224.4 | ¹H NMR: (400 MHz, DMSO) δ 10.40 - 10.35 (m, 1H), 9.36 (d, *J =* 10.8 Hz, 1H), 7.99 - 7.93 (m, 1H), 7.68 - 7.65 (m, 1H), 7.51 - 7.43 (m, 1H), 4.09 - 3.90 (m, 3H), 3.00 - 2.92 (m, 1H), 2.85 - 2.74 (m, 1H), 2.50 - 2.44 (m, 1H), 2.06 - 1.98 (m, 1H), 1.83 - 1.75 (m, 1H), 1.70 - 1.53 (m, 3H), 1.44 - 1.31 (m, 1H), 1.02 - 0.89 (m, 1H). |
| Compound **60** hydrochloride | 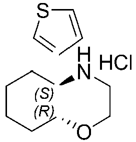 | Following the synthesis process for Compound **74** | 224.4 | ¹H NMR: (400 MHz, DMSO) δ 10.26 (s, 1H), 9.46 - 9.15 (m, 1H), 8.02 - 7.93 (m, 1H), 7.71 - 7.63 (m, 1H), 7.49 - 7.43 (m, 1H), 4.10 - 3.86 (m, 3H), 3.03 - 2.93 (m, 1H), 2.90 - 2.76 (m, 1H), 2.07 - 1.93 (m, 1H), 1.87 - 1.48 (m, 5H), 1.45 - 1.26 (m, 1H), 1.02 - 0.80 (m, 1H). |
| Compound **73** hydrochloride | 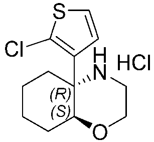 | Following the synthesis process for Compound **74** | 258.4 | ¹H NMR: (400 MHz, DMSO) δ 10.69 (br. s, 1H), 8.89 - 8.75 (m, 1H), 7.71 (d, *J =* 6.0 Hz, 1H), 7.61 (d, *J =* 6.0 Hz, 1H), 4.11 - 4.00 (m, 2H), 3.95 - 3.89 (m, 1H), 3.32 - 3.18 (m, 1H), 3.05 - 2.97 (m, 1H), 2.84 - 2.73 (m, 1H), 1.97 - 1.81 (m, 2H), 1.69 - 1.55 (m, 3H), 1.49 - 1.37 (m, 1H), 1.05 - 0.90 (m, 1H). |
| Compound **75** hydrochloride | 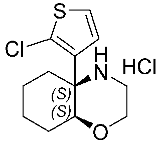 | Following the synthesis process for Compound **34** | 258.0 | ¹H NMR: (400 MHz, DMSO-*d*₆) δ 10.15 (s, 1H), 9.74 (s, 1H), 7.62 (d, *J =* 6.0 Hz, 1H), 7.39 (d, *J =* 6.1 Hz, 1H), 4.73 (s, 1H), 4.13 - 4.00 (m, 1H), 3.85 (td, *J =* 12.3, 2.9 Hz, 1H), 3.38 (s, 2H), 2.94 (dd, *J =* 13.1, 2.6 Hz, 1H), 2.67 - 2.33 (m, 3H), 1.92 - 1.76 (m, 1H), 1.74 - 1.64 (m, 1H), 1.55 - 1.44 (m, 2H), 1.40 - 1.28 (m, 1H), 1.17 (m, 1H). |
| Compound **84** hydrochloride | 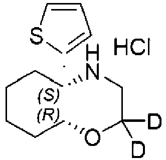 | Following the synthesis process for Compound **96** | 226.0 | ¹H NMR: (400 MHz, DMSO) δ 9.89 (br. s, 1H), 9.28 (br. s, 1H), 7.82 - 7.65 (m, 1H), 7.52 - 7.42 (m, 1H), 7.24 - 7.10 (m, 1H), 4.31 (s, 1H), 3.51 - 3.39 (m, 1H), 3.01 - 2.91 (m, 1H), 2.62 - 2.52 (m, 1H), 2.22 - 2.13 (m, 1H), 1.84 - 1.45 (m, 4H), 1.37 - 1.25 (m, 2H). |
| Compound **92** hydrochloride | 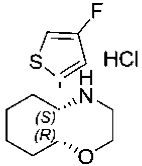 | Following the synthesis process for Compound **35** | 242.2 | ¹H NMR: (400 MHz, DMSO) δ 7.46 - 7.33 (m, 2H), 4.23 (s, 1H), 4.11 - 3.97 (m, 1H), 3.85 - 3.71 (m, 1H), 3.41 - 3.34 (m, 1H), 2.99 - 2.87 (m, 1H), 2.49 - 2.41 (m, 1H), 2.11 - 2.02 (m, 1H), 1.83 - 1.65 (m, 2H), 1.64 - 1.44 (m, 2H), 1.41 - 1.27 (m, 2H). |
| Compound **95** hydrochloride | 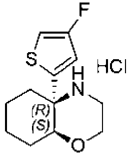 | Following the synthesis process for Compound **34** | 242.0 | ¹H NMR: (400 MHz, DMSO) δ 10.14 (br. s, 1H), 9.57 (br. s, 1H), 7.50 - 7.39 (m, 2H), 4.29 (s, 1H), 4.07 (dd, *J* = 15.6, 3.5 Hz, 1H), 3.85 (td, *J =* 26.8, 2.4 Hz, 1H), 3.40 (s, 1H), 2.97 (d, *J* = 12.8 Hz, 1H), 2.55 - 2.51 (m, 1H), 2.14 (d, *J* = 13.6 Hz, 1H), 1.80 - 1.67 (m, 2H), 1.63 - 1.43 (m, 2H), 1.37 - 1.28 (m, 2H). |
| Compound **98** hydrochloride | 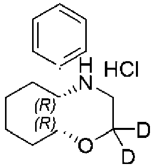 | Following the synthesis process for Compound **96** | 220.4 | ¹H NMR: (400 MHz, DMSO) δ 10.2 - 9.99 (m, 1H), 9.40 - 9.20 (m, 1H), 7.77 - 7.72 (m, 2H), 7.53 - 7.48 (m, 2H), 7.47 - 7.42 (m, 1H), 4.52 - 4.48 (m, 1H), 3.47 - 3.35 (m, 1H), 2.94 - 2.87 (m, 1H), 2.50 - 2.43 (m, 1H), 2.36 - 2.32 (m, 1H), 1.84 - 1.61 (m, 2H), 1.58 - 1.43 (m, 2H), 1.35 - 1.28 (m, 1H), 1.19 - 1.06 (m, 1H). |
| Compound **137** hydrochloride | 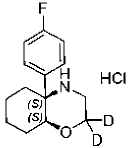 | Following the synthesis process for Compound **96** | 238.2 | ¹H NMR: (400 MHz, DMSO) δ 9.73 (br. s, 1H), 9.24 (br. s, 1H), 7.83 - 7.72 (m, 2H), 7.40 - 7.29 (m, 2H), 4.50 (s, 1H), 3.49 - 3.38 (m, 1H), 2.98 - 2.86 (m, 1H), 2.50 - 2.45 (m, 1H), 2.37 - 2.23 (m, 1H), 1.83 - 1.60 (m, 2H), 1.57 - 1.40 (m, 2H), 1.36 - 1.27 (m, 1H), 1.18 - 1.02 (m, 1H). |
| Compound **141** hydrochloride | 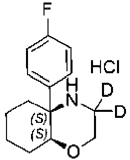 | Following the synthesis process for Compound **40** | 238.2 | ¹H NMR: (400 MHz, MeOD) δ 7.89 - 7.60 (m, 2H), 7.47 - 7.17 (, 2H), 4.54 (s, 1H), 4.23 (d, J = 13.4 Hz, 1H), 3.90 (d, J = 11.7 Hz, 1H), 2.71 (s, 1H), 2.34 (d, J = 11.9 Hz, 1H), 1.88 (s, 1H), 1.68 (dd, J = 35.7, 22.9 Hz, 3H), 1.43 (s, 1H), 1.32 (s, 1H) |
| Compound **157** hydrochloride | 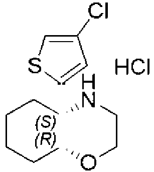 | Following the synthesis process for Compound **35** | 258.0 | ¹H NMR: (400 MHz, DMSO) δ 10.07 (s, 1H), 9.48 (s, 1H), 7.80 (d, *J* = 1.2 Hz, 1H), 7.52 (d, *J =* 1.6 Hz, 1H), 4.31 (s, 1H), 4.07 (dd, *J* = 16.0, 3.6 Hz, 1H), 3.83 (td, *J* = 27.2, 2.5 Hz, 1H), 3.41 (s, 1H), 2.98 (d, *J*= 12.8 Hz, 1H), 2.57 - 2.51 (m, 1H), 2.14 (d, *J* = 13.6 Hz, 1H), 1.82 - 1.66 (m, 2H), 1.62 - 1.43 (m, 2H), 1.38 - 1.26 (m, 2H). |
| Compound **155** hydrochloride | 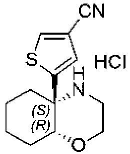 | Following the synthesis process for Compound **35** | 249.4 | ¹H NMR: (400 MHz, CDCl₃) δ 7.78 (d, *J* = 1.2 Hz, 1H), 7.21 - 7.19 (m, 1H), 3.92 - 3.86 (m, 1H), 3.84 - 3.80 (m, 1H), 3.61 - 3.54 (m, 1H), 3.17 - 3.061 (m, 1H), 2.71 - 2.66 (m, 1H), 2.48 - 2.36(m, 1H), 1.87 - 1.80 (m, 1H), 1.64 - 1.40 (m, 6H), 1.34 - 1.29 (m, 1H). |
| Compound **159** hydrochloride | 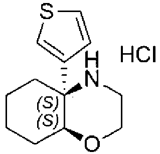 | Following the synthesis process for Compound **34** | 224.2 | ¹H NMR: (400 MHz, DMSO) δ 9.70 (s, 1H), 9.13 (s, 1H), 7.93 - 7.89 (m, 1H), 7.72 - 7.68 (m, 1H), 7.43 - 7.39 (m, 1H), 4.40 - 4.32 (m, 1H), 4.15 - 4.04 (m, 1H), 3.89 - 3.75 (m, 1H), 3.50 - 3.38 (m, 1H), 2.99 - 2.91 (m, 1H), 2.47 - 2.41 (m, 1H), 2.25 - 2.17 (m, 1H), 1.78 - 1.62 (m, 2H), 1.57 - 1.43 (m, 2H), 1.35 - 1.27 (m, 1H), 1.20 - 1.10 (m, 1H) |
| Compound **160** hydrochloride | 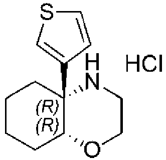 | Following the synthesis process for Compound **34** | 224.2 | ¹H NMR: (400 MHz, DMSO-*d*₆) δ 10.01 (s, 1H), 9.32 (s, 1H), 7.91 (s, 1H), 7.69 (dd, *J* = 5.2, 2.8 Hz, 1H), 7.45 (d, *J =* 5.1 Hz, 1H), 4.35 (s, 1H), 4.07 (dd, *J* = 12.5, 3.9 Hz, 1H), 3.86 (t, *J =* 11.3 Hz, 1H), 3.39 (d, *J =* 8.8 Hz, 2H), 2.92 (d, *J* = 12.8 Hz, 1H), 2.44 (dt, *J* = 12.9, 6.5 Hz, 2H), 2.25 (d, *J* = 12.6 Hz, 1H), 1.70 (dd, *J =* 34.3, 11.4 Hz, 3H), 1.51 (m, 4H), 1.35 - 1.08 (m, 4H). |
| Compound **161** hydrochloride | 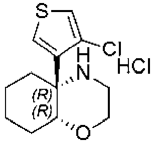 | Following the synthesis process for Compound **35** | 258.0 | ¹H NMR: (400 MHz, DMSO) δ 9.39 (d, *J =* 26. Hz, 1H), 8.14 (d, *J =* 3.6 Hz, 1H), 7.77 (d, *J =* 3.6 Hz, 1H), 4.75 (s, 1H), 4.09 (dd, *J* = 15.6, 3.2 Hz, 1H), 3.80 (td, *J* = 27.2, 2.6 Hz, 1H), 3.44 (s, 1H), 3.00 (d, *J =* 12.8 Hz, 1H), 2.63 (d, *J* = 12.0 Hz, 1H), 2.44 (dd, *J* = 13.2, 3.4 Hz, 1H), 1.84 - 1.48 (m, 4H), 1.40 - 1.17 (m, 3H). |
| Compound **163** mesylate | 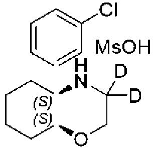 | Following the synthesis process for Compound **40** | 254.2 | ¹H NMR: (400 MHz, DMSO) δ 8.84 (br. s, 1H), 7.72 (s, 1H), 7.67 - 7.62 (m, 1H), 7.57 - 7.49 (m, 2H), 4.46 (s, 1H), 4.10 - 3.99 (m, 1H), 3.79 - 3.65 (m, 1H), 2.49 - 2.38 (m, 1H), 2.31 (s, 3H), 2.21 - 2.02 (m, 1H), 1.90 - 1.76 (m, 1H), 1.71 - 1.63 (m, 1H), 1.56 - 1.41 (m, 2H), 1.38 - 1.28 (m, 1H), 1.20 - 1.07 (m, 1H). |
| Compound **164** mesylate | 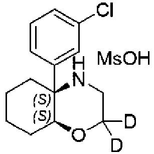 | Following the synthesis process for Compound **96** | 254.2 | ¹H NMR: (400 MHz, DMSO) δ 9.35 - 9.18 (m, 1H), 9.05 - 8.91 (m, 1H), 7.75 (s, 1H), 7.71 - 7.65 (m, 1H), 7.60 - 7.54 (m, 2H), 4.60 - 4.46 (m, 1H), 3.86 - 3.78 (m, 1H), 3.53 - 3.43 (m, 1H), 3.04 - 2.96 (m, 1H), 2.39 (s, 3H), 2.29 - 2.19 (m, 1H), 1.84 - 1.63 (m, 2H), 1.55 - 1.40 (m, 2H), 1.38 - 1.30 (m, 1H), 1.17 - 1.05 (m, 1H). |
| Compound **2-SS** mesylate | 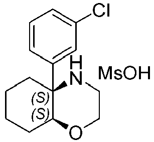 | Following the synthesis process for Compound 42 in WO2022237849 | 252.1 | ¹H NMR: (400 MHz, CDCl₃) δ 9.83 (s, 1H), 9.56 (s, 1H), 7.76 (s, 1H), 7.66 (d, *J* = 7.1 Hz, 1H), 7.44 - 7.32 (m, 2H), 4.39 (s, 0H), 3.94 (s, 2H), 3.04 (s, 1H), 2.51 (dd, *J* = 49.1, 11.3 Hz, 2H), 1.94 (s, 2H), 1.78 - 1.58 (m, 1H), 1.51 (d, *J* = 13.3 Hz, 1H), 1.41 - 1.20 (m, 2H). |
| Compound **2-RR** mesylate | 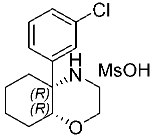 | Following the synthesis process for Compound 41 in WO2022237849 | 252.1 | ¹H NMR: (400 MHz, CDCl₃) δ 9.81 (s, 1H), 9.53 (s, 1H), 7.75 (s, 1H), 7.65 (d, *J* = 7.1 Hz, 1H), 7.36 (d, *J =* 8.9 Hz, 2H), 4.38 (s, 0H), 3.92 (s, 2H), 2.50 (dd, *J* = 49.6, 12.2 Hz, 2H), 1.88 (s, 3H), 1.79 - 1.58 (m, 1H), 1.50 (d, *J* = 13.4 Hz, 1H), 1.42 - 1.17 (m, 2H). |
| Comparative Compound **3-SS** | 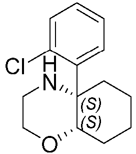 | Following the synthesis process for Compound 44 in WO2022237849 | 252.1 | ¹H NMR: (400 MHz, Chloroform-d) δ 7.68 (dd, *J* = 8.1, 1.7 Hz, 1H), 7.34 (d, *J* = 7.8 Hz, 1H), 7.31 - 7.24 (m, 1H), 7.17 (td, *J* = 7.6, 1.7 Hz, 1H), 4.41 (s, 1H), 4.02 (dt, *J* = 11.2, 7.0 Hz, 1H), 3.47 (d, *J =* 11.1 Hz, 1H), 2.77 - 2.50 (m, 4H), 1.89 - 1.61 (m, 4H), 1.49 - 1.18 (m, 3H). |
| Comparative Compound **3-RR** | 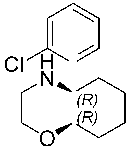 | Following the synthesis process for Compound 43 in WO2022237849 | 252.1 | ¹H NMR: (400 MHz, Chloroform-d) δ 7.70 (dd, *J* = 8.1, 1.7 Hz, 1H), 7.36 (dd, *J* = 7.9, 1.6 Hz, 1H), 7.33 - 7.25 (m, 1H), 7.20 (td, *J* = 7.6, 1.7 Hz, 1H), 4.44 (s, 1H), 4.13 - 3.98 (m, 1H), 3.50 (d, *J=* 11.2 Hz, 1H), 3.08 - 2.35 (m, 5H), 1.93 - 1.62 (m, 3H), 1.36 (m, 3H). |
| Compound **3-RS** | 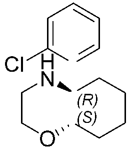 | Following the synthesis process for Compound 1 | 252.1 | ¹H NMR: (400 MHz, Chloroform-d) δ 8.13 (dd, *J* = 8.0, 1.8 Hz, 1H), 7.37 (dd, *J* = 7.7, 1.7 Hz, 1H), 7.20 (dtd, *J* = 23.2, 7.4, 1.7 Hz, 2H), 3.95 - 3.85 (m, 3H), 3.71 (dd, *J =* 13.0, 4.5 Hz, 1H), 3.15 (dd, *J =* 13.7, 3.0 Hz, 1H), 2.73 - 2.59 (m, 2H), 2.53 (dt, *J =* 12.1, 2.2 Hz, 1H), 2.11 (qd, *J=* 12.8, 4.8 Hz, 1H), 1.90 (m, 1H), 1.75 (m, 1H), 1.54 - 1.19 (m, 3H), 0.95 - 0.80 (m, 1H). |
| Racemic hydrochloride of Compound **250** | 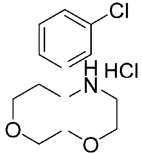 | Following the synthesis process for Compound 145 | 254.0 | ¹H NMR: (400 MHz, DMSO-d6) δ 10.94 (s, 1H), 9.64 (s, 1H), 7.94 (s, 2H), 7.92 - 7.87 (m, 1H), 7.58 - 7.5 3 (m, 2H), 4.29 (dd, *J* = 10.8, 4.8 Hz, 1H), 4.12 (d, *J* = 7.3 Hz, 4H), 3.88 (dd, *J* = 11.1, 4.7 Hz, 1H), 3.82 - 3.74 (m, 4H), 3.69 (t, *J* = 11.0 Hz, 2H), 3.04 (d, *J* = 13.1 Hz, 2H), 2.94 (td, *J* = 12.2, 1.7 Hz, 1H), 2.75 (t, *J* = 11.1 Hz, 2H), 2.60 - 2.55 (m, 1H), 2.44 (m, 1H). |

### Example 21: Preparation of Compounds 177 and 178

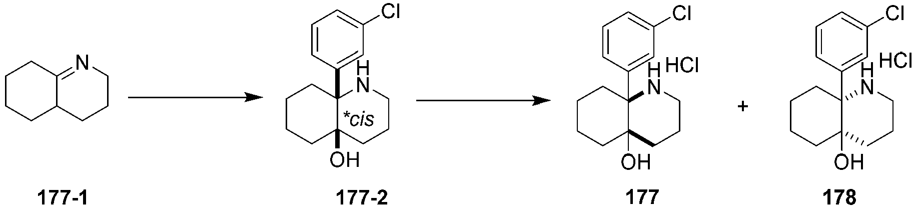

### Step I: Synthesis of Compound 177-2

At 0°C and under nitrogen atmosphere, (3-chlorophenyl)magnesium bromide (29.0 mL, 0.5 M, 14.5 mmol) was added dropwise to a solution of Compound **177-1** (1.0 g, 7.3 mmol) in diethyl ether (30 mL), and slowly heated to 35°C for 6 hrs. A saturated NH₄Cl aqueous solution (50 mL) was added, and the reaction solution was extracted with diethyl ether (50 mL x 3). The organic phases were combined, washed with saturated brine (50 mL), dried over Na₂SO₄, and filtered. The filtrate was concentrated to obtain a crude product. The crude product was purified by Prep-HPLC (Chromatographic column: Xtimate C18, 21.2*250 mm, 5 um; Mobile phase: (0.1 formic acid in) water: acetonitrile; acetonitrile: 5%-35%), and freeze dried, to obtain Compound **177-2** formate. The reaction solution was adjusted to pH = 8-9 with a sodium hydroxide aqueous solution (2 N), and extracted with EA (20 mL x 3). The organic phases were combined, washed with saturated brine (20 mL), dried over anhydrous sodium sulfate, and filtered. The filtrate was concentrated under reduced pressure to obtain Compound **177-2.** LCMS: 266.4 (M+H)⁺.

### Step II: Synthesis of Compounds 177 and 178

Compound **177-2(100** mg, 0.376 mmol) was separated by SFC. A fraction P1 (Rt = 6.48 min) and a fraction P2 (Rt = 7.12 min) were freeze dried. HCl (1 N, 50 uL) and pure water (10 mL) were respectively added, and freeze dried to obtain Compound **177** and Compound **178.**

(SFC conditions: Column: ChiralPak IGS, 250 × 40 mm I.D., 10 µm; Mobile phase: A for supercritical CO2 and B for MeOH; Gradient: 25% B; Flow rate: 130 mL /min).

### Compound 177 (RT: 6.48)

LCMS: 266.4 (M+H)⁺. ¹H NMR: N230984-385-P1 (400 MHz, DMSO) δ 8.95 (br. s, 1H), 8.74 (br. s, 1H), 8.34 (s, 1H), 8.06 (s, 1H), 7.51 - 7.42 (m, 2H), 5.51 (s, 1H), 3.30 - 3.12 (m, 1H), 3.05 - 2.89 (m, 1H), 2.46 - 2.35 (m, 1H), 2.21 - 2.05 (m, 2H), 1.99 - 1.85 (m, 1H), 1.76 - 1.34 (m, 7H), 1.14 - 0.93 (m, 1H).

### Compound 178(RT: 7.12)

LCMS: 266.2 (M+H)⁺. ¹H NMR: N230984-385-P1 (400 MHz, DMSO) δ 8.95 (br. s, 1H), 8.74 (br. s, 1H), 8.34 (s, 1H), 8.06 (s, 1H), 7.49 - 7.43 (m, 2H), 5.51 (s, 1H), 3.29 - 3.15 (m, 1H), 3.04 - 2.88 (m, 1H), 2.46 - 2.37 (m, 1H), 2.21 - 2.05 (m, 2H), 1.98 - 1.86 (m, 1H), 1.75 - 1.37 (m, 7H), 1.15 - 0.94 (s, 1H).

### Example 22: Preparation of Compound 244 hydrochloride

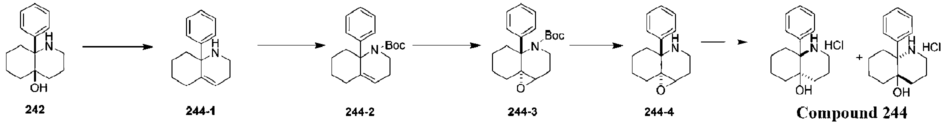

### Step I: Synthesis of Compound 244-1

At 0°C, Compound **242** (530 mg, 2.29 mmol) was dissolved in dichloromethane (15 mL), and then sulfuric acid (610 uL,11.4 mmol) was slowly added dropwise, and reacted at room temperature for 16 hrs. The reaction solution was cooled to 0°C, adjusted to pH 10 with 1N sodium hydroxide aqueous solution, and extracted two times with dichloromethane (20 mL). The organic phases were combined, washed with saturated brine (20 mL), and dried over anhydrous sodium sulfate. After filtration and concentration, a crude product was obtained. The crude product was separated and purified by flash column chromatography on silica gel to obtain Compound **244-1.** LCMS: 214.6 (M+H)⁺.

### Step II: Synthesis of Compound 244-2

At -78°C, to a solution of **244-1** (430 mg, 2.02 mmol) in tetrahydrofuran (10 mL), n-butyl lithium (926 uL, 4.03 mmol) was added, and stirred at -78°C for 0.5 hr. Di-t-butyl dicarbonate (1.21 mL, 3.02 mmol) was added dropwise to the reaction solution. Then, the reaction solution was heated to room temperature and further stirred for 16 hrs. The reaction was quenched with a saturated ammonium chloride aqueous solution (30 mL), and extracted two times with ethyl acetate (30 mL). The organic phases were combined, and dried over anhydrous sodium sulfate. After filtration and concentration, a crude product was obtained. The crude product was separated and purified by flash column chromatography on silica gel to obtain Compound **244-2.** LCMS: 336.5 (M+Na)⁺.

### Step III: Synthesis of Compound 244-3

At 0°C, to a solution of **244-2** (430 mg, 1.37 mmol) in dichloromethane (10 mL), m-chloroperbenzoic acid (550 mg, 2.71 mmol) was added. The reaction solution was then heated to room temperature and stirred for 4 hrs. The reaction was diluted with dichloromethane (50 mL), and quenched with a saturated sodium sulfite aqueous solution (20 mL). The organic phase was washed with a saturated sodium bicarbonate aqueous solution (20 mL), and dried over anhydrous sodium sulfate. After filtration and concentration, a crude product was obtained. The crude product was separated and purified by flash column chromatography on silica gel to obtain Compound **244-3**. LCMS: 352.4 (M+Na)⁺.

### Step IV: Synthesis of Compound 244-4

At room temperature, to a solution of **244-3** (430 mg, 1.30 mmol) in dichloromethane (10 mL), trifluoroacetic acid (2.00 mL, 25.9 mmol) was added. After that, the reaction solution was stirred at room temperature for 2 hrs. The reaction solution was adjusted to pH 10 with a 1 N sodium hydroxide aqueous solution, and extracted two times with dichloromethane (30 mL). The organic phases were combined, washed with saturated brine (20 mL), and dried over anhydrous sodium sulfate. After filtration and concentration, Compound **244-4** was obtained. LCMS: 230.4 (M+H)⁺.

### Step V: Synthesis of racemic hydrochloride of Compounds 244 and 245

At 0°C, to a mixture of lithium aluminium hydride (66.2 mg, 1.74 mmol) in tetrahydrofuran (2 mL), a solution of **244-4** (100 mg, 0.436 mmol) in tetrahydrofuran (2 mL) was added dropwise. After that, the reaction solution was heated to 60°C, stirred for 16 hrs, and quenched with sodium sulfate decahydrate. After filtration and concentration, a crude product was obtained. The crude product was separated and purified by flash column chromatography on silica gel, and then purified by prep-HPLC. Most of the water was removed by concentration, and then 1 M HCl was added and freeze-dried to obtain Compound **244** hydrochloride. LCMS: 232.4 (M+H)⁺.

¹H NMR (400 MHz, DMSO) δ 9.02 - 8.73 (m, 1H), 8.60 - 8.40 (m, 1H), 7.88 - 7.77 (m, 2H), 7.51 - 7.35 (m, 3H), 5.52 (s, 1H), 2.92 - 2.82 (m, 1H), 2.35 - 2.14 (m, 5H), 1.90 - 1.67 (m, 3H), 1.63 - 1.41 (m, 4H), 0.83 - 0.68 (m, 1H).

**Example 23:** The following compounds can be prepared by referring to the preparation method of the above analogues, as shown in Table **17**.

**Table 17: Characterization data for compound preparation**

| No. | Structure | Preparation method | LCMS (M+H)⁺ | HNMR |
|---|---|---|---|---|
| Compound **179** hydrochloride | 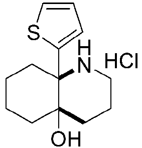 | Following the synthesis process for Compound **177.** | 238.2 | ¹HNMR: (400 MHz, MeOD) δ 7.56 - 7.51 (m, 1H), 7.50 - 7.45 (m, 1H), 7.13 - 7.05 (m, 1H), 3.39 - 3.32 (m, 1H), 3.09 - 3.01 (m, 1H), 2.64 - 2.46 (m, 2H), 2.41 - 2.25 (m, 2H), 1.95 - 1.85 (m, 1H), 1.83 - 1.73 (m, 2H), 1.65 - 1.49 (m, 5H). |
| | | Rt=9.0 min | | |
| Compound **180** hydrochloride | 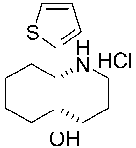 | Following the synthesis process for Compound **178.** | 238.2 | ¹HNMR: (400 MHz, MeOD) δ 7.55 - 7.51 (m, 1H), 7.49 - 7.45 (m, 1H), 7.13 - 7.07 (m, 1H), 3.37 - 3.32 (m, 1H), 3.08 - 3.00 (m, 1H), 2.62 - 2.46 (m, 2H), 2.42 - 2.25 (m, 2H), 1.95 - 1.84 (m, 1H), 1.83 - 1.73 (m, 2H), 1.64 - 1.47 (m, 5H). |
| | | Rt=11.5 min | | |
| Compound **218** hydrochloride | 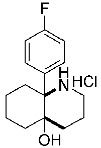 | Following the synthesis process for Compound **177.** | 250.4 | ¹H NMR: (400 MHz, DMSO) δ 9.07 (s, 1H), 8.66 (s, 1H), 8.34 - 8.20 (s, 2H), 7.24 (t, *J* = 8.8 Hz, 2H), 5.45 (s, 1H), 3.30 - 2.77 (m, 2H), 2.48 - 2.37 (m, 1H), 2.27 - 1.37 (m, 10H), 1.20 - 0.88 (m, 1H). |
| | | Rt=7.5 min | | |
| Compound **219** hydrochloride | 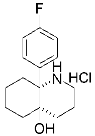 | Following the synthesis process for Compound **178.** | 250.4 | ¹H NMR: (400 MHz, DMSO) δ 9.05 (s, 1H), 8.66 (s, 1H), 8.30 - 8.19 (m, 2H), 7.24 (t, *J* = 8.8 Hz, 2H), 5.45 (s, 1H), 3.30 - 2.80 (m, 2H), 2.48 - 2.41 (m, 1H), 2.23 - 1.40 (m, 10H), 1.15 - 0.88 (m, 1H). |
| | | Rt=9.2 min | | |
| Compound 220 hydrochloride | 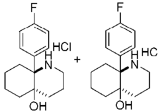 | Following the synthesis process for Compound **244** | 250.4 | **¹H NMR**:δ 9.30 - 9.10 (m, 1H), 8.55 - 8.38 (m, 1H), 8.03 - 7.92 (m, 2H), 7.33 - 7.25 (m, 2H), 5.54 (s, 1H), 2.92 - 2.83 (m, 1H), 2.35 - 2.17 (m, 5H), 1.87 - 1.59 (m, 4H), 1.54 - 1.41 (m, 3H), 0.80 - 0.67 (m, 1H). |
| Compound **242** hydrochloride | 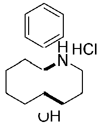 | Following the synthesis process for Compound **177.** | 232.4 | ¹H NMR: (400 MHz, DMSO) δ 9.02 (s, 1H), 8.65 (s, 1H), 8.20 (d, *J* = 7.6 Hz, 2H), 7.47 - 7.30 (m, 3H), 5.39 (s, 1H), 3.35 - 3.82 (m, 2H), 2.48 - 2.36 (m, 1H), 2.27 - 1.38 (m, 10H), 1.14 - 0.88 (m, 1H). |
| | | Rt=8.5 min | | |
| Compound **243** hydrochloride | 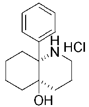 | Following the synthesis process for Compound **178.** | 232.4 | ¹H NMR: (400 MHz, DMSO) δ 9.02 (s, 1H), 8.65 (s, 1H), 8.20 (d, *J* = 7.6 Hz, 2H), 7.47 - 7.30 (m, 3H), 5.39 (s, 1H), 3.35 - 3.82 (m, 2H), 2.48 - 2.36 (m, 1H), 2.27 - 1.38 (m, 10H), 1.14 - 0.88 (m, 1H). |
| | | Rt=11.33 min | | |
| Compound **258** hydrochloride | 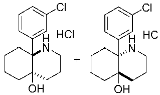 | Following the synthesis process for Compound **244** | 266.4 | ¹H NMR: (400 MHz, DMSO) δ 9.04 - 8.84 (m, 1H), 8.71 - 8.50 (m, 1H), 7.91 - 7.85 (m, 1H), 7.84 - 7.75 (m, 1H), 7.56 - 7.46 (m, 2H), 5.58 (s, 1H), 2.95 - 2.83 (m, 1H), 2.37 - 2.27 (m, 2H), 2.24 - 2.11 (m, 3H), 1.98 - 1.77 (m, 1H), 1.75 - 1.65 (m, 2H), 1.62 - 1.52 (m, 2H), 1.51 - 1.42 (m, 2H), 0.79 - 0.67 (m, 1H). |
| Compound **258a** hydrochloride | 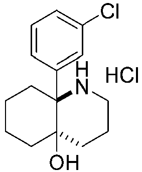 | Following the synthesis process for Compound **244.** | 266.4 | ¹H NMR (400 MHz, DMSO) δ 9.29 - 8.99 (m, 1H), 8.69 - 8.36 (m, 1H), 8.01 - 7.90 (m, 1H), 7.88 - 7.81 (m, 1H), 7.53 - 7.46 (m, 2H), 5.61 (s, 1H), 2.99 - 2.83 (m, 1H), 2.38 - 2.26 (m, 2H), 2.25 - 2.13 (m, 3H), 1.91 - 1.66 (m, 3H), 1.65 - 1.40 (m, 4H), 0.81 - 0.65 (m, 1H). |
| | | After resolution by SFC | | |
| | | RT=3.729 | | |
| Compound **258b** hydrochloride | 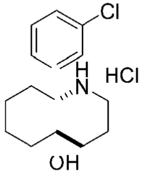 | Following the synthesis process for Compound **244** | 266.4 | ¹H NMR (400 MHz, DMSO) δ 9.29 - 8.90 (m, 1H), 8.66 - 8.41 (m, 1H), 7.99 - 7.90 (m, 1H), 7.88 - 7.78 (m, 1H), 7.53 - 7.46 (m, 2H), 5.59 (s, 1H), 2.97 - 2.83 (m, 1H), 2.38 - 2.25 (m, 2H), 2.24 - 2.11 (m, 3H), 1.89 - 1.66 (m, 3H), 1.64 - 1.40 (m, 4H), 0.81 - 0.67 (m, 1H). |
| | | After resolution by SFC | | |
| | | RT=4.409 | | |
| Compound **167a** hydrochloride | 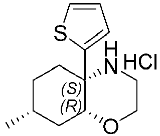 | Following the synthesis process for Compound **35.** | 238.2 | ¹H NMR: (400 MHz, DMSO) δ 9.99 - 9.44 (m, 2H), 7.67 (d, *J* = 4.4 Hz, 1H), 7.56 - 7.46 (m, 1H), 7.17 - 7.10 (m, 1H), 4.35 - 4.26 (m, 1H), 4.07 - 3.96 (m, 1H), 3.78 - 3.67 (m, 1H), 3.17 (s, 1H), 2.94 (s, 1H), 2.52 (s, 1H), 2.09 (s, 1H), 1.97 - 1.82 (m, 1H), 1.77 - 1.49 (m, 4H), 1.03 (d, *J=* 6.4 Hz, 3H). |
| Compound **167b** hydrochloride | 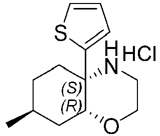 | Following the synthesis process for Compound **35.** | 238.2 | ¹H NMR: (400 MHz, DMSO) δ 9.97 (br. s, 1H), 9.29 (br. s, 1H), 7.74 (dd, *J* = 6.0, 1.2 Hz, 1H), 7.47 (dd, *J* = 4.8, 1.2 Hz, 1H), 7.21 - 7.15 (m, 1H), 4.32 (s, 1H), 4.13 - 3.99 (m, 1H), 3.91 - 3.78 (m, 1H), 3.48 - 3.37 (m, 1H), 3.01 - 2.89 (m, 1H), 2.65 - 2.55 (m, 1H), 2.25 - 2.15 (m, 1H), 1.81 - 1.65 (m, 3H), 1.32 - 1.21 (m, 2H), 1.06 - 0.93 (m, 1H), 0.73 (d, *J* = 6.0 Hz, 3H). |
| Compound **32** hydrochloride | 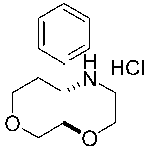 | Following the synthesis process for Compound **17 hydrochloride.** | 220.2 | ¹H NMR: (400 MHz, DMSO) δ 10.76 (s, 1H), 9.49 (s, 1H), 7.90 (d, *J =* 7.2 Hz, 2H), 7.56 - 7.41 (m, 3H), 4.30 - 4.25 (m, 1H), 4.12 - 4.09 (m, 2H), 3.91 - 3.85 (m, 1H), 3.79 - 3.70 (m, 2H), 3.01 (d, *J =* 13.2 Hz, 1H), 2.92 (t, *J* = 11.6 Hz, 1H), 2.77 - 2.66 (m, 1H), 2.59 - 2.53 (m, 1H), 2.47 - 2.38 (m, 1H). |
| | | Rt=5.43 min | | |
| Compound **29** hydrochloride | 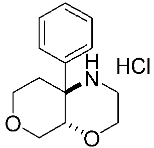 | Following the synthesis process for Compound **13 hydrochloride.** | 220.2 | ¹H NMR: (400 MHz, DMSO) δ 10.74 (s, 1H), 9.48 (s, 1H), 7.92 - 7.87 (m, 2H), 7.54 - 7.43 (m, 3H), 4.27 (dd, *J =* 11.2, 4.8 Hz, 1H), 4.13 - 4.06 (m, 2H), 3.87 (dd, *J =* 11.2, 4.8 Hz, 1H), 3.78 - 3.71 (m, 2H), 3.05 - 2.98 (m, 1H), 2.96 - 2.88 (m, 1H), 2.76 - 2.67 (m, 1H), 2.58 - 2.53 (m, 1H), 2.46 - 2.37 (m, 1H). |
| | | Rt=6.43 min | | |
| Compound **51** hydrochloride | 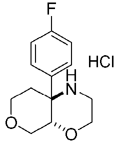 | Following the synthesis process for Compound **13 hydrochloride.** | 238.2 | ¹H NMR: (400 MHz, MeOD) δ 7.72 - 7.62 (m, 2H), 7.29 - 7.19 (m, 2H), 4.21 - 4.12 (m, 1H), 3.92 - 3.76 (m, 3H), 3.63 - 3.54 (m, 1H), 3.49 - 3.42 (m, 1H), 3.08 - 3.02 (m, 1H), 2.95 - 2.84 (m, 1H), 2.31 - 2.22 (m, 1H), 1.21 - 1.18 (m, 2H). |
| | | Rt=18.5 min | | |
| Compound **54** hydrochloride | 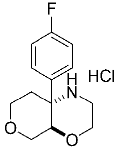 | Following the synthesis process for Compound **17 hydrochloride.** | 238.2 | ¹H NMR: (400 MHz, MeOD) δ 7.72 - 7.61 (m, 2H), 7.30 - 7.17 (m, 2H), 4.19 - 4.10 (m, 1H), 3.91 - 3.75 (m, 3H), 3.66 - 3.54 (m, 1H), 3.50 - 3.44 (m, 1H), 3.07 - 3.01 (m, 1H), 2.95 - 2.87 (m, 1H), 2.30 - 2.23 (m, 1H), 1.20 - 1.18 (m, 2H). |
| | | Rt=14.5 min | | |
| Compound 259 hydrochloride | 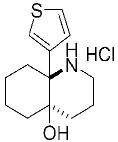 | Following the synthesis process for Compound **177.** | 238.2 | ¹H NMR: (400 MHz, DMSO) δ 9.27 - 8.87 (m, 1H), 8.56 - 8.35 (m, 1H), 7.61 (d, *J* = 4.8 Hz, 1H), 7.50 - 7.42 (m, 1H), 7.12 - 7.03 (m, 1H), 5.79 (s, 1H), 3.27 - 3.07 (m, 1H), 2.96 - 2.82 (m, 1H), 2.46 - 2.25 (m, 2H), 2.23 - 2.00 (m, 1H), 1.84 - 1.59 (m, 3H), 1.54 - 1.38 (m, 4H), 1.37 - 1.19 (m, 2H). |
| | | Rt = 12.83 min | | |
| Compound 260 hydrochloride | 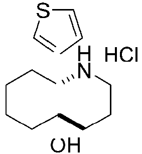 | Following the synthesis process for Compound **177.** | 238.4 | ¹H NMR: (400 MHz, DMSO) δ 9.22 - 8.96 (m, 1H), 8.54 - 8.24 (m, 1H), 7.66 - 7.55 (m, 1H), 7.49 - 7.42 (m, 1H), 7.12 - 7.00 (m, 1H), 5.79 (s, 1H), 3.24 - 3.09 (m, 1H), 3.00 - 2.82 (m, 1H), 2.44 - 2.25 (m, 2H), 2.16 - 2.01 (m, 1H), 1.79 - 1.57 (m, 3H), 1.55 - 1.38 (m, 4H), 1.37 - 1.21 (m, 2H). |
| | | Rt = 9.83 min | | |
| Comparative Compound A | 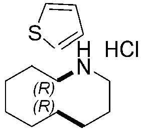 | See literature: J. Org. Chem. 1962, 27, 11, 3882-3885. | 222.4 | ¹H NMR: (400 MHz, MeOD) δ 7.63 - 7.59 (m, 1H), 7.42 - 7.38 (m, 1H), 7.17 - 7.14 (m, 1H), 2.64 - 2.53 (m, 2H), 2.16 - 2.09 (m, 2H), 2.05 - 2.00 (m, 2H), 1.86 - 1.74 (m, 4H), 1.64 - 1.56 (m, 3H), 1.51 - 1.41 (m, 2H). |
| | | Rt = 14.2 min | | |
| Comparative Compound B | 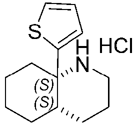 | See literature: J. Org. Chem. 1962, 27, 11, 3882-3885. | 222.4 | ¹H NMR: (400 MHz, MeOD) δ 7.64 - 7.58 (m, 1H), 7.41 - 7.37 (m, 1H), 7.19 - 7.14 (m, 1H), 2.62 - 2.51 (m, 2H), 2.16 - 2.07 (m, 2H), 2.04 - 2.01 (m, 2H), 1.88 - 1.74 (m, 4H), 1.64 - 1.57 (m, 3H), 1.49 - 1.39 (m, 2H). |
| | | Rt = 16.2 min | | |
| PDHQ (SR or RS) | 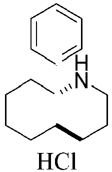 | Based on J. Med. Chem. 1992, V35, 1634-1638 | 216.3 | ¹H NMR: (400 MHz, DMSO) δ 9.57 (s, 1H), 9.06 (s, 1H), 7.82 (d, *J* = 7.6 Hz, 2H), 7.60 - 7.37 (m, 3H), 3.01 - 2.98 (m, 1H), 2.35 - 2.14 (m, 3H), 2.13 - 1.85 (m, 4H), 1.74 - 1.55 (m, 5H), 1.41 - 1.37 (m, 1H), 0.87 - 0.80 (m, 1H). |
| | | Rt=4.028 | | |
| PDHQ (RS or SR) | 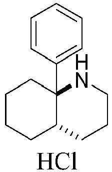 | Based on J. Med. Chem. 1992, V35, 1634-1638 | 216.3 | ¹H NMR: (400 MHz, DMSO) δ 9.56 (s, 1H), 9.03 (s, 1H), 7.80 (s, 2H), 7.48 - 7.39 (m, 3H), 3.00 - 2.95 (m, 1H), 2.32 - 2.19 (m, 3H), 2.09 - 1.87 (m, 4H), 1.80 - 1.51 (m, 5H), 1.44 - 1.31 (m, 1H), 0.89 - 0.74 (m, 1H). |
| | | RT=4.678 | | |
| PDHQ (SS or RR) | 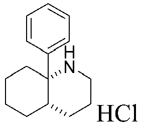 | Based on J. Med. Chem. 1992, V35, 1634-1638 | 216.3 | ¹H NMR: (400 MHz, DMSO) δ 9.16 (br. s, 2H), 7.65 (d, *J* = 7.6 Hz, 2H),7.54 - 7.45 (m, 2H),7.43 - 7.35 (m, 1H), 3.28 - 3.03 (m, 1H), 2.95 - 2.75 (m, 1H),2.72 - 2.61 (m, 1H), 2.35 - 2.10 (m, 1H), 2.09 - 1.4 (m, 9H), 1.38 - 1.05 (m, 2H). |
| | | Rt=1.274 | | |
| PDHQ (RR or SS) | 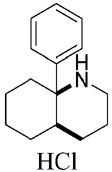 | Based on J. Med. Chem. 1992, V35, 1634-1638 | 216.3 | ¹H NMR: (400 MHz, DMSO) δ 9.00 (br. s, 1H), 8.00 - 7.73 (m, 2H), 7.71 - 7.35 (m, 3H), 3.16 - 2.92 (m, 1H), 2.85 - 2.53 (m, 2H), 2.35 - 0.91 (m, 13H). |
| | | Rt=0.524 | | |

### Example 24: Preparation of Compound 62 hydrochloride and Compound 68 hydrochloride

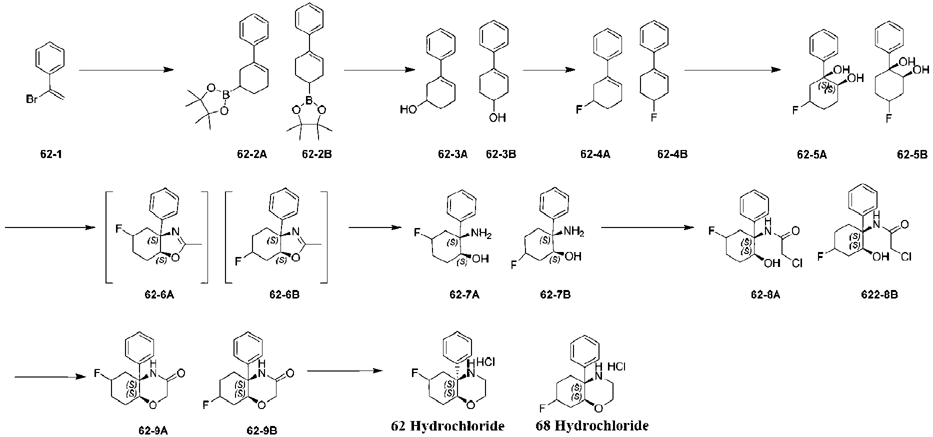

### Step I: Synthesis of Compounds 62-2A and 62-2B

At room temperature, **62-1** (20.0 g, 109 mmol), vinyl boronic acid pinacol ester (92.5mL, 546 mmol), and potassium phosphate (69.6 g, 328 mmol) were added to a mixed solvent of dioxane (200 mL) and water (4 mL). Palladium acetate (1.23 g, 5.46 mmol) and Sphos (4.49 g, 10.9 mmol) were added to the reaction solution. The system was purged with nitrogen, heated to 50°C, and reacted for 2 hrs. The reaction solution was heated to 120°C, and further reacted for 16 hrs. After filtration, the filtrate was concentrated to obtain a crude product of mixed **62-2A** and **62-2B.**

### Step II: Synthesis of Compounds 62-3A and 62-3B

At 0°C, a sodium hydroxide solution (112 mL, 224 mmol) was added to a solution of mixed **62-2A** and **62-2B** (32.0 g) in tetrahydrofuran (500 mL), and then 30% hydrogen peroxide (95.7 g, 844 mmol) was slowly added dropwise. After that, the reaction solution was further stirred at 0°C for 2 hrs, diluted with ethyl acetate (300 mL), quenched with a saturated sodium thiosulfate solution, and extracted with ethyl acetate (100 mL). The organic phases were combined, and dried over anhydrous sodium sulfate. After filtration, the filtrate was concentrated under reduced pressure to obtain a crude product. The crude product was purified by column chromatography on silica gel, to obtain a light yellow solid. The light yellow solid was slurried in petroleum ether (30 mL). After filtration, the filter cake was collected and dried to obtain **63-3B.** The filtrate was concentrated to obtain a 1:1 mixture of **62-2A** and **62-2B.**

**63-3B** :1HNMR (400 MHz, CDCl₃) δ 7.40 - 7.37 (m, 2H), 7.34 - 7.28 (m, 2H), 7.25 - 7.21 (m, 1H), 6.02 - 5.98 (m, 1H), 4.11 - 4.02 (m, 1H), 2.66 - 2.48 (m, 3H), 2.27 - 2.17 (m, 1H), 2.05 - 1.99 (m, 1H), 1.88 - 1.77 (m, 1H).

### Step III: Synthesis of Compounds 62-4A and 62-4B

At room temperature, the mixture (2.0 g) of **62-2A** and **62-2B** was dissolved in dichloromethane (40 mL). After cooling to 0°C, BAST (4.20 mL, 22.8 mmoL) was slowly added dropwise to the above reaction solution. After that, the reaction solution was further stirred at 0°C for 2 hrs, adjusted to pH 8 with a saturated sodium bicarbonate aqueous solution, and extracted with ethyl acetate (50 mL). The organic phases were combined, washed with saturated brine (50 mL), and dried over anhydrous sodium sulfate. After filtration, the filtrate was concentrated under reduced pressure to obtain a crude product. The crude product was separated and purified by column chromatography to obtain a mixture of **62-4A** and **62-4B**.

¹HNMR (400 MHz, CDCl₃) δ 7.40 - 7.36 (m, 2H), 7.34 - 7.30 (m, 2H), 7.26 - 7.22 (m, 1H), 6.15 - 6.10 (m, 1H), 5.98 - 5.93 (m, 1H), 5.14 - 4.88 (m, 1H), 2.87 - 2.59 (m, 2H), 2.53 - 2.25 (m, 2H), 2.15 - 1.92 (m, 2H).

### Step IV: Synthesis of Compounds 62-5A and 62-5B

At room temperature, potassium carbonate (1.65 g, 11.9 mmol), potassium ferricyanide (3.92 g, 11.9 mmol), methylsulfonylamide (415 mg, 4.36 mmol), potassium osmate dihydrate (73 mg, 0.20 mmol), and (DHQ)₂PHAL (619 mg, 0.795 mmol) were added to water (10 mL). The mixture (700 mg) of **62-4A** and **62-4B** was dissolved in t-butanol (6 mL), added to the above reaction solution, and stirred at room temperature for 16 hrs. The reaction solution was diluted with ethyl acetate (50 mL), and washed with water (20 mL). The organic phase was dried over anhydrous sodium sulfate. After filtration, the filtrate was concentrated under reduced pressure to obtain a crude product. The crude product was separated and purified by column chromatography to obtain a mixture (730 mg) of **62-5A** and **62-5B**.

¹HNMR (400 MHz, CDCl₃) δ 7.54 - 7.44 (m, 4H), 7.43 - 7.35 (m, 4H), 7.32 - 7.26 (m, 2H), 5.13 - 5.09 (m, 1H), 5.06 - 4.97 (m, 1H), 4.95 - 4.84 (m, 1H), 4.77 - 4.56 (m, 1H), 4.41 - 4.34 (m, 1H), 4.10 - 4.03 (m, 1H), 4.03 - 3.94 (m, 1H), 3.11 (d, J = 13.2 Hz, 1H), 2.69 - 2.68 (m, 1H), 2.67 - 2.66 (m, 1H), 2.41 - 2.21 (m, 3H), 2.12 - 1.79 (m, 8H), 1.71 - 1.60 (m, 2H), 1.55 - 1.54 (m, 1H).

### Step V: Synthesis of Compounds 62-7A and 62-7B

At -40°C, to a solution of the mixture (730 mg) of **62-5A** and **62-5B** in acetonitrile (20 mL), TfOH (670 µL, 7.63 mmol) was added dropwise. The dry ice bath was removed, and the reaction solution was naturally warmed to room temperature and reacted for 1 hr. Water (20 mL) was added to the reaction solution, and stirred for 10 min. Acetonitrile was removed by concentration under vacuum. The reaction solution was heated to 100°C, and refluxed for 16 hrs. The reaction solution was cooled to room temperature, adjusted to pH 10 with a 15% sodium hydroxide aqueous solution, and extracted with EA (50 mL). The organic phase was washed with water (50 mL x 3) and saturated brine (50 mL), and dried over anhydrous sodium sulfate. After filtration, the filtrate was concentrated under reduced pressure to obtain a crude product. The crude product was separated and purified by column chromatography to obtain a mixture of **62-7A** and **62-7B**. LCMS: 210.4 (M+H)⁺.

### Step VI: Synthesis of Compounds 62-8A and 62-8B

At 0°C, the mixture (300 mg) of **62-7A** and **62-7B** and triethyl amine (400 µL, 2.88 mmol) were added to tetrahydrofuran (10 mL). Chloroacetyl chloride (100 mL, 1.25 mmol) was slowly added dropwise, and then the reaction solution was further stirred at 0°C for 1 hr. Water (10 mL) was added, and the reaction solution was extracted with EA (30 mL x 3). The organic phases were combined, washed with saturated brine (30 mL), and dried over anhydrous sodium sulfate. After filtration and concentration, a crude product was obtained. The crude product was separated and purified by column chromatography to obtain a mixture of **62-8A** and **62-8B**. LCMS: 308.2 (M+Na)⁺.

### Step VII: Synthesis of Compounds 62-9A and 62-9B

At 0°C, potassium tert-butoxide (149 mg, 1.33 mmol) was added batchwise to a solution of the mixture (190 mg) of **62-8A** and **62-8B** in propanol (3 mL). The reaction solution was naturally warmed to room temperature and stirred for 2 hrs. The reaction solution was concentrated to obtain a crude product. The reaction solution was diluted with water (10 mL) and extracted with ethyl acetate (20 mL). The organic phases were combined, dried over anhydrous sodium sulfate, and concentrated to obtain a mixture of **62-9A** and **62-9B**. LCMS: 250.2 (M+H)⁺.

### Step VIII: Synthesis of Compound 62 hydrochloride and Compound 68 hydrochloride

At 0°C, the mixture (150 mg) of **62-9A** and **62-9B** was dissolved in THF (10 mL), and then a borane dimethyl sulfide complex (600 uL, 6.00 mmol) was added, heated to 70°C and refluxed for 16 hrs. After cooling to 0°C, the reaction solution was quenched with methanol (5 mL). At 0°C, sodium borohydride (45 mg, 1.2 mmol) was slowly added batchwise. The reaction solution was heated to 50°C, and further stirred for 16 hrs. The reaction solution was concentrated to obtain a crude product. The crude product was purified by SFC. A first fraction (Rt=12.0 min) was collected, and concentrated under reduced pressure. Compound **62** was obtained after freezing drying with pure water. LCMS: 236.3 (M+H)⁺.

¹H NMR (400 MHz, MeOD) δ 7.73 - 7.68 (m, 2H), 7.59 - 7.49 (m, 3H), 4.71 - 4.57 (m, 2H), 4.23 - 4.16 (m, 1H), 3.83 (td, J = 12.4, 2.4 Hz, 1H), 3.65 - 3.55 (m, 1H), 3.09 - 2.97 (m, 2H), 2.37 - 2.24 (m, 2H), 2.16 - 2.06 (m, 1H), 2.04 - 1.86 (m, 1H), 1.63 - 1.44 (m, 1H).

A second fraction (Rt=14.0 min) was collected, and concentrated under reduced pressure. Compound **68** was obtained after freezing drying with pure water. LCMS: 236.3 (M+H)⁺.

¹H NMR (400 MHz, MeOD) δ 7.71 - 7.66 (m, 2H), 7.52 - 7.42 (m, 3H), 5.10 - 4.95 (m, 1H), 4.68 - 4.64 (m, 1H), 4.23 - 4.16(m, 1H), 3.94 - 3.86 (m, 1H), 3.62 - 3.54 (m, 1H), 3.10 - 3.03(m, 1H), 2.94 - 2.86 (m, 1H), 2.85 - 2.70 (m, 1H), 2.11 - 1.88 (m, 3H), 1.77 - 1.69 (m, 1H).

(SFC conditions: Column: ChiralPak OD-H, 250 × 4.6mm I.D., 5µm; Mobile phase: A for n-hexane and B for ethanol; Gradient: 80% B; Flow rate: 1.0 mL /min).

### Example 25: Preparation of Compound 230 hydrochloride and Compound 231 hydrochloride

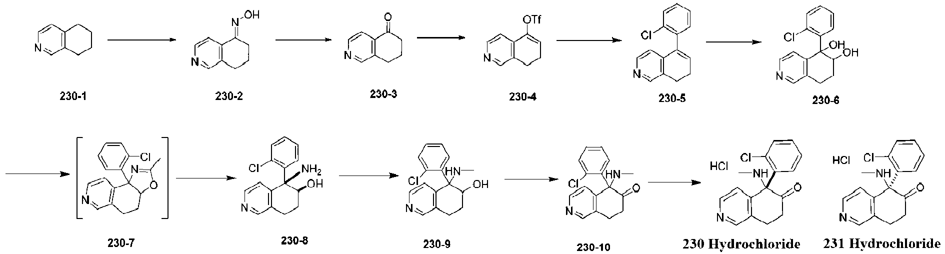

### Step I: Synthesis of Compound 230-2

At room temperature, **230-1** (11.5 g, 86.3 mmol) was dissolved in THF (300 mL), and potassium tert-butoxide (19.4 g, 174 mmol) was added to the reaction solution, and stirred at room temperature for 12 hrs. Then, tert-butyl nitrite (30.8 mL, 259 mmol) was added to the reaction solution and further reacted at room temperature for 12 hrs. The reaction solution was quenched with water (200 mL), and extracted with ethyl acetate (200 mL x 2). The organic phases were combined, washed with saturated brine (100 mL), and dried over anhydrous sodium sulfate. After filtration and concentration, a crude product was obtained. The crude product was slurried in ethanol (50 mL) and water (50 mL) to obtain **230-2.**

¹H NMR (400 MHz, DMSO) δ 11.62 (s, 1H), 8.43 (s, 1H), 8.36 (d, *J* = 5.2 Hz, 1H), 7.68 (d, *J* = 5.2 Hz, 1H), 2.75 - 2.69 (m, 2H), 2.66 (t, *J* = 6.6 Hz, 2H), 1.86 - 1.71 (m, 2H).

### Step II: Synthesis of Compound 230-3

**230-2** (8 g, 49.325 mmol) was dissolved in a mixed solution of acetone (100 mL) and HCl (2M, 60 mL). The reaction solution was heated to 100°C, refluxed for 12 hrs, and concentrated under reduced pressure to obtain a crude product, which was slurried in ethanol. The obtained solid was dissolved in a saturated sodium bicarbonate solution (100 mL), and extracted with ethyl acetate (100 mL x 2). The organic phases were combined, washed with saturated brine (100 mL), and dried over anhydrous sodium sulfate. After filtration and concentration, a crude product was obtained. The crude product was purified by column chromatography to obtain **230-3**.

¹H NMR (400 MHz, CDCl₃) δ 8.65 (d, *J* = 0.4 Hz, 1H), 8.61 (d, *J* = 5.2 Hz, 1H), 7.74 (d, *J* = 5.2 Hz, 1H), 2.96 (t, *J* = 6.0 Hz, 2H), 2.74 - 2.58 (m, 2H), 2.26 - 2.08 (m, 2H).

### Step III: Synthesis of Compound 230-4

At -78°C, to a solution of **230-3** (4.00 g, 27.8 mmol) in THF (50 mL), LDA (19.5 mL, 39.0 mmol) and 1,1,1-trifluoro-N-phenyl-N-((trifluoromethyl)sulfonyl)methansulfonamide (12.9 g, 36.2 mmol) were added. After that, the reaction solution was slowly heated to 25°C, stirred for 2 hrs, quenched with a saturated ammonium chloride solution (200 mL), and extracted with ethyl acetate (200 mL x 2). The organic phases were combined, washed with saturated brine (100 mL), and dried over anhydrous sodium sulfate. After filtration and concentration, a crude product was obtained. The crude product was purified by column chromatography to obtain **230-4**.

¹H NMR (400 MHz, CDCl₃) δ 8.55 (d, *J* = 5.2 Hz, 1H), 8.44 (d, *J* = 0.8 Hz, 1H), 7.20 (d, *J* = 5.2 Hz, 1H), 6.26 (t, *J* = 4.8 Hz, 1H), 2.89 (t, *J* = 8.4 Hz, 2H), 2.64 - 2.57 (m, 2H).

### Step IV: Synthesis of Compound 230-5

At room temperature, Na₂CO₃ (5.01 g, 47.2 mmol) and Pd(dppf)Cl₂ (0.35 g, 0.473 mmol) were added to a mixed solution of **230-4** (4.30 g, 15.7 mmol) and (2-chlorophenyl)boronic acid (3.70 g, 23.6 mmol) in 1,4-dioxane (50 mL) and water (5 mL). The reaction solution was heated to 95°C refluxed for 18 hrs, quenched with a sodium chloride solution (200 mL), and extracted with ethyl acetate (200 mL x 2). The organic phases were combined, washed with saturated brine (100 mL), and dried over anhydrous sodium sulfate. After filtration and concentration, a crude product was obtained. The crude product was purified by column chromatography to obtain **230-5**.

¹H NMR (400 MHz, CDCl₃) δ 8.41 (s, 1H), 8.31 (d, *J* = 5.2 Hz, 1H), 7.49 - 7.42 (m, 1H), 7.37 - 7.31 (m, 2H), 7.30 - 7.24 (m, 1H), 6.53 (d, *J* = 5.2 Hz, 1H), 6.26 (t, *J* = 4.4 Hz, 1H), 2.92 - 2.87 (m, 2H), 2.72 - 2.42 (m, 2H).

### Step V: Synthesis of Compound 230-6

At room temperature, **230-5** (200 mg, 0.827 mmol) was dissolved in a mixed solution of t-butanol (3 mL) and water (3 mL), and then NMO (387 mg, 1.65 mmol) and potassium osmate dihydrate (25.7 mg, 0.083 mmol) were added. The reaction solution was heated to 100°C and refluxed for 16 hrs. 1N NaOH solution (20 mL) was added, and the reaction solution was extracted with ethyl acetate (20 mL x 2). The organic phases were combined, washed with saturated brine (20 mL), and dried over anhydrous sodium sulfate. After filtration and concentration, a crude product was obtained. The crude product was purified by column chromatography to obtain **230-6**.

¹H NMR (400 MHz, DMSO) δ 8.38 (s, 1H), 8.21 (d, *J* = 5.2 Hz, 1H), 8.07 - 7.91 (m, 1H), 7.45 - 7.38 (m, 1H), 7.36 - 7.24 (m, 2H), 6.58 (d, *J* = 5.2 Hz, 1H), 5.53 (s, 1H), 4.86 (d, *J* = 7.2 Hz, 1H), 4.45 - 4.26 (m, 1H), 2.98 - 2.80 (m, 2H), 2.09 - 1.82 (m, 2H).

### Step VI: Synthesis of Compound 230-8

At -40°C, **230-6** (70.0 mg, 0.254 mmol) was dissolved in acetonitrile (10 mL), and TfOH (95.2 mg, 0.635 mmol) was added. The reaction solution was slowly heated to 80°C and refluxed for 16 hrs. The reaction solution was cooled to room temperature, added with water (10 mL), and concentrated under reduced pressure to obtain Intermediate **230-7** as a crude product. The crude product was dissolved in HCl (1 M,10 mL). Under a N₂ atmosphere, the reaction solution was heated to 100°C and refluxed for 3 hrs. The reaction solution was cooled to room temperature, and extracted with a sodium chloride solution (30 mL) and ethyl acetate (20 mL x 2). The aqueous phase was collected, adjusted to pH=10 with a saturated potassium carbonate solution, and extracted with ethyl acetate (20 mL x 2). The organic phases were combined, washed with saturated brine (10 mL), and dried over anhydrous sodium sulfate. After filtration and concentration, a crude product was obtained. The crude product was purified by column chromatography to obtain **230-8**.

¹H NMR (400 MHz, DMSO) δ 8.32 (s, 1H), 8.15 (d, *J* = 5.2 Hz, 1H), 8.04 (d, *J* = 7.6 Hz, 1H), 7.47 - 7.34 (m, 1H), 7.32 - 7.22 (m, 2H), 6.55 (d, *J* = 5.2 Hz, 1H), 4.97 (d, *J* = 5.6 Hz, 1H), 4.41 - 4.27 (m, 1H), 2.97 - 2.83 (m, 2H), 2.26 - 2.18 (m, 2H), 2.15 - 1.97 (m, 1H), 1.92 - 1.78 (m, 1H).

### Step VII: Synthesis of Compound 230-9

At room temperature, to a solution of **230-8** (50 mg, 0.182 mmol) in MeOH (5 mL), paraformaldehyde (6.56 mg, 0.218 mmol), AcOH (0.010 mL, 0.182 mmol), and NaBH₃CN (114 mg, 1.82 mmol) were sequentially added, and stirred under a N₂ atmosphere at 25°C for 16 hrs. The reaction solution was filtered, and purified by Prep-HPLC to obtain **230-9**. LCMS: 289.1 (M+H)⁺.

### Step VIII: Synthesis of Compound 230-10

In an ice bath, Jones reagent (5 mL) was added to a solution of **230-9** (100 mg, 0.346 mmol) in acetone (20 mL), and stirred under a N₂ atmosphere at 25°C for 16 hrs. The reaction solution was quenched with water (20 mL), adjusted to pH = 8 with a saturated sodium bicarbonate solution, and extracted with ethyl acetate (20 mL x 2). The organic phases were combined, washed with saturated brine (100 mL), and dried over anhydrous sodium sulfate. After filtration and concentration, a crude product was obtained. The crude product was purified by Prep-HPLC to obtain **230-10**. LCMS: 287.0 (M+H)⁺.

### Step IX: Synthesis of Compound 230 hydrochloride and Compound 231 hydrochloride

**230-10** (20.0 mg, 69.0 µmol) was purified by SFC. A first fraction (Rt=9.87 min) was collected, and concentrated under reduced pressure. 1N HCl and pure water were added and freeze dried to obtain Compound **230** hydrochloride. LCMS: 287.0 (M+H)⁺.

¹H NMR (400 MHz, MeOD) δ 8.95 (s, 1H), 8.89 (d, *J* = 6.2 Hz, 1H), 8.08 (d, *J* = 6.0 Hz, 1H), 7.59 - 7.49 (m, 3H), 7.45 (d, *J* = 8.2 Hz, 1H), 3.52 - 3.38 (m, 1H), 3.25- 3.13 (m, 1H), 3.09 - 2.92 (m, 2H), 2.66 (s, 3H).

A second fraction (Rt=13.26 min) was collected, and concentrated under reduced pressure. 1N HCl and pure water were added and freeze dried to obtain Compound **231** hydrochloride. LCMS: 287.0 (M+H)⁺.

¹H NMR (400 MHz, MeOD) δ 8.94 (s, 1H), 8.89 (d, *J* = 6.0 Hz, 1H), 8.07 (d, *J* = 6.2 Hz, 1H), 7.59 - 7.48 (m, 3H), 7.48 - 7.42 (m, 1H), 3.50 - 3.40 (m, 1H), 3.23 - 3.13 (m, 1H), 3.10 - 2.92 (m, 2H), 2.65 (s, 3H).

(SFC conditions: Column: ChiralPak IG, 250 × 50 mm I.D., 10 µm; Mobile phase: A for CO₂ and B MeOH; Gradient: 35% B; Flow rate: 140 mL /min).

### Example 26: Preparation of Compound 196 hydrochloride and Compound 199 hydrochloride

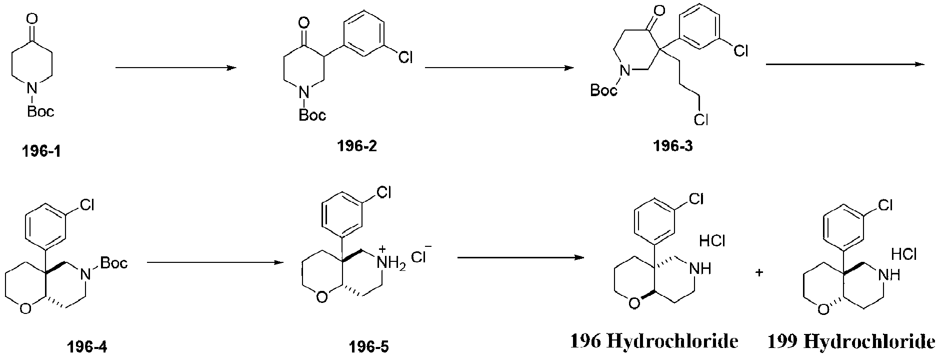

### Step I: Synthesis of Compound 196-2

At room temperature, **196-1** (4.0 g, 20.0 mmol) was dissolved in THF (40 mL), and m-chlorobromobenzene (7.7 g, 40.0 mmol), tri-t-butylphosphine (263 mg, 1.3 mmol), palladium acetate (224 mg, 1.0 mmol), and sodium tert-butoxide (2.9 g, 30.0 mmol) were sequentially added, heated to 65°C and refluxed for 24 hrs. The reaction solution was cooled to room temperature, added with a saturated sodium bicarbonate solution (40 mL), and extracted with EA (100 ml * 2). The organic phases were combined, washed with saturated brine, dried over anhydrous sodium sulfate, and concentrated, to obtain a crude product. The crude product was purified by column chromatography to obtain **196-2.** LCMS: 310.0 (M+H)⁺.

### Step II: Synthesis of Compound 196-3

**196-2** (1.0 g, 3.23 mmol) was dissolved in THF (9 mL), and cooled to 0°C. A solution of NaH (155 mg, 3.87 mmol) in THF (6 mL) was slowly added dropwise. After that, the reaction solution was heated to room stirred and stirred for 4 hrs. 1-chloro-3-iodopropane (2.64 g, 12.92 mmol) was added dropwise, and further stirred overnight at room temperature. The reaction solution was quenched with water, and extracted with EA (50 * 2). The organic phases were combined, washed with saturated brine, dried over anhydrous sodium sulfate, and concentrated, to obtain a crude product. The crude product was purified by column chromatography to obtain **196-3.**

¹H NMR (400 MHz, CDCl₃) δ 7.5 - 7.0 (m, 4H), 4.9 (dd, *J* = 75.8, 14.3 Hz, 1H), 4.2 (dd, *J* = 81.6, 12.0 Hz, 1H), 3.4 (t, *J* = 6.3 Hz, 2H), 3.1 (m, 2H), 2.5 (m, 1H), 2.3 (m, 1H), 2.0 (m, 1H), 1.7 (m, 1H), 1.7 - 1.4 (m, 11H).

### Step III: Synthesis of Compound 196-4

**196-3** (551 mg, 1.43 mmol) was dissolved in methanol (8 mL), and cooled to 0°C. Sodium borohydride (60 mg, 1.57 mmol) was added batchwise. The reaction solution was naturally warmed to room temperature, stirred for 1 hr, and concentrated under reduced pressure. The remainder was dissolved in THF (10 mL), and cooled to 0°C. NaH (69 mg, 1.72 mmol) was added batchwise. The reaction solution was naturally warmed to room temperature, stirred for 12 hrs, quenched with 1 N HCl added dropwise, and extracted with DCM (30 * 2). The organic phases were combined, dried over anhydrous sodium sulfate, and concentrated, to obtain a crude product. The crude product was purified by column chromatography to obtain **196-4**. LCMS: 252.0 (M-Boc+H)⁺.

### Step IV: Synthesis of Compound 196-5

**196-4** (206 mg, 0.59 mmol) was dissolved in DCM (5 mL), and hydrogen chloride-dioxane solution (4 M, 2 mL) was added, and stirred overnight at room temperature. After concentration under reduced pressure, **196-5** was obtained. LCMS: 252.0 (M +H)⁺.

### Step V: Synthesis of Compound 196 hydrochloride and Compound 199 hydrochloride

**196-5** (47 mg, 0.19 mmol) was separated by SFC. A first fraction (Rt=7.2 min) was collected, and concentrated under reduced pressure. 1N HCl and pure water were added and freeze dried to obtain Compound **199** hydrochloride or Compound **196** hydrochloride.

LCMS: 252.2 (M+H)⁺. ¹H NMR (400 MHz, MeOD) δ 7.85 - 7.74 (m, 1H), 7.71 - 7.59 (m, 1H), 7.33 (t, *J* = 8.0 Hz, 1H), 7.26 - 7.21 (m, 1H), 4.06 - 3.99 (m, 1H), 3.92 - 3.83 (m, 1H), 3.70 - 365 (m, 1H), 3.63 - 3.55 (m, 1H), 3.21 - 3.16 (m, 1H), 3.15 - 3.07 (m, 1H), 3.00 - 2.93 (m, 1H), 2.31 - 2.17 (m, 1H), 2.00 - 1.92 (m, 1H), 1.77 - 1.62 (m, 2H), 1.47 - 1.27 (m, 2H).

A second fraction (Rt=11.4 min) was collected, and concentrated under reduced pressure. 1N HCl and pure water were added and freeze dried to obtain Compound **196** hydrochloride or Compound **199** hydrochloride. LCMS: 252.2 (M+H)⁺.

¹H NMR (400 MHz, MeOD) δ 7.89 - 7.73 (m, 1H), 7.71 - 7.59 (m, 1H), 7.33 (t, *J* = 8.0 Hz, 1H), 7.28 - 7.20 (m, 1H), 4.08 - 3.98 (m, 1H), 3.90 - 3.83 (m, 1H), 3.72 - 3.65 (m, 1H), 3.63 - 3.55 (m, 1H), 3.21 - 3.17 (m, 1H), 3.16 - 3.07 (m, 1H), 3.00 - 2.91 (m, 1H), 2.31 - 2.16 (m, 1H), 1.99 - 1.91 (m, 1H), 1.77 - 1.61 (m, 2H), 1.46 - 1.28 (m, 2H).

(SFC conditions: Column: ChiralPak IE, 250 × 40 mm I.D., 10 µm; Mobile phase: A for n-hexane and B for ethanol; Gradient: 30% B; Flow rate: 80 mL /min).

### Example 27: Preparation of Compound 226 hydrochloride

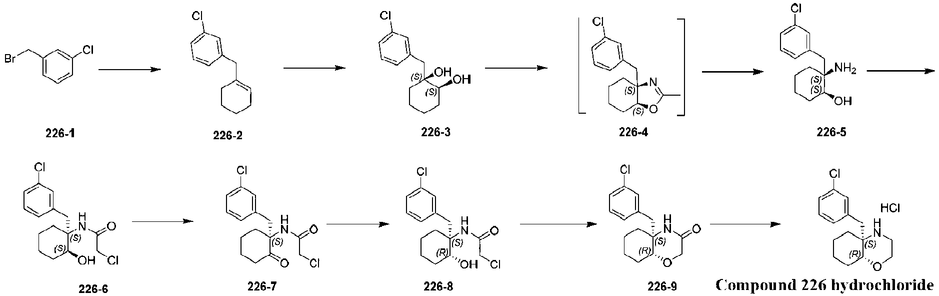

### Step I: Synthesis of Compound 226-2

At room temperature, to a mixed solution of 1-(bromomethyl)-3-chlorobenzene (1.00 g, 4.86 mmol) in 1,4-dioxane (15 mL) and water (3 mL), hex-1-en-1-ylboronic acid (740 mg, 5.84 mmol), K₂CO₃ (2.02 g, 14.6 mmol), and Pd (dppf)Cl₂ (110 mg, 0.146 mmol) were added. Under a nitrogen atmosphere, the reaction solution was stirred at 100 °C for 18 hrs, and then cooled to room temperature. A saturated sodium chloride solution (30 mL) was added, and the reaction solution was extracted two times with ethyl acetate (50 mL x 2). The organic phases were combined, washed with saturated brine (20 mL), and dried over anhydrous sodium sulfate. After filtration and concentration, a crude product was obtained. The crude product was purified by column chromatography (petroleum ether:ethyl acetate = 50: 1), to obtain Compound **226-2.**

¹H NMR: (400 MHz, CDCl₃) δ 7.24 - 7.13 (m, 3H), 7.07 - 7.02 (m, 1H), 5.56 - 5.43 (m, 1H), 3.21 (s, 2H), 2.08 - 1.97 (m, 2H), 1.90 - 1.75 (m, 2H), 1.62 - 1.47 (m, 4H).

### Step II: Synthesis of Compound 226-3

At room temperature, K₂CO₃ (940 mg, 6.79 mmol), K₃Fe(CN)₆ (2.24 g, 6.79 mmol), MeSO₂NH₂ (240 mg, 2.49 mmol), K₂OsO₄.2H₂O (40.0 mg, 0.113 mmol), and (DHQD)₂PHAL (350 mg, 0.453 mmol) were added to water (14 mL). Then, a solution of Compound **226-2** (500 mg, 2.26 mmol) in t-butanol (8.4 mL) was added, reacted for 16 hrs, added with ethyl acetate (100 mL), and washed three times with a 15% sodium hydroxide aqueous solution (50 mL). The organic phase was dried over anhydrous sodium sulfate, and filtered. The filtrate was concentrated under reduced pressure to obtain a crude product. The crude product was purified by column chromatography (petroleum ether: ethyl acetate = 0 - 25%) to obtain Compound **226-3.**

¹H NMR: (400 MHz, DMSO) δ 7.32 - 7.28 (m, 1H), 7.28 - 7.21 (m, 2H), 7.20 - 7.16 (m, 1H), 4.49 (d, *J* = 6.0 Hz, 1H), 3.92 (d, *J* = 1.2 Hz, 1H), 3.15 (dd, *J* = 13.6, 6.4 Hz, 1H), 2.82 (d, *J* = 13.2 Hz, 1H), 2.71 (d, *J* = 13.2 Hz, 1H), 1.59 - 1.46 (m, 3H), 1.43 - 1.17 (m, 3H), 1.15 - 0.95 (m, 2H).

### Step III: Synthesis of Compound 226-5

At -40°C, trifluoromethansulfonic acid (367 uL, 4.15 mmol) was added to a solution of Compound **226-3** (400 mg, 1.66 mmol) in acetonitrile (10 mL). The reaction solution was heated to room temperature, reacted for 1 hr, added with water (10 mL), and stirred for 10 min. Acetonitrile was removed by concentration under vacuum. The reaction solution was heated to 100°C, further reacted for 16 hrs, and then cooled to room temperature. The reaction solution was adjusted to pH 10 with a sodium hydroxide aqueous solution (1N), and extracted with ethyl acetate (10 mL x 3). The organic phases were combined, washed with saturated brine (10 mL), dried over anhydrous sodium sulfate, and filtered. The filtrate was concentrated under reduced pressure to obtain a crude product. The crude product was added with petroleum ether:ethyl acetate=10:1 (10 mL), and stirred (10 min). After filtration, the filer cake was collected, and dried under vacuum to obtain Compound **226-5**. LCMS: 240.0 (M+H)⁺.

### Step IV: Synthesis of Compound 226-6

At 0°C, to a solution of Compound **226-5**(100 mg, 0.0830 mmol) and triethyl amine (23 uL, 0.17 mmol) in tetrahydrofuran (2 mL), chloroacetyl chloride (7 uL, 0.09 mmol) was added dropwise. reacted for 1 hrs, added with water (5 mL), and extracted with ethyl acetate (5 mL x 3). The organic phases were combined, washed with saturated brine (5 mL), and dried over anhydrous sodium sulfate. After filtration and concentration, a crude product was obtained. The crude product was purified by column chromatography (ethyl acetate:petroleum ether = 0 - 24%) to obtain Compound **226-6**.

LCMS: 316.4 (M+H)⁺.

### Step V: Synthesis of Compound 226-7

At room temperature, Compound **226-6** (110 mg, 0.348 mmol) and PCC (225 mg, 1.04 mmol) were added to dichloromethane (5 mL), and reacted at 40°C for 16 hrs. The reaction solution was concentrated to obtain a crude product. The crude product was purified by column chromatography (ethyl acetate:petroleum ether = 0 - 18%) to obtain Compound **226-7**.

LCMS: 314.0(M+H)⁺.

### Step VI: Synthesis of Compound 226-8

At 0°C, (19 mg, 0.51 mmol) was added to a solution of Compound **226-8** (80 mg, 0.25 mmol) in methanol (2 mL), reacted at room temperature for 1 hr, and concentrated, to obtain a crude product. The crude product was purified by column chromatography (ethyl acetate:petroleum ether = 0 - 28%) to obtain Compound **226-8** (50 mg, 0.16 mmol, yield 62%).

LCMS: 316.2(M+H)⁺.

### Step VII: Synthesis of Compound 226-9

At 0°C, potassium tert-butoxide (35 mg, 0.32 mmol) was added to a solution of Compound **226-9** (50 mg, 0.16 mmol) in iso-propanol (2 mL), and reacted for 1 hr. A crude product was obtained by concentrating the reaction solution to remove most of the solvent. The crude product was added with water (2 mL), stirred for 10 min, and filtered. The filer cake was collected, and dried under vacuum to obtain Compound **226-9**.

LCMS: 280.1(M+H)⁺.

### Step VI: Synthesis of Compound 226

At 0°C, to a solution of Compound **226-9** (35 mg, 0.13 mmol) in tetrahydrofuran (5 mL), a borane dimethyl sulfide complex (63 µL, 0.63 mmol) was added dropwise. The system was heated to 70°C, and reacted for 16 hrs. The reaction solution was cooled to 0°C, and quenched with methanol (2 mL). Sodium borohydride (14 mg, 0.38 mmol) was slowly added batchwise. The reaction solution was heated to 50°C, and reacted for 32 hrs. The reaction solution was concentrated to obtain a crude product. The crude product was separated by prep-HPLC, and freeze dried to obtain Compound **226**.LCMS: 266.4 (M+H)⁺.

(Prep-HPLC conditions: Column: Xtimate C18, 21.2*250 mm, 5 um; Mobile phase: A (0.1%HCl) + B (ACN) ; Gradient: 16% B; Flow rate: 20 mL /min).

¹H NMR: (400 MHz, DMSO) δ 9.59 (s, 1H), 7.97 - 7.75 (m, 1H), 7.44 - 7.39 (m, 3H), 7.31 - 7.27 (m, 1H), 4.08 - 4.00 (m, 1H), 3.91 - 3.75 (m, 2H), 3.71 - 3.64 (m, 1H), 3.30 - 3.21 (m, 2H), 3.06 - 3.01 (m, 1H), 1.83 - 1.40 (m, 8H).

### Example 28

### Preparation of Crystal Form A of Compound 2 hydrochloride

Compound **2** (2.0 g, 7.94 mmol) was weighed into a 100 mL glass flask, and 20 mL of isopropanol was added and stirred until the compound was dissolved. 3.66 mL of hydrochloric acid in isopropanol (0.8 mL concentrated hydrochloric acid diluted 5 times with isopropanol, 1.1 eq) was added, stirred at room temperature for 24 hrs, and filtered. The filter cake was dried under vacuum at 25°C for 48 hrs, to obtain Crystal Form A of Compound **2** hydrochloride. The salt forming ratio is 1: 1. ¹H-NMR (400 MHz, DMSO) δ 10.41 (s, 1H), 9.42 (s, 1H), 7.98 - 7.93 (m, 2H), 7.58 - 7.53 (m, 2H), 4.14 - 4.01 (m, 3H), 3.01 (d, J=12,1H), 2.79 - 2.73 (m, 1H), 2.57 - 2.51 (m, 1H), 2.01- 1.77 (m, 4H), 1.67 - 1.55 (m, 2H), 0.89 - 0.79 (m, 1H).

### Preparation of Crystal Form B of Compound 2 phosphate

Compound **2** (2.0 g, 7.94 mmol) was weighed into a 100 mL glass flask, and 26 mL of acetone was added and stirred until the compound was dissolved. 2.93 mL of phosphoric acid in acetone (0.8 mL phosphoric acid diluted 5 times with acetone, 1.1 eq) was added, stirred at room temperature for 24 hrs, and filtered. The filter cake was dried under vacuum at 25°C for 48 hrs, to obtain Crystal Form B of Compound **2** phosphate. The salt forming ratio is 1: 1. ¹H-NMR (400 MHz, DMSO) δ7.84 - 7.75 (m, 2H), 7.45 - 7.35 (m, 2H), 0.94 - 3.90 (m, 1H), 3.80 - 3.64 (m, 2H), 2.61-2.49(m, 2H), 2.33 - 2.30 (m, 1H), 1.71 - 1.60 (m, 4H), 1.47-1.39 (m, 2H), 0.82 - 0.78 (m, 1H).

### Preparation of Crystal Form C of Compound 2 mesylate

Method I: Compound **2** (3.0 g, 11.91 mmol) was added to a 100 mL glass flask, and 54 mL of a mixed solution of methyl t-butyl ether/acetone (7:2) was added and stirred until the compound was dissolved. 5.36 mL of methansulfonic acid in methyl t-butyl ether (1.2 mL methansulfonic acid diluted 5 times with methylt-butyl ether) was added, stirred at room temperature for 24 hrs, and filtered. The filter cake was dried under vacuum at 25°C for 48 hrs, to obtain Crystal Form C of Compound **2** mesylate. The salt forming ratio is 1: 1. ¹H-NMR (400 MHz, DMSO) δ 9.49 (s, 1H), 9.13 (s, 1H), 7.95 - 7.93 (m, 2H), 7.60 - 7.57 (m, 2H), 4.17- 4.13 (m, 1H), 3.95 - 3.88 (m, 2H), 3.09 - 3.06 (m, 1H), 2.79 - 2.52 (m, 2H), 2.36 (s, 3H), 1.90 - 1.56 (m, 6H), 0.90 - 0.83 (m, 1H).

Method II: 200 mg of Crystal Form D of Compound **2** mesylate was added to EA (2 mL) to form a suspension, and stirred for 24 hrs. The filter cake was dried under vacuum at 25°C for 48 hrs, to obtain Crystal Form C of Compound **2** mesylate.

Method III. 200 mg of Crystal Form D of Compound **2** mesylate was added to methanol (2 mL) to form a suspension, and stirred for 24 hrs. The filter cake was dried under vacuum at 25°C for 48 hrs, to obtain Crystal Form C of Compound **2** mesylate.

### Preparation of Crystal Form D of Compound 2 mesylate

Method I: Crystal Form C of Compound **2** mesylate (50 mg, 0.20 mmol) was weighed into a 2 mL glass flask, and 0.1 mL of methanol was added and stirred until the compound was dissolved to obtain a clear solution. The solvent was evaporated off in a semi-closed environment at room temperature, to obtain Crystal Form D of Compound **2** mesylate.

Method II: 200 mg of Crystal Form C of Compound **2** mesylate was added to ethanol: water (volume ratio: 1: 1) (2 mL), to form a suspension, and stirred for 24 hrs. The filter cake was dried under vacuum at 25°C for 48 hrs, to obtain Crystal Form D of Compound **2** mesylate.

### Preparation of Crystal Form E of Compound 2 mesylate

Crystal Form C of Compound **2** mesylate (50 mg, 0.20 mmol) was weighed into a 2 mL glass flask, and 0.1 mL of a mixed solvent of ethanol/water (3:1) was added and stirred until a clear solution was obtained. The solvent was evaporated off in a semi-closed environment at room temperature, to obtain Crystal Form E of Compound **2** mesylate.

### Preparation of Crystal Form F of Compound 2 formate

Compound **2**(50 mg, 0.20 mmol) was weighed into to a 5 mL glass flask, and 0.1 mL of methyl t-butyl ether was added and stirred until the compound was dissolved. Formic acid (1.1 eq) was added and stirred at room temperature for 24 hrs. 0.02 mL of n-heptane was added, stirred at 40°C for 12 hrs, and filtered. The filter cake was dried under vacuum at 25°C for 24 hrs, to obtain Crystal Form F of Compound **2** formate. The salt forming ratio is 1: 1. ¹H-NMR (400 MHz, DMSO) δ 8.18 (s, 1H), 7.79 - 7.71 (m, 2H), 7.41 - 7.29 (m, 2H), 3.87- 3.84 (m, 1H), 3.67 - 3.54 (m, 2H), 2.53 - 2.43 (m, 3H), 1.75 - 1.66 (m, 3H), 1.44 - 1.41 (m, 3H), 0.87 - 0.80 (m, 1H).

### Preparation of Crystal Form L of Compound 2 oxalate

Compound **2** (500 mg, 1.97mmol) was weighed into a 10 mL glass flask, and 5 mL of methyl t-butyl ether and 2 mL of acetone were added and stirred until the compound was dissolved. Oxalic acid (0.5 eq) was added, and stirred at room temperature for 24 hrs. The solvent was evaporated off in a semi-closed environment at room temperature, to obtain Crystal Form L of Compound **2** oxalate. ¹H-NMR (400 MHz, DMSO) δ 7.85 (s, 2H), 7.80 (d, *J* = 7.6 Hz, 2H), 7.49 - 7.36 (m, 4H), 4.00 - 3.94 (m, 2H), 3.80 - 3.68 (m, 4H), 2.71 (d, *J* = 10.8 Hz, 2H), 2.65 - 2.55 (m, 2H), 2.39 (d, *J* = 13.2 Hz, 2H), 1.82 - 1.60 (m, 7H), 1.51 - 1.40 (m, 3H), 0.89 - 0.75 (m, 2H).

### Preparation of Crystal Form M of Compound 2 pamoate

Compound **2** (500 mg, 1.97mmol) was weighed into a 10 mL glass flask, and 5 mL of methyl t-butyl ether and 2 mL of acetone were added and stirred until the compound was dissolved. Pamoic acid (0.5 eq) was added, and stirred at room temperature for 24 hrs. The solvent was evaporated off in a semi-closed environment at room temperature, to obtain Crystal Form M of Compound **2** pamoate. ¹H-NMR (400 MHz, DMSO) δ 8.30 (s, 2H), 8.17 (d, *J* = 8.4 Hz, 2H), 7.98 - 7.88 (m, 3H), 7.74 (d, *J* = 7.6 Hz, 2H), 7.54 - 7.51 (m, 2H), 7.26 - 7.18 (m, 2H), 7.09 (t, *J* = 14.8 Hz, 2H), 4.73 (s, 2H), 4.21 - 4.06 (m, 1H), 4.03 - 3.86 (m, 3H), 3.09 - 3.01 (m, 4H), 2.82 - 2.71 (m, 1H), 2.65 - 2.57 (m, 1H), 1.93 - 1.76 (m, 4H), 1.71 - 1.53 (m, 3H), 1.49 - 1.36 (m, 1H), 1.11 (s, 8H), 0.90 - 0.76 (m, 1H).

### Preparation of Crystal Form G of Compound 3 hydrochloride

Compound **3** (400 mg, 1.59 mmol) was weighed into a 10 mL glass flask, and 2 mL of acetone was added and stirred until the compound was dissolved. 1.49 mL of hydrochloric acid in acetone (1.49 mL concentrated hydrochloric acid diluted 10 times with acetone) was added, stirred at room temperature for 20 hrs, and filtered. The filter cake was dried under vacuum at 25°C for 16 hrs, to obtain Crystal Form G of Compound **3** hydrochloride. The salt forming ratio is 1: 1. ¹H NMR (400 MHz, DMSO) δ 11.07 (m, 1H), 8.30-8.27(m, 1), 8.06(br. s, 1H), 7.65-7.61 (m, 1H), 7.53-7.50 (m, 2H), 4.23 - 4.03 (m, 3H), 3.41-3.35 (m, 1H), 3.12-3.08 (m, 1H), 2.71 - 2.69 (m, 1H), 2.71-2.69 (m, 1H), 2.07 - 1.96 (m, 3H), 1.73 - 1.46 (m, 3H), 0.81 - 0.71 (m, 1H).

### Preparation of Crystal Form H of Compound 3 hydrochloride

Compound **3** (400 mg, 1.59 mmol) was weighed into a 10 mL glass flask, and 1 mL of EA was added and stirred until the compound was dissolved. 0.44 mL of hydrogen chloride/EA (4 M) was added, stirred at room temperature for 6 hrs, and filtered. The filter cake was dried under vacuum at 25°C for 16 hrs, to obtain Crystal Form H of Compound **3** hydrochloride.

### Preparation of Crystal Form I of Compound 3 phosphate

Compound **3** (400 mg, 1.59 mmol) was weighed into a 10 mL glass flask, and 2 mL of acetone was added and stirred until the compound was dissolved. 0.118 mL of phosphoric acid aqueous solution (85% w/w) was added, stirred at room temperature for 6 hrs, and filtered. The filter cake was dried under vacuum at 25°C for 16 hrs, to obtain Crystal Form I of Compound **3** phosphate. The salt forming ratio is 1.0: 1.19. ¹H NMR (400 MHz, DMSO) δ8.13-8.11(m, 1), 7.48-7.46 (m, 1H), 7.37-7.28 (m, 2H), 3.85 - 3.82 (m, 1H), 3.75-3.63 (m, 2H), 3.10 - 3.06(m, 2H), 2.55 - 2.50 (m, 2H), 2.03-1.99 (m, 1H), 1.84 - 1.68 (m, 2H), 1.46 - 1.35 (m, 3H), 0.77 - 0.72 (m, 1H).

### Preparation of Crystal Form J of Compound 3 mesylate

Compound **3** (400 mg, 1.59 mmol) was weighed into a 10 mL glass flask, and 1 mL of DCM was added and stirred until the compound was dissolved. 170.4 mg of methansulfonic acid was added, stirred at room temperature for 6 hrs, and filtered. The filter cake was dried under vacuum at 25°C for 16 hrs, to obtain Crystal Form J of Compound **3** mesylate. ¹H NMR (400 MHz, DMSO) δ 10.00 (m, 1H), 8.29-8.27(m, 1), 8.08(br. s, 1H), 7.66-7.63 (m, 1H), 7.55-7.50 (m, 2H), 4.11-4.07 (m, 1H), 4.02 - 3.96 (m, 2H), 3.42-3.35 (m, 1H), 3.15-3.12 (m, 1H), 2.76-2.70 (m, 1H), 2.34 (s, 3H), 2.08 - 2.01 (m, 2H), 1.75 - 1.47 (m, 4H), 0.82 - 0.75 (m, 1H).

### Preparation of Crystal Form K of Compound 3 formate

Compound **3** (100 mg, 0.40 mmol) was weighed into a 10 mL glass flask, and 1 mL of EA was added and stirred until the compound was dissolved. 0.336 mL of formic acid in EA (0.1 mL formic acid diluted 10 times with EA) was added and stirred at 70°C for 5 hrs. 0.8 mL of n-heptane was added, stirred at 60°C for 10 hrs, and filtered. The filter cake was dried under vacuum at 25°C for 24 hrs, to obtain Crystal Form K of Compound **3** formate. ¹H NMR (400 MHz, DMSO) δ8.59(s, 1), 8.23-8.20 (m, 1H), 7.49-7.47 (m, 1H), 7.43-7.36 (m, 2H), 4.13 - 3.91 (m, 2H), 3.70-3.54 (m, 2H), 3.39 - 3.34(m, 1H), 2.32 - 2.12 (m, 2H), 1.95-1.91 (m, 2H), 1.75-1.40 (m, 3H), 0.77 - 0.72 (m, 1H).

### Preparation of Crystal Form N of Compound 3 oxalate

Compound **3** (250 mg, 1.0 mmol) was weighed into a 10 mL glass flask, and 3.3 mL of methyl t-butyl ether and 1.2 mL of acetone were added and stirred until the compound was dissolved. Oxalic acid (0.5 eq) was added, and stirred at room temperature for 24 hrs, and filtered. The filter cake was dried under vacuum at 25°C for 48 hrs, to obtain Crystal Form N of Compound **3** oxalate. ¹H NMR (400 MHz, DMSO) δ 8.25 - 8.04 (m, 1H), 7.60 - 7.51 (m, 1H), 7.47 - 7.35 (m, 2H), 3.96 - 3.89 (m, 3H), 3.27 - 3.21 (m, 1H), 2.92 - 2.84 (m, 1H), 2.67 - 2.55 (m, 1H), 2.09 - 1.98 (m, 1H), 1.96 - 1.85 (m, 1H), 1.72 - 1.57 (m, 2H), 1.53 - 1.36 (m, 2H), 0.84 - 0.65 (m, 1H).

### Preparation of Crystal Form O of Compound 3 pamoate

Compound **3** (250 mg, 1.0 mmol) was weighed into a 10 mL glass flask, and 3.3 mL of methyl t-butyl ether and 1.2 mL of acetone were added and stirred until the compound was dissolved. Pamoic acid (0.5 eq) was added, and stirred at room temperature for 24 hrs, and filtered. The filter cake was dried under vacuum at 25°C for 48 hrs, to obtain Crystal Form O of Compound **3** pamoate. ¹H NMR (400 MHz, DMSO) δ 8.43 (s, 2H), 8.28 - 8.23 (m, 1H), 8.14(d, *J* = 8.8 HZ, 2H), 7.85 (d, *J* = 8.0 Hz, 2H), 7.65 - 7.57 (m, 1H), 7.52 - 7.44 (m, 3H), 7.37 - 7.31 (m, 2H), 7.23 - 7.16 (m, 2H), 4.78 (s, 2H), 4.09 - 4.00 (m, 4H), 3.08 (s, 1H), 2.78 - 2.63 (m, 2H), 2.12 - 1.91 (m, 3H), 1.86 - 1.76 (m, 1H), 1.73 - 1.65 (m, 1H), 1.60 - 1.53 (m, 1H), 1.49 - 1.38 (m, 1H), 1.11 (s, 4H), 0.82 - 0.66 (m, 1H).

### Example 29: Solubility test

The solubilities of different forms of the compounds in media with different pH values were tested. The compound sample was weighed into a glass flask, and 1 mL or 2 mL of phosphate buffer pH=6.0, pure water, phosphate buffer pH=8.0, and/or 0.9% physiological saline respectively was added respectively, and magnetically stirred at 20°C for 24 hrs. A sample was taken for solubility test, and the purity of the solution over 24 hrs was tested.

**Table 18: Solubility test results of different crystal forms of the salts of Compound 2 in media with different pH values**

| | Crystal Form A of Compound **2** hydrochloride (99.99%) | | Crystal Form B of Compound **2** phosphate (99.98%) | | Crystal Form C of Compound **2** mesylate (99.97%) | |
|---|---|---|---|---|---|---|
| Vehicle | 24-h solubility (mg/mL) | Purity (24 h) | 24-h solubility (mg/mL) | Purity (24 h) | 24-h solubility (mg/mL) | Purity (24 h) |
| Phosphate buffer pH=6.0 | 38.26 | 99.92% | 13.30 | 99.95% | 44.00 | 99.53% |
| Phosphate buffer pH=8.0 | 25.83 | 99.93% | 18.70 | 99.98% | 27.02 | 99.88% |
| Pure water pH=6.8 | 35.32 | 99.99% | 26.96 | 99.70% | 57.57 | 99.98% |
| 0.9% physiological saline | 17.79 | 99.98% | 31.20 | 99.88% | 58.49 | 99.98% |

**Table 19: Solubility test results of different crystal forms of the salts of Compound 3 in media with different pH values**

| | Crystal Form G of Compound **3** hydrochloride (99.97%) | | Crystal Form I of Compound **3** phosphate (99.91%) | | Crystal Form J of Compound **3** mesylate (99.91%) | |
|---|---|---|---|---|---|---|
| Vehicle | 24-h solubility (mg/mL) | Purity (24 h) | 24-h solubility (mg/mL) | Purity (24 h) | 24-h solubility (mg/mL) | Purity (24 h) |
| Phosphate buffer pH=6.0 | ≥10 | 99.89% | ≥10 | 99.88% | ≥10 | 99.88% |
| Phosphate buffer pH=8.0 | 2.5 | 99.56% | ≥10 | 99.81% | ≥10 | 99.81% |
| Pure water pH=6.8 | ≥10 | 99.95% | ≥10 | 99.86% | ≥10 | 99.86% |

The solubility test results show that Crystal Form A of the hydrochloride, Crystal Form B of the phosphate, and Crystal Form C of the mesylate of Compound **2**, and Crystal Form G of the hydrochloride, Crystal Form I of the phosphate, and Crystal Form J of the mesylate of Compound **3** show excellent solubilities in media with different pH values, and the solution stability is good.

### Example 30: Solid stability test

Crystal Form A of the hydrochloride, Crystal Form B of the phosphate, and Crystal Form C of the mesylate of Compound **2** and Crystal Form G of the hydrochloride, Crystal Form I of the phosphate, and Crystal Form J of the mesylate of Compound **3** were respectively packaged and stored at an acceleration condition of 40°C/75% RH, a routing condition of 25°C/60% RH, and a high-temperature condition of 60°C, and the purity, crystal form, and appearance of the compounds were evaluated at different times.

**Table 20. Solid stability test results of Crystal Form A of Compound 2 hydrochloride**

| Test condition | Time | Crystal Form | Purity | Appearance |
|---|---|---|---|---|
| | 0D | Crystal Form A | 99.99% | Dry white solid powder |
| 60°C | 5D | Crystal Form A | 99.98% | White solid powder |
| | 10D | Crystal Form A | 99.99% | White solid powder |
| | 30D | Crystal Form A | 99.99% | White solid powder |
| 25°C/60% RH | 5D | Crystal Form A | 99.99% | White solid powder |
| | 10D | Crystal Form A | 99.99% | White solid powder |
| | 30D | Crystal Form A | 99.99% | White solid powder |
| 40°C/75% RH | 5D | Crystal Form A | 99.99% | White solid powder |
| | 10D | Crystal Form A | 99.99% | White solid powder |
| | 30D | Crystal Form A | 99.98% | White solid powder |

**Table 21. Solid stability test results of Crystal Form B of Compound 2 phosphate**

| Test condition | Time | Crystal Form | Purity | Appearance |
|---|---|---|---|---|
| | 0D | Crystal Form B | 99.98% | Dry white solid powder |
| 60°C | 5D | Crystal Form B | 99.99% | White solid powder |
| | 10D | Crystal Form B | 99.99% | White solid powder |
| | 30D | Crystal Form B | 99.99% | White solid powder |
| 25°C/60% RH | 5D | Crystal Form B | 99.97% | White solid powder |
| | 10D | Crystal Form B | 99.98% | White solid powder |
| | 30D | Crystal Form B | 99.98% | White solid powder |
| | 5D | Crystal Form B | 99.99% | White solid powder |
| 40°C/75% RH | 10D | Crystal Form B | 99.98% | White solid powder |
| | 30D | Crystal Form B | 99.98% | White solid powder |

**Table 22. Solid stability test results of Crystal Form C of Compound 2 mesylate**

| Test condition | Time | Crystal Form | Purity | Appearance |
|---|---|---|---|---|
| | 0D | Crystal Form C | 99.97% | Dry white solid powder |
| 60°C | 5D | Crystal Form C | 99.97% | White solid powder |
| | 10D | Crystal Form C | 99.95% | White solid powder |
| | 30D | Crystal Form C | 99.98% | White solid powder |
| 25°C/60% RH | 5D | Crystal Form C | 99.99% | White solid powder |
| | 10D | Crystal Form C | 99.99% | White solid powder |
| | 30D | Crystal Form C | 99.99% | White solid powder |
| 40°C/75% RH | 5D | Crystal Form C | 99.96% | White solid powder |
| | 10D | Crystal Form C | 99.99% | White solid powder |
| | 30D | Crystal Form C | 99.99% | White solid powder |

**Table 23. Solid stability test results of Crystal Form G of Compound 3 hydrochloride**

| Test condition | Time | Crystal Form | Purity | Appearance |
|---|---|---|---|---|
| | 0D | Crystal Form G | 99.97% | Dry white solid powder |
| 60°C | 7D | Crystal Form G | 99.99% | White solid powder |
| 40°C/75% RH | 7D | Crystal Form G | 99.98% | White solid powder |

**Table 24. Solid stability test results of Crystal Form I of Compound 3 phosphate**

| Test condition | Time | Crystal Form | Purity | Appearance |
|---|---|---|---|---|
| | 0D | Crystal Form I | 99.91% | Dry white solid powder |
| 60°C | 7D | Crystal Form I | 99.95% | White solid powder |
| 40°C/75% RH | 7D | New crystal form | 99.92% | White solid powder |

**Table 25. Solid stability test results of Crystal Form J of Compound 3 mesylate**

| Test condition | Time | Crystal Form | Purity | Appearance |
|---|---|---|---|---|
| | 0D | Crystal Form J | 99.95% | Dry white solid powder |
| 60°C | 7D | Crystal Form J | 99.97% | White solid powder |
| | 30D | Crystal Form J | 99.98% | White solid powder |
| 40°C/75% RH | 7D | Crystal Form J | 99.96% | White solid powder |
| | 30D | Crystal Form J | 99.95% | White solid powder |

The stability research result show that the various crystal forms of the salts of Compound **2** and **3** according to the present invention have excellent stability under the high-temperature and acceleration conditions, and the purity of the compound does not change significantly. Except for Crystal Form I of Compound **3** phosphate that undergoes crystal transformation, all other crystal forms remain stable.

### Evaluation of biological and chemical performances

The present invention will be further described and explained below in conjunction with test example, but these examples are not meant to limit the scope of the present invention.

### Test Example 1: Test of NMDA receptor-mediated on HEK293 cells

### 1. Expression of human NMDA receptor GluN1 and GluN2A subunits in HEK293 cells

The day before transfection, HEK293 cells were inoculated into a 24-well plate with a 10 mm glass slide, and the glass slide was coated with polylysine in advance to increase the cell attachment. DMEM medium containing 10% FBS was added, and the cells were cultured in an incubator at 37°C and 5% CO₂. When the cells were grown to 60%-70% confluency after 18-24 hrs, TRE-GluN1-GluN2A containing human GluN1 and GluN2A, and the plasmid cDNAs of tTA and pCAG-EGFP were transfected into HEK293 cells by lipofectin transfection (TRE-GluN1-GluN2A: tTA: pCAG-EGFP was 0.2 µg : 0.2 µg : 0.02 µg). Before transfection, a new DMEM medium was changed, and 1 µg/mL Dox (doxycycline) was added to the medium to induce stable expression. 100 µM D-APV and 10 mM MgCl₂ were added to avoid excitotoxicity to cells caused by NMDAR overexpression. About 24 hrs after transfection, the cells with green fluorescence were selected for electrophysiological recording.

### 2. Whole-cell patch-clamp recording of HEK293 cells

The glass slide inoculated with HEK293 cells was placed in a recording chamber, and an extracellular solution (NaCl 140 mM, KCl 2.8 mM, HEPES 10 mM, CaCl₂ 1 mM, glycine 0.1 mM, pH7.2) was perfused at a speed of 4 mL/ min. The channels of the ALA-VC³ eight-channel pressurized perfusion system were filled with an extracellular solution for washing, an extracellular solution containing glutamic acid (100 µM), and an extracellular solution containing glutamic acid and the compound to be tested respectively. The dripping rate was approximately 1 drop per sec, with a volume of 10 µL per drop. HEK293 cells with moderate green fluorescence intensity and good attachment were recorded under a fluorescence microscope. The delivery glass microtube of the perfusion system was positioned at a distance of approximately ten cell diameters away from the cell to be recorded. The drug was sprayed on the cell surface under air pressure (0.02 MPa). The glass electrode with a resistance of 4-6 MΩ was drawn with a microelectrode drawing instrument, and filled with an internal solution (CsCl 125 mM, HEPES 10 mM, EGTA 11 mM). Using a microelectrode manipulator, the glass electrode was slowly pushed to the cells. After the electrode was attached to the cell membrane, a negative pressure was applied, to form a high-resistance seal of 1 GΩ or higher between the electrode tip and the cell membrane, and then a negative pressure was applied to rupture the cell member and establish a whole-cell recording configuration. After the whole cell recording configuration was formed, the cell membrane potential was clamped at -70 mV. The extracellular solution containing 100 µM glutamic acid, the extracellular solution containing 100 µM glutamic acid and the compound to be tested, and the extracellular fluid were sprayed by the perfusion system on the surface of the cell membrane in sequence, for a time of 10 s, 20 s, and 10 s respectively. The NMDA receptor-mediated current was recorded to evaluate the inhibitory effect of the compound to be tested on NMDAR containing GluN2A. The test process was controlled by pCLAMP 10.6, and the stimulation signal is generated and the feedback signal is collected by using Digidata 1440A digital-to-analog converter.

In the test, the inhibition (%) on NMDAR-mediated current was evaluated after the compound was administered at a concentration of 10 µM, or the inhibition of the compound at 10, 3, 1, 0.3, 0.1, and 0.03 µM were tested, and fitted to obtain the IC₅₀ value.

The inhibition percentage (inhibition rate) of the compound of the present invention on NMDAR-mediated current = (1- NMDAR-mediated current after administration/NMDAR-mediated current before administration)*100%. The inhibition rates of the compound at 10, 3, 1, 0.3, 0.1, and 0.03 µM were imported into Graphpad prism software, and subjected to Nonlinear Regression analysis. The IC₅₀ value was automatically calculated by curve fitting.

The in-vitro activity test results of the compound of the present invention are shown in Table 26.

**Table 26: Inhibition of each compound on NMDAR-mediated current**

| Compound | % (10 µM) | IC₅₀ (µM) | Compound | % (10 µM) | IC₅₀ (µM) |
|---|---|---|---|---|---|
| Compound **2-RR** hydrochloride | NA | 2.966 | Compound **74** hydrochloride | 78% | NA |
| Compound **1** | NA | 1.413 | Compound **80** hydrochloride | 91% | NA |
| Compound **2** | NA | 0.654 | Compound **84** hydrochloride | 95% | NA |
| Compound **2** mesylate | NA | 0.746 | Compound **92** hydrochloride | 74% | NA |
| Compound **3** hydrochloride | NA | 0.849 | Compound **95** hydrochloride | 85% | NA |
| Compound **13** hydrochloride | 77% | NA | Compound **96** hydrochloride | 70% | NA |
| Compound **17** hydrochloride | 70% | NA | Compound **98** hydrochloride | 81% | NA |
| Compound **33** hydrochloride | NA | 0.134 | Compound **144** hydrochloride | 69% | NA |
| Compound **36** hydrochloride | NA | 0.302 | Compound **159** hydrochloride | 78% | NA |
| Compound **39** hydrochloride | 86% | NA | Compound **160** hydrochloride | 87% | NA |
| Compound **40** hydrochloride | 74% | NA | Compound **161** hydrochloride | 88% | NA |
| Compound **45** hydrochloride | NA | 0.193 | Compound **177** hydrochloride | 96% | NA |
| Compound **46** hydrochloride | 94% | NA | Compound **178** hydrochloride | 88% | NA |
| Compound **54** hydrochloride | 66% | NA | Compound **179** hydrochloride | 73% | NA |
| Compound **55** hydrochloride | NA | 0.247 | Compound **180** hydrochloride | 77% | NA |
| Compound **58** hydrochloride | NA | 0.434 | Comparative Compound PDHQ (SR or RS) | NA | 0.896 |
| Compound **59** hydrochloride | 96% | NA | Comparative Compound PDHQ (RS or SR) | NA | 2.11 |
| Compound **60** hydrochloride | 83% | NA | Comparative Compound A | 30% | NA |
| Compound **73** hydrochloride | 93% | NA | Comparative Compound B | 32% | NA |

| | | | | | |
|---|---|---|---|---|---|
| NA: not tested | | | | | |

The result shows that the compound of the present invention has excellent inhibition on NMDAR-mediated current.

### Test Example 2: Mouse behavioral test

Test animals: C57BL/6J male mice aged 10-12 weeks were purchased from Speifu (Suzhou) Biotechnology Co., Ltd. The animals arrived at the test center before the test and acclimated in the feeding room for more than 7 days. 5 animals per cage was bred at a temperature of 23-24°C, under a 12-hr light/12-hr dark cycle. Before the behavioral test, the animals were taken to the behavioral test chamber 1 hour in advance, to acclimate to the environment.

Forced swimming test is a standard behavioral test to evaluate the depressive behavior of rodents. The experimental animals were placed in a confined environment, and the animals struggled desperately in an attempt to escape but failed to do so, to provide an inescapable stressful environment. After a period of time, the animal showed a typical "immobility state", reflecting a state known as "behavioral despair". It is generally accepted that this behavioral despair is similar to the depressive symptoms in humans. The objective of this test was to evaluate the antidepressant efficacy of the compound of the present invention in C57 mice.

In the FST test, the number of animals in each group (N) was 15, the administration mode was intraperitoneal injection (i.p.) or oral gavage (p.o.), the dosage of administration was 5, 10 or 20 mg/kg (depending on the experimental needs), and the volume of administration was 10 mL/kg. The vehicle was 0.9% physiological saline.

Test process: 1. The test was carried out in a quiet environment. The animals were weighed and acclimated in the test chamber for at least 30 min before the test.

2. The compound to be tested was dissolved in 0.9% physiological saline, and the corresponding volume of the compound was given by intraperitoneal injection (i.p.) or oral gavage (p.o.) according to the weight of mice and used as the test group. 0.9% physiological saline was used as the control group, and the dosage was the target dosage of administration. The administration process was double-blinded administration.

3. 1 hr after administration, the mice were placed in a transparent plexiglass water tank with a diameter of 15 cm and a height of 30 cm, in which water was injected to a height of about 15 cm, and the water temperature was 23-25°C. The total recording time was 6 min.

4. The immobility time of mice within later 4 min was analyzed, with which the degree of helplessness and despair of mice was reflected.

5. The immobility time of mice between the administration group and the control group was compared, and the antidepressant activity (1-h inhibition rate) of the compound to be tested was analyzed. The difference was analyzed by unpaired t-test to have statistical significance, P<0.05 means that there was a significant difference. * P<0.05, **P<0.01, ***P<0.001. 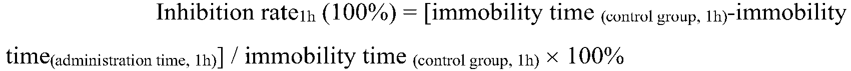 Inhibition rate1h (100%) = [immobility time (control group, 1h)-immobility time(administration time, 1h)] / immobility time (control group, 1h) × 100%

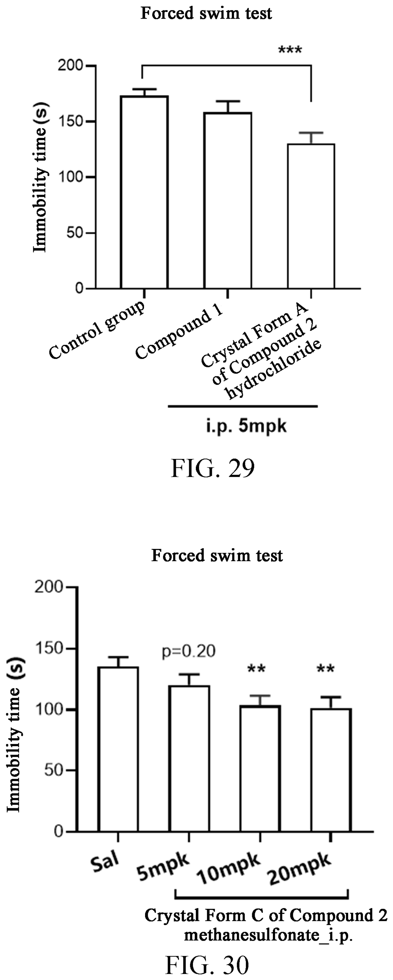

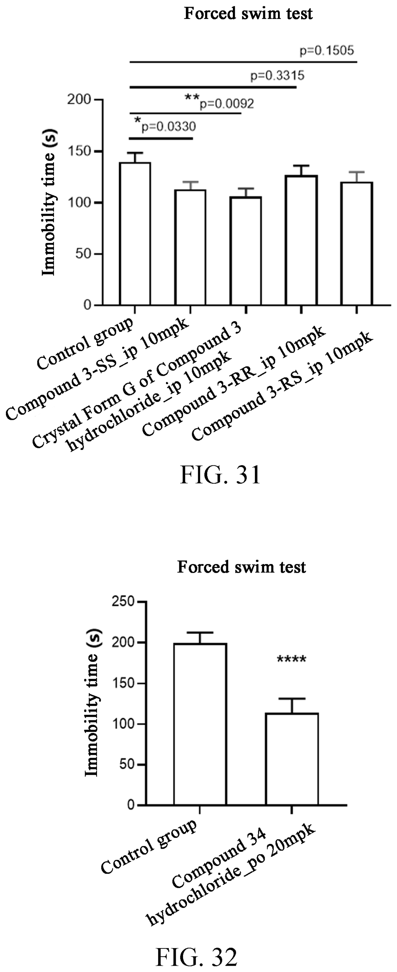

Results: FIGs. 29-32 and Table 27 show that Crystal Form A of Compound **2** hydrochloride at 5 mg/kg i.p. has obvious antidepressant effect; and Crystal Form C of Compound **2** mesylate at 10 mg/kg and 20 mg/kg i.p. has obvious antidepressant effect. Crystal Form G of Compound **3** hydrochloride at 10mg/kg i.p. has obvious antidepressant effect that is significantly better than the effect of the three isomer compounds **3-SS** hydrochloride, **3-RR** hydrochloride, and **3-RS** hydrochloride. Compound **60** hydrochloride, **73** hydrochloride, **80** hydrochloride, and **95** hydrochloride at 10mg/kg i.p. have obvious antidepressant effect. Compound **34** hydrochloride, **177** hydrochloride, **178** hydrochloride, **179** hydrochloride, and **180** hydrochloride at 10 mg/kg p.o. have significant antidepressant effect.

**Table 27: Result of antidepressant activity of the compound of the present invention after intraperitoneal/oral administration**

| Compound | Route of administration | Dose of administration | 1-h inhibition rate | P value |
|---|---|---|---|---|
| Crystal Form A of Compound **2** hydrochloride | i.p. | 5 mg/kg | 24.6% | 0.0005 |
| Crystal Form C of Compound **2** mesylate | i.p. | 5 mg/kg | 11.2% | 0.20 |
| | i.p. | 10 mg/kg | 23.3% | 0.0067 |
| | i.p. | 20 mg/kg | 24.9% | 0.0063 |
| Crystal Form G of Compound **3** hydrochloride | i.p. | 10 mg/kg | 24.0% | 0.0092 |
| Comparative compound **3-SS** hydrochloride | i.p. | 10 mg/kg | 19.1% | 0.033 |
| Comparative compound **3-RR** hydrochloride | i.p. | 10 mg/kg | 9.1% | 0.33 |
| Compound **3-RS** hydrochloride | i.p. | 10 mg/kg | 13.8% | 0.15 |
| Compound **34** hydrochloride | p.o. | 20 mg/kg | 42.8% | 0.0005 |
| Compound **60** hydrochloride | i.p. | 10 mg/kg | 36.5% | 0.0002 |
| Compound **73** hydrochloride | i.p. | 10 mg/kg | 38.6% | <0.0001 |
| Compound **80** hydrochloride | i.p. | 10 mg/kg | 27.4% | 0.012 |
| Compound **95** hydrochloride | i.p. | 10 mg/kg | 58.2% | <0.0001 |
| Compound **177** hydrochloride | p.o. | 10 mg/kg | 42.6% | 0.0009 |
| Compound **178** hydrochloride | p.o. | 10 mg/kg | 26.2% | 0.028 |
| Compound **179** hydrochloride | p.o. | 10 mg/kg | 53.6% | <0.0001 |
| Compound **180** hydrochloride | p.o. | 10 mg/kg | 32.1% | 0.019 |

| | | | | |
|---|---|---|---|---|
| i.p.: intraperitoneal injection; p.o.: oral administration | | | | |

The result shows that the compound of the present invention has excellent in-vivo antidepressant efficacy.

### Test Example 3: Evaluation of analgesic effects of compounds

The writhing test by intraperitoneal injection of acetic acid was a method to induce acute inflammatory pain by chemical stimulation. That is, acetic acid was intraperitoneally injected into the test animals to induce pain, and the writhing reaction of the test animals was observed to determine whether there was pain, to evaluate the efficacy of the analgesic drugs. The animal information was the same as that in Test Example 2.

In the acetic acid-induced writhing test, the number of animals in each group (N) was 10, the administration mode was intraperitoneal injection (i.p.) or oral gavage (p.o.), and the vehicle was 0.9% physiological saline.

Test process: 1. The test was carried out in a quiet environment. The animals were weighed and acclimated in the test chamber for at least 30 min before the test.

2. The compound to be tested was administered by intraperitoneal injection (left side of the abdominal cavity) or oral gavage. The administration process was double-blinded administration.

3. 30 min after administration, 0.6% acetic acid (prepared immediately before use) was given at the other side of the abdominal cavity (right side of the abdominal cavity, administration volume: 10 ml/kg), and then the mice were placed in an observation chamber and recorded for 30 min.

4. The writhing times of mice within 30 min after 0.6% acetic acid was given were statistically counted. The writhing times of mice between the administration group and the control group was compared, and the analgesic activity (analgesic rate) of the compound to be tested was analyzed. The difference was analyzed by unpaired t-test to have statistical significance, where P<0.05 means that there was a significant difference. * P<0.05, **P<0.01, ***P<0.001,**** P<0.0001. 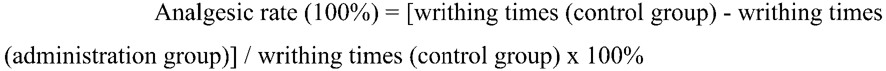 Analgesic rate (100%) = [writhing times (control group) - writhing times (administration group)] / writhing times (control group) x 100%

The result shows that after intraperitoneal or oral administration, the compound of the present invention shows obvious analgesic effect at the dosage of 2 mg/kg to 5 mg/kg, and the analgesic rate (%) is 50-74%, with P<0.05.

The compound of the present invention has excellent in-vivo analgesic effect.

### Test Example 4: Toxicity test of compounds

The exploration test of non-toxic dose of the compound is an important index to evaluate the safety of the compound. High safety will bring a higher treatment window. The animal information was the same as that in Test Example 2.

### Test process:

1. The test was carried out in a quiet environment. The animals were weighed and acclimated in the test chamber for at least 30 min before the test.
2. The compound to be tested was dissolved in 0.9% physiological saline, and the corresponding volume of the compound was intraperitoneally injected (i.p.) according to the weight of mice. The dosage was the target dosage of administration. The number of animals in each group was 2 or 3.
3. The mice after administration were placed in a transparent observation chamber, and the behavior changes of the mice were video recorded and observed for about one hour. (Note: During the observation period, if the mouse was lying still, the mouse was turned over by hand, and whether the righting reflex (RR) of the mouse disappeared was checked. If disappeared, the mouse was in an anesthetic state, and the time of anesthesia of the mouse was recorded).
4. After the one-hour observation, the mice were returned to the rearing cage. The observation chamber was wiped with 75% alcohol, to avoid the influence of the information left by animals in the last round (such as the smell of animals' feces and urine) on the next test result. The next round of animals were changed to continue the test.

The epilepsy score was obtained according to the epilepsy rating scale for rodents shown in Table 24 below.

**Table C. Epilepsy-like behavior rating scale for rodents**

| Score | Behavioral phenotype |
|---|---|
| 0 | No abnormal behavior |
| 1 | Walking unsteadily/losing the motor ability/immobility |
| 2 | Generalized clonus/tremor/writhing |
| 3 | Tail rigidity/tail flicking |
| 4 | Forelimb clonus |
| 5 | Generalized spasm/convulsion |
| 6 | Startle or running-bouncing clonic seizure |
| 7 | Generalized tonic-clonic seizure |
| 8 | Death |

The test results are shown in Table 28.

**Table 28: Summary of safety after administration in mice**

| | Dosage and score in observation period | | |
|---|---|---|---|
| Compound | NOAEL dosage (mg/kg) (score 0) | Minimum toxic dose (mg/kg) (score 1) | STD10 (mg/kg) (score 8) |
| Comparative Compound 2-SS mesylate | 25 | 35 | not tested |
| Comparative Compound 2-RR mesylate | 45 | 60 | not tested |
| Crystal Form A of Compound **2** hydrochloride | 44 | not tested | 263 |
| Crystal Form C of Compound **2** mesylate | 60 | 75 | 275 |
| Compound **34** hydrochloride | 20 | 40 | 250 |
| Comparative Compound PDHQ (SS or RR) | not tested | not tested | 75 |
| Comparative Compound PDHQ (RR or SS) | not tested | not tested | 100 |

| | | | |
|---|---|---|---|
| *NOAEL: no observed adverse effect level; STD10: dose at which 10% of the animals have severe toxic reaction. | | | |

Conclusions: The compound of the present invention has excellent in-vivo safety. The NOAEL doses of Crystal Form A of the hydrochloride and Crystal Form C of the mesylate of Compound **2** are higher than that of Comparative Compound 2-SS mesylate. The minimum toxic dose (score 1) of Crystal Form C of Compound **2** mesylate is significantly higher than those of the two Comparative Compounds 2-SS and 2-RR. The STD10 doses of all the compound of the present invention are significantly higher than those of PDHQ (SS or RR) and PDHQ(RR or SS).

### Test Example 5: Open field test of mice

Open field test is a standard behavioral method to evaluate the voluntary movement behavior and the exploratory behavior, which is widely used in the research of neurological and psychiatric diseases such as depression, anxiety, Parkinson's disease and screening of drugs acting on central nervous system.

Due to the limited tests to evaluate the hallucinogenic effect of NMDAR antagonists, the open field test is used in the present invention to evaluate whether the compound affects the spontaneous locomotor activity of mice and also used to indirectly evaluate whether the compound has hallucinogenic effect. Furthermore, the increase of spontaneous locomotor activity of mice is also considered as one of the positive symptoms of schizophrenia. Therefore, the open field test can also be used to evaluate whether the compound has the psychotomimetic-like side effects.

The animal information was the same as that in Test Example 2. The number of animals in each group (N) was 10, the administration mode was intraperitoneal injection or oral administration, the dosage of administration was 5 or 10 mg/kg, and the volume of administration was 10 mL/kg.

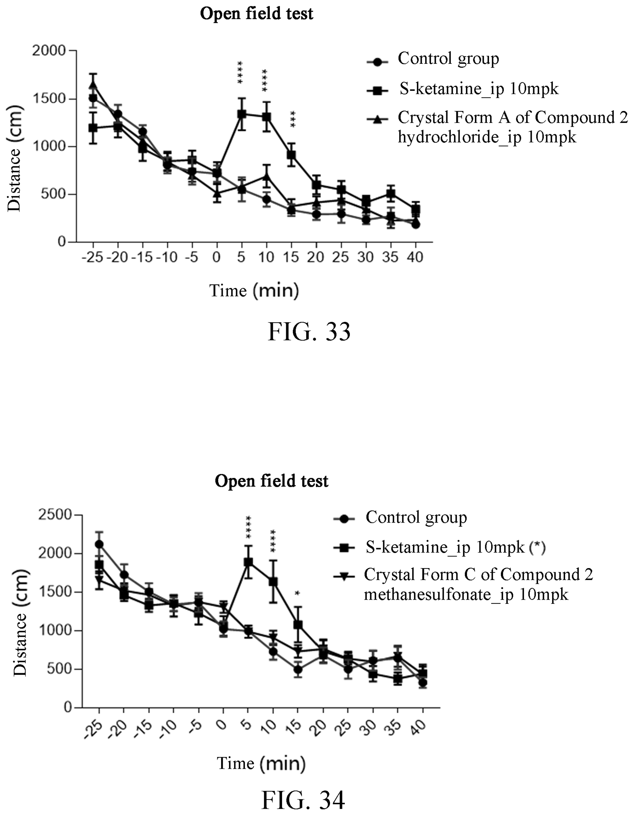

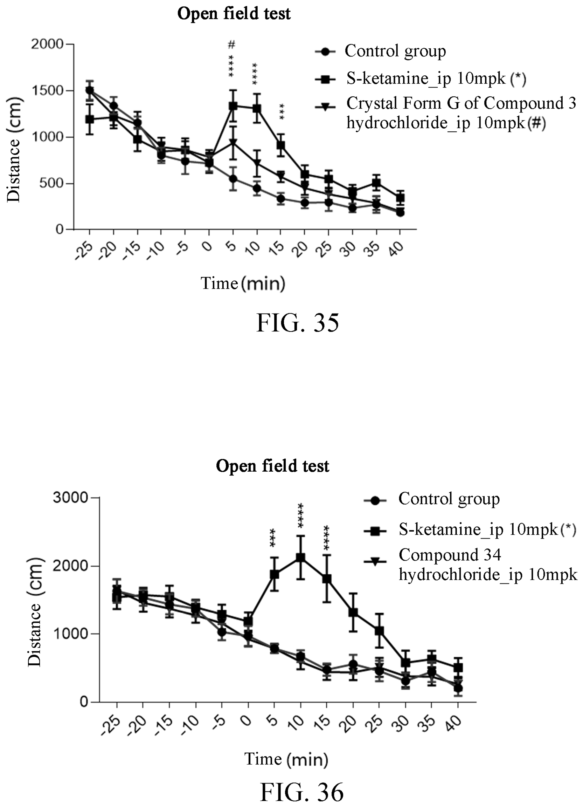

Test process: The open field test chamber was a soundproof chamber made of opaque plexiglass and having an inner diameter of 40*40*40 cm, and the analysis software was purchased from Noldus (Ethovision XT 11.5). The test was carried out in a quiet environment. The animals were weighed and acclimated in the test chamber for at least 30 min before the test. Before the test, the software EthoVision XT 11.5 was set, the wild-type mice were placed at the center of the bottom of the chamber, and the activity within 30 min or 20 min was recorded as the baseline. After 30 min, the mice were taken out and given corresponding doses of solvent (control group) or test compound according to their body weight. Immediately after administration, the mice were placed in the same observation chamber as that in the baseline stage, and their activities within 40 min after administration were recorded. The instrument was wiped with 75% alcohol after each test, to avoid the influence of the information left by animals in the last round (such as the smell of animals' feces and urine) on the next test result. The next round of animals were changed to continue the test. After the test, data were collected for statistical analysis. The difference was analyzed by unpaired t-test to have statistical significance, where P<0.05 means that there was a significant difference. ^{#} P<0.05, * P<0.05, **P<0.01, ***P<0.001, **** P<0.0001. After the test, the distance (cm) traveled by the mice in the control and test compound groups every 5 min was statistically analyzed. The representative results of the open field evaluation test of the compound of the present invention are shown in FIGs. 33 to 36.

Conclusions: according to the analysis of the open field test results, compared with the saline group, 10 mg/kg of S-ketamine significantly increases the distance traveled by the mice within 5-20 min after administration. The distance traveled by the mice after intraperitoneal administration of 10 mg/kg of Crystal Form A of the hydrochloride and Crystal Form C of the mesylate of Compound **2**, Crystal Form G of Compound **3** hydrochloride, and Compound **34** hydrochloride, and oral administration of 10 mg/kg of Compound **177** hydrochloride and Compound **178** hydrochloride, and 5 mg/kg of Compound **179** hydrochloride has no significant difference compared with the vehicle group.

The compound of the present invention has no hallucinogenic side effect.

### Test Example 6: Pre-pulse inhibition test

Pre-pulse inhibition phenomenon is an important parameter to evaluate the sensorimotor gating function, and an index to evaluate schizophrenia-like behavior. This phenomenon means that a weak stimulus occurring before a strong stimulus will reduce the degree of animal response to the strong stimulus. In the present invention, the PPI test was used to evaluate whether the compound could induce psychotomimetic-like behavior in mice.

The animal information was the same as that in Test Example 2. The number of animals in each group (N) was 10, and the administration mode was intraperitoneal injection or oral administration (p.o.). The dosage of S-ketamine was 20 mg/kg, the dosage of other compounds was 45 mg/kg, and the administration volume was 10 mL/kg. The dosage for oral administration was 10 mg/kg.

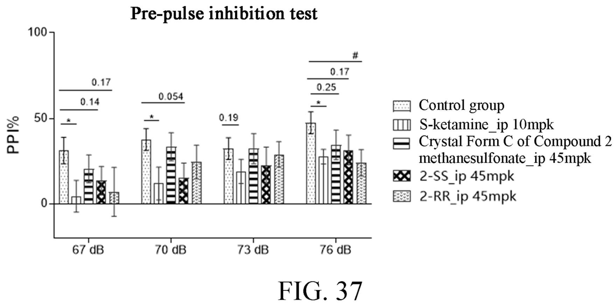

Test process: The test was carried out in a quiet environment. The mice were weighed and acclimated in the test chamber for at least 30 min before the test. The PPI test was divided into four stages, including: acclimation stage, Block 1, Block 2, and Block 3. At the beginning of the test, the mice were given corresponding doses of solvent (control group) or test compound according to their body weight. Then the mice were placed in a startle response chamber and allowed to acclimate for 5 min without stimulation. Then in Block 1-3, the mice were given startle stimuli of varying decibel intensities. In Block 1 and Block 3, 10 trials were set, and each trial had no pre-pulse stimuli. The startle stimulus was set to be white noise of 120 dB. The time interval between trials varies randomly from 10 to 15 sec. Block 2 was set to have 56 trials. There were three types of stimuli: (1) a continuous startle stimulus of 120 dB was given in 8 trials; (2) a pre-pulse stimulus of 67, 70, 73, or 76 dB was given first and then a startle stimulus of 120 dB was given in 32 trials, and only pre-pulse stimulus (two trials for each of the four pre-pulse stimuli) was given in 8 trials; and (3) no stimulus was given in 8 trials, and the duration was 200 ms. The duration of pre-pulse stimulus was 20 ms, and the fixed interval was 100 ms. The duration of startle stimulus in all Block was 40 ms, and the background noise was set to 62dB. The instrument was wiped with 75% alcohol after each test, to avoid the influence of the information left by animals in the last round (such as the smell of animals' feces and urine) on the next test result. The difference was analyzed by unpaired t-test to have statistical significance, where P<0.05 means that there was a significant difference. ^{#} P<0.05, * P<0.05, ** P<0.01, *** P<0.001. The representative result of the PPI test of the compound of the present invention is shown in FIG. 37. The result shows that at a dose of 45 mg/kg i.p., Crystal Form C of Compound **2** mesylate of the present invention does not affect the PPI of native C57 mice, which is superior to S-ketamine and significantly superior to its two comparative molecular isomers 2SS and 2RR. Compound 2SS mesylate tends to cause loss of PPI at a pre-pulse level of 67 dB and 70 dB. Compound 2RR mesylate causes loss of PPI at a pre-pulse level of 76 dB. In addition, Compound **177** hydrochloride, **178** hydrochloride, and **179** hydrochloride, at a dosage of 10 mg/kg p.o., will not affect the PPI of mice, and will not induce psychotomimetic-like behavior in mice.

Experimental conclusions: The compound of the invention will not induce psychotomimetic-like behavior.

### Test Example 7: Evaluation of anti-pruritic effect of compounds

In the test, male C57BL/6 mice were used to establish a pruritus model to evaluate the anti-pruritic effect of the compound of the present invention.

Test process: Male mice aged 6-8 weeks were used, which were raised adaptively for 7 days after purchase and allowed to free access to water and food during the period. 3 days before the test, hair of 2 cm×3 cm on the neck and back of the tested animals was shaved off, and the animals were habituated to touch by test personnels to reduce the stress responses to the test personnels. The compound of the present invention is administered once, and a histamine-induced pruritus model was established 30 min after administration.

Histamine-induced pruritus model: Before the model was established, the animals were placed in a transparent test chamber (30 cm x 30 cm x 15 cm), and acclimated to the environment for 15 min. A syringe was used to intradermally inject a histamine solution (100 µg histamine dissolved in 50 µL physiological saline) into the back of the neck of mice.

Behavioral test: The mice were placed back into the test chamber to observe their itching behavior for 30 min, and their scratching number and time were recorded. One number of scratching is defined as: the mouse lifts its hind paw one or more times to scratch the injection site on the back of the neck, and then puts the hind paw in the mouth or on the ground.

Data analysis: The scratching number and time of mice were recorded. After the test, the animals were euthanized.

The test results show that in the group administered with the compound of the present invention, the total scratching number and scratching time decrease significantly within 30 min compared with the model group, and the compound of the invention has obvious anti-pruritic effect.

### Test Example 8: Pharmacokinetic test in mice

The test was to investigate the pharmacokinetic characteristics of the compound of the present invention in C57BL/6J male mice after oral administration (oral gavage) and intravenous injection.

Male C57BL/6J mice aged 10-12 weeks were given the solution of the compound by intravenous injection (i.v.) and oral administration (p.o.) respectively. The dosage was shown in the table below. The vehicle for intravenous injection was 0.9% physiological saline for injection. The administration dosage volume was 5 mL/kg. The animals were allowed to normal access to food and water before administration. The vehicle for oral administration was 0.9% physiological saline for injection. The administration dosage volume was 10 mL/kg. The animals were fasted for 16 hrs before administration. 0.0333, 0.1667, 0.3330, 1, 2, 4, 6, 8, 24 hrs after administration, about 70 µL of blood was collected from the facial veins of the two groups of animals, transferred to an anticoagulant tube with EDTA-K2, placed on ice, and then centrifuged at 4 Celsius degrees in 1 hr to separate the plasma (4500 g, 15 min). The drug concentration was analyzed by LC-MS/MS, and the related pharmacokinetic parameters were calculated through the linear-log trapezoidal method by non-compartmental analysis using PKSolver2.0 kinetic software.

The test results are shown in Table 29.

**Table 29: Pharmacokinetic data**

| | Intravenous administration | | | Oral administration | | | |
|---|---|---|---|---|---|---|---|
| Compound | Dose (mg/kg) | Cl (mL/min/kg) | AUC0-last (h*ng/mL) | Dose (mg/kg) | T_{1/2} (h) | AUC0-last (h*ng/mL) | Bioavailability (F%) |
| Compound **34** hydrochloride | 1 | 68 | 241 | 20 | 1.02 | 1951 | 40% |
| Compound **196** hydrochloride | 1 | 93 | 176 | 50 | 1.21 | 5862 | 66% |
| Compound **250** hydrochloride | 1 | 102 | 159 | 100 | 2.98 | 13911 | 87% |
| Compound **177** hydrochloride | 1 | 109 | 149 | 10 | 3.38 | 449 | 30% |
| Compound **178** hydrochloride | 1 | 117 | 141 | 10 | 3.43 | 773 | 55% |
| Compound **179** hydrochloride | 1 | 55 | 291 | 5 | 6.16 | 821 | 56% |
| Compound **180** hydrochloride | 1 | 46 | 348 | 10 | 1.85 | 1954 | 56% |

Experimental conclusions: The compound of the present invention has good in-vivo metabolic stability, excellent oral drug absorption and exposure, and good oral drug absorption and bioavailability.

## Claims

1. A compound of Formula II: 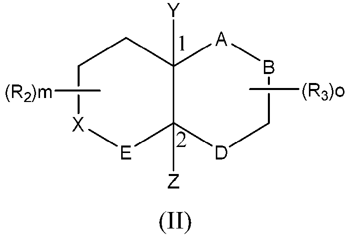
or a pharmaceutically acceptable salt, enantiomer, diastereomer, tautomer, solvate, isotopologue, polymorph, prodrug, or metabolite thereof,
wherein,
X is -O- or -CH₂-;
E is -O- or -CH₂-;
A is -NRₐ- or -CH₂-;
B is -NRₐ- or -CH₂-;
Rₐ is selected from H or C₁₋₆ alkyl;
D is -O- or -CH₂-;
Y is selected from a substituted or unsubstituted 6- to 14-membered aryl, a substituted or unsubstituted 5- to 14-membered heteroaryl, or a substituted or unsubstituted C₁₋₆ alkyl, and Y is optionally substituted with 1, 2, 3, 4 or 5 R₁, where each R₁ is independently selected from deuterium, cyano, C₁₋₃ alkyl, halogen, a 5- to 14-membered heteroaryl optionally substituted with 1, 2, 3, 4 or 5 halogens, or a 6- to 14-membered aryl optionally substituted with 1, 2, 3, 4 or 5 halogens, and the 5- to 14-membered heteroaryl comprises 1, 2, 3 or 4 heteroatoms selected from N, O or S;
Z is selected from H, hydroxyl, C₁₋₆ alkyl, or C₁₋₆ alkoxy;
R₂ is selected from deuterium, C₁₋₃ alkyl, or halogen;
R₃ is selected from deuterium, C₁₋₃ alkyl, or halogen;
m is 0, 1, 2 or 3;
o is 0, 1, 2 or 3;
the chiral carbon atom at position 1 is in S or R configuration; and
the chiral carbon atom at position 2 is in S or R configuration;
provided that the compound of Formula II is not the following compounds and salt thereof: (4aR,8aS)-4α-(2-thienyl)octahydro-2H-benzo[b][1,4]oxazine, (4aR,8aR)-4α-(2-chloro-3-thienyl)octahydro-2H-benzo[b][1,4]oxazine, (4aS,8aR)-4α-(2-thienyl)octahydro-2H-benzo[b][1,4]oxazine, (4aR,8aS)-4α-(3-methyl-2-thienyl)octahydro-2H-benzo[b][1,4]oxazine, (4aR,8aR)-4α-(4-methyl-3-thienyl)octahydro-2H-benzo[b][1,4]oxazine, (4aR,8aR)-4α-(3-chlorophenyl)octahydro-2H-benzo[b][1,4]oxazine, (4aS,8aS)-4α-(3-chlorophenyl)octahydro-2H-benzo[b][1,4]oxazine, (4aR,8aR)-4α-(2-chlorophenyl)octahydro-2H-benzo[b][1,4]oxazine, (4aS,8aS)-4α-(2-chlorophenyl)octahydro-2H-benzo[b][1,4]oxazine, 4a-phenyloctahydro-2H-benzo[b][1,4]oxazine, 4a-(2-methoxyphenyl)octahydro-2H-benzo[b][1,4]oxazine, 4a-(3-fluorophenyl)octahydro-2H-benzo[b][1,4]oxazine, 4a-(4-fluorophenyl)octahydro-2H-benzo[b][1,4]oxazine, 4a-(2,3-difluorophenyl)octahydro-2H-benzo[b][1,4]oxazine, 4a-(2-fluorophenyl)octahydro-2H-benzo[b][1,4]oxazine, 6-methyl-4a-phenyloctahydro-2H-benzo[b][1,4]oxazine, 6-ethyl-4a-phenyloctahydro-2H-benzo[b][1,4]oxazine, 4a-(3-chlorophenyl)octahydro-2H-benzo[b][1,4]oxazine, 4a-(3-methylphenyl)octahydro-2H-benzo[b][1,4]oxazine, 4a-(2-chlorophenyl)octahydro-2H-benzo[b][1,4]oxazine, 4a-(4-chlorophenyl)octahydro-2H-benzo[b][1,4]oxazine, 4a-(4-methylphenyl)octahydro-2H-benzo[b][1,4]oxazine, 4a-(3-(trifluoromethyl)phenyl)octahydro-2H-benzo[b][1,4]oxazine, 8-methyl-4a-phenyloctahydro-2H-benzo[b][1,4]oxazine, 5-methyl-4a-phenyloctahydro-2H-benzo[b][1,4]oxazine, 7-methyl-4a-phenyloctahydro-2H-benzo[b][1,4]oxazine, 4-methyl-4a-phenyloctahydro-2H-benzo[b][1,4]oxazine, 3,3-dimethyl-5a-phenyldecahydrobenzo[b] [1,4]olanzapine, 4a-(3-methoxyphenyl)octahydro-2H-benzo[b][1,4]oxazine, 4a-(3-(trifluoromethoxy)phenyl)octahydro-2H-benzo[b][1,4]oxazine, 4*a*-(4-(trifluoromethyl)phenyl)octahydro-2*H*-benzo[*b*][1,4]oxazine, 4*a*-(2,6-dimethylphenyl)octahydro-2*H*-benzo[*b*][1,4]oxazine, 4*a*-(4-(t-butyl)phenyl)octahydro-2*H-*benzo[*b*][1,4]oxazine, 4*a*-(2,3-dichlorophenyl)octahydro-2*H*-benzo[*b*][1,4]oxazine, 4*a*-(2-isopropylphenyl)octahydro-2*H*-benzo[*b*] [1,4]oxazine, 4*a*-(2,5-dimethylphenyl)octahydro-2*H*-benzo[*b*][1,4]oxazine, 4*a*-(2-chloro-3-fluorophenyl)octahydro-2*H*-benzo[*b*][1,4]oxazine, 4*a*-(3,4-difluorophenyl)octahydro-2*H*-benzo[*b*][1,4]oxazine, 6,6-dimethyl-4*a-*phenyloctahydro-2*H*-benzo[*b*][1,4]oxazine, 4*a*-(2-chloro-5-fluorophenyl)octahydro-2*H-*benzo[*b*][1,4]oxazine, 4*α*-(3-ethoxyphenyl)octahydro-2*H*-benzo[*b*][1,4]oxazine, 4*α*-(2-chloro-4-methoxyphenyl)octahydro-2*H*-benzo[*b*][1,4]oxazine, and 4a-(2-chloro-6-fluorophenyl)octahydro-2H-benzo[b] [1,4]oxazine.

2. The compound or the pharmaceutically acceptable salt, enantiomer, diastereomer, tautomer, solvate, isotopologue, polymorph, prodrug, or metabolite thereof according to claim 1, having a structure of Formula A': 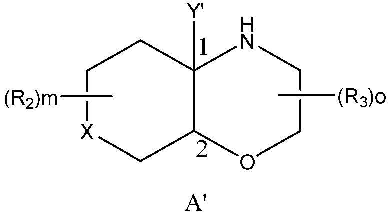 wherein,
Y' is a C₁₋₆ alkyl optionally substituted with a 6- to 14-membered aryl, where the 6- to 14-membered aryl is optionally substituted with 1, 2, 3, 4 or 5 halogens.

3. The compound or the pharmaceutically acceptable salt, enantiomer, diastereomer, tautomer, solvate, isotopologue, polymorph, prodrug, or metabolite thereof according to claim 1, having a structure of Formula II-1: 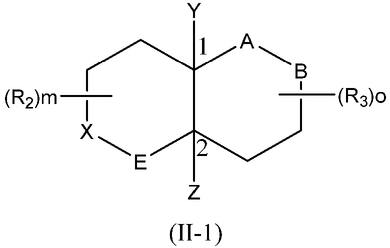 wherein,
Y is selected from a substituted or unsubstituted 6- to 14-membered aryl or a substituted or unsubstituted 5- to 14-membered heteroaryl, and Y is optionally substituted with 1, 2, 3, 4 or 5 R₁, where each R₁ is independently selected from deuterium, cyano, C₁₋₃ alkyl, or halogen, and the 5- to 14-membered heteroaryl comprises 1, 2, 3 or 4 heteroatoms selected from N, O or S.

4. The compound or the pharmaceutically acceptable salt, enantiomer, diastereomer, tautomer, solvate, isotopologue, polymorph, prodrug, or metabolite thereof according to claim 1, having a structure of Formula A: 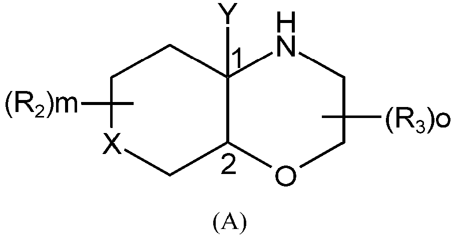 wherein:
Y is a 6- to 14-membered aryl or a 5- to 7-membered heteroaryl optionally substituted with 1, 2, 3, 4 or 5 R₁ substituents, where each R₁ is independently selected from deuterium, cyano, C₁₋₃ alkyl, and halogen.

5. The compound or the pharmaceutically acceptable salt, enantiomer, diastereomer, tautomer, solvate, isotopologue, polymorph, prodrug, or metabolite thereof according to claim 1, wherein the pharmaceutically acceptable salt is selected from a formate, a hydrochloride, a sulfate, a phosphate, a citrate, a succinate, a D-tartrate, a fumarate, a maleate, a p-toluenesulfonate, a mesylate, a benzenesulfonate, an oxalate, or a pamoate, preferably selected from a formate, a hydrochloride, a phosphate, or a mesylate.

6. The compound according to claim 1 or the pharmaceutically acceptable salt, enantiomer, diastereomer, tautomer, solvate, isotopologue, polymorph, prodrug, or metabolite thereof according to claim 1, wherein the compound of Formula II is selected from the following compounds:

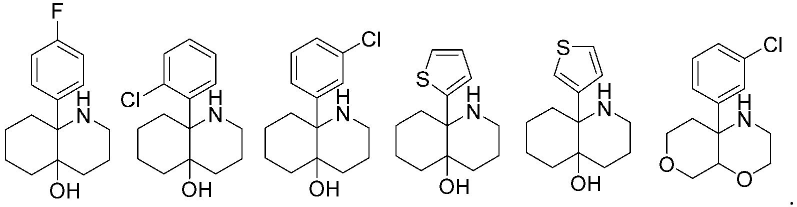

7. The compound or the pharmaceutically acceptable salt, enantiomer, diastereomer, tautomer, solvate, isotopologue, polymorph, prodrug, or metabolite thereof according to claim 1, wherein the compound of Formula II is selected from the following compounds: 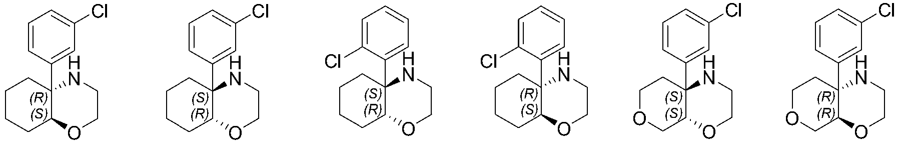 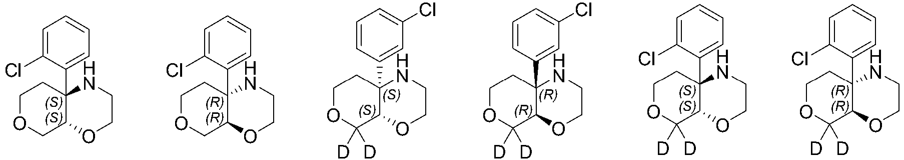 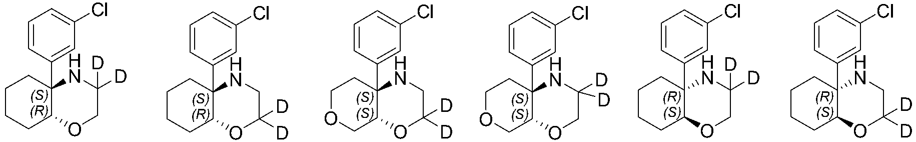 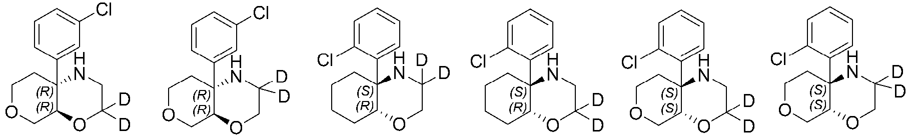 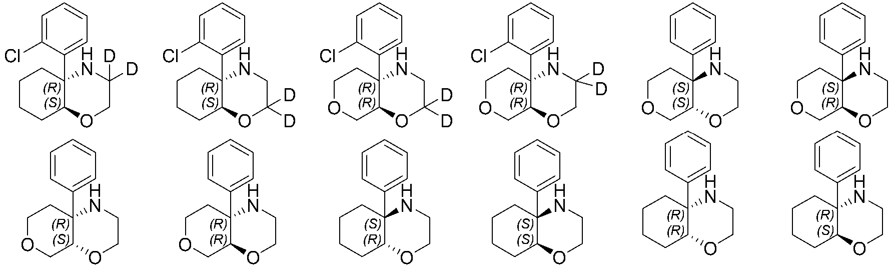 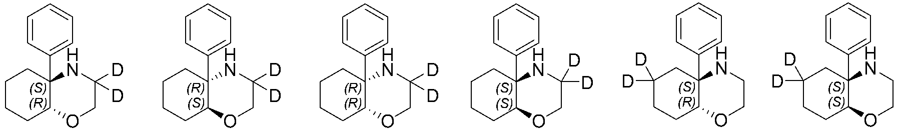 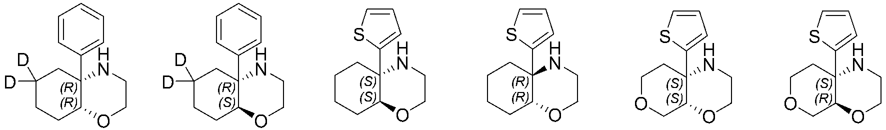 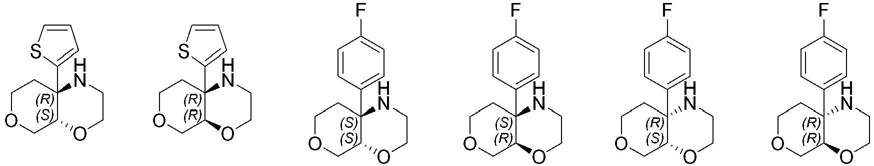 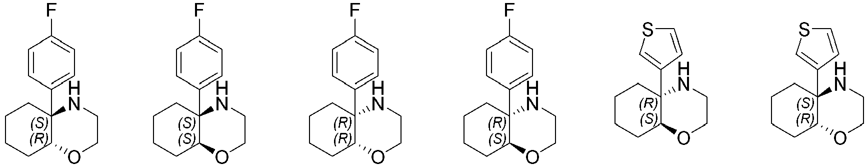 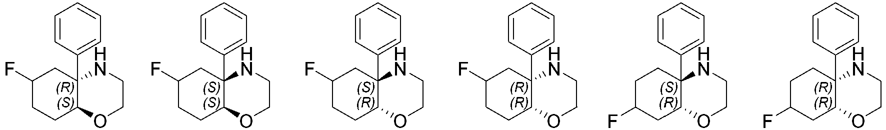 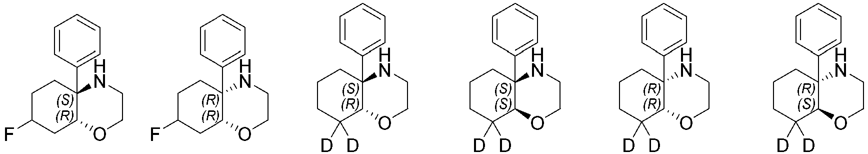 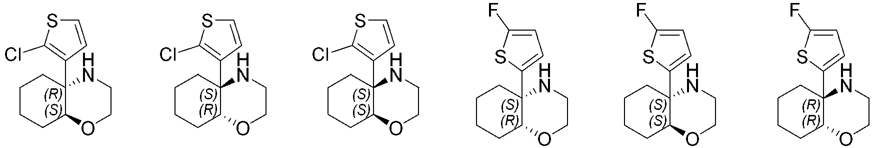 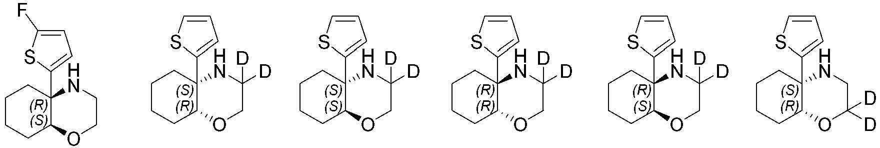 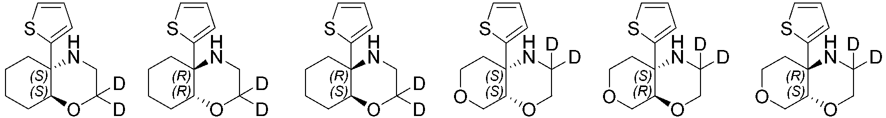 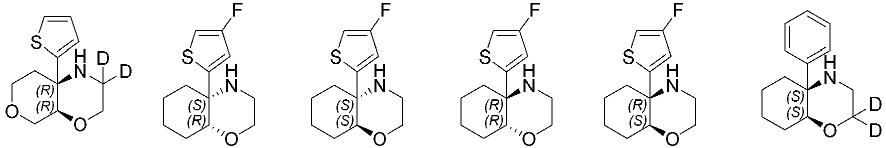 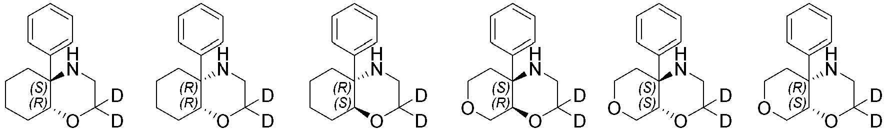 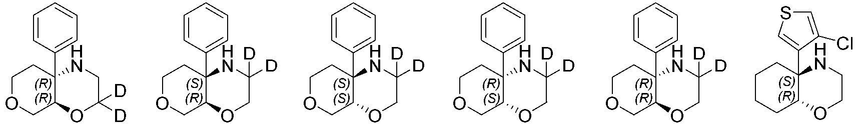 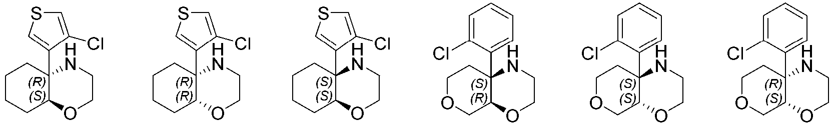 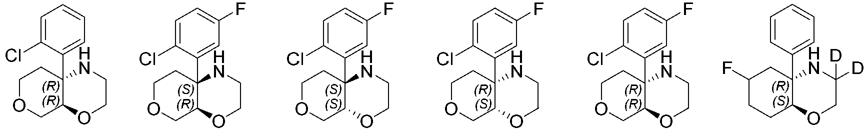 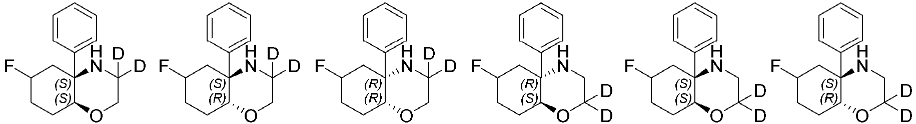 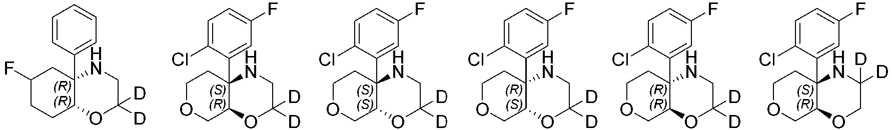 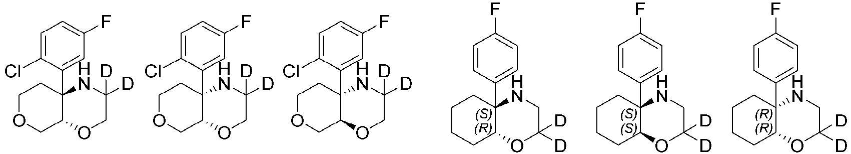 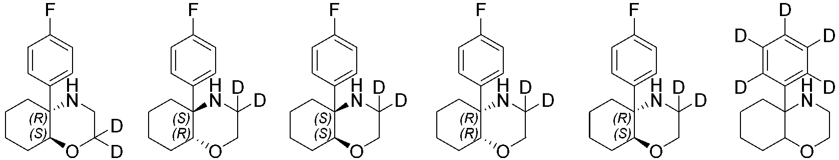 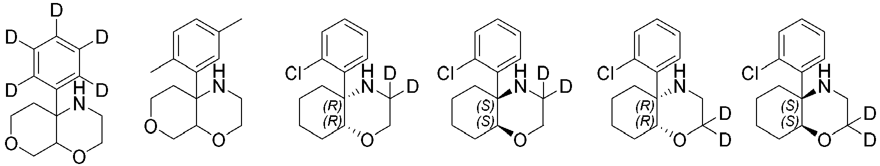 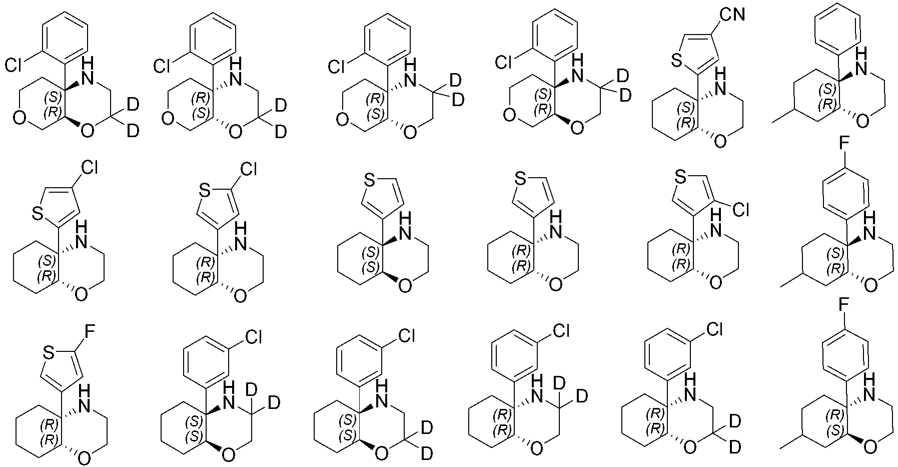 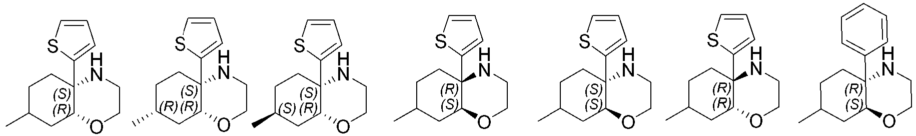 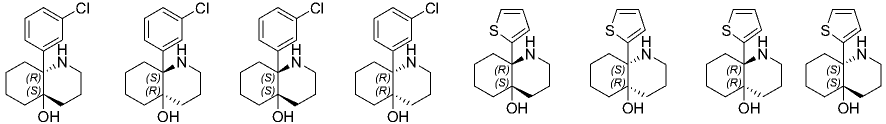 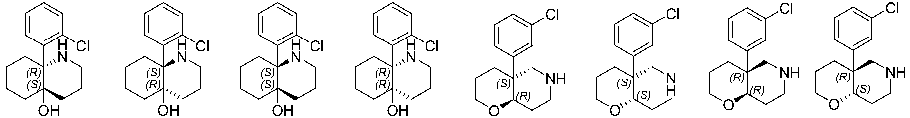 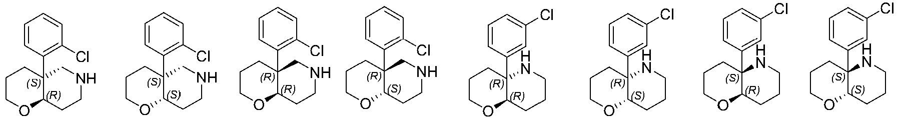 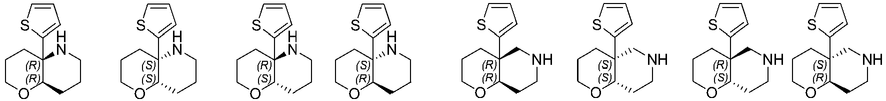 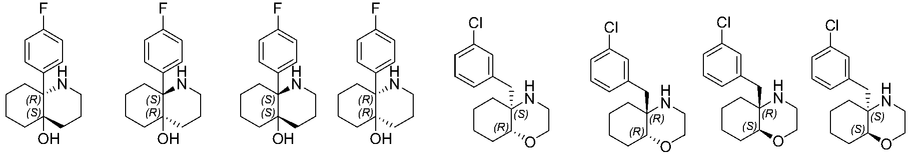 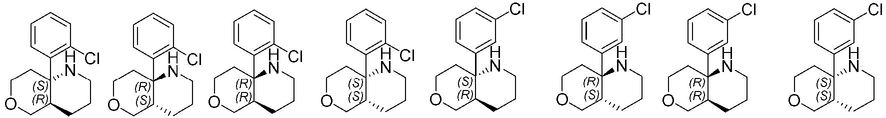 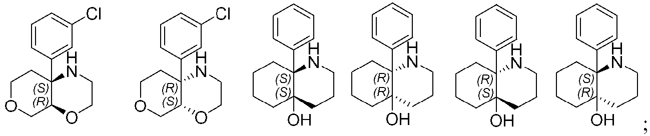 and
optionally, the pharmaceutically acceptable salt of the compound of Formula II is selected from Compound **2** hydrochloride, Compound **2** phosphate, Compound **2** mesylate, Compound **2** formate, Compound **2** oxalate, Compound **2** pamoate, Compound **3** hydrochloride, Compound **3** phosphate, Compound **3** mesylate, Compound **3** formate, Compound **3** oxalate, Compound **3** pamoate, Compound **13** hydrochloride, Compound **17** hydrochloride, Compound **33** hydrochloride, Compound **36** hydrochloride, Compound **34** hydrochloride, Compound **35** hydrochloride, Compound **39** hydrochloride, Compound **40** hydrochloride, Compound **45** hydrochloride, Compound **46** hydrochloride, Compound **74** hydrochloride, Compound **80** hydrochloride, Compound **96** hydrochloride, Compound **98** hydrochloride, Compound **144** hydrochloride, Compound **145** hydrochloride, Compound **158** hydrochloride, Compound **55** hydrochloride, Compound **56** hydrochloride, Compound **57** hydrochloride, Compound **58** hydrochloride, Compound **84** hydrochloride, Compound **92** hydrochloride, Compound **98** hydrochloride, Compound **139** hydrochloride, Compound **141** hydrochloride, Compound **155** hydrochloride, Compound **156** hydrochloride, Compound **157** hydrochloride, Compound **158** hydrochloride, Compound **159** hydrochloride, Compound **160** hydrochloride, Compound **161** hydrochloride, Compound **177** hydrochloride, Compound **178** hydrochloride, Compound **179** hydrochloride, Compound **180** hydrochloride, Compound **218** mesylate, Compound **219** mesylate, Compound **242** mesylate, Compound **243** mesylate, Compound **167**a mesylate, Compound **167**b mesylate, Compound **29** mesylate, Compound **32** mesylate, Compound **51** mesylate, Compound **54** mesylate, Compound **62** mesylate, Compound **68** mesylate, Compound **230** mesylate, Compound **231** mesylate, Compound **196** mesylate, Compound **199** mesylate, Compound **226** mesylate, Compound **14** mesylate, Compound **163** mesylate, or Compound **164** mesylate.

8. A compound of Formula III: 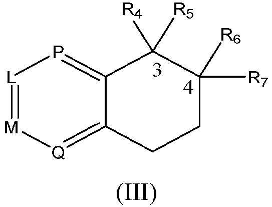
or a pharmaceutically acceptable salt, enantiomer, diastereomer, tautomer, solvate, isotopologue, polymorph, prodrug, or metabolite thereof,
wherein,
L, M, P, and Q are each independently selected from N or CH;
R₄, R₅, R₆, and R₇ are each independently selected from C₁₋₆ alkyl, C₁₋₆ alkoxy, -NR_{b}R_{c}, a substituted or unsubstituted 6- to 14-membered aryl, or a substituted or unsubstituted 5- to 14-membered heteroaryl, where the 6- to 14-membered aryl and the 5- to 14-membered heteroaryl are optionally substituted with a group selected from halogen, deuterium, C₁₋₆ alkyl, or C₁₋₆ alkoxy, and the 5- to 14-membered heteroaryl comprises 1, 2, 3 or 4 heteroatoms selected from N, O or S; R₄ is further selected from oxo, provided that R₅ is absent; and R₆ is further selected from oxo, provided that R₇ is absent;
R_{b} and R_{c} are each independently selected from H or C₁₋₆ alkyl; and
the carbon atom at position 3 or 4 is a chiral carbon atom that is in S configuration or R configuration.

9. The compound or the pharmaceutically acceptable salt, enantiomer, diastereomer, tautomer, solvate, isotopologue, polymorph, prodrug, or metabolite thereof according to claim 8, wherein the compound of Formula III is selected from the following compounds:

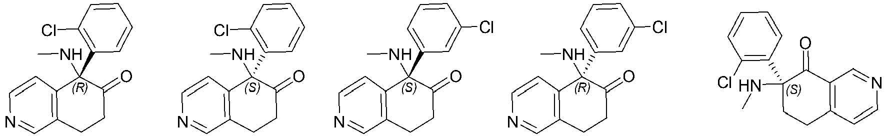

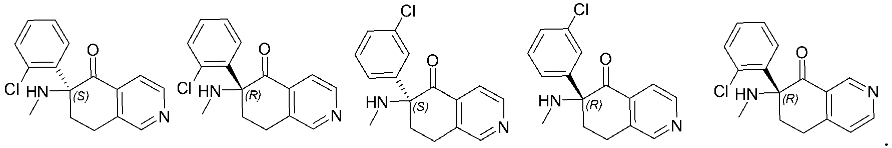

10. A crystal form of a salt of Compound 2, selected from Crystal Forms A, B, C, D, E, F, L or M, 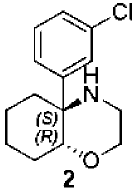 wherein
the X-ray powder diffraction pattern of Crystal Form A of the hydrochloride comprises characteristic peaks at the following 2θ angles: 14.44° ± 0.20°, 17.16° ± 0.20°, 20.59° ± 0.20°, and 23.38° ± 0.20°;
the X-ray powder diffraction pattern of Crystal Form B of the phosphate comprises characteristic peaks at the following 2θ angles: 5.48° ± 0.20°, 18.48° ± 0.20°, 22.49° ± 0.20°, and 22.92° ± 0.20°;
the X-ray powder diffraction pattern of Crystal Form C of the mesylate comprises characteristic peaks at the following 2θ angles: 8.05° ± 0.20°, 14.37± 0.20°, 16.18° ± 0.20°, 18.49° ± 0.20°, and 22.65± 0.20°;
the X-ray powder diffraction pattern of Crystal Form D of the mesylate comprises characteristic peaks at the following 2θ angles: 12.13° ± 0.20°, 14.80° ± 0.20°, 20.54° ± 0.20°, 23.37° ± 0.20°, and 26.09° ± 0.20°;
the X-ray powder diffraction pattern of Crystal Form E of the mesylate comprises characteristic peaks at the following 2θ angles: 14.81° ± 0.20°, 16.30° ± 0.20°, 18.60° ± 0.20°, and 22.75° ± 0.20°;
the X-ray powder diffraction pattern of Crystal Form F of the formate comprises characteristic peaks at the following 2θ angles: 11.35° ± 0.20°, 13.53° ± 0.20°, and 23.76° ± 0.20°;
the X-ray powder diffraction pattern of Crystal Form L of the oxalate comprises characteristic peaks at the following 2θ angles: 9.44° ± 0.20°, 11.11° ± 0.20°, 15.61° ± 0.20°, 19.42° ± 0.20°, and 26.30° ± 0.20°;
the X-ray powder diffraction pattern of Crystal Form M of the pamoate comprises characteristic peaks at the following 2θ angles: 5.44° ± 0.20°, 6.49° ± 0.20°, 10.75° ± 0.20°, and 21.01° ± 0.20°.

11. The crystal form of the salt of Compound 2 according to claim 10, wherein
the X-ray powder diffraction pattern of Crystal Form A of the hydrochloride further comprises a characteristic peak(s) at one or more of the following 2θ angles: 11.44° ± 0.20°, 15.00° ± 0.20°, 16.36° ± 0.20°, 24.11° ± 0.20°, 24.30° ± 0.20°, and 26.72° ± 0.20°; preferably, the X-ray powder diffraction pattern of Crystal Form A of the hydrochloride further comprises a characteristic peak(s) at one or more of the following 2θ angles: 6.79° ± 0.20°, 13.15° ± 0.20°, and 25.74° ± 0.20°;
the X-ray powder diffraction pattern of Crystal Form B of the phosphate further comprises a characteristic peak(s) at one or more of the following 2θ angles: 13.49° ± 0.20°, 15.44° ± 0.20°, 16.50° ± 0.20°, 20.63° ± 0.20°, and 27.64° ± 0.20°; preferably, the X-ray powder diffraction pattern of Crystal Form B of the phosphate further comprises a characteristic peak(s) at one or more of the following 2θ angles: 10.96° ± 0.20°, 12.32° ± 0.20°, and 14.83° ± 0.20°;
the X-ray powder diffraction pattern of Crystal Form C of the mesylate further comprises a characteristic peak(s) at one or more of the following 2θ angles: 13.91° ± 0.20°, 15.03° ± 0.20°, and 24.42° ± 0.20°; preferably, the X-ray powder diffraction pattern of Crystal Form C of the mesylate further comprises a characteristic peak(s) at one or two of the following 2θ angles: 9.71° ± 0.20°, and 12.00° ± 0.20°;
the X-ray powder diffraction pattern of Crystal Form D of the mesylate further comprises a characteristic peak(s) at one or more of the following 2θ angles: 17.14° ± 0.20°, 18.08° ± 0.20°, 22.70° ± 0.20°, and 24.66° ± 0.20°; preferably, the X-ray powder diffraction pattern of Crystal Form D of the mesylate further comprises a characteristic peak(s) at one or more of the following 2θ angles: 6.28° ± 0.20°, 11.33° ± 0.20°, 12.53° ± 0.20°, 21.46° ± 0.20°, and 23.93° ± 0.20°;
the X-ray powder diffraction pattern of Crystal Form E of the mesylate further comprises a characteristic peak(s) at one or more of the following 2θ angles: 12.13° ± 0.20°, 14.48° ± 0.20°, 17.15° ± 0.20°, 20.53° ± 0.20°, 21.45° ± 0.20°, and 21.62° ± 0.20°; preferably, the X-ray powder diffraction pattern of Crystal Form E of the mesylate further comprises a characteristic peak(s) at one or more of the following 2θ angles: 6.24° ± 0.20°, 8.16° ± 0.20°, 12.47° ± 0.20°, and 26.08° ± 0.20°;
the X-ray powder diffraction pattern of Crystal Form F of the formate further comprises a characteristic peak(s) at one or more of the following 2θ angles: 11.79° ± 0.20°, 12.99° ± 0.20°, 15.89° ± 0.20°, 17.34° ± 0.20°, and 22.87° ± 0.20°; preferably, the X-ray powder diffraction pattern of Crystal Form F of the formate further comprises a characteristic peak(s) at one or two of the following 2θ angles: 5.85° ± 0.20°, and 22.37° ± 0.20°;
the X-ray powder diffraction pattern of Crystal Form L of the oxalate further comprises a characteristic peak(s) at one or more of the following 2θ angles: 18.98° ± 0.20°, 23.17° ± 0.20°, and 29.92° ± 0.20°; preferably, the X-ray powder diffraction pattern of Crystal Form L of the oxalate further comprises a characteristic peak at the following 2θ angle: 31.69° ± 0.20°;
the X-ray powder diffraction pattern Crystal Form M of the pamoate further comprises a characteristic peak(s) at one or more of the following 2θ angles: 5.35° ± 0.20°, 10.00° ± 0.20°, and 12.51° ± 0.20°.

12. The crystal form of the salt of Compound 2 according to claim 10, wherein
Crystal Form A of the hydrochloride has an X-ray powder diffraction (XPRD) pattern substantially as shown in FIG. 1, and/or a differential scanning calorimetry (DSC) profile substantially as shown in FIG. 2 and/or a thermogravimetric analysis (TGA) curve substantially as shown in FIG. 2;
Crystal Form B of the phosphate has an X-ray powder diffraction (XPRD) pattern substantially as shown in FIG. 3, and/or a differential scanning calorimetry (DSC) profile substantially as shown in FIG. 4 and/or a thermogravimetric analysis (TGA) curve substantially as shown in FIG. 4;
Crystal Form C of the mesylate has an X-ray powder diffraction (XPRD) pattern substantially as shown in FIG. 5, and/or a differential scanning calorimetry (DSC) profile substantially as shown in FIG. 6 and/or a thermogravimetric analysis (TGA) curve substantially as shown in FIG. 6;
Crystal Form D of the mesylate has an X-ray powder diffraction (XPRD) pattern substantially as shown in FIG. 7, and/or a differential scanning calorimetry (DSC) profile substantially as shown in FIG. 8 and/or a thermogravimetric analysis (TGA) curve substantially as shown in FIG. 9;
Crystal Form E of the mesylate has an X-ray powder diffraction (XPRD) pattern substantially as shown in FIG. 10, and/or a differential scanning calorimetry (DSC) profile substantially as shown in FIG. 11, and/or a thermogravimetric analysis (TGA) curve substantially as shown in FIG. 12;
Crystal Form F of the formate has an X-ray powder diffraction (XPRD) pattern substantially as shown in FIG. 13, and/or a differential scanning calorimetry (DSC) profile substantially as shown in FIG. 14, and/or a thermogravimetric analysis (TGA) curve substantially as shown in FIG. 14;
Crystal Form L of the oxalate has an X-ray powder diffraction (XPRD) pattern substantially as shown in FIG. 15; and
Crystal Form M of the pamoate has an X-ray powder diffraction (XPRD) pattern substantially as shown in FIG. 16.

13. A crystal form of a salt of Compound 3, selected from Crystal Forms G, H, I, J, K, N, and O, 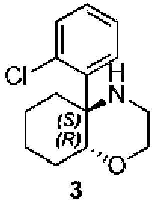 wherein,
the X-ray powder diffraction pattern of Crystal Form G of the hydrochloride comprises characteristic peaks at the following 2θ angles: 6.18° ± 0.20°, 11.42° ± 0.20°, 12.45° ± 0.20°, 20.02° ± 0.20°, and 23.41° ± 0.20°;
the X-ray powder diffraction pattern of Crystal Form H of the hydrochloride comprises characteristic peaks at the following 2θ angles: 6.64° ± 0.20°, 13.12° ± 0.20°, 13.31° ± 0.20°, 21.83° ± 0.20°, and 23.06° ± 0.20°;
the X-ray powder diffraction pattern of Crystal Form I of the phosphate comprises characteristic peaks at the following 2θ angles: 5.94° ± 0.20°, 12.29° ± 0.20°, 13.34° ± 0.20°, and 17.99° ± 0.20°;
the X-ray powder diffraction pattern of Crystal Form J of the mesylate comprises characteristic peaks at the following 2θ angles: 14.75° ± 0.20°, 15.81° ± 0.20°, and 21.89° ± 0.20°;
the X-ray powder diffraction pattern of Crystal Form K of the formate comprises characteristic peaks at the following 2θ angles: 11.92° ± 0.20°, 15.15° ± 0.20°, and 24.04° ± 0.20°;
the X-ray powder diffraction pattern of Crystal Form N of the oxalate comprises characteristic peaks at the following 2θ angles: 6.20° ± 0.20°, 7.49° ± 0.20°, 12.25° ± 0.20°, 12.47° ± 0.20°, 16.36° ± 0.20°, and 23.43° ± 0.20°;
the X-ray powder diffraction pattern of Crystal Form O of the pamoate comprises characteristic peaks at the following 2θ angles: 6.18° ± 0.20°, 9.41° ± 0.20°, 11.10° ± 0.20°, 15.59° ± 0.20°, and 26.25° ± 0.20°.

14. The crystal form of the salt of Compound 3 according to claim 13, wherein
the X-ray powder diffraction pattern of Crystal Form G of the hydrochloride further comprises a characteristic peak(s) at one or more of the following 2θ angles: 10.42° ± 0.20°, 13.57° ± 0.20°, 14.15° ± 0.20°, 17.14° ± 0.20°, 19.42° ± 0.20°, 24.50° ± 0.20°, and 24.88° ± 0.20°; preferably, the X-ray powder diffraction pattern of Crystal Form G of the hydrochloride further comprises a characteristic peak(s) at one or more of the following 2θ angles: 8.52° ± 0.20°, 10.96° ± 0.20°, 20.46° ± 0.20°, and 26.74° ± 0.20°;
the X-ray powder diffraction pattern of Crystal Form H of the hydrochloride further comprises a characteristic peak(s) at one or more of the following 2θ angles: 14.16° ± 0.20°, 18.37° ± 0.20°, 21.48° ± 0.20°, 23.71° ± 0.20°, 26.17° ± 0.20°, 29.21° ± 0.20°, and 33.70° ± 0.20°; preferably, the X-ray powder diffraction pattern of Crystal Form H of the hydrochloride further comprises a characteristic peak(s) at one or more of the following 2θ angles: 12.64° ± 0.20°, 14.43° ± 0.20°, 16.99° ± 0.20°, and 26.61° ± 0.20°;
the X-ray powder diffraction pattern of Crystal Form I of the phosphate further comprises a characteristic peak(s) at one or more of the following 2θ angles: 14.94° ± 0.20°, 19.20° ± 0.20°, 21.54° ± 0.20°, 23.80° ± 0.20°, 24.12° ± 0.20°, and 24.54° ± 0.20°; preferably, the X-ray powder diffraction pattern of Crystal Form I of the phosphate further comprises a characteristic peak at 7.21° ± 0.20°;
the X-ray powder diffraction pattern of Crystal Form J of the mesylate further comprises a characteristic peak(s) at one or more of the following 2θ angles: 15.27° ± 0.20°, 16.75° ± 0.20°, 18.96° ± 0.20°, 23.51° ± 0.20°, and 24.44° ± 0.20°; preferably, the X-ray powder diffraction pattern of Crystal Form J of the mesylate further comprises a characteristic peak(s) at one or more of the following 2θ angles: 7.87° ± 0.20°, 11.94° ± 0.20°, 12.32° ± 0.20°, 18.21° ± 0.20°, 27.96° ± 0.20°, and 30.06° ± 0.20°;
the X-ray powder diffraction pattern of Crystal Form K of the formate further comprises a characteristic peak(s) at one or more of the following 2θ angles: 13.91° ± 0.20°, 14.38° ± 0.20°, 18.41° ± 0.20°, and 30.61° ± 0.20°; preferably, the X-ray powder diffraction pattern of Crystal Form K of the formate further comprises a characteristic peak(s) at one or two of the following 2θ angles: 10.76° ± 0.20°, and 24.90° ± 0.20°;
the X-ray powder diffraction pattern of Crystal Form N of the oxalate further comprises a characteristic peak(s) at one or more of the following 2θ angles: 11.45° ± 0.20°, 14.00° ± 0.20°, 15.06° ± 0.20°, and 23.78° ± 0.20°; preferably, the X-ray powder diffraction pattern of Crystal Form N of the oxalate further comprises a characteristic peak(s) at one or two of the following 2θ angles: 24.07° ± 0.20°, and 24.95° ± 0.20°;
the X-ray powder diffraction pattern of Crystal Form O of the pamoate further comprises a characteristic peak(s) at one or more of the following 2θ angles: 10.40° ± 0.20°, 12.46° ± 0.20°, 18.96° ± 0.20°, 19.34° ± 0.20°, 23.12° ± 0.20°, 23.43° ± 0.20°, 29.90° ± 0.20°, and 31.46° ± 0.20°; preferably, the X-ray powder diffraction pattern of Crystal Form O of the pamoate further comprises a characteristic peak at 25.00° ± 0.20°.

15. The crystal form of the salt of Compound 3 according to claim 13, wherein
Crystal Form G of the hydrochloride has an X-ray powder diffraction (XPRD) pattern substantially as shown in FIG. 17, and/or a differential scanning calorimetry (DSC) profile substantially as shown in FIG. 18 and/or a thermogravimetric analysis (TGA) curve substantially as shown in FIG. 18;
Crystal Form H of the hydrochloride has an X-ray powder diffraction (XPRD) pattern substantially as shown in FIG. 19, and/or a differential scanning calorimetry (DSC) profile substantially as shown in FIG. 20 and/or a thermogravimetric analysis (TGA) curve substantially as shown in FIG. 20;
Crystal Form I of the phosphate has an X-ray powder diffraction (XPRD) pattern substantially as shown in FIG. 21, and/or a differential scanning calorimetry (DSC) profile substantially as shown in FIG. 22 and/or a thermogravimetric analysis (TGA) curve substantially as shown in FIG. 22;
Crystal Form J of the mesylate has an X-ray powder diffraction (XPRD) pattern substantially as shown in FIG. 23, and/or a differential scanning calorimetry (DSC) profile substantially as shown in FIG. 24 and/or a thermogravimetric analysis (TGA) curve substantially as shown in FIG. 24;
Crystal Form K of the formate has an X-ray powder diffraction (XPRD) pattern substantially as shown in FIG. 25, and/or a differential scanning calorimetry (DSC) profile substantially as shown in FIG. 26 and/or a thermogravimetric analysis (TGA) curve substantially as shown in FIG. 26;
Crystal Form N of the oxalate has an X-ray powder diffraction (XPRD) pattern substantially as shown in FIG. 27;
Crystal Form O of the pamoate has an X-ray powder diffraction (XPRD) pattern substantially as shown in FIG. 28.

16. A pharmaceutical composition, comprising a pharmaceutically acceptable carrier and
(i) the compound or the pharmaceutically acceptable salt, enantiomer, diastereomer, tautomer, solvate, isotopologue, polymorph, prodrug, or metabolite thereof according to any one of claims 1 to 9, and a pharmaceutically acceptable carrier; or
(ii) the crystal form of the salt of Compound 2 according to any one of claims 10 to 12; or
(iii) the crystal form of the salt of Compound 3 according to any one of claims 13 to 15.

17. Use of the compound or the pharmaceutically acceptable salt, enantiomer, diastereomer, tautomer, solvate, isotopologue, polymorph, prodrug, or metabolite thereof according to any one of claims 1 to 9, the crystal form of the salt of Compound **2** according to any one of claims 10 to 12, the crystal form of the salt of Compound **3** according to any one of claims 13 to 15, or the pharmaceutical composition according to claim 16 in the manufacture of a medicament for treating or preventing NMDA receptor-mediated diseases, or in the manufacture of an anesthetic, an analgesic, or a sedative (for human or animal use); preferably, the NMDA receptor-mediated diseases are selected from: cerebral ischemia, traumatic brain injury, infarction, stroke, Alzheimer's disease, Parkinson's disease, Huntington's diseases, depression, anxiety, bipolar disorder, schizophrenia, autism, epilepsy, anti-NMDA receptor encephalitis, neuropathic pain, anorexia, sleep disorder or pruritus related diseases, and other nervous system events or neurodegeneration caused by the activation of NMDA receptors; preferably, the NMDA receptor-mediated disease is depression, schizophrenia or epilepsy; preferably, the sleep disorder related disease is insomnia, sleep apnea syndrome, atypical circadian rhythm sleep-wake disorder or Down syndrome.
